# EUROPEAN PATENT APPLICATION

(11) **EP 3 597 653 A1**
(43) Date of publication of application: **22.01.2020**
(21) Application number: 18184450.7
(22) Date of filing: 19.07.2018
(51) Int. Cl.: C07D 513/04, A61K 31/407, C07D 513/20, C07D 515/04, C07D 515/20, C07D 285/28, C07D 291/08, A61P 31/20

(54) **CYCLIC INHIBITORS OF HEPATITIS B VIRUS**

(71) Applicant: IRBM S.P.A., 00071 Pomezia (RM) (IT); Promidis S.r.l., 20132 Milano MI (IT); Ospedale San Raffaele S.r.l., 20132 Milano (IT); Istituto Nazionale Di Genetica Molecolare-INGM, 20122 Milano (IT)
(72) Inventor: De Francesco, Raffaele, 20146 Milano (IT); Donnici, Lorena, 20122 Milano (IT); Guidotti, Luca, 20132 Milano (IT); Iannacone, Matteo, 20132 Milano (IT); Di Fabio, Romano, 00071 Pomezia (IT); Summa, Vincenzo, 00071 Pomezia (IT); Bencheva, Leda Ivanova, 20132 Milano (IT); De Matteo, Marilenia, 20132 Milano (IT); Ferrante, Luca, 20132 Milano (IT); Prandi, Adolfo, 20132 Milano (IT); Randazzo, Pietro, 20132 Milano (IT)
(74) Representative: Capasso, Olga

(57) **Abstract**

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

SEQUENCE LISTING

<110>  PROMIDIS S.R.L.
       IRBM SCIENCE PARK S.P.A.
       OSPEDALE SAN RAFFAELE S.R.L.
       ISTITUTO NAZIONALE DI GENETICA MOLECOLARE - INGM

<120>  INHIBITORS OF HEPATITIS B VIRUS

<130>  BE138505

<160>  2

<170>  PatentIn version 3.5

<210>  1
<211>  22
<212>  DNA
<213>  Artificial Sequence

<220>
<223>  synthetic primer

<400>  1
atttgttcag tggttcgtag gg                                                22


<210>  2
<211>  22
<212>  DNA
<213>  Artificial Sequence

<220>
<223>  synthetic primer

<400>  2
cggtaaaaag ggactcaaga tg                                                22

## Description

### FIELD OF THE INVENTION

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

### BACKGROUND OF THE INVENTION

The Hepatitis B virus (HBV) is an enveloped, partially double-stranded DNA (dsDNA) virus of the hepadnaviridae family that is spread by contact with infected blood and body fluids and causes acute and chronic necroinflammatory liver diseases of varying severity (Guidotti LG, Chisari FV. Annu Rev Pathol. 2006; 1:23-61.). The HBV lipid envelope contains 3 in-frame viral envelope proteins (large, middle and small), each of which possesses the hepatitis B virus surface antigen (HBsAg) determinant (Seeger C, Mason WS.Virology. 2015 May; 479-480:672-86). This envelope encloses a protein shell, or capsid, that is composed of 240 monomers of the core protein and each monomer possesses the hepatitis B virus core antigen (HBcAg or Cp) determinant. The capsid in turn encloses a partially double-stranded, relaxed circular DNA (rcDNA) form of the viral genome as well as a molecule of the viral polymerase. Upon entry onto susceptible cells (i.e. the hepatocytes) via the interaction of the large envelope protein with specific receptors on the hepatocellular membrane, the capsid is released into the cytoplasm and transported at the nuclear membrane. The rcDNA is then released into the nucleus and repaired by cellular polymerases into an episomal "minichromosome", termed covalently closed circular DNA (cccDNA), which represents the viral transcriptional template. The minus strand of the viral DNA encodes 3.5, 2.4, 2.1 and 0.7 kb mRNA species that are translated into structural (envelope and core) and nonstructural (polymerase, precure and X) proteins of the virus. Following transport into the cytoplasm, one of the 3.5 kb RNAs (termed pregenomic RNA) is selectively packaged into a nascent capsid by interacting with the core and polymerase proteins that have been translated from their respective mRNAs. Within these capsids, the viral polymerase reverse transcribes the pregenomic RNA into a single minus strand DNA molecule that serves as template for the viral polymerase-mediated DNA plus strand synthesis and the cohesive structure of the linear DNA intermediates converts them into a relaxed circular double stranded molecule. A fraction of these HBV DNA-containing "mature" capsids are transported back to the nucleus where second strand synthesis is completed and the ends of both strands are ligated, leading to amplification of the pool of cccDNA. Another fraction of the capsids binds to viral envelope proteins that have been independently translated and translocated to membranes of endoplasmic reticulum (ER)-like structures. Following binding, the enveloped capsids bud into the lumen of the ER and exit the cell as infectious virions to initiate new cycles of infection.

Thus, the HBV core protein and the related capsids are essential components and regulators of the HBV life cycle. The full-length core protein Cp183, or its N-terminal domain Cp149, predominantly assembles into a T = 4 icosahedral capsids. Due to its critical roles in capsid assembly, pregenomic RNA packaging, and cccDNA maintenance, it is not surprising that the HBV core protein and the related capsids have been widely recognized as attractive antiviral targets (Durantel D, Zoulim F; J Hepatol. 2016 Apr;64(1 Suppl):S117-S131).

According to World Health Organization (WHO) statistics, HBV infection is one of the major medical scourges of our time. As a sexually transmitted disease that is also transferred by intravenous drug abuse and from mother to infant at birth, over one third of the world's population has been infected by HBV at some point in their lives (Burns GS, Thompson AJ; Cold Spring Harb Perspect Med. 2014 Oct 30;4(12)). While most of these people have successfully cleared the virus, more than 240 million people remain persistently infected and almost 800,000 of these individuals die annually from the complications of chronic infection (i.e. cirrhosis and/or hepatocellular carcinoma). HBV infection is highly endemic in sub-Saharan Africa, the Pacific, and particularly Asia. Regions with high rates of chronic HBV infection also include the Middle East, the Indian subcontinent, areas of South and Central America, and the southern parts of Eastern and Central Europe. In recent years the number of chronic carriers has increased steadily in the western world as well, mostly because of the influx of immigrants from endemic areas. Additionally, HBV acts as a helper virus to hepatitis delta virus (HDV) and it should be noted that the more than 15 million people co-infected with HBV and HDV have an increased risk of rapid progression to cirrhosis and hepatic decompensation (Hughes, S.A. et al. Lancet 2011, 378, 73-85).

Well-tolerated vaccines that elicit neutralizing antibodies to HBsAg efficiently prevent de novo HBV infection, but have no therapeutic potential for the millions of people that are already persistently infected (Zoulim, Durantel D; Cold Spring Harb Perspect Med. 2015 Apr 1;5(4)). Therapy for these individuals mainly relies on direct acting antiviral (DAA) drugs (e.g. tenofovir, lamivudine, adefovir, entecavir or telbivudine) that suppress virus production but do not eradicate HBV from the liver, requiring lifelong treatment. Cohorts of patients still receive a therapy based on pegylated interferon-a (PEG-IFN-α), which has the advantages of limited treatment duration and higher rates of HBsAg seroconversion but the relevant disadvantage of greater adverse effects. As such, the number of patients receiving PEG-IFN-α is progressively decreasing.

Different chemical classes of inhibitors targeting the encapsidation process of HBV (also termed capsid assembly modulators or CAMs) are under development, and they include heteroaryldihydropyrimidines (HAPs) and sulfamoylbenzamides (SBAs). For instance, Novira Therapeutics recently utilized a humanized mouse model of HBV infection to show that a combination of CAM and PEG-IFN-α has higher antiviral activity than that previously observed with DAAs. This class of CAM is now in Phase 2 clinical development, with compound NVR3-778 that is administered in patients in combination with DAAs or PEG-IFN-α (Gastroenterology 2018; 154: 652-662).

WO2013/006394, published on January 10, 2013, relates to a subclass of sulfamoyl-arylamides having general fomula A, useful for the treatment of Hepatitis B virus (HBV) infection:

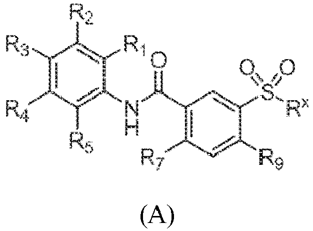

WO2013/096744, published on June 26, 2013 relates to sulfamoyl-arylamides of formula B active against HBV:

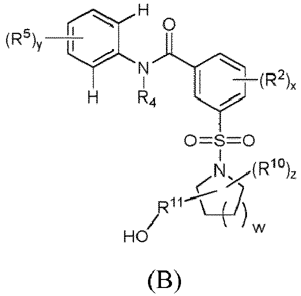

WO2014/106019, published on July 3, 2014, relates to compounds of formula C, useful as nucleocapsid assembly inhibitors for the treatment of viruses, especially but not exclusively, including pregenomic RNA encapsidation inhibitors of HBV for the treatment of Hepatitis B virus (HBV) infection and related conditions:

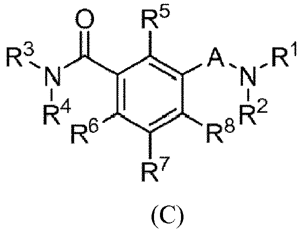

WO2014/165128, published on October 9, 2014, WO2015/109130 published on July 23, 2015, US2015274652, published on October 1, 2015, all relate to sulfamoyl-arylamides compounds active against HBV.

WO2015/120178, published on August 13, 2015, relates to sulfamoyl-arylamides compounds used in combinantion therapy with peginterferon alfa-2a, or another interferon analog for the treatment of HBV infection.

WO2016/089990, published on June 9, 2016, relates to sulfide alkyl and pyridyl reverse sulphonamide compounds for HBV treatment.

US2016185748, published on June 30, 2016, relates to pyridyl reverse sulfonamides for HBV treatment.

US2016151375, published on June 2, 2016 relates to sulfide alkyl compounds for HBV treatment. WO2017/001655A1, published on January 5, 2017, relates to cyclized sulfamoylarylamide derivatives having structure:

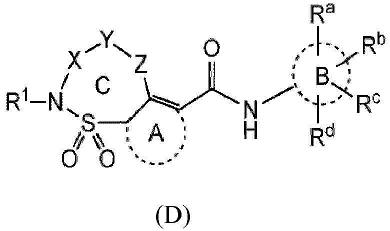

Amongst the problems which HBV direct antivirals may encounter are toxicity, mutagenicity, lack of selectivity, poor efficacy, poor bioavailability, low solubility and/or off-target activity, and until now there are no compounds in any of the structural classes identified above approved as drugs for the treatment of HBV patients.

There is a need for additional HBV inhibitors that may overcome at least one of these disadvantages or that have additional advantages such as increased potency, increased bioavailability or an increased safety window.

The present invention provides small molecule drugs obtained through chemical modification of the known sulfamoyl arylamides derivatives. The chemotype discovered in the present invention results in potent HBV inhibitors with improved pharmacokinetic properties, good kinetic solubility, stability in mouse and human hepatocytes, low in vivo clearance and positive liver-to-plasma concentration. Given the liver's key role in metabolic regulation and the fact that it is the principal tissue affected by hepatitis B disease, designing HBV inhibitors with hepatoselective distribution profiles is an important strategy in developing safe drug candidates (Tu M. et al., Current Topics in Medicinal Chemistry, 2013, 13, 857-866).

### DESCRIPTION OF THE INVENTION

The compounds of this invention are inhibitors of hepatitis B virus (HBV).

It is therefore an object of the present invention a compound of general formula (I): 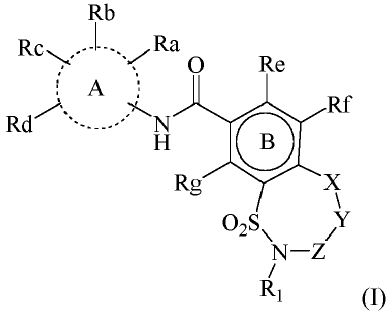 wherein:
A is aryl or heteroaryl;
B is a 6-membered aryl optionally containing one or more N atoms;
X is N optionally substituted with R₄; or is O;
Y is a single bond; a double bond; a C₁₋₄alkanediyl or a C₂₋₄alkenediyl each of said C₁₋₄alkanediyl or C₂₋₄alkenediyl being independently optionally substituted with R₅;
Z is CR₂R₃ or C(O);
R₁ is selected from:
   - hydrogen;
   - OH;
   - C₁₋₆alkyl optionally substituted with one or more substituents each independently selected from the group consisting of OH, halogen, CN, NH₂, NH(R₆), N(R₆)₂, aryl, heteroaryl, 3-7 membered saturated ring and 5-7 membered unsaturated ring, each of said saturated or unsaturated ring optionally containing one or more heteroatoms selected from the group consisting of O, N, S and each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
   - aryl or heteroaryl ring, each of said aryl or heteroaryl ring being optionally substituted with one or more substituents selected from OH, halogen, haloC₁₋₆alkyl, CN, haloC₁₋₆alkoxy and C₁₋₆alkoxy; and
   - a 3-8 membered saturated or partially unsaturated ring optionally containing one or more heteroatoms each independently selected from the group consisting of O, S and N, the 3-8 membered saturated or partially unsaturated ring being optionally substituted with one, two or more substituents each independently selected from the group consisting of OH, halogen, CN, C₁₋₄alkyl, hydroxyC₁₋₄alkyl, C(O)OR₆, C(O)R₆, haloC₁₋₆alkyl, haloC₁₋₆alkoxy and C₁₋₆alkoxy;
R₂ is selected from:
   - hydrogen;
   - deuterium;
   - C₁₋₆alkyl; C₁₋₆alkoxy; C₂₋₆alkenyl; each of said C₁₋₆alkyl, C₁₋₆alkoxy or C₂₋₆alkenyl being optionally substituted with one or more substituents independently selected from OH, halogen, NH₂, NH(R₆), N(R₆)₂, NHC(O)R₆, C₁₋₆alkoxy, haloC₁₋₆alkoxy, CN, aryl, heteroaryl, 3-7 membered saturated ring and 5-7 membered unsaturated ring, each of said 3-7 membered saturated or 5-7 membered unsaturated ring optionally containing one or more heteroatoms selected from the group consisting of O, N, S, each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
   - NH₂;
   - NHC(O)R₆;
   - a 3-8 membered saturated ring optionally containing one or more heteroatoms each independently selected from the group consisting of O, S and N, the 3-8 membered saturated ring being optionally substituted with one or more substituents each independently selected from the group consisting of OH, halogen, C(O)OR₆, C(O)R₆, C₁₋₆alkyl and CN; and
   - an aryl or heteroaryl ring optionally substituted with one, two, three or more substituents each independently selected from the group consisting of C₁₋₆alkyl, haloC₁₋₆alkyl, halogen, C₁₋₆alkoxy and haloC₁₋₆alkoxy;
R₃ is absent, hydrogen, deuterium or C₁₋₆alkyl;
R₄ is hydrogen, C₁₋₄alkyl, C₃₋₆cycloalkyl or OH;
R₅ is C₁₋₃alkyl optionally substituted with OH;
R₆ is C₁₋₄alkyl;
or R₁ and R₂ taken together form with the atoms to which they are respectively attached a heterocyclic ring optionally substituted with one or more substituents each independently selected from the group consisting of C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl ring optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or any two residues of R₁, R₂, R₃, R₄, R₅ which are not geminal or vicinal group, taken together represent a single bond, or represent a C₁₋₄alkanediyl or a C₂₋₄alkenediyl, each of said C₁₋₄alkanediyl or a C₂₋₄alkenediyl being independently optionally substituted with one or more substituents each independently selected from C₁₋₄ alkyl and halogen; said single bond or said optionally substituted C₁₋₄alkanediyl or C₂₋₄alkenediyl forming together with the bridging atoms to which they are respectively linked a 4-10 membered saturated or partially unsaturated ring;
or R₂ and R₃ taken together form with the carbon atom to which they are attached a 3-7 membered saturated ring optionally containing one or more heteroatoms each independently selected from the group consisting of O, S and N, the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)R₆;
or R₂ and R₃ taken together form with the carbon atom to which they are attached an indoline-2-one or an azaindoline-2-one, each being optionally substituted with one or more substituents selected from halogen, hydroxy, C₁₋₆alkyl, haloC₁₋₆alkyl, C₁₋₆alkoxy and haloC₁₋₆alkoxy;
or R₂ and R₅ taken together form with the carbon atoms to which they are respectively attached a 3-8 membered saturated ring or a 5-10 membered partially saturated ring, each ring optionally containing one or more heteroatoms each independently selected from the group consisting of O, S and N, each ring optionally being substituted with one, two or more substituents each independently selected from the group consisting of halogen, OH, CN, C(O)OR₆, C(O)R₆, C₁₋₆alkyl, C₁₋₆alkoxy, haloC₁₋₆alkyl and haloC₁₋₆alkoxy;
or, when Y is a bond, R₂ and R₄ taken together form with the atoms to which they are attached a 5-6 membered heteroaryl ring optionally substituted with C₁₋₃alkyl;
Ra is selected from the group consisting of hydrogen, halogen, CN, C₁₋₃alkyl, C₁₋₃alkoxy, haloC₁₋₃alkyl and haloC₁₋₃alkoxy; or is absent;
Rb is selected from the group consisting of hydrogen, halogen, CN, C₁₋₃alkyl, C₁₋₃alkoxy, haloC₁₋₃alkyl and haloC₁₋₃alkoxy; or is absent;
Rc is selected from the group consisting of hydrogen, halogen, CN, C₁₋₃alkyl, C₁₋₃alkoxy, haloC₁₋₆alkyl and haloC₁₋₃alkoxy; or is absent
Rd is selected from the group consisting of hydrogen, halogen, CN, C₁₋₃alkyl, C₁₋₃alkoxy, haloC₁₋₃alkyl and haloC₁₋₃alkoxy; or is absent;
Re is selected from the group consisting of hydrogen, halogen, C₁₋₃alkyl, NH₂, NH(CH₃), N(CH₃)₂, NHC(O)CH₃, OH, and CN; or is absent;
Rf is selected from the group consisting of hydrogen, halogen, C₁₋₃alkyl, haloC₁₋₃alkyl, C₁₋₃alkoxy and haloC₁₋₃alkoxy; or is absent;
Rg is selected from the group consisting of hydrogen, halogen, C₁₋₃alkyl, haloC₁₋₃alkyl, C₁₋₃alkoxy and haloC₁₋₃alkoxy; or is absent;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate or 4-cyclopropyl-N-phenyl-3,4-dihydro-2H-benzo[e][1,2,4]thiadiazine-7-carboxamide 1,1-dioxide
and pharmaceutically acceptable salts, tautomers, isomers, stereoisomers thereof.

Preferably, A is phenyl. Preferably, B is phenyl. Preferably, A and B are both phenyl.

Preferably, X is N substituted with R₄. Preferably, Y is a single bond or CH₂.

Preferably, R₁ is selected from: hydrogen; C₁₋₆alkyl (in particular methyl); haloC₁₋₆alkyl (in particular CH(CF₃)CH₃); and a 3-8 membered saturated or partially unsaturated ring optionally containing one or more of O and/or N, said 3-8 membered saturated or partially unsaturated ring being optionally substituted with one, two or more substituents each independently selected from the group consisting of OH, C₁₋₄alkyl (in particular methyl) and hydroxyC₁₋₄alkyl (in particular CH₂OH). Preferably, said 3-8 membered saturated or partially unsaturated ring is selected from the group consisting of: piperidyl, oxetanyl, cyclobutyl, cyclohexyl and cyclohexenyl. Also preferably, R₁ and R₂ taken together form with the atoms to which they are respectively attached piperidyl optionally substituted with one or more substituents each independently selected from the group consisting of C(O)OR₆, C(O)CH₃, halogen, OH, haloC₁₋₆alkyl, CN, C₁₋₆alkyl, C₁₋₆alkoxy and haloC₁₋₆alkoxy.

Preferably, R₂ is selected from the group consisting of: hydrogen; deuterium; C₁₋₆alkyl optionally substituted one or more of NH₂ or NHC(O)R₆ (in particular methyl, CH₂NH₂ or CH2NHC(O)R₆); haloC₁₋₆alkyl (in particular CHF₂); C₁₋₆alkoxy (in particular CH₂CH₂OiPr); hydroxyC₁₋₆alkyl (in particular CH₂OH, CH₂CH₂OH, CH(OH)CH₃ or CH(OH)CH₂CH₃); a 3-8 membered saturated ring (in particular piperidyl) optionally substituted with C(O)OR₆; and an aryl optionally substituted with one or more substituents independently selected from the group consisting of: C₁₋₆alkyl (in particular methyl), haloC₁₋₆alkyl (in particular trifluoromethyl) and halogen (in particular F or C1). Also preferably, R₂ and R₃ taken together form with the carbon atom to which they are attached a 3-7 membered saturated ring optionally containing one or more of O and/or N and optionally being substituted with one or more substituents each independently selected from the group consisting of halogen, OH, halo C₁₋₆alkyl, CN, C₁₋₆alkyl, C₁₋₆alkoxy, haloC₁₋₆alkoxy, C(O)OR₆ and C(O)R₆. More preferably, R₂ and R₃ taken together form with the carbon atom to which they are attached a 3-7 membered saturated ring selected from: cyclobutyl, tetrahydro-2H-pyranyl, piperidinyl). Still preferably, R₂ and R₃ taken together form with the carbon atom to which they are attached an azaindoline-2-one.

Preferably, R₃ is hydrogen, deuterium or C₁₋₆alkyl (in particular methyl). Preferably, R₄ is hydrogen. Preferably, R₆ is tert-butyl or methyl.

Preferably, Ra is H. Preferably Rb and Rd are selected from the group consisting of: hydrogen, halogen (in particular F or Cl), C₁₋₃alkyl (in particular methyl) and haloC₁₋₃alkyl (in particular CH₂F). Preferably, Rc is halogen. More preferably, Rc is F. Preferably, Rd is hydrogen or halogen (in particular F or Cl).

Preferably, Re is selected from the group consisting of: hydrogen, halogen (in particular Cl, Br or F) and C₁₋₃alkyl (in particular methyl). Preferably, Rf is hydrogen or C₁₋₃alkyl (in particular methyl). Preferably, Rg is hydrogen.

In another embodiment, the invention provides the compound of general formula (I) as defined above, wherein:
A is aryl or heteroaryl;
B is phenyl or pyridyl;
X is N optionally substituted with R₄; or is O;
Y is a single bond; a double bond; or a C₁₋₄ alkanediyl optionally substituted with R₅;
Z is CR₂R₃ or C(O);
R₁ is selected from:
   - hydrogen;
   - OH;
   - C₁₋₆alkyl, hydroxyC₁₋₆alkyl, haloC₁₋₆alkyl; and
   - a 3-8 membered saturated or partially unsaturated ring optionally containing one or more heteroatoms each independently selected from the group consisting of O, S and N, the 3-8 membered saturated or partially unsaturated ring being optionally substituted with one, two, three or more substituents each independently selected from the group consisting of OH, halogen, CN, C₁₋₆alkyl, hydroxyC₁₋₄alkyl, C(O)OR₆, C(O)CH₃, haloC₁₋₆alkyl, haloC₁₋₆alkoxy and C₁₋₆alkoxy;
R₂ is selected from:
   - hydrogen;
   - C₁₋₆alkyl optionally substituted with one or more substituents selected from OH, halogen, NH₂, NHC(O)CH₃ and C₁₋₆alkoxy;
   - NH₂;
   - NHC(O)CH₃;
   - a 3-8 membered saturated ring optionally containing one or more heteroatoms each independently selected from the group consisting of O, S and N, the 3-8 membered saturated ring optionally substituted with one or more substituents each independently selected from the group consisting of OH, halogen and C₁₋₆alkyl; and
   - an aryl or heteroaryl ring, each of said aryl or heteroaryl ring optionally substituted with one, two, three or more substituents each independently selected from C₁₋₆alkyl, haloC₁₋₆alkyl and halogen;
R₃ is absent, hydrogen or C₁₋₆alkyl;
R₄ is hydrogen, C₁₋₄alkyl, C₃₋₆cycloalkyl or OH;
R₅ is C₁₋₃alkyl;
R₆ is C₁₋₄alkyl;
or R₁ and R₂ taken together form with the atoms to which they are respectively attached a heterocyclic ring selected from the group consisting of aziridinyl, azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₁ and R₄, or R₁ and R₅ taken together represent a C₁₋₄ alkanediyl optionally substituted with one or more substituents each independently selected from C₁₋₄ alkyl and halogen;
said C₁₋₄ alkanediyl forming together with the bridging atoms to which it is linked a 4-10 heterocyclic ring;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
or, when Y is a bond, R₂ and R₄ taken together form with the atoms to which they are attached a 5-6 membered heteroaryl ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl; or is absent when B is pyridyl;
Rf is selected from the group consisting of hydrogen, halogen and methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide; or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate; or 4-cyclopropyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

Preferably, the invention relates to a compound of general formula (I) as defined above,
wherein:
A is phenyl or pyridyl;
B is phenyl or pyridyl;
X is N optionally substituted with R₄; or is O;
Y is a single bond, C₁₋₂alkanediyl optionally substituted with R₅;
Z is CR₂R₃ or C(O);
R₁ is selected from:
   - hydrogen;
   - OH;
   - C₁₋₆alkyl, hydroxyC₁₋₆alkyl, haloC₁₋₆alkyl; and
   - a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
   - hydrogen;
   - C₁₋₆alkyl optionally substituted with one or more substituents selected from OH, halogen, NH₂, NHC(O)CH₃ and C₁₋₆alkoxy;
   - NH₂;
   - NHC(O)CH₃;
   - a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one or more substituents each independently selected from OH, halogen, C(O)OR₆, C(O)CH₃, C₁₋₆alkyl and CN; and
   - an aryl or heteroaryl ring selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl, each of said aryl or heteroaryl ring being optionally substituted with one, two, three or more substituents each independently selected from C₁₋₆alkyl, haloC₁₋₆alkyl and halogen;
R₃ is absent, hydrogen or methyl;
R₄ is hydrogen, C₁₋₄alkyl or OH;
R₅ is C₁₋₃alkyl;
R₆ is C₁₋₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄, or R₁ and R₅ taken together represent a C₁₋₄ alkanediyl optionally substituted with one or more substituents each independently selected from C₁₋₄ alkyl and halogen; said C₁₋₄ alkanediyl forming together with the bridging atoms to which it is linked a 4-10 heterocyclic ring;
or R₂ and R₅ taken together form with the carbon atoms to which they are respectively attached a 3-8 membered saturated ring selected from the group consisting of cyclobutyl, cyclopentyl and cyclohexyl, said 3-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
Ra is selected from the group consisting of hydrogen, halogen and CN;
Rb is selected from the group consisting of hydrogen, halogen and CN;
Rc is selected from the group consisting of hydrogen, halogen and CN;
Rd is selected from the group consisting of hydrogen, halogen and CN;
Re is selected from the group consisting of hydrogen, halogen, C₁₋₃alkyl, NH₂, NH(CH₃), N(CH₃)₂, NHC(O)CH₃, OH and CN; or is absent when B is pyridyl;
Rf is selected from the group consisting of hydrogen, halogen and methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

All the definitions of substituents, such as for example "alkyl", "alkoxy", "aryl", "heteroaryl" and so on, are reported herein below and apply to formula (I), (Ia), (Ib) and (Ic).

In a further embodiment the invention relates to a compound having general formula (I) wherein: 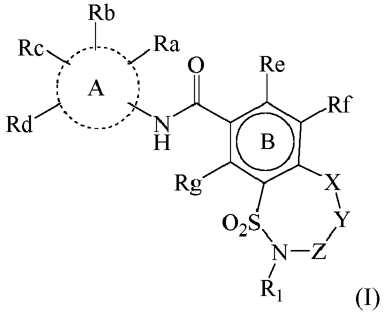
A is aryl or heteroaryl;
B is phenyl or pyridyl;
X is N optionally substituted with R₄; or is O;
Y is a single bond; a double bond; or a C₁₋₄ alkanediyl optionally substituted with R₅;
Z is CR₂R₃ or C(O);
   - R₁ is selected from:
   - hydrogen;
   - OH;
   - C₁₋₆alkyl;
   - hydroxyC₁₋₆alkyl;
   - haloC₁₋₆alkyl; and
   - a 3-8 membered saturated ring optionally containing one or more heteroatoms each independently selected from the group consisting of O, S and N, the 3-8 membered saturated ring optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy; the 3-8 membered saturated ring being preferably selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl; in one embodiment the ring is selected from the group consisting of cyclopropyl, oxetanyl, piperidinyl, cyclobutanolyl, cyclohexanolyl, pyrrolidinyl, 1-methylpyrrolidinyl, or pyrrolidinyl N substituted with C(O)OR₆;
R₂ is selected from:
   - hydrogen;
   - C₁₋₆alkyl optionally substituted with one or more substituents selected from OH, halogen, NH₂, NHC(O)CH₃ and C₁₋₆alkoxy;
   - NH₂;
   - NHC(O)CH₃;
   - a 3-8 membered saturated ring optionally containing one or more heteroatoms selected from the group consisting of O, S and N; the 3-8 membered saturated ring being preferably selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl; in one embodiment the 3-8 membered saturated ring is piperidinyl optionally substituted with C(O)OR₆;
   - an aryl or heteroaryl ring, said ring being optionally substituted with one, two, three or more substituents each independently selected from C₁₋₆alkyl, haloC₁₋₆alkyl and halogen; in a preferred embodiment the aryl or heteroaryl ring is selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl or oxazolyl; in one embodiment the aryl is selected from the group consisting of phenyl, pyridyl, pyrimidinyl, pyrazinyl, thienyl, thiazolyl, thiadiazolyl, pyrazolyl, imidazolyl, isoxazolyl and oxazolyl;
R₃ is absent, hydrogen, C₁₋₆alkyl; in particular hydrogen or methyl;
R₄ is hydrogen, C₁₋₄alkyl, C₃₋₆cycloalkyl or OH; in particular hydrogen or methyl;
R₅ is C₁₋₃alkyl;
R₆ C₁₋₄alkyl; in particular *t*-butyl;
or R₁ and R₂ taken together form with the atoms to which they are respectively attached a heterocyclic ring selected from the group consisting of aziridinyl, azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy; in one embodiment the heteroaryl is imidazole;
or any two residues of R₁ and R₄, or R₁ and R₅ taken together represent a C₁₋₄ alkanediyl optionally substituted with one or more substituents each independently selected from C₁₋₄ alkyl and halogen; said C₁₋₄ alkanediyl forming together with the bridging atoms to which it is linked a 4-10 heterocyclic ring;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, each ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃; in a preferred embodiment the 3-7 membered saturated ring is selected from cyclobutyl, oxetanyl, piperidinyl and tetrahydro-2H-pyranyl, the 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃; the 4-8 membered saturated ring is preferably selected from the group consisting of cyclobutyl, cyclopentyl and cyclohexyl; in one embodiment the 4-8 membered saturated ring is cyclopentyl;
or, when Y is a bond, R₂ and R₄ taken together form with the atoms to which they are attached a 5-6 membered heteroaryl ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl; or is absent when B is pyridyl;
Rf is selected from the group consisting of hydrogen, halogen and methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate; or 4-cyclopropyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

In a further preferred embodiment the invention relates to a compound of formula (I) wherein:
A is phenyl or pyridyl;
B is phenyl or pyridyl;
X is N optionally substituted with R₄; or is O;
Y is a single bond, C₁₋₂alkanediyl optionally substituted with R₅;
Z is CR₂R₃ or C(O);
R₁ is selected from:
   - hydrogen;
   - OH;
   - C₁₋₆alkyl;
   - hydroxyC₁₋₆alkyl;
   - haloC₁₋₆alkyl; and
   - a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
   - hydrogen;
   - C₁₋₆alkyl optionally substituted with one or more substituents selected from OH, halogen, NH₂, NHC(O)CH₃ and C₁₋₆alkoxy;
NH₂;
NHC(O)CH₃;
a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, each of said ring being optionally substituted with one or more substituents each independently selected from OH, C(O)OR₆, C(O)CH₃, C₁₋₆alkyl and CN; and
an aryl or heteroaryl ring selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl, each ring optionally substituted with one, two, three or more substituents each independently selected from C₁₋₆alkyl, haloC₁₋₆alkyl and halogen;
R₃ is absent, hydrogen or methyl;
R₄ is hydrogen, C₁₋₄alkyl or OH; in particular hydrogen or methyl;
R₅ is C₁₋₃alkyl;
R₆ is C₁₋₄alkyl; in particular *t*-butyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl, pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy, haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl and thiazolidinyl, thiolane 1,1-dioxide and pyrrolidinyl, each ring optionally being substituted with one or more substituents independently selected from halogen, OH, haloC₁₋₆alkyl, CN, C₁₋₆alkyl, haloC₁₋₆alkoxy, C(O)OR₆ and C(O)CH₃; or R₂ and R₃ taken together form with the carbon atom to which they are attached an indoline-2-one or an azaindoline-2-one;
or any two residues of R₁ and R₄, or R₁ and R₅ taken together represent a C₁₋₄ alkanediyl optionally substituted with one or more substituents each independently selected from C₁₋₄ alkyl and halogen;
said C₁₋₄ alkanediyl forming together with the bridging atoms to which it is linked a 4-10 heterocyclic ring;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃,
in a preferred embodiment R₂ and R₅ taken together form with the carbon atoms to which they are respectively attached a saturated ring selected from the group consisting of cyclobutyl, cyclopentyl and cyclohexyl, each ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
Ra is selected from the group consisting of hydrogen, halogen and CN;
Rb is selected from the group consisting of hydrogen, halogen and CN;
Rc is selected from the group consisting of hydrogen, halogen and CN;
Rd is selected from the group consisting of hydrogen, halogen and CN;
Re is selected from the group consisting of hydrogen, halogen, C₁₋₃alkyl, NH₂, NH(CH₃), N(CH₃)₂, NHC(O)CH₃, OH, OR, CN; or is absent when B is pyridyl;
Rf is selected from the group consisting of hydrogen, halogen or methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆, Re, Rf and Rg are according to any of the definitions reported above; A is phenyl; B is phenyl; at least one of Ra, Rb, Rc and Rd are F and the other(s) is/are hydrogen;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆, Re, Rf and Rg are according to any of the definitions reported above; A is phenyl; B is phenyl; at least two of Ra, Rb, Rc and Rd are F and the other(s) is/are hydrogen; and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆, Re, Rf and Rg are according to any of the definitions reported above for formula (I); A is phenyl; B is phenyl; at least three of Ra, Rb, Rc and Rd are F and the other is hydrogen;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆, Re, Rf and Rg are according to any of the definitions reported above for formula (I); A is phenyl; B is phenyl; Ra, Rc and Rd are each independently hydrogen or F; Rb is selected from the group consisting of methyl, Cl, CF₃, CHF₂ and CN;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆ are according to any of the definitions reported above for formula (I); A is phenyl; B is phenyl; at least two of Ra, Rb, Rc and Rd are F and the other(s) is/are hydrogen; Re, Rf and Rg are hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆ are according to any of the definitions reported above for formula (I); A is phenyl; B is phenyl; Ra, Rb, Rc, Rd are each independently hydrogen or F; Re is selected from the group consisting of methyl, Cl, Br or F; Rf is hydrogen; Rg is hydrogen;
and pharmaceutically acceptable salts, tautomers, isomers, stereoisomers thereof.

Another embodiment of the present invention relates to the compounds of formula (I) wherein X, Y, Z, R₁, R₂, R₃, R₄, R₅, R₆ are according to any of the definitions reported above for formula (I); A is phenyl; B is phenyl; Ra, Rb, Rc and Rd are each independently hydrogen or F; Re is hydrogen; Rf is methyl; Rg is hydrogen;
and pharmaceutically acceptable salts, tautomers, isomers, stereoisomers thereof.

In a further embodiment the invention relates to a compound with Y being a single bond, having formula (Ia): 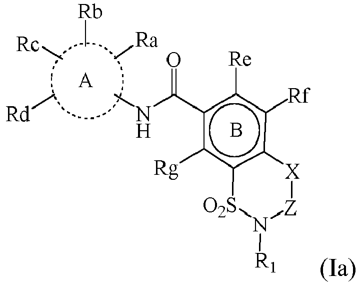 wherein:
A is phenyl or pyridyl;
B is phenyl or pyridyl;
Z is CR₂R₃ or C(O);
X is N optionally substituted with R₄; or is O;
R₁ is selected from:
   - hydrogen;
   - OH;
   - C₁₋₆alkyl, hydroxyC₁₋₆alkyl or haloC₁₋₆alkyl; and
   - a 3-8 membered saturated ring selected from the group consisting of cyclopropyl, oxetanyl, piperidinyl, cyclobutanolyl, cyclohexanolyl, pyrrolidinyl and 1-methylpyrrolidinyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
   - hydrogen;
   - C₁₋₆alkyl optionally substituted with one or more substituents selected from halogen, OH, NH₂, NHC(O)CH₃, C₁₋₆alkoxy;
   - NH₂;
   - NHC(O)CH₃;
   - piperidinyl optionally substituted with C(O)OR₆; and
   - an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from C₁₋₆alkyl, haloC₁₋₆alkyl and halogen;
R₃ is absent, hydrogen, or methyl;
R₄ is hydrogen or methyl;
R₆ is C₁₋₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a 5-membered heteroaryl optionally substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclobutyl, oxetanyl, piperidinyl and tetrahydro-2H-pyranyl; said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄ taken together represent a C₁₋₄ alkanediyl forming together with the bridging atoms to which it is linked a 4-10 heterocyclic ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

In a further embodiment the invention relates to a compound with X being NH, Z being C(O) and Y being a single bond, having formula (Ib): 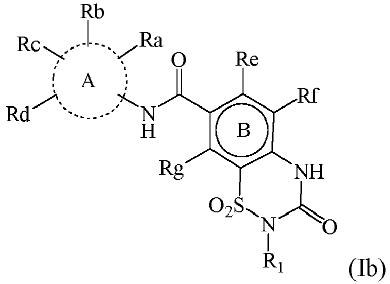 wherein:
A is phenyl;
B is phenyl;
R₁ is selected from:
   - hydrogen;
   - methyl;
   - CH(CH₃)CF₃; and
   - a 3-8 membered saturated ring selected from cyclopropyl, oxetanyl, piperidinyl, pyrrolidinyl, 1-methylpyrrolidinyl, cyclobutanolyl and cyclohexanolyl, said 3-8 membered saturated ring being substituted with one, two or more substituents each independently selected from the group consisting of C₁₋₄alkyl, OH, C(O)OR₆ and C(O)CH₃;
R6 is C₁₋₄ alkyl;
Ra is selected from the group consisting of hydrogen, methyl and halogen;
Rb is selected from the group consisting of hydrogen, methyl and halogen;
Rc is selected from the group consisting of hydrogen, methyl and halogen;
Rd is hydrogen;
Re, Rf and Rg are each hydrogen;
provided that the compound is not: tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof

In a further embodiment the invention relates to a compound with Z being CR₂R₃ and Y being a single bond, having formula (Ic): 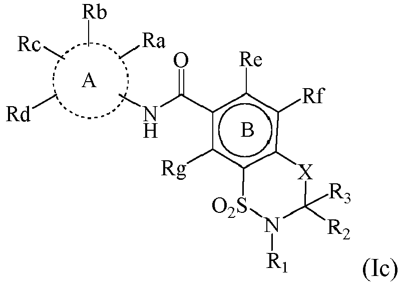 wherein:
A is phenyl or pyridyl;
B is phenyl or pyridyl;
X is N optionally substituted with R₄;
R₁ is selected from hydrogen, methyl, OH, CH₂CH₂OH, cyclopropyl, cyclohexanolyl and piperidinyl;
R₂ is selected from:
   - hydrogen;
   - methyl, CF₃, CHF₂, CH₂OH, CH₂CH₂OH, CHOHCH₂OH, CH(CH)₃OH, CH₂NH₂, CH₂NHC(O)CH₃, CH₂CH₂OCH(CH₃)₂;
   - piperidinyl optionally substituted with C(O)OR₆; and
   - an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from methyl, CF₃, Cl, F and Br;
R₃ is hydrogen or methyl;
R₄ is hydrogen, methyl or OH;
R₆ is C₁₋₄ alkyl;
or R₁ and R₂ taken together form with the atom to which they are respectively attached a pyrrolidinyl;
or R₂ and R₃ taken together with the atom to which they are respectively attached form a ring selected from the group consisting of cyclobutyl, oxetanyl, piperidinyl and tetrahydro-2H-pyranyl;
or R₂ and R₃ taken together form with the carbon atom to which they are attached an indoline-2-one or an azaindoline-2-one;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

In a further embodiment the invention relates to a compound having formula (Ic): wherein X, R₁, R₂, R₃, R₄, R₆, Ra, Rb, Rc and Rd are as defined above for fomula (Ic); A is phenyl, B is phenyl; Re is selected from the group consisting of hydrogen, halogen and methyl; Rf is selected from the group consisting of hydrogen, halogen and methyl; Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

Another embodiment of the present invention relates to the compounds of formula (I), (Ia), (Ib), (Ic) wherein X, Y, Z, R₁, R₂, R₃, R₄, R₅, R₆ are as defined above; A is phenyl; B is phenyl; Ra, Rb, Rc and Rd are each independently hydrogen or F; Re is selected from the group consisting of hydrogen, halogen and methyl; Rf is selected from the group consisting of hydrogen, halogen and methyl; Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

Another embodiment of the present invention relates to the compounds of formula (I), (Ia), (Ib), (Ic) wherein X, Y, Z, R₁, R₂, R₃, R₄, R₅, R₆ are as defined above in any of formula (I), (Ia), (Ib), (Ic); A is phenyl; B is phenyl; at least one of Ra, Rb, Rc and Rd are F and the other(s) is/are hydrogen; Re is selected from the group consisting of hydrogen, halogen and methyl; Rf is selected from the group consisting of hydrogen, halogen and methyl; Rg is hydrogen;
and pharmaceutically acceptable salts, tautomers, isomers, stereoisomers thereof.

Another embodiment of the present invention relates to the compounds of formula (I), (Ia), (Ib) or (Ic) wherein X, Y, Z, R₁, R₂, R₃, R₄, R₅, R₆ are as defined above in any of formula (I), (Ia), (Ib), (Ic); A is phenyl; B is phenyl; at least two of Ra, Rb, Rc and Rd are F and the other(s) is/are hydrogen; Re, Rf and Rg are hydrogen;
and pharmaceutically acceptable salts, tautomers, isomers, stereoisomers thereof.

Another embodiment of the present invention relates to the compounds of formula (I), (Ia), (Ib), or (Ic) wherein X, Y, Z, R₁, R₂, R₃, R₄, R₅, R₆ are as defined above in any of formula (I), (Ia), (Ib), (Ic); A is phenyl; B is phenyl; at least three of Ra, Rb, Rc and Rd are F and the other is hydrogen; Re, Rf and Rg are hydrogen;
and pharmaceutically acceptable salts, tautomers, isomers, stereoisomers thereof.

Another embodiment of the present invention relates to the compounds of formula (I), (Ia), (Ib) or (Ic) wherein X, Y, Z, R₁, R₂, R₃, R₄, R₅, R₆ are as defined above in any of formula (I), (Ia), (Ib), (Ic); A is phenyl; B is phenyl; Ra, Rb, Rc, Rd are each independently hydrogen or F; Re is selected from the group consisting of hydrogen, methyl, Cl, Br and F; Rf and Rg are hydrogen;
and pharmaceutically acceptable salts, tautomers, isomers, stereoisomers thereof.

Another embodiment of the present invention relates to the compounds of formula (I), (Ia), (Ib) or (Ic) wherein X, Y, Z, R₁, R₂, R₃, R₄, R₅, R₆ are as defined above in any of formula (I), (Ia), (Ib), (Ic); A is phenyl; B is phenyl; Ra, Rb, Rc, Rd are each independenlty hydrogen or F; Re is hydrogen; Rf is methyl; Rg is hydrogen;
and pharmaceutically acceptable salts, tautomers, isomers, stereoisomers thereof.

Another embodiment of the present invention relates to the compounds of (I), (Ia), (Ib) or (Ic) wherein X, Y, Z, R₁, R₂, R₃, R₄, R₅, R₆ are as defined above in any of formula (I), (Ia), (Ib), (Ic); Ra, Rc and Rd are each independently hydrogen or F; Rb is selected from the group consisting of methyl, Cl, CF₃, CHF₂ and CN;

Another embodiment of the present invention relates to the compounds of formula (I), (Ia), (Ib) or (Ic) wherein X, Y, Z, R₁, R₂, R₃, R₄, R₅, R₆ are as defined in any of formula (I), (Ia), (Ib), (Ic); A is phenyl; B is phenyl; Ra is F; Rb is Cl; Rc and Rd are hydrogen; Re is selected from the group consisting of hydrogen, halogen and methyl; Rf is selected from the group consisting of hydrogen, halogen and methyl; Rg is hydrogen;
and pharmaceutically acceptable salts, tautomers, isomers, stereoisomers thereof.

Preferred compounds exhibit an HBV inhibition and/or an EC₅₀, as defined hereinbelow, greater than 50%. In particular, preferred compounds are selected from the following list:
- N-(3,4,5-trifluorophenyl)-3,4-dihydro-2H-benzo[e][1,2,4]thiadiazine-7-carboxamide 1,1-dioxide;
- N-(3,4,5-trifluorophenyl)-3,4-dihydro-2H-benzo[e][1,2,4]thiadiazine-3,3-d₂-7-carboxamide 1,1-dioxide;
- 6-chloro-N-(3,4,5-trifluorophenyl)-3,4-dihydro-2H-benzo[e][1,2,4]thiadiazine-7-carboxamide 1,1-dioxide;
- 6-bromo-N-(3,4,5-trifluorophenyl)-3,4-dihydro-2H-benzo[e][1,2,4]thiadiazine-7-carboxamide 1,1-dioxide;
- 6-methyl-N-(3,4,5-trifluorophenyl)-3,4-dihydro-2H-benzo[e][1,2,4]thiadiazine-7-carboxamide 1,1-dioxide;
- 6-chloro-3-methyl-N-(3,4,5-trifluorophenyl)-3,4-dihydro-2H-benzo[e][1,2,4]thiadiazine-7-carboxamide 1,1-dioxide;
- N-(3,4-difluorophenyl)-2H,4H-spiro[benzo[e][1,2,4]thiadiazine-3,1'-cyclobutane]-7-carboxamide 1,1-dioxide;
- N-(3,4,5-trifluorophenyl)-2',3',5',6'-tetrahydro-2H,4H-spiro[benzo[e][1,2,4]thiadiazine-3,4'-pyran]-7-carboxamide 1,1-dioxide;
- N-(3,4,5-trifluorophenyl)-2H,4H-spiro[benzo[e][1,2,4]thiadiazine-3,4'-piperidine]-7-carboxamide 1,1-dioxide;
- 3-(2-isopropoxyethyl)-N-(3,4,5-trifluorophenyl)-3,4-dihydro-2H-benzo[e][1,2,4]thiadiazine-7-carboxamide 1,1-dioxide;
- 3-(difluoromethyl)-N-(3,4,5-trifluorophenyl)-3,4-dihydro-2H-benzo[e][1,2,4]thiadiazine-7-carboxamide 1,1-dioxide;
- N-(3,4-difluorophenyl)-3,4-dihydro-2H-benzo[e][1,2,4]thiadiazine-7-carboxamide 1,1-dioxide;
- N-(3,4-difluorophenyl)-3-methyl-3,4-dihydro-2H-benzo[e][1,2,4]thiadiazine-7-carboxamide 1,1-dioxide;
- 3-methyl-N-(3,4,5-trifluorophenyl)-3,4-dihydro-2H-benzo[e][1,2,4]thiadiazine-7-carboxamide 1,1-dioxide;
- 3-(2-hydroxyethyl)-N-(3,4,5-trifluorophenyl)-3,4-dihydro-2H-benzo[e][1,2,4]thiadiazine-7-carboxamide 1,1-dioxide;
- 3-((S)-1-hydroxyethyl)-N-(3,4,5-trifluorophenyl)-3,4-dihydro-2H-benzo[e][1,2,4]thiadiazine-7-carboxamide 1,1-dioxide;
- 3-((R)-1,2-dihydroxyethyl)-N-(3,4,5-trifluorophenyl)-3,4-dihydro-2H-benzo[e][1,2,4]thiadiazine-7-carboxamide 1,1-dioxide;
- 3,3-dimethyl-N-(3,4,5-trifluorophenyl)-3,4-dihydro-2H-benzo[e][1,2,4]thiadiazine-7-carboxamide 1,1-dioxide;
- 3-((R)-1-hydroxyethyl)-N-(3,4,5-trifluorophenyl)-3,4-dihydro-2H-benzo[e][1,2,4]thiadiazine-7-carboxamide 1,1-dioxide;
- 3-(hydroxymethyl)-N-(3,4,5-trifluorophenyl)-3,4-dihydro-2H-benzo[e][1,2,4]thiadiazine-7-carboxamide 1,1-dioxide;
- 3-(aminomethyl)-N-(3,4,5-trifluorophenyl)-3,4-dihydro-2H-benzo[e][1,2,4]thiadiazine-7-carboxamide 1,1-dioxide;
- 3-(acetamidomethyl)-N-(3,4,5-trifluorophenyl)-3,4-dihydro-2H-benzo[e][1,2,4]thiadiazine-7-carboxamide 1,1-dioxide;
- tert-butyl 4-(1,1-dioxido-7-((3,4,5-trifluorophenyl)carbamoyl)-3,4-dihydro-2H-benzo[e][1,2,4]thiadiazin-3-yl)piperidine-1-carboxylate;
- 3-(piperidin-4-yl)-N-(3,4,5-trifluorophenyl)-3,4-dihydro-2H-benzo[e][1,2,4]thiadiazine-7-carboxamide 1,1-dioxide;
- 3-(2,6-difluorophenyl)-N-(3,4,5-trifluorophenyl)-3,4-dihydro-2H-benzo[e][1,2,4]thiadiazine-7-carboxamide 1,1-dioxide;
- 6-chloro-3-(thiazol-2-yl)-N-(3,4,5-trifluorophenyl)-3,4-dihydro-2H-benzo[e][1,2,4]thiadiazine-7-carboxamide 1,1-dioxide;
- 3-(1-methyl-1H-imidazol-5-yl)-N-(3,4,5-trifluorophenyl)-3,4-dihydro-2H-benzo[e][1,2,4]thiadiazine-7-carboxamide 1,1-dioxide;
- N-(3,4-difluorophenyl)-3-(2,4-dimethyloxazol-5-yl)-3,4-dihydro-2H-benzo[e][1,2,4]thiadiazine-7-carboxamide 1,1-dioxide;
- 3-methyl-3-(pyridin-4-yl)-N-(3,4,5-trifluorophenyl)-3,4-dihydro-2H-benzo[e][1,2,4]thiadiazine-7-carboxamide 1,1-dioxide;
- 3-(thiazol-5-yl)-N-(3,4,5-trifluorophenyl)-3,4-dihydro-2H-benzo[e][1,2,4]thiadiazine-7-carboxamide 1,1-dioxide;
- 3-(thiazol-4-yl)-N-(3,4,5-trifluorophenyl)-3,4-dihydro-2H-benzo[e][1,2,4]thiadiazine-7-carboxamide 1,1-dioxide;
- 3-(6-(trifluoromethyl)pyridin-3-yl)-N-(3,4,5-trifluorophenyl)-3,4-dihydro-2H-benzo[e][1,2,4]thiadiazine-7-carboxamide 1,1-dioxide;
- 3-(thiophen-2-yl)-N-(3,4,5-trifluorophenyl)-3,4-dihydro-2H-benzo[e][1,2,4]thiadiazine-7-carboxamide 1,1-dioxide;
- 3-(pyridin-2-yl)-N-(3,4,5-trifluorophenyl)-3,4-dihydro-2H-benzo[e][1,2,4]thiadiazine-7-carboxamide 1,1-dioxide;
- 3-(1,2,3-thiadiazol-5-yl)-N-(3,4,5-trifluorophenyl)-3,4-dihydro-2H-benzo[e][1,2,4]thiadiazine-7-carboxamide 1,1-dioxide;
- 3-(1H-pyrazol-5-yl)-N-(3,4,5-trifluorophenyl)-3,4-dihydro-2H-benzo[e][1,2,4]thiadiazine-7-carboxamide 1,1-dioxide;
- N-(3,4-difluorophenyl)-3-(pyridin-3-yl)-3,4-dihydro-2H-benzo[*e*][1,2,4]thiadiazine-7-carboxamide 1,1-dioxide;
- 3-(pyridin-3-yl)-N-(3,4,5-trifluorophenyl)-3,4-dihydro-2H-benzo[*e*][1,2,4]thiadiazine-7-carboxamide 1,1-dioxide;
- 3-(thiazol-2-yl)-N-(3,4,5-trifluorophenyl)-3,4-dihydro-2H-benzo[*e*][1,2,4]thiadiazine-7-carboxamide 1,1-dioxide;
- N-(3,4-difluorophenyl)-3-(thiazol-2-yl)-3,4-dihydro-2H-benzo[*e*][1,2,4]thiadiazine-7-carboxamide 1,1-dioxide;
- 3-(6-chloropyridin-3-yl)-N-(3,4,5-trifluorophenyl)-3,4-dihydro-2H-benzo[e][1,2,4]thiadiazine-7-carboxamide 1,1-dioxide;
- 3-(5-chloropyridin-2-yl)-3-methyl-N-(3,4,5-trifluorophenyl)-3,4-dihydro-2H-benzo[e][1,2,4]thiadiazine-7-carboxamide 1,1-dioxide;
- 3-(pyrimidin-2-yl)-N-(3,4,5-trifluorophenyl)-3,4-dihydro-2H-benzo[e][1,2,4]thiadiazine-7-carboxamide 1,1-dioxide;
- 3-methyl-3-(pyrazin-2-yl)-N-(3,4,5-trifluorophenyl)-3,4-dihydro-2H-benzo[e][1,2,4]thiadiazine-7-carboxamide 1,1-dioxide;
- 2'-oxo-N-(3,4,5-trifluorophenyl)-2H,4H-spiro[benzo[e][1,2,4]thiadiazine-3,3'-indoline]-7-carboxamide 1,1-dioxide;
- 2'-oxo-N-(3,4,5-trifluorophenyl)-1',2'-dihydro-2H,4H-spiro[benzo[e][1,2,4]thiadiazine-3,3'-pyrrolo[2,3-b]pyridine]-7-carboxamide 1,1-dioxide;
- N-(3-chloro-4-fluorophenyl)-3,4-dihydro-2H-benzo[e][1,2,4]thiadiazine-7-carboxamide 1,1-dioxide;
- 6-fluoro-N-(3,4,5-trifluorophenyl)-3,4-dihydro-2H-benzo[e][1,2,4]thiadiazine-7-carboxamide 1,1-dioxide;
- 2-methyl-3-oxo-N-(3,4,5-trifluorophenyl)-3,4-dihydro-2H-benzo[e][1,2,4]thiadiazine-7-carboxamide 1,1-dioxide;
- (S)-3-oxo-N-(3,4,5-trifluorophenyl)-2-(1,1,1-trifluoropropan-2-yl)-3,4-dihydro-2H-benzo[e][1,2,4]thiadiazine-7-carboxamide 1,1-dioxide;
- *trans*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide;
- 3-oxo-2-(piperidin-4-yl)-N-(3,4,5-trifluorophenyl)-3,4-dihydro-2H-benzo[e][1,2,4]thiadiazine-7-carboxamide 1,1-dioxide;
- 2-(oxetan-3-yl)-3-oxo-N-(3,4,5-trifluorophenyl)-3,4-dihydro-2H-benzo[e][1,2,4]thiadiazine-7-carboxamide 1,1-dioxide;
- 2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- *cis*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide;
- 5-methyl-3-(thiazol-2-yl)-N-(3,4,5-trifluorophenyl)-3,4-dihydro-2H-benzo[e][1,2,4]thiadiazine-7-carboxamide 1,1-dioxide;
- N-(3,4,5-trifluorophenyl)-1,3,4,11,12,12a-hexahydro-2H-benzo[f]pyrido[1,2-b][1,2,5]thiadiazepine-8-carboxamide 6,6-dioxide;
- (R)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide;
- N-(3,4-difluorophenyl)-3-(hydroxymethyl)-3,4-dihydro-2H-benzo[e][1,2,4]thiadiazine-7-carboxamide 1,1-dioxide;
- N-(3,4-difluorophenyl)-3,3-dimethyl-3,4-dihydro-2H-benzo[e][1,2,4]thiadiazine-7-carboxamide 1,1-dioxide;
- trans-2-(4-hydroxycyclohexyl)-6-methyl-3-oxo-N-(3,4,5-trifluorophenyl)-3,4-dihydro-2H-benzo[e][1,2,4]thiadiazine-7-carboxamide 1,1-dioxide;
- N-(3-chloro-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide;
- Cis-2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- Trans-2(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- (R)-2'-oxo-N-(3,4,5-trifluorophenyl)-2H,4H-spiro[benzo[e][1,2,4]thiadiazine-3,3'-indoline]-7-carboxamide 1,1-dioxide (single enantiomer; second eluted);
- N-(3-(difluoromethyl)-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide;
- N-(4-fluoro-3-methylphenyl)-2-((1r,4r)-4-hydroxycyclohexyl)-3-oxo-3,4-dihydro-2H-benzo[e][1,2,4]thiadiazine-7-carboxamide 1,1-dioxide;
- 2-(1-methylpiperidin-4-yl)-3-oxo-N-(3,4,5-trifluorophenyl)-3,4-dihydro-2H-benzo[e][1,2,4]thiadiazine-7-carboxamide 1,1-dioxide;
- 2-(4-(hydroxymethyl)cyclohex-3-en-1-yl)-3-oxo-N-(3,4,5-trifluorophenyl)-3,4-dihydro-2H-benzo[e][1,2,4]thiadiazine-7-carboxamide 1,1-dioxide
and pharmaceutically acceptable salts, tautomers, isomers, stereoisomers thereof.

It is an object of the invention a compound of formula (I), (Ia), (Ib) or (Ic) as defined above for medical use. Preferably, the compound as defined above is for use in the treatment and/or prevention of a HBV infection.

It is therefore an object of the present invention a compound of formula (I) for use in the treatment of hepatitis B virus (HBV) infection, as defined below: 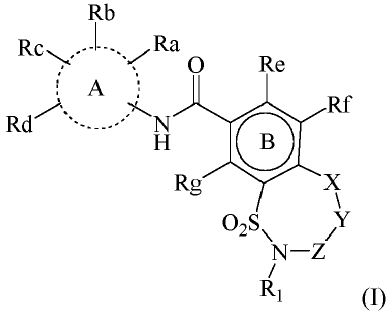 wherein:
A is aryl or heteroaryl;
B is a 6-membered aryl optionally containing one or more N atoms;
X is N optionally substituted with R₄; or is O;
Y is a single bond; a double bond; a C₁₋₄alkanediyl or a C₂₋₄alkenediyl each of said C₁₋₄alkanediyl or C₂₋₄alkenediyl being independently optionally substituted with R₅;
Z is CR₂R₃ or C(O);
R₁ is selected from:
   - hydrogen;
   - OH;
   - C₁₋₆alkyl optionally substituted with one or more substituents each independently selected from the group consisting of OH, halogen, CN, NH₂, NH(R₆), N(R₆)₂, aryl, heteroaryl, 3-7 membered saturated ring and 5-7 membered unsaturated ring, each of said saturated or unsaturated ring optionally containing one or more heteroatoms selected from the group consisting of O, N, S and each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
   - aryl or heteroaryl ring, each of said aryl or heteroaryl ring being optionally substituted with one or more substituents selected from OH, halogen, haloC₁₋₆alkyl, CN, haloC₁₋₆alkoxy and C₁₋₆alkoxy; and
   - a 3-8 membered saturated or partially unsaturated ring optionally containing one or more heteroatoms each independently selected from the group consisting of O, S and N, the 3-8 membered saturated or partially unsaturated ring being optionally substituted with one, two or more substituents each independently selected from the group consisting of OH, halogen, CN, C₁₋₄alkyl, hydroxyC₁₋₄alkyl, C(O)OR₆, C(O)R₆, haloC₁₋₆alkyl, haloC₁₋₆alkoxy and C₁₋₆alkoxy;
R₂ is selected from:
   - hydrogen;
   - deuterium;
   - C₁₋₆alkyl; C₁₋₆alkoxy; C₂₋₆alkenyl; each of said C₁₋₆alkyl, C₁₋₆alkoxy or C₂₋₆alkenyl being optionally substituted with one or more substituents independently selected from OH, halogen, NH₂, NH(R₆), N(R₆)₂, NHC(O)R₆, C₁₋₆alkoxy, haloC₁₋₆alkoxy, CN, aryl, heteroaryl, 3-7 membered saturated ring and 5-7 membered unsaturated ring, each of said 3-7 membered saturated or 5-7 membered unsaturated ring optionally containing one or more heteroatoms selected from the group consisting of O, N, S, each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
   - NH₂;
   - NHC(O)R₆;
   - a 3-8 membered saturated ring optionally containing one or more heteroatoms each independently selected from the group consisting of O, S and N, the 3-8 membered saturated ring being optionally substituted with one or more substituents each independently selected from the group consisting of OH, halogen, C(O)OR₆, C(O)R₆, C₁₋₆alkyl and CN; and
   - an aryl or heteroaryl ring optionally substituted with one, two, three or more substituents each independently selected from the group consisting of C₁₋₆alkyl, haloC₁₋₆alkyl, halogen, C₁₋₆alkoxy and haloC₁₋₆alkoxy;
R₃ is absent, hydrogen, deuterium or C₁₋₆alkyl;
R₄ is hydrogen, C₁₋₄alkyl, C₃₋₆cycloalkyl or OH;
R₅ is C₁₋₃alkyl optionally substituted with OH;
R₆ is C₁₋₄alkyl;
or R₁ and R₂ taken together form with the atoms to which they are respectively attached a heterocyclic ring optionally substituted with one or more substituents each independently selected from the group consisting of C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl ring optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or any two residues of R₁, R₂, R₃, R₄, R₅ which are not geminal or vicinal group, taken together represent a single bond, or represent a C₁₋₄alkanediyl or a C₂₋₄alkenediyl, each of said C₁₋₄alkanediyl or a C₂₋₄alkenediyl being independently optionally substituted with one or more substituents each independently selected from C₁₋₄ alkyl and halogen; said single bond or said optionally substituted C₁₋₄alkanediyl or C₂₋₄alkenediyl forming together with the bridging atoms to which they are respectively linked a 4-10 membered saturated or partially unsaturated ring;
or R₂ and R₃ taken together form with the carbon atom to which they are attached a 3-7 membered saturated ring optionally containing one or more heteroatoms each independently selected from the group consisting of O, S and N, the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)R₆;
or R₂ and R₃ taken together form with the carbon atom to which they are attached an indoline-2-one or an azaindoline-2-one, each being optionally substituted with one or more substituents selected from halogen, hydroxy, C₁₋₆alkyl, haloC₁₋₆alkyl, C₁₋₆alkoxy and haloC₁₋₆alkoxy;
or R₂ and R₅ taken together form with the carbon atoms to which they are respectively attached a 3-8 membered saturated ring or a 5-10 membered partially saturated ring, each ring optionally containing one or more heteroatoms each independently selected from the group consisting of O, S and N, each ring optionally being substituted with one, two or more substituents each independently selected from the group consisting of halogen, OH, CN, C(O)OR₆, C(O)R₆, C₁₋₆alkyl, C₁₋₆alkoxy, haloC₁₋₆alkyl and haloC₁₋₆alkoxy;
or, when Y is a bond, R₂ and R₄ taken together form with the atoms to which they are attached a 5-6 membered heteroaryl ring optionally substituted with C₁₋₃alkyl;
Ra is selected from the group consisting of hydrogen, halogen, CN, C₁₋₃alkyl, C₁₋₃alkoxy, haloC₁₋₃alkyl and haloC₁₋₃alkoxy; or is absent;
Rb is selected from the group consisting of hydrogen, halogen, CN, C₁₋₃alkyl, C₁₋₃alkoxy, haloC₁₋₃alkyl and haloC₁₋₃alkoxy; or is absent;
Rc is selected from the group consisting of hydrogen, halogen, CN, C₁₋₃alkyl, C₁₋₃alkoxy, haloC₁₋₆alkyl and haloC₁₋₃alkoxy; or is absent
Rd is selected from the group consisting of hydrogen, halogen, CN, C₁₋₃alkyl, C₁₋₃alkoxy, haloC₁₋₃alkyl and haloC₁₋₃alkoxy; or is absent;
Re is selected from the group consisting of hydrogen, halogen, C₁₋₃alkyl, NH₂, NH(CH₃), N(CH₃)₂, NHC(O)CH₃, OH, and CN; or is absent;
Rf is selected from the group consisting of hydrogen, halogen, C₁₋₃alkyl, haloC₁₋₃alkyl, C₁₋₃alkoxy and haloC₁₋₃alkoxy; or is absent;
Rg is selected from the group consisting of hydrogen, halogen, C₁₋₃alkyl, haloC₁₋₃alkyl, C₁₋₃alkoxy and haloC₁₋₃alkoxy; or is absent;
and pharmaceutically acceptable salts, tautomers, isomers, stereoisomers thereof, for use in the treatment and/or prevention of a HBV infection.

In a preferred embodiment, the compound for use in the treatment and/or prevention of a HBV infection has a general formula (I) wherein:
A is aryl or heteroaryl;
B is phenyl or pyridyl;
X is N optionally substituted with R₄; or is O;
Y is a single bond; a double bond; or a C₁₋₄ alkanediyl optionally substituted with R₅;
Z is CR₂R₃ or C(O);
R₁ is selected from:
   - hydrogen;
   - OH;
   - C₁₋₆alkyl; hydroxyC₁₋₆alkyl, haloC₁₋₆alkyl; and
   - a 3-8 membered saturated or partially unsaturated ring optionally containing one or more heteroatoms each independently selected from the group consisting of O, S and N, the 3-8 membered saturated or partially unsaturated ring being optionally substituted with one, two, three or more substituents each independently selected from the group consisting of OH, halogen, CN, C₁₋₆alkyl, hydroxyC₁₋₄alkyl, C(O)OR₆, C(O)CH₃, haloC₁₋₆alkyl, haloC₁₋₆alkoxy and C₁₋₆alkoxy;
R₂ is selected from:
   - hydrogen;
   - C₁₋₆alkyl optionally substituted with one or more substituents selected from OH, halogen, NH₂, NHC(O)CH₃ and C₁₋₆alkoxy;
   - NH₂;
   - NHC(O)CH₃;
   - a 3-8 membered saturated ring optionally containing one or more heteroatoms each independently selected from the group consisting of O, S and N, the 3-8 membered saturated ring optionally substituted with one or more substituents each independently selected from the group consisting of OH, halogen and C₁₋₆alkyl; and
   - an aryl or heteroaryl ring, each of said aryl or heteroaryl ring optionally substituted with one, two, three or more substituents each independently selected from C₁₋₆alkyl, haloC₁₋₆alkyl and halogen;
R₃ is absent, hydrogen or C₁₋₆alkyl;
R₄ is hydrogen, C₁₋₄alkyl, C₃₋₆cycloalkyl or OH;
R₅ is C₁₋₃alkyl;
R₆ is C₁₋₄alkyl;
or R₁ and R₂ taken together form with the atoms to which they are respectively attached a heterocyclic ring selected from the group consisting of aziridinyl, azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₁ and R₄, or R₁ and R₅ taken together represent a C₁₋₄ alkanediyl optionally substituted with one or more substituents each independently selected from C₁₋₄ alkyl and halogen;
said C₁₋₄ alkanediyl forming together with the bridging atoms to which it is linked a 4-10 heterocyclic ring;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
or, when Y is a bond, R₂ and R₄ taken together form with the atoms to which they are attached a 5-6 membered heteroaryl ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl; or is absent when B is pyridyl;
Rf is selected from the group consisting of hydrogen, halogen and methyl; or is absent when B is pyridyl;
Rg is hydrogen; or is absent when B is pyridyl;
and pharmaceutically acceptable salts, tautomers, isomers, stereoisomers thereof.

In a further preferred embodiment, the compound for use in the treatment and/or prevention of a HBV infection has a general formula (I) wherein:
A is phenyl or pyridyl;
B is phenyl or pyridyl;
X is N optionally substituted with R₄; or is O;
Y is a single bond, C₁₋₂alkanediyl optionally substituted with R₅;
Z is CR₂R₃ or C(O);
R₁ is selected from:
   - hydrogen;
   - OH;
   - C₁₋₆alkyl, hydroxyC₁₋₆alkyl, haloC₁₋₆alkyl; and
   - a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
   - hydrogen;
   - C₁₋₆alkyl optionally substituted with one or more substituents selected from OH, halogen, NH₂, NHC(O)CH₃ and C₁₋₆alkoxy;
   - NH₂;
   - NHC(O)CH₃;
   - a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one or more substituents each independently selected from OH, halogen, C(O)OR₆, C(O)CH₃, C₁₋₆alkyl and CN; and
   - an aryl or heteroaryl ring selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl, each of said aryl or heteroaryl ring being optionally substituted with one, two, three or more substituents each independently selected from C₁₋₆alkyl, haloC₁₋₆alkyl and halogen;
R₃ is absent, hydrogen or methyl;
R₄ is hydrogen, C₁₋₄alkyl or OH;
R₅ is C₁₋₃alkyl;
R₆ is C₁₋₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄, or R₁ and R₅ taken together represent a C₁₋₄ alkanediyl optionally substituted with one or more substituents each independently selected from C₁₋₄ alkyl and halogen; said C₁₋₄ alkanediyl forming together with the bridging atoms to which it is linked a 4-10 heterocyclic ring; or R₂ and R₅ taken together form with the carbon atoms to which they are respectively attached a 3-8 membered saturated ring selected from the group consisting of cyclobutyl, cyclopentyl and cyclohexyl, said 3-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
Ra is selected from the group consisting of hydrogen, halogen and CN;
Rb is selected from the group consisting of hydrogen, halogen and CN;
Rc is selected from the group consisting of hydrogen, halogen and CN;
Rd is selected from the group consisting of hydrogen, halogen and CN;
Re is selected from the group consisting of hydrogen, halogen, C₁₋₃alkyl, NH₂, NH(CH₃), N(CH₃)₂, NHC(O)CH₃, OH and CN; or is absent when B is pyridyl;
Rf is selected from the group consisting of hydrogen, halogen and methyl; or is absent when B is pyridyl;
Rg is hydrogen; or is absent when B is pyridyl;
and pharmaceutically acceptable salts, tautomers, isomers, stereoisomers thereof.

In a preferred embodiment, the compound for use in the treatment and/or prevention of a HBV infection has a general formula (Ia) wherein: 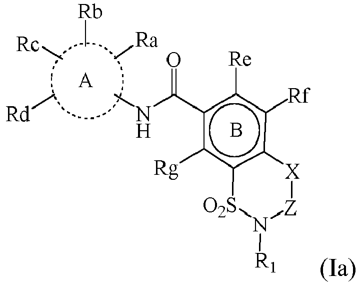 wherein:
A is phenyl or pyridyl;
B is phenyl or pyridyl;
Z is CR₂R₃ or C(O);
X is N optionally substituted with R₄; or is O;
R₁ is selected from:
   - hydrogen;
   - OH;
   - C₁₋₆alkyl, hydroxyC₁₋₆alkyl or haloC₁₋₆alkyl; and
   - a 3-8 membered saturated ring selected from the group consisting of cyclopropyl, oxetanyl, piperidinyl, cyclobutanolyl, cyclohexanolyl, pyrrolidinyl and 1-methylpyrrolidinyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
   - hydrogen;
   - C₁₋₆alkyl optionally substituted with one or more substituents selected from halogen, OH, NH₂, NHC(O)CH₃, C₁₋₆alkoxy;
   - NH₂;
   - NHC(O)CH₃;
   - piperidinyl optionally substituted with C(O)OR₆; and
   - an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from C₁₋₆alkyl, haloC₁₋₆alkyl and halogen;
R₃ is absent, hydrogen, or methyl;
R₄ is hydrogen or methyl;
R₆ is C₁₋₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a 5-membered heteroaryl optionally substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclobutyl, oxetanyl, piperidinyl and tetrahydro-2H-pyranyl; said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄ taken together represent a C₁₋₄ alkanediyl forming together with the bridging atoms to which it is linked a 4-10 heterocyclic ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
and pharmaceutically acceptable salts, tautomers, isomers, stereoisomers thereof.

Still preferably, the compound for use in the treatment and/or prevention of a HBV infection has a general formula (Ib) wherein: 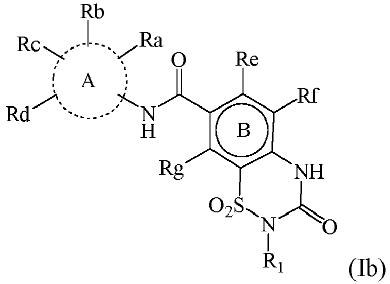 wherein:
A is phenyl;
B is phenyl;
R₁ is selected from:
   - hydrogen;
   - methyl;
   - CH(CH₃)CF₃; and
   - a 3-8 membered saturated ring selected from cyclopropyl, oxetanyl, piperidinyl, pyrrolidinyl, 1-methylpyrrolidinyl, cyclobutanolyl and cyclohexanolyl, said 3-8 membered saturated ring being substituted with one, two or more substituents each independently selected from the group consisting of C₁₋₄alkyl, OH, C(O)OR₆ and C(O)CH₃;
R6 is C₁₋₄ alkyl;
Ra is selected from the group consisting of hydrogen, methyl and halogen;
Rb is selected from the group consisting of hydrogen, methyl and halogen;
Rc is selected from the group consisting of hydrogen, methyl and halogen;
Rd is hydrogen;
Re, Rf and Rg are each hydrogen;
and pharmaceutically acceptable salts, tautomers, isomers, stereoisomers thereof.

In a preferred embodiment, the compound for use in the treatment and/or prevention of a HBV infection has a general formula (Ic) wherein: 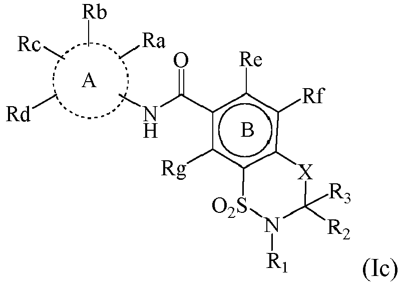 wherein:
A is phenyl or pyridyl;
B is phenyl or pyridyl;
X is N optionally substituted with R₄;
R₁ is selected from hydrogen, methyl, OH, CH₂CH₂OH, cyclopropyl, cyclohexanolyl and piperidinyl;
R₂ is selected from:
   - hydrogen;
   - methyl, CF₃, CHF₂, CH₂OH, CH₂CH₂OH, CHOHCH₂OH, CH(CH)₃OH, CH₂NH₂, CH₂NHC(O)CH₃, CH₂CH₂OCH(CH₃)₂;
   - piperidinyl optionally substituted with C(O)OR₆;
   - an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from methyl, CF₃, Cl, F and Br;
R₃ is hydrogen or methyl;
R₄ is hydrogen, methyl or OH;
R₆ is C₁₋₄ alkyl;
or R₁ and R₂ taken together form with the atom to which they are respectively attached a pyrrolidinyl;
or R₂ and R₃ taken together with the atom to which they are respectively attached form a ring selected from the group consisting of cyclobutyl, oxetanyl, piperidinyl and tetrahydro-2H-pyranyl; or R₂ and R₃ taken together form with the carbon atom to which they are attached an indoline-2-one or an azaindoline-2-one;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
and pharmaceutically acceptable salts, tautomers, isomers, stereoisomers thereof.

In a preferred embodiment, the compounds of the invention are intended for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

Preferably, the compound as defined above is for use in combination with at least one further therapeutic agent. Preferably, said use in combination comprises the administration of at least one therapeutic agent.

It is an object of the invention a pharmaceutical composition comprising the compound as defined above, alone or in combination with at least one further therapeutic agent, and at least one pharmaceutically acceptable excipient.

Preferably, the at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof.

Preferably, the therapeutic vaccine is selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell.

Preferably, the RNA interference therapeutic is a siRNA, a ddRNA or a shRNA. Preferably, the RNA interference therapeutic is selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404.

Preferably, the immunomodulator is a TLR agonist. Preferably the TLR agonist is a TLR7, TLR8 or TLR9 agonist. Preferably, the TLR7, TLR8 or TLR9 agonist is selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598.

Preferably, the RIG-I modulator is SB-9200. Preferably, the IL agonist or other immune acting protein is INO-9112 or recombinant IL12. Preferably, the immune checkpoint modulator/inhibitor is BMS-936558 (Opdivo (nivolumab)) or pembrolizumab. Preferably, the HBV entry inhibitor is Myrcludex B, IVIG-Tonrol or GC-1102.

Preferably, the cccDNA modulator is selected from: a direct cccDNA inhibitor, an inhibitor of cccDNA formation or maintenance, a cccDNA epigenetic modifier and an inhibitor of cccDNA transcription.

Preferably, the capsid assembly inhibitor/modulator, core or X protein targeting agent, direct cccDNA inhibitor, inhibitor of cccDNA formation or maintenance, or cccDNA epigenetic modifier is selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389.

Preferably, the interferon or modified interferon is selected from: interferon alpha (IFN-α), pegylated interferon alpha (PEG-IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter. Particularly preferred are: peginterferon alpha-2a, peginterferon alpha-2b, glycosylated interferon alpha-2b, peginterferon beta-la, and peginterferon lambda-1. More particularly preferred is peginterferon alpha-2a.

Preferably, the HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259.

Preferably, the cyclophilin inhibitor is selected from: OCB-030 (NVP-018), SCY-635, SCY-575 and CPI-431-32.

Preferably, said HBV polymerase inhibitor is selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir. Preferably, tenofovir is in a salt form. Preferably, tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157.

Preferably, the dinucleotide is SB9200. Preferably, the SMAC inhibitor is Birinapant. Preferably, the HDV targeting agent is Lonafamib.

Preferably, the HBV RNA destabilizer or other small-molecule inhibitor of HBV protein expression is RG7834 or AB-452.

Preferably, the at least one further therapeutic agent is an agent useful in the treatment and prevetion of hepatitis B. Preferably, the at least one further therapeutic agent is an anti-HDV agent, an anti-HCV agent and/or an anti-HIV agent.

Preferably the at least one further therapeutic agent is selected from the group consisting of: HBV polymerase inhibitor, interferon, viral entry inhibitor, BAY 41-4109, reverse transcriptase inhibitor, a TLR-agonist, AT-61 ((E)-N-(1-chloro-3-oxo-1-phenyl-3-(piperidin-1-yl)prop-1-en-2-yl)benzamide), AT-130 ((E)-N-(1-bromo-1-(2-methoxyphenyl)-3-oxo-3-(piperidin-1-yl)prop-1-en-2-yl)-4-nitrobenzamide), and a combination thereof, wherein the HBV polymerase inhibitor is preferably at least one of Lamivudine, Entecavir, Tenofovir, Adefovir, Telbivudine, Clevudine; and wherein the TLR agonist is preferably selected from the group consisting of SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-purin-9-yl)propyl][3-(4-morpholinyl)propyl] amino }methyl)phenyl] acetate) and a combination thereof.

Preferably, the compound of the invention is for use in combination with one, two or more further therapeutic agent(s) as defined above.

Preferably, the pharmaceutical composition of the invention comprises one, two or more further therapeutic agent(s) as defined above.

Preferably, said pharmaceutical composition is for use in the treatment and/or prevention of a HBV infection. Even more preferably, said pharmaceutical composition is for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection. In an embodiment, the invention provides a kit comprising at least one pharmaceutically acceptable vial or container containing one or more doses of a compound of the invention or of a pharmaceutical composition of the invention and optionally a) instructions for use thereof in mammals and/or b) an infusion bag or container containing a pharmaceutically acceptable diluent. It is a further object of the invention a process for the synthesis of a compound of general formula (I), (Ia), (Ib) or (Ic) according to the synthetic Schemes included in the description of the invention. It is a further object of the invention a pharmaceutical composition comprising an effective amount of one or more compounds as defined above or a pharmaceutically acceptable prodrug thereof, alone or in combination with other active compounds, and at least one pharmaceutically acceptable excipient.

The present invention includes within its scope prodrugs of the compounds of formula (I), (Ia), (Ib) or (Ic) above. In general, such prodrugs will be functional derivatives of the compounds of formula (I), (Ia), (Ib), (Ic) which are readily convertible *in vivo* into the required compound of formula (I), (Ia), (Ib), (Ic). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The disclosure also includes all suitable isotopic variations of a compound of the disclosure. Examples of isotopes that can be incorporated into compounds of the disclosure include isotopes such as ²H, ³H, ¹³C, ¹⁴C, ¹⁵N, ¹⁷O, ¹⁸0, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the disclosure can generally be prepared by conventional procedures such as by the illustrative methods or by the preparations described in the examples hereafter using appropriate isotopic variations of suitable reagents.

The present invention includes within its scope solvates of the compounds of (I), (Ia), (Ib) or (Ic) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted. The compounds may exist in different isomeric forms, all of which are encompassed by the present invention.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

The expressions "one or more substituents" and "one, two, three or more substituents" refer to in particular to 1, 2, 3, 4 or more substituents, in particular to 1, 2, 3 or 4 substituents, more in particular 1, 2 or 3 substituents.

As used herein "Y is a single bond" or "Y is a double bond", each indicates that X is directly linked to Z via, respectively, a single or a double bond.

As used herein, "alkyl" is intended to include both branched and straight-chain saturated aliphatic hydrocarbon groups having the specified number of carbon atoms. For example, "C₁₋₆alkyl" is defined to include groups having 1, 2, 3, 4, 5 or 6 carbons in a linear or branched arrangement. For example, "C₁₋₆ alkyl" specifically includes methyl, ethyl, *n*-propyl, *i*-propyl, *n*-butyl, *t*-butyl, *i*-butyl, pentyl, hexyl, and so on. "C₁₋₄alkyl" is defined to include groups having 1, 2, 3 or 4 carbons in a linear or branched arrangement. "C₁₋₃alkyl" is defined to include groups having 1, 2, or 3 carbons in a linear or branched arrangement. Preferred alkyl groups are methyl, ethyl, *i*-propyl or *t*-butyl.

As used herein, "alkoxy" represents an alkyl group of indicated number of carbon atoms attached through an oxygen bridge. "Alkoxy" therefore encompasses the definitions of alkyl above. C₁₋₆ alkoxy group is preferably a linear or branched C₁₋₄ alkoxy group, more preferably a C₁₋₃alkoxy group, still more preferably a C₁₋₂ alkoxy group. Examples of suitable alkoxy groups include, but are not limited to methoxy, ethoxy, *n*-propoxy, *i*-propoxy, *n*-butoxy, *s*-butoxy or *t*-butoxy. The preferred alkoxy group is methoxy.

As used herein, the terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. HaloC₁₋₆alkoxy group is preferably a linear or branched haloC₁₋₄alkoxy group, more preferably a haloC₁₋₃alkoxy group, still more preferably a haloC₁₋₂alkoxy group. HaloC₁₋₆alkyl group is preferably a linear or branched haloC₁₋₃alkyl group, more preferably a haloC₁₋₂alkyl group for example, CF₃, CHF₂, CH₂F, CH₂CH₂F, CH₂CHF₂, CH₂CF₃, CH(CH₃)CF₃, OCF₃, OCHF₂, OCH₂F, OCH₂CH₂F, OCH₂CHF₂ or OCH₂CF₃, and most especially CF₃, CHF₂, CH(CH₃)CF₃, OCF₃ or OCHF₂.

As used herein, the term "hydroxyC₁₋₆alkyl" means a C₁₋₆alkyl group in which one or more (in particular, 1 to 3) hydrogen atoms have been replaced by hydroxy groups. Similarly, the term "hydroxyC₁₋₄alkyl" means a C₁₋₄alkyl group in which one or more (in particular, 1 to 2) hydrogen atoms have been replaced by hydroxy groups. Illustrative examples include, but are not limited to CH₂OH, CH₂CH₂OH, CH(CH)₃OH and CHOHCH₂OH.

As used herein, the term "C₂₋₆alkenyl" refers to a non-aromatic hydrocarbon radical, straight or branched, containing from 2 to 6 carbon atoms and at least one carbon to carbon double bond. Preferably one carbon to carbon double bond is present, and up to four non-aromatic carbon-carbon double bonds may be present. Alkenyl groups include, but are not limited to ethenyl, propenyl, butenyl and 2-methylbutenyl. The straight or branched portion of the alkenyl group may contain double bonds and may be substituted if a substituted alkenyl group is indicated. Preferred alkenyl groups are ethenyl and propenyl.

As used herein, the term "aryl" means a monocyclic or polycyclic aromatic ring comprising carbon atoms, and hydrogen atoms. If indicated, such aromatic ring may include one or more heteroatoms, then also referred to as "heteroaryl", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Illustrative examples of aryl groups are optionally substituted phenyl. Illustrative examples of heteroaryl groups according to the invention include optionally substituted thiophene, oxazole, thiazole, thiadiazole, imidazole, pyrazole, pyrimidine, pyrazine and pyridine. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, indolyl, indolizinyl, isoindolinyl, indazolyl, isobenzofuranyl, isoindolyl, isoquinolyl, quinazolinyl, quinolyl, quinoxalinyl, quinolizinyl, naphtyl, naphthyridinyl and phthalazinyl. A preferred aromatic ring according to the present invention is phenyl. A preferred heteroaryl according to the present invention is pyridyl.

Heterocycle, heterocyclic compound or ring structure is a cyclic compound that has atoms of at least two different elements as members of its ring(s).

As used herein, the term "heterocyclic ring" is a saturated or partially saturated non aromatic ring system, of 4 to 10 members which contains one or more heteroatoms selected from N, O or S.

Examples include, but are not limited to azetidinyl, piperazinyl, piperidinyl, morpholinyl, thiomorpholinyl, thiazolidinyl, pyrrolidinyl, azepanyl, diazepanyl, oxazepanyl, thiazepanyl, azocanyl and oxazocanyl.

A substituent on a saturated, partially saturated or unsaturated heterocycle can be attached at any substitutable position.

It is also understood that the expression "is absent" (referring to a given substituent) means that the atom to which the substituent would be bound is a heteroatom, preferably nitrogen, which is comprised in a heteroaryl ring, such as a pyridine or a pyrimidine ring.

As used herein, the term "C₁₋₆ alkanediyl" as group or part of a group defines bivalent straight or branched chained saturated hydrocarbon radicals having from 1 to 4 carbon atoms. C₁₋₆ alkanediyl group, is preferably a C₁₋₄ alkanediyl group, a C₁₋₃ alkanediyl or more preferably a C₁₋₂ alkanediyl. Examples include, but are not limited to methanediyl, ethanediyl, propanediyl, butanenediyl, pentanediyl and hexanediyl. Preferred are methanediyl, ethanediyl and propanediyl.

As used herein, the term "C₂₋₄ alkenediyl" as group or part of a group defines bivalent straight or branched chained saturated hydrocarbon radicals having from 2 to 4 carbon atoms and having at least one double bond, preferably one double bond, such as, but not limited to ethenediyl, propenediyl and butenediyl.

As used herein, the terms "3-7 membered saturated ring", "3-8 membered saturated ring" and "4-8 membered saturated ring" mean saturated cyclic hydrocarbon (cycloalkyl) with 3 or 4, 5, 6 or 7 or 8 carbon atoms and is generic to cyclopropyl, cyclobutyl, cyclopentyl, cyclohexyl, cycloheptyl or cyclooctyl. Said saturated ring optionally contains one or more heteroatoms (also referred to as heterocyclyl), such that at least one carbon atom is replaced by a heteroatom selected from N, O and S, in particular from N and O. Examples include, but are not limited to oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl or oxazocanyl. Preferred are saturated cyclic hydrocarbons with 3 or 4 carbon atoms and 1 oxygen or 1 nitrogen atom. Examples include oxetanyl, tetrahydrofuranyl, tetrahydro-2H-pyranyl, piperidinyl or pyrrolidinyl.

As used herein, the term "4-10 membered unsaturated ring" means a non aromatic mono or bicyclic ring. In a preferred embodiment, the invention relates to 5-10 membered unsaturated rings. In another preferred embodiment, the invention relates to 5-7 membered unsaturated rings. Suitable examples include but are not limited to cyclopentenyl, cyclohexenyl, cyclohexadienyl or cycloheptenyl.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

Indoline-2-one is an indoline carrying an oxo group at position 2.

Azaindoline-2-one is an azaindoline carring an oxo group at position 2. Suitable example is a pyrrolo[2,3-b]pyridine-2-one.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, the term "C₃₋₆ cycloalkyl" means saturated cyclic hydrocarbon (cycloalkyl) with 3 or 4, 5 or 6 carbon atoms and is generic to cyclopropyl, cyclobutyl, cyclopentyl or cyclohexyl.

Included in the instant invention is the free base of compounds of formula (I), (Ia), (Ib) and (Ic), as well as the pharmaceutically acceptable salts and stereoisomers thereof. Some of the specific compounds exemplified herein are the protonated salts of amine compounds. Compounds of formula (I), (Ia), (Ib) or (Ic) containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of formula (I), (Ia), (Ib) or (Ic).The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reacting a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I), (Ia), (Ib) or (Ic) and 1, 2 or 3 equivalent of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the present invention find use in a variety of applications for human and animal health. The compounds of the present invention are inhibitors of hepatitis B virus (HBV). The compounds of the present invention are inhibitors of hepatitis B virus (HBV) useful for the treatment and/or prevention of HBV infection. In particular the compounds of the present invention are inhibitors of hepatitis B virus (HBV) core (HBc) protein useful for the treatment and/or prevention of a HBV infection.

The compounds, compositions and methods provided herein are particularly deemed useful for treating, ameliorating or preventing HBV infection and related conditions, including chronic hepatitis B, HBV/HDV co-infection, HBV/HCV co-infection, HBV/HIV co-infection.

In the present invention, the expression "HBV infection" comprises any and all conditions deriving from infection with HBV, including but not limited to hepatitis B, preferably chronic hepatitis B, HBV/HDV co-infection, HBV/HCV coinfection, HBV/HIV coinfection.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulstion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of formula (I), (Ia), (Ib) or (Ic) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of formula (I), (Ia), (Ib) or (Ic) are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

When a compound according to this invention is administered into a human subject, the daily dosage will normally be determined by the prescribing physician with the dosage generally varying according to the age, weight, sex and response of the individual patient, as well as the severity of the patient's symptoms.

In one exemplary application, a suitable amount of compound is administered to a mammal undergoing anti HBV treatment. Administration generally occurs in an amount between about: 0.01 mg/kg of body weight to about 60 mg/kg of body weight per day, preferably of between 0,5 mg/kg of body weight to about 40 mg/kg of body weight per day.

The instant compounds are also useful in combination with known therapeutic agents for simultaneous, separate or sequential administration.

In an embodiment, the compounds of the present invention may be used in combination with at least one or more additional therapeutic agents, in particular anti-HBV agents.

The indication that compounds of the invention are for use in the treatment and/or prevention of a HBV infection indicates that the compounds are efficacious for treating, eradicating, reducing, slowing or inhibiting an HBV infection.

The term "anti-HBV agent", or more simply "HBV antiviral(s)" also includes compounds that are therapeutic nucleic acids, antibodies or proteins either in their natural form or chemically modified and/or stabilized. The term therapeutic nucleic acid includes but is not limited to nucleotides and nucleosides, oligonucleotides, polynucleotides, of which non limiting examples are antisense oligonucleotides, miRNA, siRNA, shRNA, therapeutic vectors and DNA/RNA editing components.

The term anti-HBV agent also includes compounds capable of treating HBV infection via immunomodulation, i.e. immunomodulators or immunomodulating compounds. Examples of immunomodulators are interferon-a (IFN-α), pegylated interferon-α or stimulants of the innate immune system such as Toll-like receptor 7 and/or 8 agonists and therapeutic or prophylactic vaccines. One embodiment of the present invention relates to combinations of a compound of formula (I) or (Ia) or any subgroup thereof, as specified herein, with an immunomodulating compound, more specifically a Toll-like receptor 7 and/or 8 agonist.

The additional HBV antiviral(s) can be selected for example, from therapeutic vaccines; RNA interference therapeutic/antisense oligonucleotides (e.g. siRNA, ddRNA, shRNA); immunomodulators (such as TLR agonists (e.g. TLR7, TLR8 or TLR9 agonists); STING agonists; RIG-I modulators; NKT modulators; IL agonists; Interleukin or other immune active proteins, therapeutic and prophylactic vaccines and immune checkpoint modulators; HBV entry inhibitors; cccDNA modulators (such as for example direct cccDNA inhibitors, inhibtors of cccDNA formation or maintenance, cccDNA epigenetic modifiers, inhibitors of cccDNA transcription); inhibitors of HBV protein espression; agents targeting HBV RNA; capsid assembly inhibitors/modulators; core or X protein targeting agents; nucleotide analogues; nucleoside analogues; interferons or modified interferons; HBV antivirals of distinct or unknown mechanism; cyclophilin inhibitors; sAg release inhibitors; HBV polymerase inhibitors; dinucleotides; SMAC inhibitors; HDV targeting agents; viral maturation inhibitors; reverse transcriptase inhibitors and HBV RNA destabilizers and other small-molecule inhibitors of HBV protein expression.

In particular, the combination of previously known anti-HBV agents, such as interferon-a (IFN-α), pegylated interferon-α, 3TC, tenofovir, lamivudine, entecavir, telbivudine, and adefovir or a combination thereof, and a compound of formula (I) or (Ia) or any subgroup thereof can be used as a medicine in a combination therapy. Additional examples of further therapeutic agents that may be combined with the compounds of the present invention include: Zidovudine, Didanosine, Zalcitabine, Stavudine, Abacavir, ddA Emtricitabine, Apricitabine, Atevirapine, ribavirin, acyclovir, valacyclovir, famciclovir, ganciclovir, valganciclovir, cidofovir, Efavirenz, Nevirapine, Delavirdine and Etravirine.

Particular examples of such HBV antiviral(s) include, but are not limited to:
- RNA interference (RNAi) therapeutics: TKM-HBV (also known as ARB-1467), ARB-1740, ARC-520, ARC-521, BB-HB-331, REP-2139, ALN-HBV, ALN-PDL, LUNAR-HBV, GS3228836, and GS3389404;
- HBV entry inhibitors: Myrcludex B, IVIG-Tonrol, GC-1102;
- HBV capsid inhibitor/modulators, core or X protein targeting agents, direct cccDNA inhibitors, inhibitors of cccDNA formation or maintenance, or cccDNA epigenetic modifiers: BAY 41-4109, NVR 3-778, GLS-4, NZ-4 (also known as W28F), Y101, ARB-423, ARB-199, ARB-596, AB-506, JNJ-56136379, ASMB-101 (also known as AB-V102), ASMB-103, CHR-101, CC-31326, AT-130, RO7049389.
- HBV polymerase inhibitors: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir (in particular tenofovir disoproxil fumarate (Viread), tenofovir alafenamide fumarate (TAF)), tenofovir disoproxil orotate (also known as DA-2802), tenofovir disopropxil aspartate (also known as CKD-390), AGX-1009, and CMX157);
- HBV RNA destabilizers and other small-molecule inhibitors of HBV protein expression: RG7834, AB-452;
- cyclophilin inhibitors: OCB-030 (also known as NVP-018), SCY-635, SCY-575, and CPI-431-32;
- dinucleotides: SB9200;
- compounds of distinct or unknown mechanism, such as but not limited to AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), and similar analogs; REP-9AC (also known as REP-2055), REP-9AC' (also known as REP-2139), REP-2165 and HBV-0259;
- TLR agonists (TLR7, 8 and/or 9): RG7795 (also known as RO-6864018), GS-9620, SM360320 (9-benzyl-8-hydroxy-2-(2-methoxy-ethoxy)adenine) and AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate); ARB- 1598;
- RIG-I modulators: SB-9200;
- SMAC inhibitor: Birinapant
- Immune Check Point inhibitors: BMS-936558 (Opdivo (nivolumab)), KEYTRUDA® (pembrolizumab);
- therapeutic vaccines: HBsAG-HBIG, HB-Vac, ABX203, NASVAC, GS-4774, GX- 110 (also known as HB-110E), CVI-HBV-002, RG7944 (also known as INO-1800), TG-1050, FP-02 (Hepsyn-B), AIC649, VGX-6200, KW-2, TomegaVax-HBV, ISA-204, NU-500, INX-102-00557 HBV MVA, PepTcell;
- IL agonists and immune acting proteins: INO-9112; recombinant IL12;
- interferons: interferon alpha (IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta- la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA, interferon alpha biobetter; in particular, peginterferon alpha-2a, peginterferon alpha-2b, glycosylated interferon alpha-2b, peginterferon beta-la, and peginterferon lambda-1; more in particular, peginterferon alpha-2a;
- HDV targeting agent: Lonafamib.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

In some embodiments, pulsed administration is more effetctive than continuous treatment because total pulsed doses are often lower than would be expected from continuous administration of the same composition. Each pulse dose can be reduced and the total amount of drug administered over the course of treatment is minimized. Individual pulses can be delivered to the patient continuously over a period of several hours, such as about 2, 4, 6, 8, 10, 12, 14 or 16 hours, or several days, such as 2, 3, 4, 5, 6 or 7 days.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The present invention will be described by means of the following non-limiting examples and biological data are presented.

### MATERIALS AND METHODS

### Chemistry

### General

Unless otherwise indicated, commercially available reagents and solvents (HPLC grade) were used without further purification.

Specifically, the following abbreviations may have been used in the descriptions of the experimental methods:
NMR: Nuclear Magnetic Resonance; ¹H: proton; MHz: Megahertz; Hz: Hertz; HPLC: High Performance Liquid Chromatography; LC-MS: Liquid Chromatography Mass Chromatography Spectrum; s: second(s); min: minute(s); h: hour(s); mg: milligram(s); g: gram(s); Ml: microliter(s); mL: millilitre(s); mmol: millimole(s); nm: nanometer(s) µM: micromolar; M: molarity or molar concentration; Rt: retention time in minutes; MW: microwave; Boc: *tert*-butyloxycarbonyl protecting group; DMF: dimethylformamide; DMSO: dimethylsulfoxide; MeOH: methanol; EtOH: ethanol; EtOAc: ethyl acetate; DCM: dichloromethane; PE: Petroleum Ether; TFA: trifluoroacetic acid; (g): gas; eq.: equivalent(s);; RT: room temperature; DIPEA: *N,N-*diisopropylethylamine; DIAD: Diisopropyl azodicarboxylate; sat.aq.: saturated aqueous solution; TEA: triethylamine; THF: tetrahydrofuran; IPA: isopropylamine.; *p*TSA: para toluene sulfonic acid; TBDMS: *tert*-butyldimethylsilyl; LiHMDS: Lithium bis(trimehtylsilyl)amide; TBTU: 2-(1H-Benzotriazole-1-yl)-1,1,3,3-tetramethylaminium tetrafluoroborate;
Except where indicated otherwise, all temperatures are expressed in °C (degrees centigrade) or K (Kelvin).

The ¹H-NMR spectra were acquired with an Avance II 300 MHz Bruker spectrometer. The chemical shifts are expressed in parts per million (ppm, δ units). The coupling constants are expressed in Hertz (Hz) and the splitting patterns are described as s (singlet), bs (broad signal), d (doublet), t (triplet), q (quartet), quint (quintet), m (multiplet).

The LC-MS analyses were performed by means of an UPLC Acquity Waters System equipped with the SQD spectrometer, single quadrupole mass detector, and a TUV detector, using column 1: ACQUITY UPLC BEH SHIELD, RP₁₈ (2.1x50mm, id=1.7 µm); column2: ACQUITY UPLC HSS T3, RP₁₈ (2.1x50mm, id=1.8 µm) and column3: ACQUITY UPLC BEH SHIELD, RP₁₈ (2.1x100mm, id=1.7 µm). Column temperature 40°C. Sample temperature 25°C. Phase A was composed by water (HiPerSolv Chromanorm Water VWR for HPLC-MS) + 0,05% Trifluoroacetic Acid; Phase B by CH₃CN (HiPerSolv Chromanorm Acetonitrile SuperGradient VWR, suitable for UPLC/UHPLC instruments) + 0,05% Trifluoroacetic Acid; flow rate: 0,5 mL/min; UV detection (DIODE array) 200 nm; ESI+ and ESI- detection in the 100-1000 m/z range.
Method 1: column 1, run time: 3 minutes, run gradient: 5%B to 100%B in 2.80 min + 100%B for 0.2 min, equilibration time: 0,8 min, ionization mode: ESI⁺.
Method 2: column 2, run time: 4 minutes, run gradient: 0%B to 45%B in 3.5 min + 45%B to 100%B in 0.05 min +100%B for 0.45 min, equilibration time: 0,8 min, ionization mode: ESI⁺.
Method 3: column 3, run time: 6 minutes, run gradient: 5%B to 100%B in 5 min + 100%B for 1 min, equilibration time: 2 min.
Method 4: column 3, run time: 6 minutes, run gradient: 5%B to 50%B in 5 min + 50%B to 100%B in 0.2 min 100%B for 0.8 min, equilibration time: 2 min, ionization mode: ESI⁺.
Method 5: column 1, run time: 3 minutes, run gradient: 5%B to 100%B in 2.80 min + 100%B for 0.2 min, equilibration time: 0,8 min, ionization mode: ESI⁺.
Method 6: column 2, run time: 4 minutes. run gradient: 0%B to 45%B in 3.5 min + 45%B to 100%B in 0.05 min +100%B for 0.45 min. Equilibration time: 0,8 min, ionization mode: ESI⁺.
Method 7: column 3, run time: 6 minutes, run gradient: 5%B to 100%B in 5 min + 100%B for 1 min, equilibration time: 2 min, ionization mode: ESI⁺.
Method 8: column 3, run time: 6 minutes, run gradient: 5%B to 50%B in 5 min + 50%B to 100%B in 0.2 min 100%B for 0.8 min, Equilibration time: 2 min, ionization mode: ESI⁺.
Method 9: column 1. run time: 4 minutes, column 1, run time: 4 minutes, run gradient:5%B to 100%B in 3.00 min + 100%B for 1 min, equilibration time: 0,8 min, ionization mode: ESI⁺.
Method 10: column 1. run time: 4 minutes, run gradient: 5%B to 100%B in 3.00 min + 100%B for 1 min, equilibration time: 0,8 min, Ionization Mode: ESI⁻.
Method 11: column 1, run time: 3 minutes, run gradient: 40%B to 100%B in 2.80 min + 100%B for 0.2 min, equilibration time: 0,8 min. Ionization Mode: ESI⁺.
Method 12: column 3, run time: 6 minutes, run gradient: 25%B to 70%B in 5 min + 100%B for 1 min, equilibration time: 2 min, Flow: 0,5 mL/min, ionization mode: ESI⁺.

### Synthesis

According to a further aspect of the invention there is provided a process for the preparation of compounds of formula (I), (Ia), (Ib), (Ic) or salts thereof. The following schemes are examples of synthetic schemes that may be used to synthesise the compounds of the invention. In the following schemes reactive groups can be protected with protecting groups and deprotected according to well established techniques. In the following schemes unless otherwise indicated A, B, X, Z, R₁, R₁', R₂, R₃, R₄, Ra, Rb, Rc, Rd, Re, Rf, Rg are as defined herein above in formula (I), (Ia), (Ib) or (Ic).

It will be understood by those skilled in the art that certain compounds of the invention can be converted into other compounds of the invention according to standard chemical methods. Compounds of the invention may be prepared according to the general routes indicated in the following Scheme 1: 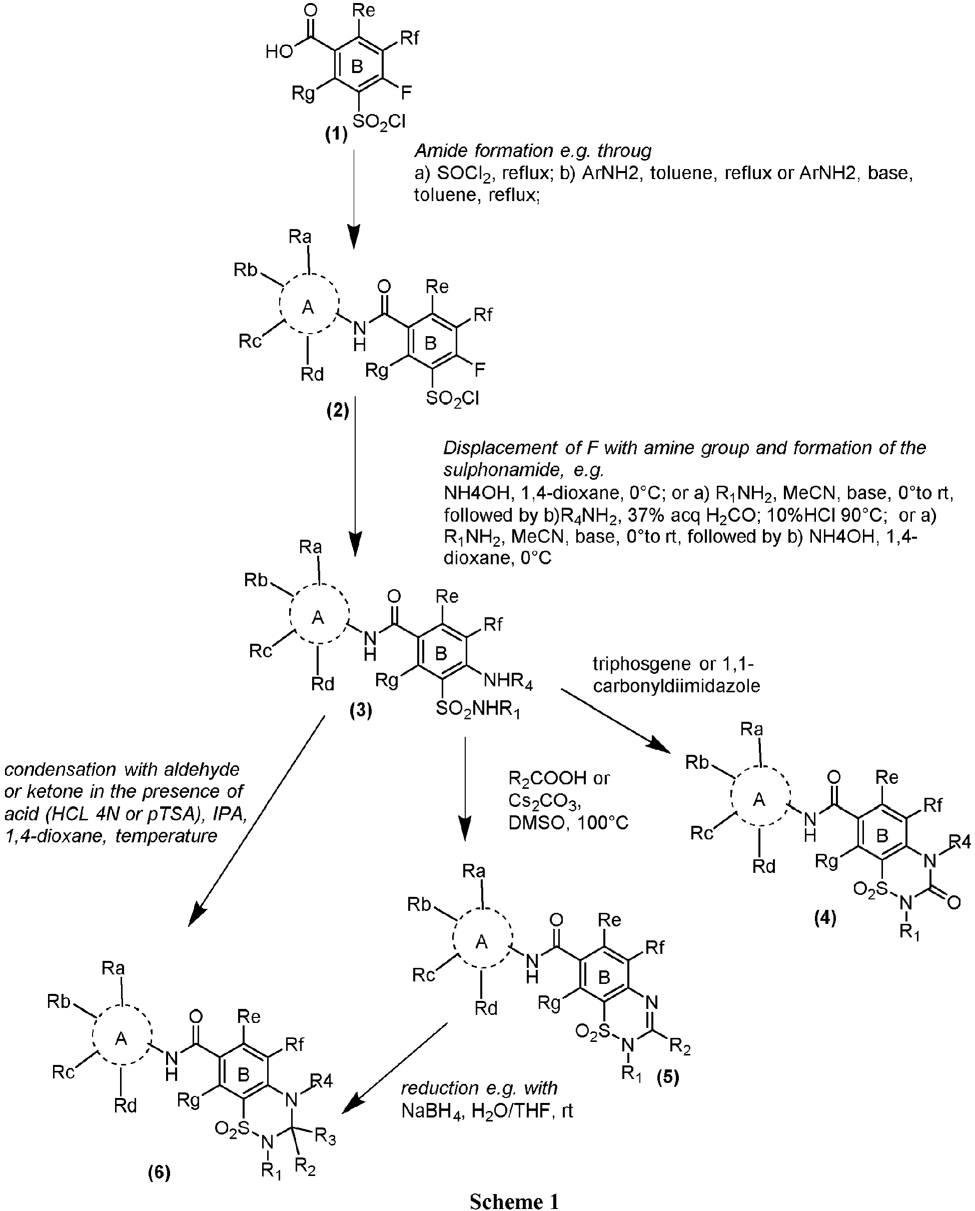 According to Scheme 1, compounds of formula (2) can be prepared by reacting a compound of formula (1) with an aromatic or heteroaromatic amine. The reaction can be generally carried out by reacting a carboxylic acid of formula (1) with thionyl chloride at reflux to obtain the corresponding acyl chloride, and further reacting the acyl chloride with the selected amine (ArNH₂) in toluene at reflux. A base such as triethyl amine may also be added. Compounds of formula (3), wherein R₁ and R₄ are both hydrogen atoms, may be obtained by reacting a compound of formula (2) with an excess of NH₄OH in the presence of a base at 0°C. Compounds of formula (3), wherein R₁ is not hydrogen and R₄ is hydrogen, may be obtained by stepwise reaction of a compound of formula (2) with R₁NH₂ in acetonitrile in the presence of a base and then with NH₄OH in dioxane. Alternatively, compounds of formula (3) wherein both R₁ and R₄ are not hydrogen, may be obtained by using in the first step R₁NH₂ and in the second step R₄NH₂ respectively.

The compound of formula (3) may be converted into a compound of formula (4) by reaction with triphosgene in dichloromethane at 0°C, eventually in the presence of a base, or by reaction with 1,1-carbonyldiimidazole and trimethylamine in DMF at about 150°C.

The compound of formula (3) may also be converted into a compound of formula (5) by reaction with a carboxylic acid of formula R₂COOH at 80-100 °C, or, when Re=F or Rf=F and R2=H, or when R₂=NH₂ or NH(CO)CH₃, by reaction with cesium carbonate in DMSO at 100°C under microwave irradiation.

Compounds of formula (6), wherein R₃ is hydrogen, may be obtained from a compound of formula (5) by reduction with a reducing agent such as sodium borohydride in water/THF at room temperature.

Alternatively, a compound of formula (6) may be obtained from a compound of formula (3) by reaction with an aldehyde, a ketone, or the relative acetal or ketal, in the presence of an acid in the appropriate solvent. Tipically the reaction is carried out at a temperature comprised between 65 °C and 140 °C, by using 4N HCl in isopropyl alcohol, 4N HCl in dioxane or para-toluenesulfonic acid in dioxane in the presence of magnesium sulfate. In some case microwave irradiation can be used to accelerate the reaction.

Protecting groups such as TBDMS, TES, SEM, BOC may be present during any of the reactions steps, and can be removed by standard chemistry.

In some cases the reactions steps indicated in Scheme 1 may be slightly modified either in the order or in the starting materials employed, as for example in the synthesis of compounds **E88** and **E89** (Scheme 2):

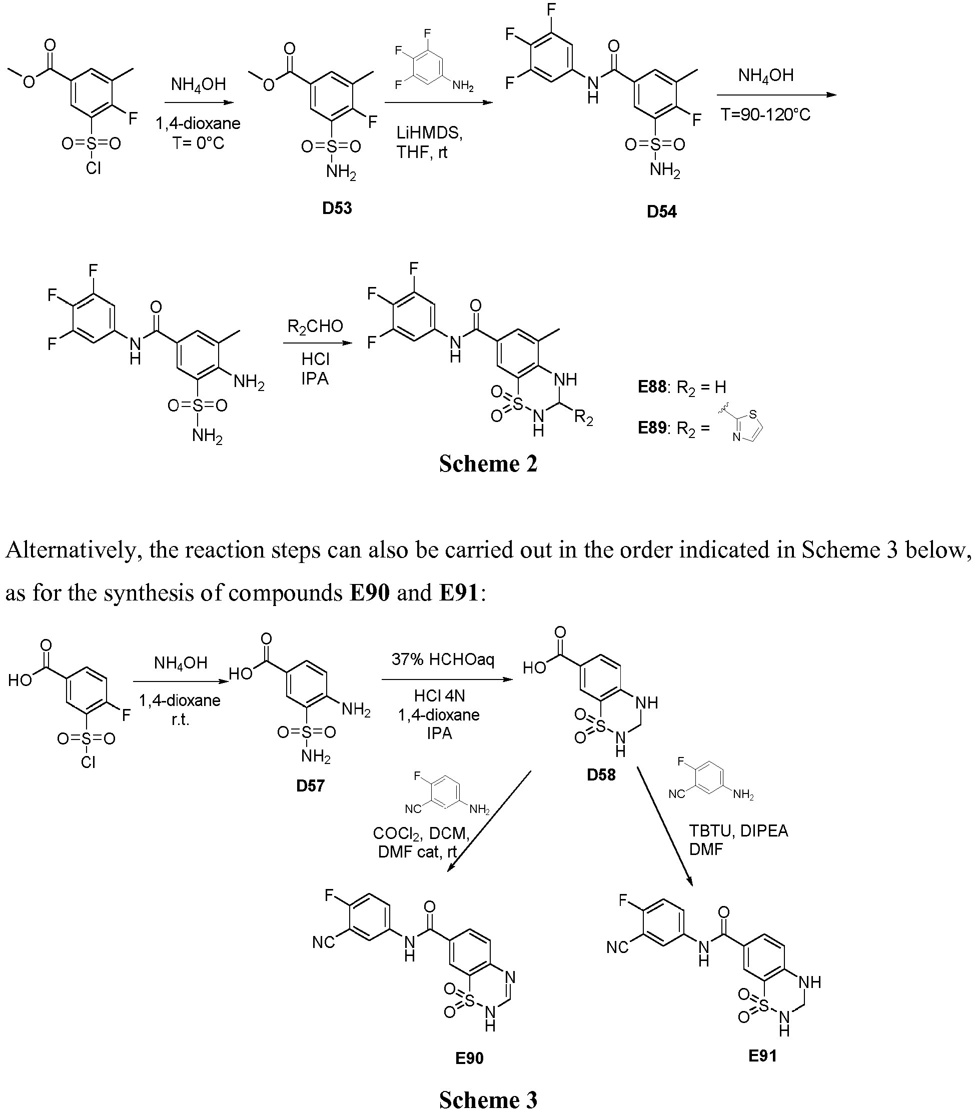

Compound **E92** may be prepared according to Scheme 4:

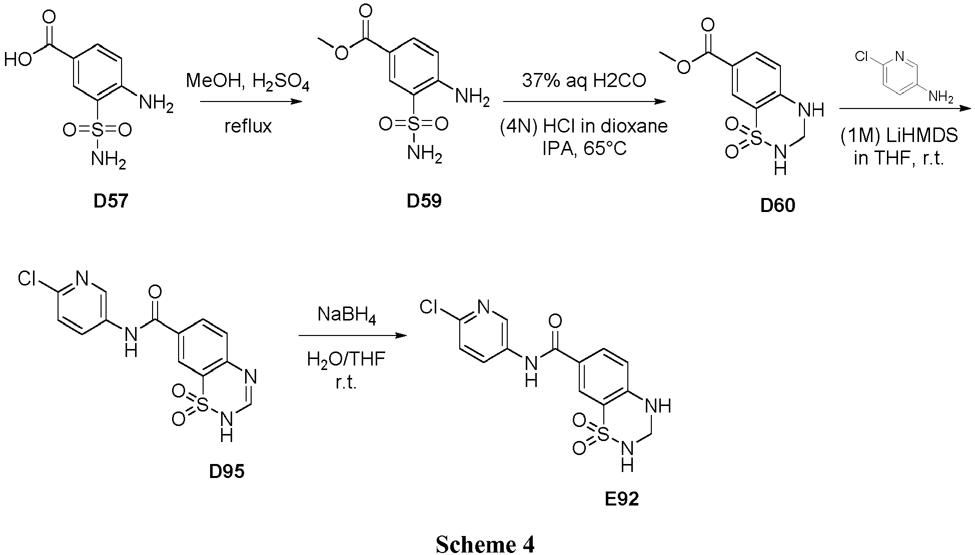

In a further alternative preparation, compound **E93** may be prepared according to the following Scheme 5:

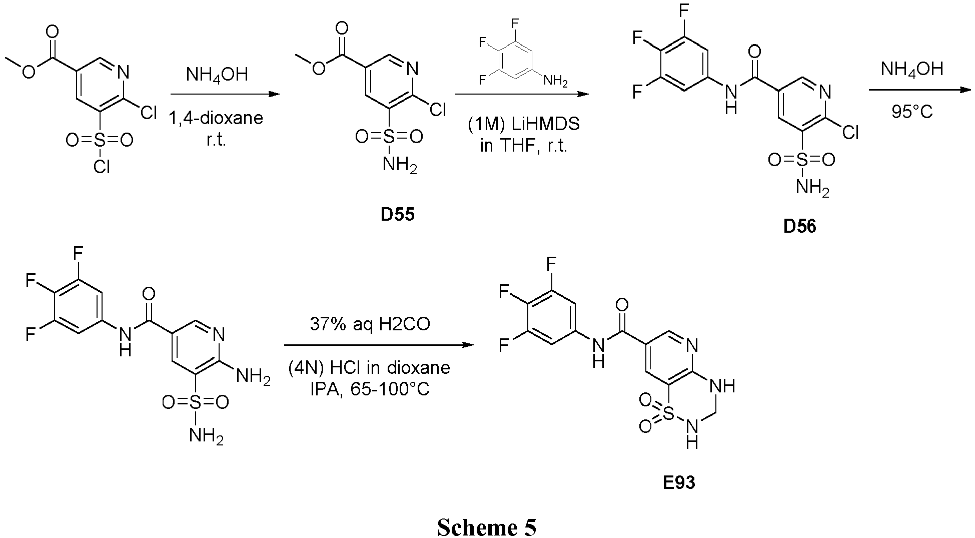

Compounds **E94-E109** and **E111-E118** can be prepared according to the routes indicated in Scheme 6:

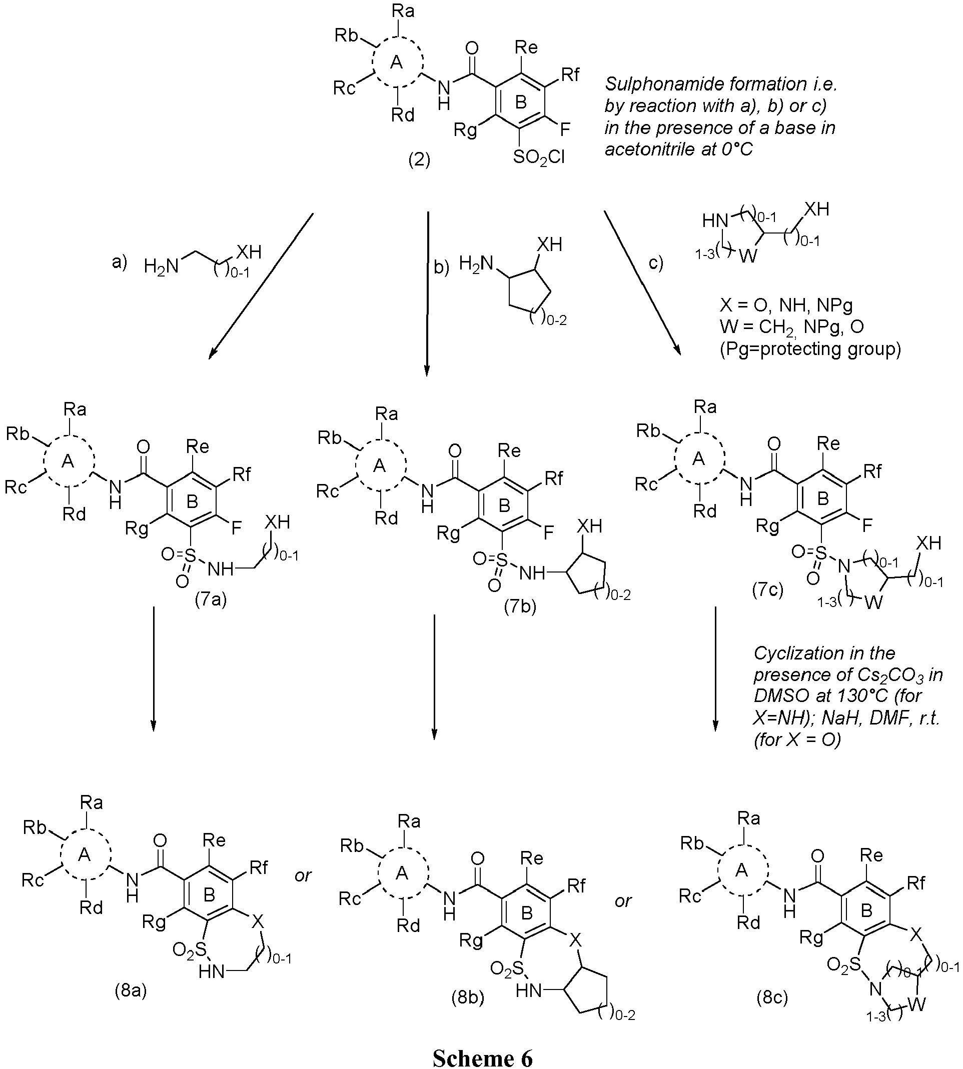

In Scheme 6, the synthetic strategy is to use a bifunctional amine in the formation of the sulphonamide, thus obtaining compounds of general formula (7a), (7b) or (7c), wherein X is NH, O or a protected nitrogen (like, for example, NBoc). The subsequent cyclization is carried out in the presence of the suitable base such as cesium carbonate or sodium hydride. A deprotection step is required when the amino functionality is protected.

Compound **E110** may be prepared according to Scheme 7:

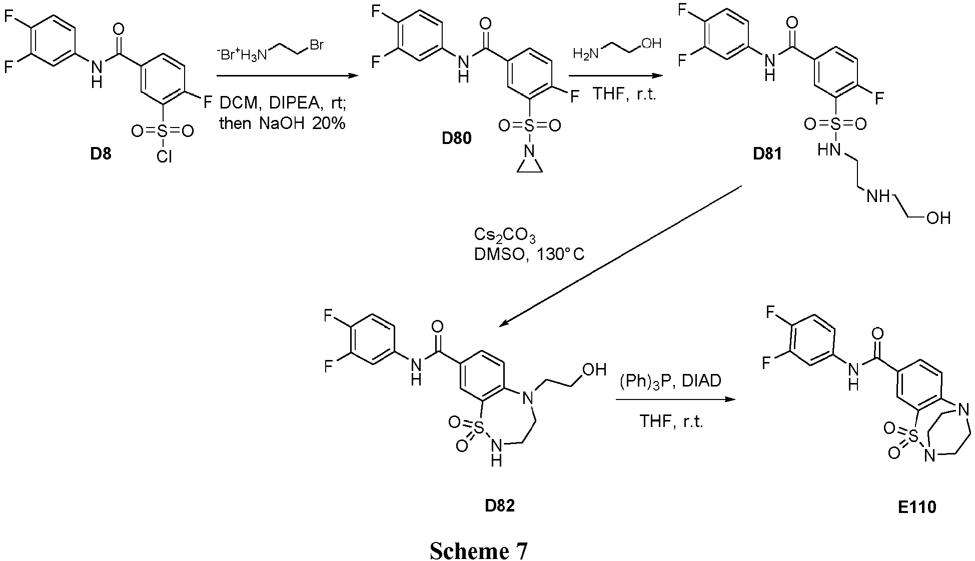

Compound **E138** can be prepared as described in the following Scheme 8, from the commercially available carboxylic acid indicated below:

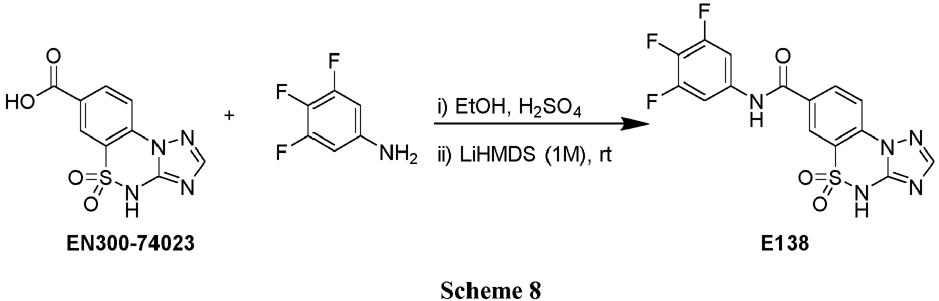

In some circumstances the desired inhibitors can be converted into other compounds of the invention by simple functional group manipulations known to those skilled in the art. For instance, protected amines can be deprotected and functionalised with reactions such as couplig to carboxylic acid derivatives or by reductive amination reactions.

The following Examples illustrate the preparation of certain compounds of formula (I), (Ia), (Ib) or (Ic) and salts thereof. The Descriptions 1 to 130 illustrate the preparation of intermediates used to make compounds of formula (I), (Ia), (Ib) or (Ic) and salts thereof. In the procedures that follow, for the starting material reference is typically provided to a procedure in the Descriptions or in the Examples. This is provided merely for assistance to the skilled chemist. The starting material may not necessarily have been prepared from the batch of the Description or the Example referred to.

### Examples

### Description 1: 3-(chlorosulfonyl)-4-fluorobenzoyl chloride (D1)

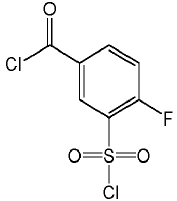

3-Chlorosulfonyl-4-fluoro-benzoic acid (Fluorochem, cat n° 037319) (14.2g, 59.51mmol) was added to thionyl chloride (80.39mL, 1106.9mmol) in a single portion. The resulting yellowish solution was heated to reflux for 4hrs, giving a slurry. Solvent was removed *in vacuo* by coevaporation with toluene, giving the title compound **D1** (15.4g, 59.91mmol) as a brown oil.

### Description 2: 2-chloro-5-(chlorosulfonyl)-4-fluorobenzoyl chloride (D2)

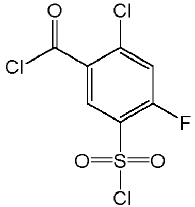

Similarly prepared according to procedure described for the preparation of compound **D1**, starting from 2-Chloro-5-chlorosulfonyl-4-fluoro-benzoic acid (Enamine, cat. n° EN300-01843).

### Description 3: 2-bromo-5-(chlorosulfonyl)-4-fluorobenzoyl chloride (D3)

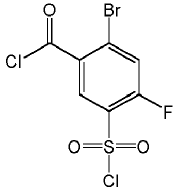

Similarly prepared according to procedure described for the preparation of **D1**, starting from 2-Bromo-5-(chlorosulfonyl)-4-fluorobenzoic acid (Enamine, cat. n° EN300-52736).

### Description 4: 5-(chlorosulfonyl)-2,4-difluorobenzoyl chloride (D4)

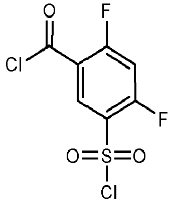

Similarly prepared according to procedure described for the preparation of **D1**, starting from 5-(Chlorosulfonyl)-2,4-difluorobenzoic acid (Enamine, cat. n° EN300-59276).

### Description 5: 3-(chlorosulfonyl)-4,5-difluorobenzoyl chloride (D5)

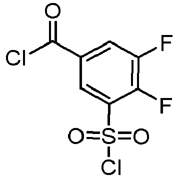

Similarly prepared according to the procedure described for the preparation of **D1**, starting from 3-(Chlorosulfonyl)-4,5-difluorobenzoic acid (Enamine, cat. n° EN300-107773).

### Description 6: 5-(chlorosulfonyl)-4-fluoro-2-methylbenzoyl chloride (D6):

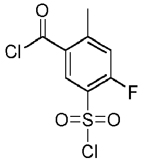

Similarly prepared according to procedure described for the preparation of **D1**, starting from 5-(chlorosulfonyl)-4-fluoro-2-methylbenzoic acid (Enamine, cat. n° EN300-114063).

### Description 7: 2-fluoro-5-((3,4,5-trifluorophenyl)carbamoyl)benzenesulfonyl chloride (D7)

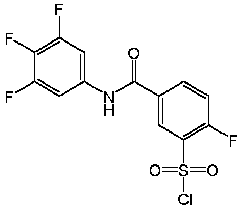

**D1** (15.4g, 59.91mmol) was dissolved in toluene (140mL), heated to reflux and then a solution of 3,4,5-trifluoroaniline (8.81g, 59.91mmol) in toluene (50 mL) was added dropwise over 10 min. A suspension was formed and refluxed for 1h. The reaction was cooled to room tempearture, filtered and the cake obtained washed with a small amount of toluene, giving the title compound **D7** (14.5g, 39.43mmol) as off-white solid. ¹H NMR (300 MHz, DMSO-*d₆*) δ ppm 7.32 (t, J 9.03 Hz, 1 H) 7.68 - 7.81 (m, 2 H) 7.90 - 8.03 (m, 1 H) 8.30 (dd, J=6.83, 2.43 Hz, 1 H) 10.71 (s, 1 H). Method 1: Rt=2.43min, m/z=368.32 (M+H)⁺.

### Description 8: 5-((3,4-difluorophenyl)carbamoyl)-2-fluorobenzenesulfonyl chloride (D8)

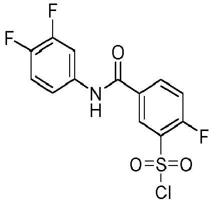

To a solution of **D1** (1.8 g, 7.0 mmol) in dry toluene (13.5 mL) at 90°C, triethylamine (1 mL, 7.175 mmol) and a solution of 3,4-difluoroaniline (0.904 g, 7.0 mmol) in dry toluene (3.5 mL) were added dropwise. Reaction mixture was stirred at reflux for 30 min, then was allowed to cool at room temperature and diluted with dichloromethane. Organic layer was washed with brine, dried over Na₂SO₄, filtered and evaporated under reduced pressure to give a beige solid (2.39 g) as crude product was suspended in DCM (5 mL) and sonicated. The resulting solid was filtered, washed with DCM and dried at vacuum pump to afford the title compound **D8** as white powder (1.73 g). ¹H NMR (300 MHz, DMSO-*d₆*) δ ppm 7.26 - 7.48 (m, 3 H) 7.52 - 7.61 (m, 1 H) 7.91 - 7.99 (m, 2 H) 8.30 (dd, *J*=6.88, 2.38 Hz, 1 H) 10,59 (s, 1 H). Method 1: Rt=2.43min, m/z=350.02 (M+H)⁺.

### Description 9: 2,4-difluoro-5-[(3,4,5-triuorophenyl)carbamoyl]benzene-1-sulfonyl chloride (D9)

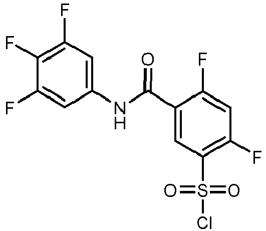

To a stirred solution of **D4** (1.02 g, 3.70 mmol) in dry toluene (6 mL) at 70°C, a solution of dry DIPEA (0.650 mL, 3.70 mmol) and 3,4,5-Trifluoroaniline (0,545 g, 3.70 mmol) in dry toluene (2 mL) were added dropwise. Temperature was increased to 90°C. After 30 mixture was allowed to cool at room temperature, diluted with dichloromethane and the organic layer was washed with brine, then was dried over Na₂SO₄, filtered and evaporated under reduced pressure to give the title compound **D9** as beige solid (1.52 g). Method 1: Rt=2.37min, m/z=386.12 (M+H)⁺.

### Description 10: 2,3-difluoro-5-[(3,4,5-trifluorophenyl)carbamoyl]benzene-1-sulfonyl chloride (D10)

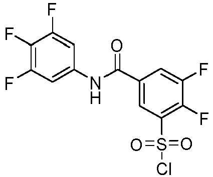

Similarly prepared according to the procedure described for the preparation of **D8**, starting from **D5**. Method 1; Rt=2.48min, m/z=385.89 (M+H)⁺.

### Description 11: 5-[(3-chloro-4-fluorophenyl)carbamoyl]-2-fluorobenzene-1-sulfonyl chloride (D11)

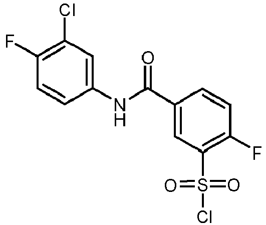

Similarly prepared according to procedure described for the preparation of **D8** starting from 3-(chlorosulfonyl)-4-fluorobenzoyl chloride **D1** and using 3-Chloro-4-fluoroaniline instead of 3,4-Difluoroaniline, to give the title compound **D11**. Method 1: Rt=2.43min, m/z=366.01 (M+H)⁺.

### Description 12: 2-fluoro-5-((4-fluoro-3-(trifluoromethyl)phenyl)carbamoyl)benzenesulfonyl chloride (D12)

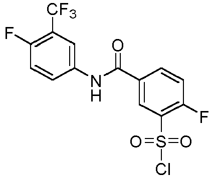

Similarly prepared according to procedure described for the preparation of **D7**, using as starting material **D1** (500mg, 1.94mmol) and 4-fluoro-3-(trifluoromethyl)aniline (0.25mL, 1.94mmol) instead of 3,4,5-trifluoroaniline. The reaction mixture was concentrated in vacuo to give to give the title compound **D12** (805mg) as beige solid, that was used in the next synthetic step directly.

### Description 13: 5-((3-cyano-4-fluorophenyl)carbamoyl)-2-fluorobenzenesulfonyl chloride (D13)

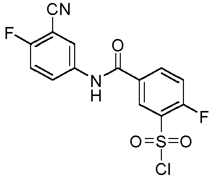

Similarly prepared according to procedure described for the preparation of **D7**, using as starting materials **D1** and 5-Amino-2-fluorobenzonitrile instead of 3,4,5-trifluoroaniline.

### Description 14: 5-((3-(difluoromethyl)-4-fluorophenyl)carbamoyl)-2-fluorobenzenesulfonyl chloride (D14)

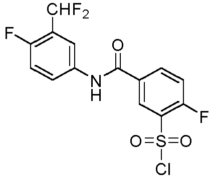

Similarly prepared according to procedure described for the preparation of **D7**, using as starting materials **D1** and 3-(Difluoromethyl)-4-fluoroaniline instead of 3,4,5-trifluoroaniline.

### Description 15: 5-((6-chloropyridin-3-yl)carbamoyl)-2-fluorobenzenesulfonyl chloride (D15)

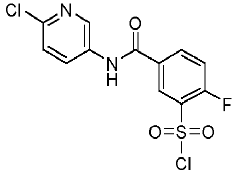

**D1** (307mg, 1.19mmol) was dissolved in Toluene (2.7mL) and heated to 90°C. A suspension of 6-chloropyridin-3-amine (153.5mg, 1.19mmol) in Toluene (1.2mL) was slowly added and the reaction mixture was refluxed at 110°C for 20min. DIPEA (0.31mL, 1.79mmol) was then added, and the reaction was refluxed at 110°C for another 1h50min. The mixture was concentrated in vacuo to give the crude title compound **D15** (702mg) as brown oil, that was used in the next synthetic step directly. Method 1; Rt=2.17min, m/z=349.00 (M+H)⁺.

### Description 16: 2-fluoro-4-methyl-5-((3,4,5-trifluorophenyl)carbamoyl)benzenesulfonyl chloride (D16)

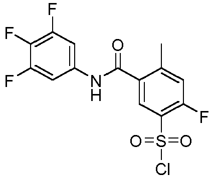

Similarly prepared according to procedure described for the preparation of **D7**, starting from 5-(chlorosulfonyl)-4-fluoro-2-methylbenzoyl chloride **D6**. Method 1; Rt=2.49min, m/z=382.11 (M+H)⁺.

### Description 17: 2-fluoro-5-((4-fluoro-3-methylphenyl)carbamoyl)benzenesulfonyl chloride (D17)

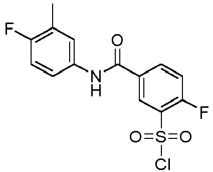

Similarly prepared according to procedure described for the preparation of **D7**, using as starting materials **D1** and 4-Fluoro-3-methylaniline instead of 3,4,5-trifluoroaniline. Method 1; Rt=2.38min, m/z=346.17 (M+H)⁺.

### Description 18: 5-((3,5-difluoro-4-methylphenyl)carbamoyl)-2-fluorobenzenesulfonyl chloride (D18)

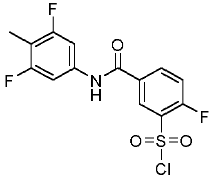

Similarly prepared according to procedure described for the preparation of **D7**, using as starting materials **D1** and 3,5-Difluoro-4-methylaniline instead of 3,4,5-trifluoroaniline. Method 1; Rt=2.54min, m/z=364.20 (M+H)⁺.

### Description 19: 2-fluoro-5-((2,3,4-trifluorophenyl)carbamoyl)benzenesulfonyl chloride (D19)

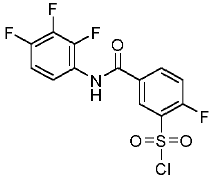

Similarly prepared according to procedure described for the preparation of **D7**, using as starting materials **D1** and 2,3,4-trifluoroaniline instead of 3,4,5-trifluoroaniline. Method 1; Rt=2.29min, m/z=368.19 (M+H)⁺.

### Description 20: 2-fluoro-5-((2,4,5-trifluorophenyl)carbamoyl)benzenesulfonyl chloride (D20)

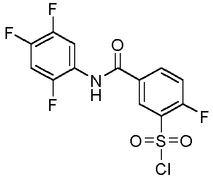

Similarly prepared according to procedure described for the preparation of **D7**, using as starting materials **D1** and 2,4,5-trifluoroaniline instead of 3,4,5-trifluoroaniline. Method 1; Rt=2.32min, m/z=368.39 (M+H)⁺.

### Description 21: 2-fluoro-5-((2,4,5-trifluorophenyl)carbamoyl)benzenesulfonyl chloride (D21)

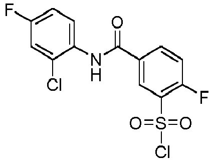

Similarly prepared according to procedure described for the preparation of **D7**, using as starting materials **D1** and 2-chloro-4-fluoroaniline instead of 3,4,5-trifluoroaniline. Method 1; Rt=2.32min, m/z=366.03 (M+H)⁺.

### Description 22: 4-fluoro-3-sulfamoyl-N-(3,4,5-trifluorophenyl)benzamide (D22)

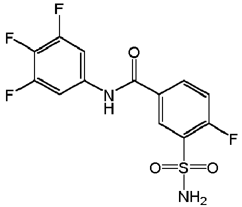

**D7** (3g, 8.16mmol) was dissolved in 1,4-dioxane (20mL,0.23mol) and added in a single portion to ammonia (10.42mL,163.18mmol) at 0°C. The reaction was stirred 15min then diluted with water and extracted with 2Me-THF. The organic layer was washed with 6N HCl and evaporated giving a brown solid (2.5g) that was triturated with DCM giving the title compound **D22** (2g, 5.74mmol) as off-white solid. Method 1; Rt: 1.85min. m/z: 349.21 (M+H)⁺.

### Description 23: 2-chloro-4-fluoro-5-sulfamoyl-N-(3,4,5-trifluorophenyl)benzamide (D23)

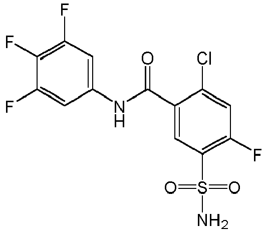

A 20mL vial was charged with 3,4,5-trifluoroaniline (245.4mg, 1.67mmol) then a solution of **D2** (221.04mg, 0.76mmol) in toluene (5mL) was added in a single portion. The vial was sealed and the reaction mixture stirred for 10min at room temperature, resulting in a white slurry. The reaction was diluted with toluene (3mL) and heated by microwave irradiation at 70°C for 20min. The reaction slurry was cooled to room temperature and poured into a flask containing acqu. NH₄OH (aprox. 30mL) and stirred vigourously at room temperature overnight. The resulting white slurry, was diluted with DCM, treated with ice and acidified with 6N HCl until pH=1. The organic layer was diluted with EtOAc, washed with 6N HCl two times and brine, dried over MgSO₄ (dry), filtered and finally evaporated giving the title compound **D23** (250mg, 0.65mmol) as white solid. ¹H NMR (300 MHz, DMSO-*d₆*) δ ppm 7.13 - 7.30 (m, 1 H) 7.53 - 7.66 (m, 2 H) 7.89 - 8.02 (m, 3 H) 11.05 (s, 1 H). Method 9; Rt: 2.02min. m/z: 383.05 (M+H)⁺.

### Description 24: 2-bromo-4-fluoro-5-sulfamoyl-N-(3,4,5-trifluorophenyl)benzamide (D24)

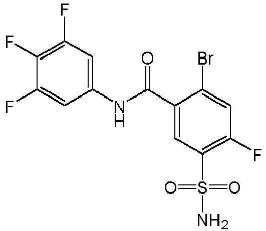

A 20mL vial was charged with **D3** (500mg, 1.49mmol), 3,4,5-trifluoroaniline (437.85mg, 2.98mmol) and toluene (4mL). The vial was sealed and stirred at room temperature giving after 10min a white suspension. The reaction was diluted with toluene (3mL) and the reaction mixture was heated by microwave irradiation at 100°C for 15min, diluted with toluene (5mL) and poured into 37% NH₄OH (20mL). MeCN (2mL) was added and the resulting mixture stirred for 4hrs at room temperature.

The reaction mixture was diluted with EtOAc and extracted with EtOAc; the organic layer was dried over MgSO₄ (dry), filtered and evaporated giving the title compound **D24** (700mg, purity 75%). This intermediate was used in the next step without any purification. Method 1; Rt: 1.93min. m/z: 427.16 (M+H)⁺.

### Description 25: N-(3-chloro-4-fluorophenyl)-4-fluoro-3-sulfamoylbenzamide (D25)

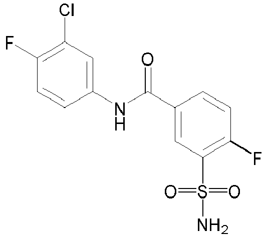

**D11** (200mg, 0,55mmol) was suspended in MeCN (2mL), cooled to 0°C with ice bath, then treated with a single portion of aqueous ammonia (2.18mL, 10.92mmol). The reaction was stirred 5min at 0°C giving a yellowish solution. Solvent was removed *in vacuo* and the residue was diluted with water and extracted with EtOAc. Solvent was removed *in vacuo* affording the title compound **D25** (200mg, 0,55mmol). Method 1; Rt: 1.88min. m/z: 346.98(M+H)⁺.

### Description 26: 4-fluoro-3-(methylsulfamoyl)-N-(3,4,5-trifluorophenyl)benzamide (D26)

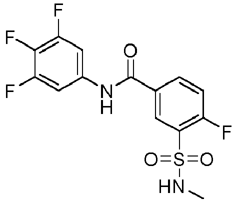

To a solution of **D7** (150 mg, 0.41 mmol) in MeCN (3.55 mL) cooled to 0°C, DIPEA (217.17 uL, 1.22 mmol) and methanamine (27.54 mg, 0.41 mmol) were added and the reaction mixture was stirred at room temperature for 1.5h. Water (5 mL) was added and the reaction mixture was stirred for 15 min. DCM (5 mL) was added and the stirring was continued for further 15 min. The solution was filtered on phase separator and the organic layer was concentrated under vacuo to give the crude title compound **D26** (151 mg) which was used without purification. Method 1; Rt=2.01min, m/z=362.79 (M+H)⁺.

### Description 27: 3,4-difluoro-5-[(iminomethyl)sulfamoyl]-N-(3,4,5-trifluorophenyl)benzamide (D27)

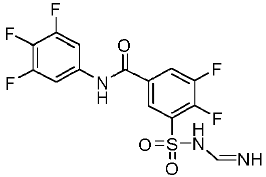

To a solution of **D10** (1.95mmol) in MeCN (20mL) formamidine hydrochloride (313.22mg, 3.89mmol) and diisopropylethylamine (1.02mL, 5.84mmol) were added at 0°C. The reaction mixture was stirred at RT for 1h. The reaction was concentrated under vacuo, the residue was taken up with DCM and washed with water, 5% citric acid solution, and aq.sat. NaHCO₃ solution. Organic layer was dried over Na₂SO₄, filtered and concentrated under vacuo. The residue was triturated with DCM/EtOAc, to obtain the title compound **D27** (406mg) as white solid. Method 1; Rt=1.93min, m/z=394.00 (M+H)⁺.

### Description 28: 2,4-difluoro-5-(N-(iminomethyl)sulfamoyl)-N-(3,4,5-trifluorophenyl)benzamide (D28)

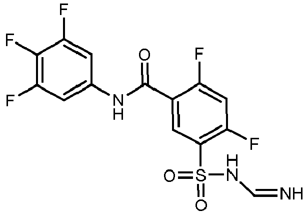

Similarly prepared according to the procedure described for the preparation ofof **D27**, using as starting material **D9** (187 mg, 0.485 mmol), to afford the title compound **D28** as white solid (90 mg). Method 1; Rt: 1.81min. m/z: 394.06 (M+H)⁺.

### Description 29: 3-(carbamimidoylsulfamoyl)-4-fluoro-N-(3,4,5-trifluorophenyl)benzamide (D29)

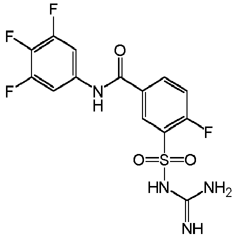

A 20mL vial was charged with **D7** (200mg, 0,544mmol), guanidine hydrochloride (60mg, 0.628mmol) and MeCN (2mL). The vial was sealed and evacuated. The reddish suspension was cooled to 0°C with ice bath then a solution of DIPEA (200uL, 1.14mmol) in MeCN (1mL) was added dropwise at the same temperature. The reaction mixture (a suspension) was stirred for 5min at 0°C then at room temperature for 1h. Solvent was removed *in vacuo* giving the title compound **D29** (212mg, 0.326mmol). Method 9; Rt: 1.78min. m/z: 391.09 (M+H)⁺.

### Description 30: 3-[(N-acetylcarbamimidoyl)sulfamoyl]-4-fluoro-N-(3,4,5-trifluorophenyl)benzamide (D30)

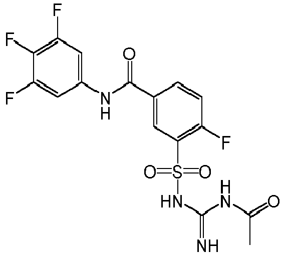

Similarly prepared according to procedure described for the preparation of **D29**, using as starting material compound **D7** and N-carbamimidoylacetamide instead of guanidine hydrochloride. Method 9; Rt: 2.00min. m/z: 433.17 (M+H)⁺.

### Description 31: (R)-4-fluoro-N-(3,4,5-trifluorophenyl)-3-(N-(1,1,1-trifluoropropan-2-yl)sulfamoyl)benzamide (D31)

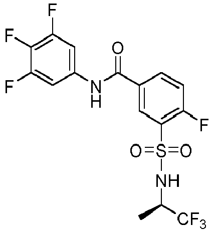

**D7** (100mg, 0.27mmol) was suspended in MeCN (2mL), DIPEA (0.24mL, 1.36mmol) and (2R)-1,1,1-trifluoro-2-propanamine hydrochloride (81.34mg, 0.54mmol) were added, and the resulting solution was stirred at RT for 3h. The reaction was diluted with EtOAc and washed twice with 3N HCl solution. Organic layer was dried over Na₂SO₄, filtered and concentrated under vacuo, to obtain the crude title compound **D31** (88mg) that was used in the next step without further purification. Method 1; Rt=2.32min, m/z=445.06 (M+H)⁺.

### Description 32: (S)-4-fluoro-N-(3,4,5-trifluorophenyl)-3-(N-(1,1,1-trifluoropropan-2-yl)sulfamoyl)benzamide (D32)

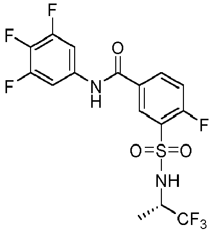

Similarly prepared according to procedure described for the preparation of compound **D31**, using (2S)-1,1,1-trifluoro-2-propanamine hydrochloride instead of (2R)-1,1,1-Trifluoro-2-propanamine hydrochloride. Method 1; Rt=2.32min, m/z=445.13 (M+H)⁺.

### Description 33: 3-(N-cyclopropylsulfamoyl)-4-fluoro-N-(3,4,5-trifluorophenyl)benzamide (D33)

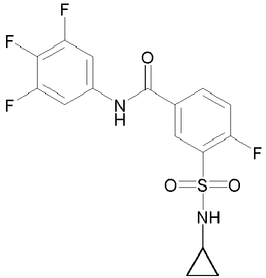

A suspension of cyclopropanamine hydrochloride (113.06uL, 1.63mmol) in MeCN (1mL) was treated at 0°C with DIPEA and then with **D7** (150mg, 0.41mmol). The resulting yellow solution was stirred at room temperature. Solvent was removed *in vacuo,* the residue was dissolved in 1/1 EtOAc/2-Methyl-THF and poured into a separating funnel. The organic layer was washed with 6N HCl, NaHCO₃ aq.sat. solution and brine, dried over Na₂SO₄, filtered and finally evaporated giving the title compound **D33** (125mg) as white solid. Method 1; Rt: 2.16min. m/z: 389.13(M+H)⁺.

### Description 34: trans-4-fluoro-3-(N-(4-hydroxycyclohexyl)sulfamoyl)-N-(3,4,5-trifluorophenyl)benzamide (D34)

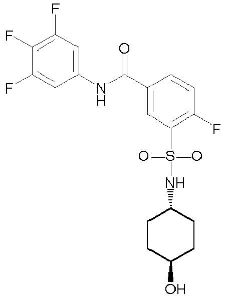

A suspension of *trans*-4-aminocyclohexan-1-ol hydrochloride (80mg, 0,53mmol) in MeCN (1mL) was treated at 0°C with triethylamine (197.92uL, 1.43mmol) and then with **D7** (150mg, 0.41mmol) The resulting solution become a white suspension and it was stirred at room temperature. Solvent was removed *in vacuo*, the residue was dissolved in 1/1 EtOAc/2-Methyl-THF and poured into a separating funnel. The organic layer was washed with 6N HCl, NaHCO₃ aq.sat.solution and brine, dried over Na₂SO₄, filtered and finally evaporated giving the title compound **D34** (100mg) as white solid. Method 1; Rt: 1.90min. m/z: 447.16(M+H)⁺.

### Description 35: cis-4-fluoro-3-(N-(4-hydroxycyclohexyl)sulfamoyl)-N-(3,4,5-trifluorophenyl)benzamide (D35)

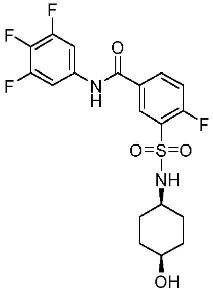

Similarly prepared according to procedure described for the preparation of **D31**, using *cis*-4-amino-cyclohexanol hydrochloride instead of (2R)-1,1,1-trifluoro-2-propanamine hydrochloride. Method 1; Rt=2.00min, m/z=447.22 (M+H)⁺.

### Description 36: trans-4-fluoro-5-(N-((4-hydroxycyclohexyl)sulfamoyl)-2-methyl-N-(3,4,5-trifluorophenyl)benzamide (D36)

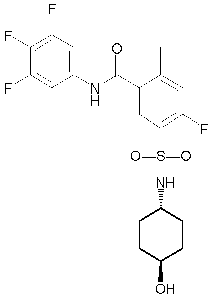

**D16** (100mg, 0.26mmol) was dissolved in MeCN (1.3mL) and cooled to 0°C. Triethylamine (0.13mL, 0.92mmol) and trans-4-aminocyclohexanol hydrochloride (43.7mg, 0.29mmol) were added, and the reaction mixture was stirred at RT for 1h. EtOAc and few drops of MeOH were added, and the mixture was washed with 5% citric acid solution and sat. NaHCO₃ solution. Organic layer was dried over Na₂SO₄, filtered and concentrated under vacuo, to obtain crude **D36** (122mg) as white solid. Method 1; Rt: 2.03min. m/z: 461.34 (M+H)⁺.

### Description 37: 4-fluoro-3-(N-((tetrahydro-2H-pyran-2-yl)oxy)sulfamoyl)-N-(3,4,5-trifluorophenyl)benzamide (D37)

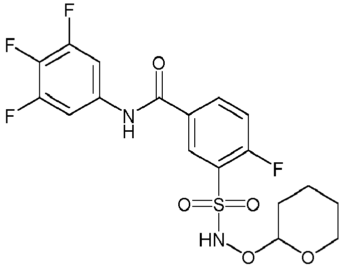

O-(tetrahydro-2H-pyran-2-yl)hydroxylamine (318.6mg, 2.72mmol) was dissolved in water (278uL) and cooled to 0°C. A solution of potassium hydrogen carbonate (136.14mg, 1.36mmol) in water (417uL) was added dropwise keeping temperature below 10°C. The reaction mixture was stirred for 15 min at the same temperature, then MeOH (348uL) and THF (1.4mL) were added. **D7** (500mg, 1.36mmol) was added portionwise over 10min. The reaction was stirred at room temperature for 1h then extracted with 2-Methyl-THF, washed with brine two times, dried over Na₂SO₄ (dry), filtered and finally evaporated, giving the title compound **D37** (600mg, 1.34mmol). Method 5; Rt: 2.25min. m/z: 561.24 (M+CF₃COO)⁻.

### Description 38: tert-butyl 4-((2-fluoro-5-((3,4,5-trifluorophenyl)carbamoyl)phenyl)sulfonamido)piperidine-1-carboxylate (D38)

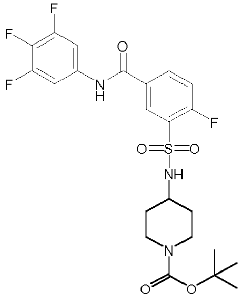

Tert-butyl 4-aminopiperidine-1-carboxylate (245.11mg, 1.22mmol) was suspended in MeCN (2mL), cooled to 0°C and treated with a single portion of **D7** (150mg, 0.41mmol). The resulting white suspension was stirred at room temperature for 1h. Solvent was removed *in vacuo*, the residue was dissolved in 1/1 EtOAc/2-Methyl-THF and poured into a separating funnel. The organic layer was washed with 6N HCl, NaHCO₃ sat. solution and brine, dried over Na₂SO₄, filtered and finally evaporated giving the title compound **D38** (140mg) as white solid. Method 1; Rt: 2.37min. m/z: 432.23(M-Boc)⁺.

### Description 39: 3-(N-(4,4-diethoxybutyl)sulfamoyl)-4-fluoro-N-(3,4,5-trifluorophenyl)benzamide (D39)

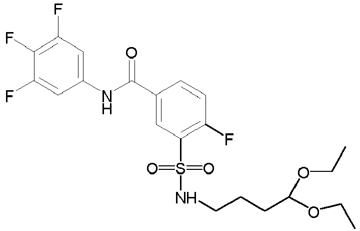

**D7** (150mg, 0.41mmol) was dissolved in MeCN (1mL) and treated with a single portion of 4-aminobutyraldehyde diethylacetal (204.4uL, 1.18mmol) dissolved in MeCN (0.3mL). The reaction was stirred at room temperature for 15min. The reaction solution was diluted with DCM, washed with 0,5N HCl and evaporated under reduced pressure. The residue was dissolved in DCM, washed with 0,5N HCl, brine, 1N NaOH, dried over Na₂SO₄, filtered and evaporated. The residue (187mg, yellow oil) was dissolved in toluene (1mL) and treated with PE (5mL) giving a milky suspension. After sonication a sticky precipitate appeared; the mother liquor was removed by decantation giving the title compound **D39** (110mg) as sticky gum. Method 1; Rt: 2.34min. m/z: 401.22 (M-2OEt)⁺.

### Description 40: 4-fluoro-3-(N-(pyridin-4-yl)sulfamoyl)-N-(3,4,5-trifluorophenyl)benzamide (D40)

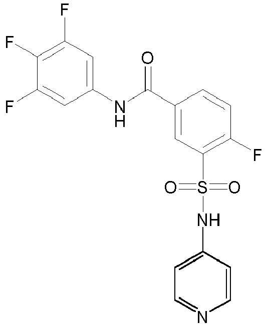

**D7** (50mg, 0.14mmol) was suspended in MeCN (1mL), treated with triethylamine (56.55uL, 0.41mmol) and pyridin-4-amine (40mg, 0.43mmol) resulting in a yellowish solution. The reaction was diluted with 2-Methyl-THF and EtOAc and acidified with 1N HCl to pH=1 (by paper), poured into a separating funnel and the acqueous layer separated. The aqueous layer was basified with NaHCO₃ and extracted with EtOAc; the combined organic extracts were evaporated, giving a residue containing the crude product (60mg). The crude product was suspended in DCM and filtered giving the title compound **D40** as yellowish solid. Method 1; Rt: 1.57min. m/z: 426.15 (M+H)⁺.

### Description 41: tert-butyl ((1S,2S)-2-((2-fluoro-5-((3,4,5-trifluorophenyl)carbamoyl)phenyl)sulfonamido)cyclopentyl)carbamate (D41)

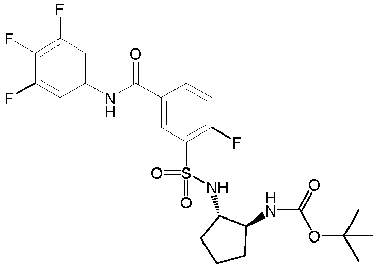

A solution of **D7** (70mg, 0.19mmol was dissolved in MeCN (400uL) and treated with a solution oftert-Butyl [(1S,2S)-2-aminocyclopentyl]carbamate (76.26mg, 0.38mmol) in MeCN (500uL) at 0°C. The reaction solution was stirred for 5min, resulting in a white suspension. The reaction was filtered and the filtrate was discarded. The mother liquor was evaporated, dissolved in DCM, washed with 1M NaOH and 5% citric acid aq. solution, dried over Na₂SO₄, filtered and finally evaporated yielding the title compound **D41** (60mg,0.11 mmol) as yellowish oil. Method 1; Rt: 2.42min. m/z: 432.16 (M-Boc)⁺.

### Description 42: 3-(N-((1S,2S)-2-aminocyclopentyl)sulfamoyl)-4-fluoro-N-(3,4,5-trifluorophenyl)benzamide (D42)

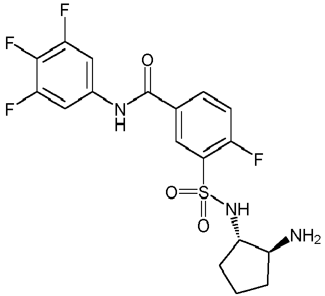

A solution of **D41** (60mg, 0.11mmol) in DCM (1mL) was treated at room temperature with TFA (1.04mL, 13.54mmol) and stirred at the same temperature for 1h. Solvent was removed giving the title compound **D42** TFA salt (70mg) as brownish solid. Method 1; Rt: 1.50min. m/z: 432.23(M+H)+.

### Description D43: 4-fluoro-3-(N-(oxetan-3-yl)sulfamoyl)-N-(3,4,5-trifluorophenyl)benzamide (D43)

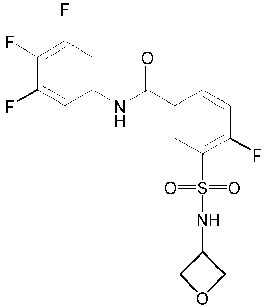

A solution **of D7** (300mg, 0.82mmol) in MeCN (2mL) was added to 3-oxetanamine (0.06mL, 0.82mmol) in a single portion at room temperature. The resulting white suspension was stirred at the same temperature for 1hr. Solvent was removed *in vacuo,* the residue dissolved in DCM, washed with 5% citric acid aqueous solution, dried over Na₂SO₄, filtered and finally evaporated giving the title compound **D43** (300mg,0.74mmol) as white solid. Method 1; Rt: 1.99min. m/z: 405.14(M+H)⁺.

### Description 44: tert-butyl (S)-3-((2-fluoro-5-((3,4,5-trifluorophenyl)carbamoyl)phenyl)sulfonamido)pyrrolidine-1-carboxylate (D44)

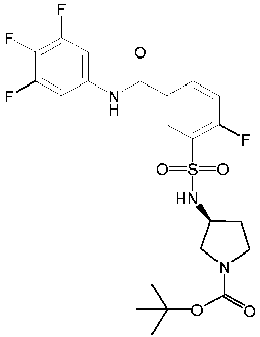

A solution of **D7** (200mg, 0,54mmol) in MeCN (2mL) was added to tert-butyl (S)-3-aminopyrrolidine-1-carboxylate (202.61mg, 1.09mmol in a single portion at room temperature. The resulting white suspension was stirred at room temperature for 1hr. Solvent was removed *in vacuo,* the residue dissolved in DCM, washed with 5% citric acid aqueous solution, dried over Na₂SO₄, filtered and finally evaporated giving the title compound **D44** (280mg, 0.54mmol)as oil. Method 1; Rt: 2.35min. m/z: 518.22(M+H)⁺.

### Description 45: 4-fluoro-3-(N-(3-hydroxycyclobutyl)sulfamoyl)-N-(3,4,5-trifluorophenyl)benzamide (D45)

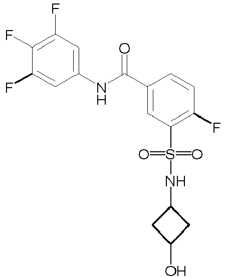

A solution of 3-aminocyclobutanol hydrochloride (134.44mg, 1.09mmol)in MeCN (2mL) was treated with a single portion of DIPEA (229.33uL, 1.63mmol) and then with **D7** (200mg, 0,54mmol). The resulting solution was stirred at room temperature for 1h. Solvent was removed *in vacuo,* the residue dissolved in DCM, washed with 5% citric acid aqueous solution, dried over Na₂SO₄, filtered and finally evaporated giving the title compound **D45** (220mg, 0.526mmol) as oil. Method 1; Rt: 1.89min. m/z: 419.19(M+H)⁺.

### Description 46: 4-fluoro-3-(N-((1S,3S)-3-hydroxycyclopentyl)sulfamoyl)-N-(3,4,5-trifluorophenyl)benzamide (D46)

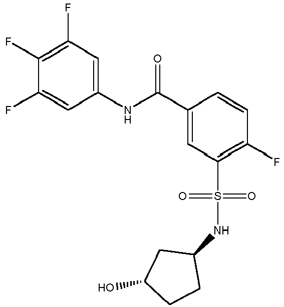

A suspension of (1S,3S)-3-Aminocyclopentan-1-ol hydrochloride 56.14mg,0.410mmol (cod. I-9981, Advanced ChemBlocks) in MeCN (1.5mL) was treated with N-ethyl-N-isopropylpropan-2-amine (198.95uL,1.14mmol) and stirred at room temperature for 15min. The reaction solution was cooled with ice bath and treated with a single portion of **D7** (100.mg,0.270mmol) and stirred for 5min at 0°C and at room temperature for 10min giving a yellow solution. Solvent was removed in vacuo, the residue was partitioned in DCM and citric acid (5% acq. solution). The organic layer was dried over Na₂SO₄(anh.), filtered and evaporated, giving **D46** (105mg).

### Description 47: tert-butyl (2-((2-fluoro-5-((3,4,5-trifluorophenyl)carbamoyl)phenyl)sulfonamido)ethyl)carbamate (D47)

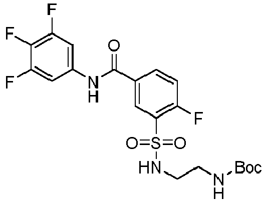

**D7** (80mg, 0.220mmol) was dissolved in MeCN (1.2mL) and cooled to 0°C. Triethylamine (0.06mL, 0.440mmol) and tert-butyl(2-aminoethyl)carbamate (0.07mL, 0.440mmol) were added and the reaction mixture was stirred at 0°C for 10min then at RT for 40min. The reaction was diluted with EtOAc and washed twice with 5% citric acid solution. Organic layer was dried over Na₂SO₄, filtered and concentrated under vacuo. The resulting crude product was purified by flash chromatography on silica (DCM/MeOH) to obtain the title compound **D47** (84mg) as white solid. Method 1; Rt=2.23min, m/z=492.14 (M+H)⁺. ¹H NMR (300 MHz, DMSO-*d*₆) δ ppm 1.35 (s, 9 H) 2.79 - 3.07 (m, 4 H) 6.57 - 6.91 (m, 1 H) 7.53 - 7.82 (m, 3 H) 8.04 (br s, 1 H) 8.23 - 8.32 (m, 1 H) 8.38 (dd, *J*=6.88, 2.29 Hz, 1 H) 10.77 (br s, 1 H).

### Description 48: 3-(N-(2-aminoethyl)sulfamoyl)-4-fluoro-N-(3,4,5-trifluorophenyl)benzamide (D48)

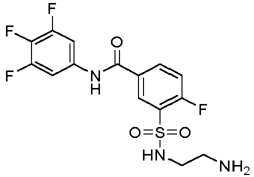

**D47** (82mg, 0.170mmol) was dissolved in DCM (1.5mL) and MeOH (0.3mL) and HCl 4N in dioxane (1.04mL, 0.17mmol) was added. The reaction mixture was stirred at RT for 1h. The reaction was concentrated under vacuo to obtain the title compound **D48** as HCl salt (70mg) as white solid. Method 9; Rt=1.43min, m/z=392.17 (M+H)⁺. ¹H NMR (300 MHz, DMSO-*d*₆) δ ppm 2.89 (t, *J*=6.60 Hz, 2 H) 3.13 (t, *J*=6.79 Hz, 2 H) 7.68 - 7.83 (m, 3 H) 8.02 (br s, 3 H) 8.30 - 8.39 (m, 1 H) 8.43 (dd, *J*=6.88, 2.29 Hz, 1 H) 10.92 (s, 1 H).

### Description 49: cis-4-amino-3-(N-(4-hydroxycyclohexyl)sulfamoyl)-N-(3,4,5-trifluorophenyl)benzamide (D49)

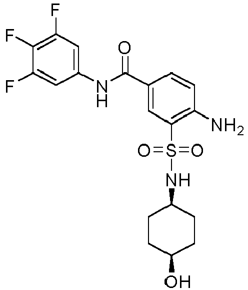

A pressure vessel was charged with **D35**, 1,4-dioxane and 33% aqueous ammonia. The pressure vessel was sealed and the reaction mixture was stirred 5 min at RT, then heated at 95° for 8h. The reaction was diluted with EtOAc and water, organic layer was dried over Na₂SO₄, filtered and concentrated under vacuo. The resulting crude product was purified by preparative HPLC. Method 3; Rt=3.24min, m/z=444.25 (M+H)⁺. ¹H NMR (300 MHz, DMSO-*d*₆) δ ppm 1.35 (m, *J*=3.30 Hz, 4 H) 1.43 - 1.62 (m, 4 H) 2.82 - 3.08 (m, 1 H) 3.49 - 3.58 (m, 2 H) 6.52 (br s, 2 H) 6.87 (d, *J*=8.62 Hz, 1 H) 7.53 - 7.79 (m, 3 H) 7.88 (dd, *J*=8.71, 2.29 Hz, 1 H) 8.23 (d, *J*=2.20 Hz, 1 H) 10.33 (s, 1 H).

### Description 50: trans-4-amino-5-(N-(4-hydroxycyclohexyl)sulfamoyl)-2-methyl-N-(3,4,5-trifluorophenyl)benzamide (D50)

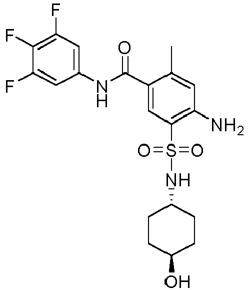

Similarly prepared according to the procedure described for the preparation of compound **D49**, starting from **D36.** Method 3; Rt=2.03min, m/z=461.34 (M+H)⁺.

### Description 51: 4-amino-3-(N-(4,4-diethoxybutyl)sulfamoyl)-N-(3,4,5-trifluorophenyl)benzamide (D51)

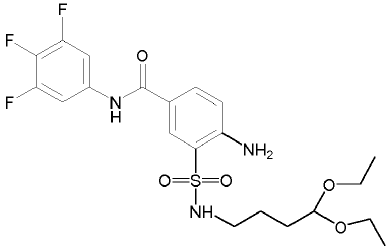

**D39** (110mg, 0.22mmol) was dissolved in dioxane (0,5mL), treated with aqueous ammonia (527.2uL, 4.47mmol) and heated at 100°C for 5hrs in a closed vial. Solvent was removed *in vacuo* giving the title compound **D51** (100mg). Method 1; Rt: 2.35min. m/z: 512.15.16 (M+Na)⁺.

### Description 52: 4-amino-3-(N-((tetrahydro-2H-pyran-2-yl)oxy)sulfamoyl)-N-(3,4,5-trifluorophenyl)benzamide (D52)

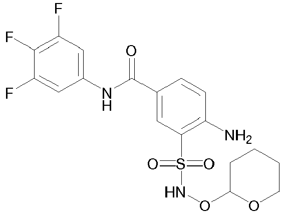

A mixture of **D37** (80mg, 0.18mmol) and aq. ammonia (500uL) in 1,4-Dioxane (500uL) was heated at 100°C-120°C for 4hrs. The reaction was partitioned between water and 2-Methyl-THF. The organic layer was dried over Na₂SO₄, filtered and finally evaporated giving the title compound **D52** (79mg, 0.18mmol) which was used in the next step without any purification. Method 1; Rt: 2.16min. m/z: 446.21(M+H)⁺.

### Description 53: methyl 4-fluoro-3-methyl-5-sulfamoylbenzoate (D53)

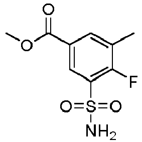

Prepared by reaction of methyl 3-(chlorosulfonyl)-4-fluoro-5-methylbenzoate with ammonia according to procedure described for the preparation of the preparation of **D22**; Rt=1.39min, m/z=248.14 (M+H)⁺.

### Description 54: Synthesis of compound: 4-fluoro-3-methyl-5-sulfamoyl-N-(3,4,5-trifluorophenyl)benzamide (D54)

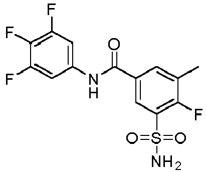

To a solution of methyl 4-fluoro-3-methyl-5-sulfamoylbenzoate **D53** (150mg, 0.610mmol) and 3,4,5-trifluoroaniline (116mg 0.79mmol) in dry THF (3mL), lithium bis(trimethylsilyl)amide 1M in THF (3.52mL, 3.52mmol) was added dropwise. The reaction mixture was stirred at RT for 3h, then more 3,4,5-trifluoroaniline (50mg, 0.340mmol) and lithium bis(trimethylsilyl)amide 1M in THF (1mL, 1mmol) were added, and the reaction mixture was stirred at RT overnight. The reaction was quenched with sat. NH₄Cl solution, and EtOAc was added. Organic layer was washed with brine, then was dried over Na₂SO₄, filtered and concentrated under vacuo. The residue was triturated with PE, to obtain the title compound **D54** (218mg) as light-brown solid, that was used without further purification. Method 1; Rt=1.97min, m/z=363.12 (M+H)⁺. ¹H NMR (300 MHz, DMSO-*d*₆) δ ppm 2.40 (d, *J*=2.11 Hz, 3 H) 7.63 - 8.01 (m, 4 H) 8.12 - 8.19 (m, 1 H) 8.19 - 8.25 (m, 1 H) 10.79 (br s, 1 H).

### Description 55: methyl 6-chloro-5-sulfamoylnicotinate (D55)

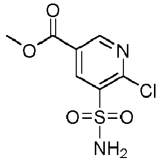

Methyl 6-chloro-5-(chlorosulfonyl)nicotinate (Enamine, cat.n°EN300-41733) (525mg, 1.94mmol) was dissolved in 1,4-dioxane (2mL), 33% aqueous ammonia (2.29mL,19.44mmol) was added and the reaction mixture was stirred 20min at RT. The reaction was diluted with EtOAc and water, the phases were separated, the organic layer was dried over Na₂SO₄, filtered and concentrated under vacuo to obtain crude title compound **D55** (285mg) that was used without further purification. Method 1; Rt=1.20min, m/z=250.91 (M+H)⁺.

### Description D56: 6-chloro-5-sulfamoyl-N-(3,4,5-trifluorophenyl)nicotinamide (D56)

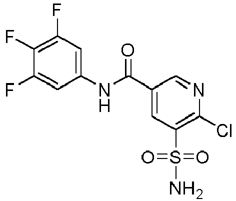

To a solution of crude compound **D55** (144mg, 0,570mmol) and 3,4,5-trifluoroaniline (109.9mg, 0.750mmol) in dry THF (2.5mL), lithium bis(trimethylsilyl)amide 1M in THF (3.33mL, 3.33mmol) was added dropwise. The reaction mixture was stirred at RT for 1h. The reaction was quenched with sat. NH₄Cl solution, and EtOAc was added. The organic layer was dried over Na₂SO₄, filtered and concentrated under vacuo. The resulting crude product was purified by preparative HPLC (H₂O/CH₃CN+1%TFA) to obtain, after lyophilization, the title compound **D56** (40mg) as light-yellow solid. Method 3; Rt: 3.17min, m/z: 366.10 (M+H)⁺. ¹H NMR (300 MHz, DMSO-*d*₆) δ ppm 7.61 - 7.82 (m, 2 H) 8.01 (s, 2 H) 8.81 (d, *J*=2.29 Hz, 1 H) 9.13 (d, *J*=2.29 Hz, 1 H) 11.00 (s, 1 H).

### Description 57: 4-amino-3-sulfamoylbenzoic acid (D57)

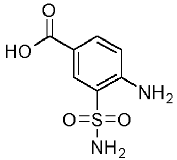

To a solution of 3-(chlorosulfonyl)-4-fluorobenzoic acid (Fluorochem, cat n° 037319) (1 g, 4.19 mmol) in 1,4-dioxane (10.61 mL) aqueous ammonia (5.31 mL, 136.35 mmol) was added and the reaction mixture was stirred in a sealed vial at 90°C for 6h, at 120°C for 17 h. An aliquota of aqueous ammonia (5.31 mL, 136.35 mmol) was added and the stirring continued overnight at 130°C. The solvent was evaporated under reduced pressure to give the crude product which was dissolved in 2N HCl solution up to acid pH. The solid precipitate was filtered, washed with water and dried out to afford the title compound **D57** (704.33 mg) as solid which was used without purification. Method 1; Rt=0.82min, m/z=217.19 (M+H)⁺.

### Description 58: 3,4-dihydro-2H-benzo[e][1,2,4]thiadiazine-7-carboxylic acid 1,1-dioxide (D58)

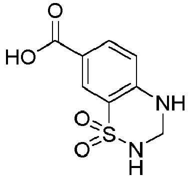

In a pressure vessel, to a solution of **D57** (300 mg, 1.39 mmol) in IPA (13.61 mL) were added formaldehyde (152.91 uL, 5.55 mmol) and 4N HCl solution in 1,4-dioxane (1 drop). The reaction mixture was stirred at 65°C for 1h. Satured aqueous K₂CO₃ solution and EtOAc were added and the two phases separated. The aqueous layer was acidified with 2N HCl solution. The precipitate was filtered to give the title compound **D58** (223.7 mg) as light yellow solid. The solid was used without purification. Method 1; Rt=0.93min, m/z=229.15 (M+H)⁺.

### Description 59: methyl 4-amino-3-sulfamoylbenzoate (D59)

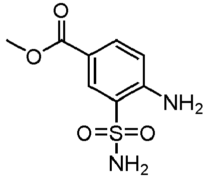

A mixture of 4-amino-3-sulfamoylbenzoic acid **D57** (30 mg, 0.14 mmol) and sulfuric acid (16.9 uL, 0.32 mmol) in methanol (706 uL) was heated at reflux for 6 h. The solvent was removed under reduced pressure. The residue was suspended in water and the resulting precipitate was collected by filtration, washed with water and dried to afford the crude title compound **D59** (9.98 mg) which was used without purification. Method 1; Rt=1.13min, m/z=231.24 (M+H)⁺.

### Description 60: methyl 3,4-dihydro-2H-benzo[e][1,2,4]thiadiazine-7-carboxylate 1,1-dioxide (D60)

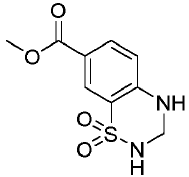

Similarly prepared according to procedure described for the preparation of the preparation of **D58** starting from methyl 4-amino-3-sulfamoylbenzoate **D59**. The crude title compound **D60** was used without purification. Method 1; Rt=1.26min, m/z=243.00 (M+H)⁺.

### Description 61: trans-4-amino-3-(N-(4-hydroxycyclohexyl)sulfamoyl)-N-(3,4,5-trifluorophenyl)benzamide (D61)

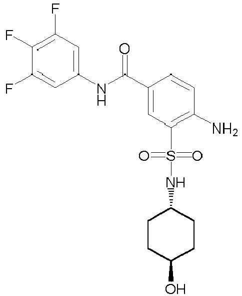

A solution of **D34** (100mg, 0.22mmol) in aqueous ammonia (801.08uL, 2.24mmol)and 1,4-dioxane (1mL) was heated at 100°C for 8 hrs in a closed vial. Solvent was removed *in vacuo,* the residue was treated with toluene and further evaporated. Solvent traces were removed by high vacuum pump, giving a residue as off-white solid (108mg). A sample of this crude material (20mg) was purified by preparative HPLC (H₂O/CH₃CN+1‰TFA), affording the title compound **D61** (9mg) as white solid. Method 3; Rt: 3.11. m/z: 444.25 (M+H)⁺. ¹H NMR (300 MHz, DMSO-*d*₆) δ ppm 0.87 - 1.35 (m, 4 H) 1.50 - 1.86 (m, 4 H) 2.78 - 2.97 (m, 1 H) 3.20 - 3.34 (m, 2 H) 6.51 (br s, 2 H) 6.88 (d, *J*=8.71 Hz, 1 H) 7.54 - 7.82 (m, 3 H) 7.89 (dd, *J*=8.71, 2.11 Hz, 1 H) 8.23 (d, *J*=2.11 Hz, 1 H) 10.34 (s, 1 H). ¹H NMR (300 MHz, DMSO-d*₆*+TFA) δ ppm 0.87 - 1.37 (m, 4 H) 1.45 - 1.86 (m, 4 H) 2.79 - 3.01 (m, 1 H) 3.14 - 3.43 (m, 1 H) 6.88 (d, *J*=8.71 Hz, 1 H) 7.55 - 7.82 (m, 3 H) 0.00 (dd, *J*=8.50, 2.20 Hz, 1 H) 8.23 (d, *J*=2.11 Hz, 1 H) 10.33 (s, 1 H).

### Description 62: tert-butyl 4-(1,1-dioxido-3-oxo-7-((3,4,5-trifluorophenyl)carbamoyl)-3,4-dihydro-2H-benzo[e][1,2,4]thiadiazin-2-yl)piperidine-1-carboxylate (D62)

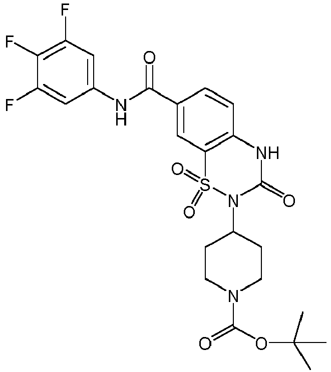

**D124** (50mg, 0.09mmol) and di(1H-imidazol-1-yl)methanone (43.2mg, 0.27mmol) were mixed in DMF (500uL) and heated at 150°C for 11min. Solvent was removed *in vacuo,* diluted with EtOAc and washed with 0,5N HCl. The organic were dried over Na₂SO₄, filtered and finally evaporated giving the title compound **D62** (23mg). Method 1; Rt: 2.45min. m/z: 455.26(M-Boc)⁺.

### Description 63: 4-(hydroxyamino)-3-sulfamoyl-N-(3,4,5-trifluorophenyl)benzamide (D63)

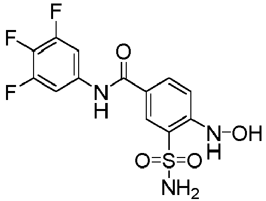

A solution of **D22** (40mg, 0.11mmol), hydroxylamine chloridrate (23.94mg, 0.34mmol) and sodium bicarbonate (33.77mg, 0.4mmol) in DMSO (1mL) were charged in a 5mL vial and stirred at room temperature overnight then at 100°C for 4hrs. The reaction was cooled to room temperature, poured into water and extracted with 2-Methyl-THF. The combined organic extracts were washed with brine, dried over Na₂SO₄, filtered and evaporated *in vacuo,* giving the title compound **D63** (48mg, 0.13mmol) as oil. Method 1; Rt: 1.80min. m/z: 362.04.16 (M+H)⁺.

### Description 64: tert-butyl ((1-((2-fluoro-5-((3,4,5-trifluorophenyl)carbamoyl)phenyl)sulfonyl)piperidin-2-yl)methyl)carbamate (D64)

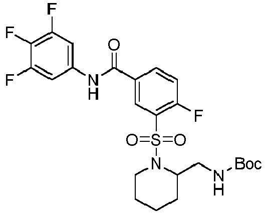

Similarly prepared according to the procedure described for the preparation of compound **D47**, using tert-butyl(piperidin-2-ylmethyl)carbamate instead of tert-butyl (2-aminoethyl)carbamate. Method 3; Rt=4.25min, m/z=546.11 (M+H)⁺. ¹H NMR (300 MHz, DMSO-*d*₆) δ ppm 0.94 - 1.18 (m, 1 H) 1.19 - 1.31 (m, 1 H) 1.35 (s, 9 H) 1.41 - 1.67 (m, 4 H) 2.98 - 3.25 (m, 3 H) 3.57 - 3.80 (m, 1 H) 3.88 - 4.16 (m, 1 H) 6.67 - 6.89 (m, 1 H) 7.58 - 7.82 (m, 3 H) 8.20 - 8.34 (m, 1 H) 8.36 - 8.49 (m, 1 H) 10.79 (s, 1 H).

### Description 65: 3-((2-(aminomethyl)piperidin-1-yl)sulfonyl)-4-fluoro-N-(3,4,5-trifluorophenyl)benzamide (D65)

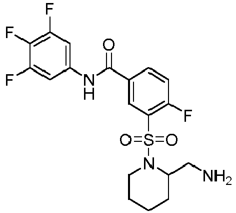

Similarly prepared according to the procedure described for the preparation of compound **D48**, using as starting material **D64**. The title compound **D65** was obtained as HCl salt. Method 3; Rt=2.71min, m/z=446.28 (M+H)⁺. ¹H NMR (300 MHz, DMSO-*d*₆) δ ppm 0.98 - 1.33 (m, 2 H) 1.38 - 1.60 (m, 3 H) 1.60 - 1.75 (m, 1 H) 2.98 - 3.14 (m, 2 H) 3.15 - 3.28 (m, 1 H) 3.70 - 3.90 (m, 1 H) 4.14 - 4.33 (m, 1 H) 7.70 - 7.82 (m, 3 H) 7.88 (br s, 2 H) 8.30 - 8.42 (m, 1 H) 8.47 (dd, *J*=6.88, 2.29 Hz, 1 H) 10.98 (br s, 1 H).

### Description 66: tert-butyl (S)-((1-((2-fluoro-5-((3,4,5-trifluorophenyl)carbamoyl)phenyl)sulfonyl)piperidin-2-yl)methyl)carbamate (D66)

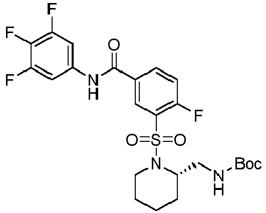

Similarly prepared according to the procedure described for the preparation of compound **D47**, using (S)-tert-butyl(piperidin-2-ylmethyl)carbamate instead of tert-butyl(2-aminoethyl)carbamate, and diisopropylethylamine instead of triethylamine. Method 1; Rt=2.49min, m/z=546.18 (M+H)⁺. ¹H NMR (300 MHz, DMSO-*d*₆) δ ppm 0.98 - 1.19 (m, 1 H) 1.21 - 1.30 (m, 1 H) 1.35 (s, 9 H) 1.43 - 1.67 (m, 4 H) 2.98 - 3.26 (m, 3 H) 3.55 - 3.85 (m, 1 H) 3.88 - 4.14 (m, 1 H) 6.69 - 6.92 (m, 1 H) 7.53 - 7.84 (m, 3 H) 8.17 - 8.36 (m, 1 H) 8.37 - 8.51 (m, 1 H) 10.77 (s, 1 H).

### Description 67: (S)-3-((2-(aminomethyl)piperidin-1-yl)sulfonyl)-4-fluoro-N-(3,4,5-trifluorophenyl)benzamide (D67)

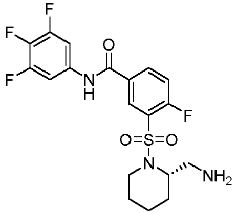

Similarly prepared according to the procedure described for the preparation of compound **D48**, using as starting material **D66**. Product obtained as HCl salt. Method 1; Rt=1.52min, m/z=446.14 (M+H)⁺. ¹H NMR (300 MHz, DMSO-*d*₆) δ ppm 0.99 - 1.31 (m, 2 H) 1.36 - 1.60 (m, 3 H) 1.60 - 1.78 (m, 1 H) 2.98 - 3.15 (m, 2 H) 3.15 - 3.28 (m, 1 H) 3.71 - 3.90 (m, 1 H) 4.16 - 4.32 (m, 1 H) 7.68 - 7.84 (m, 3 H) 7.99 (br s, 3 H) 8.29 - 8.42 (m, 1 H) 8.43 - 8.53 (m, 1 H) 10.97 (s, 1 H).

### Description 68: tert-butyl (R)-((1-((2-fluoro-5-((3,4,5-trifluorophenyl)carbamoyl)phenyl)sulfonyl)piperidin-2-yl)methyl)carbamate (D68)

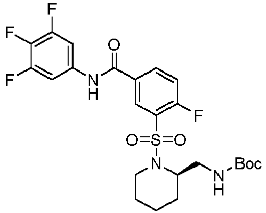

Similarly prepared according to the procedure described for the preparation of compound **D47**, using (R)-tert-butyl(piperidin-2-ylmethyl)carbamate instead of tert-butyl(2-aminoethyl)carbamate, and diisopropylethylamine instead of triethylamine. Method 1; Rt=2.49min, m/z=546.38 (M+H)⁺. ¹H NMR (300 MHz, DMSO-*d*₆) δ ppm 0.98 - 1.18 (m, 1 H) 1.24 - 1.41 (m, 10 H) 1.42 - 1.69 (m, 4 H) 3.00 - 3.25 (m, 3 H) 3.60 - 3.82 (m, 1 H) 3.90 - 4.16 (m, 1 H) 6.69 - 6.90 (m, 1 H) 7.60 - 7.82 (m, 3 H) 8.21 - 8.35 (m, 1 H) 8.36 - 8.50 (m, 1 H) 10.79 (s, 1 H).

### Description 69: (R)-3-((2-(aminomethyl)piperidin-1-yl)sulfonyl)-4-fluoro-N-(3,4,5-trifluorophenyl)benzamide (D69)

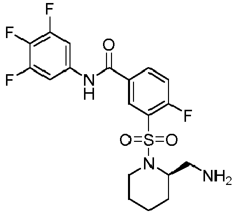

Similarly prepared according to the procedure described for the preparation of compound **D48**, starting from **D68**. Product obtained as HCl salt. Method 1; Rt=1.52min, m/z=446.21 (M+H)⁺.

### Description 70: tert-butyl (S)-(1-((2-fluoro-5-((3,4,5-trifluorophenyl)carbamoyl)phenyl)sulfonyl)pyrrolidin-3-yl)carbamate (D70)

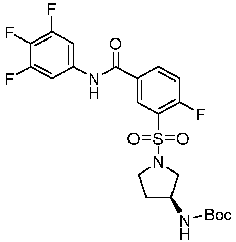

Similarly prepared according to the procedure described for the preparation of compound **D47**, using tert-butyl(S)-pyrrolidin-3-ylcarbamate instead of tert-butyl(2-aminoethyl)carbamate, and diisopropylethylamine instead of triethylamine. Method 1; Rt=2.34min, m/z=518.28 (M+H)⁺. ¹H NMR (300 MHz, DMSO-*d*₆) δ ppm 1.36 (s, 9 H) 1.65 - 1.85 (m, 1 H) 2.00 (s, 1 H) 3.08 - 3.23 (m, 1 H) 3.34 - 3.54 (m, 3 H) 3.81 - 3.99 (m, 1 H) 6.99 - 7.18 (m, 1 H) 7.62 - 7.80 (m, 3 H) 8.27 - 8.36 (m, 1 H) 8.39 (dd, *J*=6.60, 2.11 Hz, 1 H) 10.80 (s, 1 H).

### Description 71: (S)-3-((3-aminopyrrolidin-1-yl)sulfonyl)-4-fluoro-N-(3,4,5-trifluorophenyl)benzamide (D71)

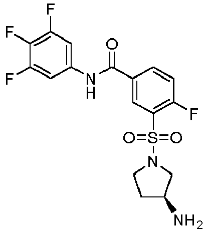

Similarly prepared according to the procedure described for the preparation of compound **D48**, using as starting material **D70**. Product obtained as HCl salt. Method 1; Rt=1.52min, m/z=418.31 (M+H)⁺. ¹H NMR (300 MHz, DMSO-*d*₆) δ ppm 1.77 - 2.01 (m, 1 H) 2.05 - 2.26 (m, 1 H) 3.33 - 3.41 (m, 2 H) 3.45 - 3.67 (m, 2 H) 3.73 - 3.89 (m, 1 H) 7.63 - 7.85 (m, 3 H) 8.07 (br s, 3 H) 8.30 - 8.48 (m, 2 H) 10.91 (s, 1 H).

### Description 72: tert-butyl ((1-((2-fluoro-5-((3,4,5-trifluorophenyl)carbamoyl)phenyl)sulfonyl)pyrrolidin-2-yl)methyl)carbamate (D72)

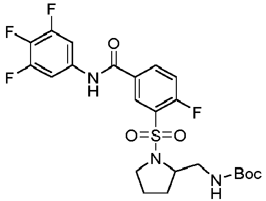

Similarly prepared according to the procedure described for the preparation of compound D47, using tert-butyl N-(pyrrolidin-2-ylmethyl)carbamate instead of tert-butyl(2-aminoethyl)carbamate, and diisopropylethylamine instead of triethylamine. Method 1; Rt=2.43min, m/z=532.20 (M+H)⁺. ¹H NMR (300 MHz, DMSO-*d*₆) δ ppm 1.36 (s, 9 H) 1.51 - 1.70 (m, 2 H) 1.70 - 1.97 (m, 2 H) 2.85 - 3.05 (m, 1 H) 3.11 - 3.27 (m, 2 H) 3.38 - 3.48 (m, 1 H) 3.70 - 3.90 (m, 1 H) 6.84 - 7.03 (m, 1 H) 7.61 - 7.80 (m, 3 H) 8.23 - 8.35 (m, 1 H) 8.36 - 8.49 (m, 1 H) 10.81 (s, 1 H).

### Description 73: 3-((2-(aminomethyl)pyrrolidin-1-yl)sulfonyl)-4-fluoro-N-(3,4,5-trifluorophenyl)benzamide (D73)

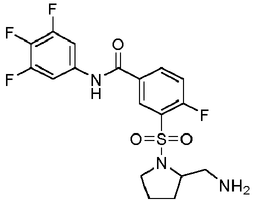

Similarly prepared according to the procedure described for the preparation of compound **D48**, starting from **D72**. Product obtained as HCl salt. Method 1; Rt=1.47min, m/z=432.23 (M+H)⁺. ¹H NMR (300 MHz, DMSO-d*₆*+TFA) δ ppm 1.56 - 1.78 (m, 2 H) 1.80 - 1.98 (m, 2 H) 2.86 - 3.15 (m, 2 H) 3.22 - 3.37 (m, 1 H) 3.37 - 3.51 (m, 1 H) 3.92 - 4.06 (m, 1 H) 7.64 - 7.81 (m, 3 H) 7.92 (br s, 3 H) 8.27 - 8.41 (m, 1 H) 8.42 - 8.52 (m, 1 H) 10.88 (s, 1 H).

### Description 74: tert-butyl ((1-((2-fluoro-5-((3,4,5-trifluorophenyl)carbamoyl)phenyl)sulfonyl)azetidin-2-yl)methyl)carbamate (D74)

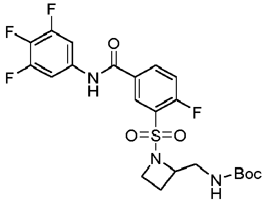

Similarly prepared according to the procedure described for the preparation of compound **D47**, using 2-(N-Boc-aminomethyl)azetidine hydrochloride instead of tert-butyl(2-aminoethyl)carbamate, and diisopropylethylamine instead of triethylamine. Method 1; Rt=2.37min, m/z=518.08 (M+H)⁺. ¹H NMR (300 MHz, DMSO-*d*₆) δ ppm 1.35 (s, 9 H) 1.91 - 2.15 (m, 2 H) 3.08 - 3.23 (m, 1 H) 3.24 - 3.31 (m, 1 H) 3.60 - 3.85 (m, 2 H) 4.04 - 4.20 (m, 1 H) 6.78 - 7.01 (m, 1 H) 7.63 - 7.84 (m, 3 H) 8.24 - 8.47 (m, 2 H) 10.85 (br s, 1 H).

### Description 75: 3-((2-(aminomethyl)azetidin-1-yl)sulfonyl)-4-fluoro-N-(3,4,5-trifluorophenyl)benzamide (D75)

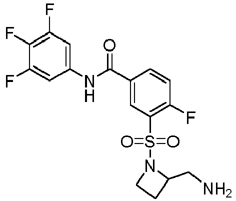

Similarly prepared according to the procedure described for the preparation of compound **D48**, using starting material **D74**. The title compound **D75** was obtained as HCl salt. Method 1; Rt=1.43min, m/z=418.18 (M+H)⁺ .

### Description 76: tert-butyl ((4-((2-fluoro-5-((3,4,5-trifluorophenyl)carbamoyl)phenyl)sulfonyl)morpholin-3-yl)methyl)carbamate (D76)

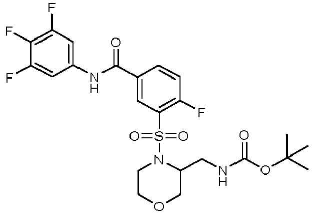

Similarly prepared according to the procedure described for the preparation of compound **D47**, using tert-butyl-(morpholin-3-ylmethyl)carbamate instead of tert-butyl(2-aminoethyl)carbamate, and diisopropylethylamine instead of trimethylamine. Method 1; Rt=2.31min, m/z=548.48 (M+H)⁺.

### Description 77: 3-[3-(aminomethyl)morpholine-4-sulfonyl]-4-fluoro-N-(3,4,5-trifluorophenyl)benzamide (D77)

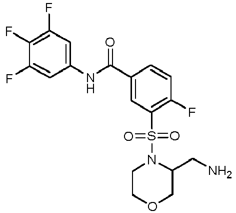

Similarly prepared according to the procedure described for the preparation of compound **D48**, using as starting material **D76.** Method 1; Rt=1.42min, m/z=448.18 (M+H)⁺.

### Description 78: tert-butyl N-[[1-[2-fluoranyl-5-[[3,4,5-tris(fluoranyl)phenyl]carbamoyl]phenyl]sulfonylimidazol-2-yl]methyl]carbamate (D78)

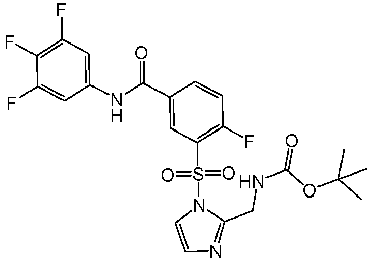

1H-imidazol-2-ylmethanamine dihydrochloride (300mg, 1.76mmol) was suspended in DCM (10mL), treated with N,N-dimethylpyridin-4-amine (76mg, 0.62mmol), triethylamine (1.35mL, 9.7mmol) and finally with di-tert-butyl dicarbonate (1.156g, 5.29mmol). The reaction was stirred at room temperature for 20min, heated at 50°C for 5hrs, cooled and stored at room temperature for 48hrs. The reaction was diluted with DCM and washed with brine. The organic layer was evaporated, the residue dissolved in DCM/MeCN/TFA and stirred at room temperature for one night. The reaction was basified with NaHCO₃ (sat. solution) then the organic layer was separated giving after evaporation tert-butyl [(1H-imidazol-2-yl)methyl]carbamate (300mg, 1.76mmol) as yellowish oil.

**D7** (85mg, 0.23mmol) was treated with a solution of tert-butyl [(1H-imidazol-2-yl)methyl]carbamate (45.59mg, 0.23mmol) in DCM (3mL) and stirred at room temperature. To the resulting suspension, triethylamine (193.32uL, 1.39mmol) was added in two equal portions and the reaction mixture was magnetically stirred at room temperature for 1h. The resulting yellow reaction solution was washed with NaHCO₃ sat. solution, aq. 5% citric acid then with brine. The organic layer was dried over Na₂SO₄ (dry), filtered and finally evaporated yielding the title compound **D78** (100mg, 0.189mmol) as cristalline white solid. Method 9; Rt: 2.41min. m/z: 529.36 (M+H)⁺. ¹H NMR (300 MHz, DMSO-*d*₆) δ ppm 1.21 - 1.43 (m, 3 H) 1.31 (s, 9 H) 4.32 (br d, *J*=5.78 Hz, 2 H) 7.00 - 7.10 (m, 1 H) 7.10 - 7.24 (m, 1 H) 7.64 - 7.86 (m, 4 H) 8.33 - 8.52 (m, 1 H) 8.58 - 8.80 (m, 1 H) 10.87 (s, 1 H)

### Description 79: 4-fluoro-3-[2-(hydroxymethyl)piperidine-1-sulfonyl]-N-(3,4,5-trifluorophenyl)benzamide (D79)

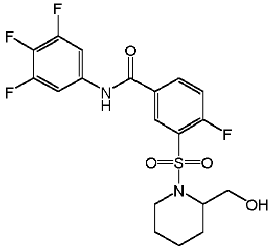

**D7** (50mg,0.14mmol) was dissolved in MeCN (1.5mL), treated with triethylamine (56.55uL,0.41mmol) then with piperidin-2-ylmethanol (18.79mg, 0.16mmol) then stirred at room temperature for 15min. A second portion of piperidin-2-ylmethanol (18.79mg, 0.16mmol) was added and the reaction was further stirred at room temperature for 15min; then a third portion of triethylamine (56.55uL, 0.41mmol) was added and the reaction further stirred for 15min at room temperature. The reaction was stopped by water addition. The solvent was removed in vacuo and the residue partitioned between water and DCM. The organic layer was washed with 0,5N HCl then dried over Na₂SO₄, filtered and finally evaporated yielding the title compound **D79** (40mg, 0.09mmol) as cristalline white solid. Method 1; Rt: 2.11min. m/z: 447.16 (M+H)⁺.

### Description 80: 3-(aziridin-1-ylsulfonyl)-N-(3,4-difluorophenyl)-4-fluorobenzamide (D80)

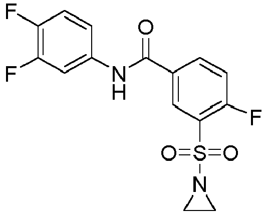

In a vial (3 mL) **D8** (198 mg, 0,566 mmol), and 2-bromanylethanamine hydrobromide (116 mg, 0,566 mmol) were weighed and the vial was sealed. The the solvent was added, DCM (1mL). The suspension was stirred at room temperature and DIPEA (0.118 mL, 0.679 mmol) was added. The mixture turned into a yellow solution immediately. The reaction mixture was treated with NaOH 20% (1 mL) dropwise and shaken. The product was extracted in DCM twice, the organic layer dried over Na₂SO₄ and the solvent was removed under vacuum to give the crude title compound **D80** (200 mg) which was dried under vacuum overnight.

### Description 81: N-(3,4-difluorophenyl)-4-fluoro-3-(N-(2-((2-hydroxyethyl)amino)ethyl)sulfamoyl)benzamide (D81)

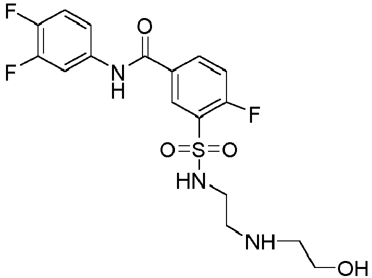

In a vial (3 mL) **D80** (200 mg, 0,561 mmol), was dissolved in dry THF, and ethanolamine (0.6 mL, 9.9 mmol) was added dropwise over 20 minutes and stirred at room temperature. Then the mixture was dried under vacuum and the mixture was diluted with 200 µL of acetonitrile and 200 µL of water and purified by preparative HPLC/MS (H₂O/CH₃CN+0.1% TFA) to give the title compound **D81** as white powder (49 mg).

### Description 82: N-(3,4-difluorophenyl)-5-(2-hydroxyethyl)-2,3,4,5-tetrahydrobenzo[f][1,2,5]thiadiazepine-8-carboxamide 1,1-dioxide (D82)

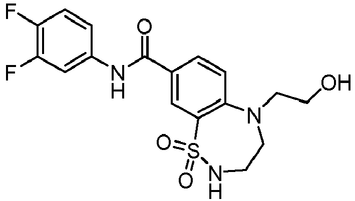

In a vial (3 mL) **D81** (16 mg, 0.038 mmol), and cesium carbonate (118 mg, 0.360 mmol) were dissolved in DMSO (2 mL). The suspension was treated at 130 °C for 6h in microwave reactor. The crude product was purified by preparative HPLC/MS (H₂O/CH₃CN+0.1% TFA) to give the title compound **D82** as white powder (4.4 mg).

### Description 83: 4-fluoro-3-[(2R)-2-(hydroxymethyl)piperidine-1-sulfonyl]-N-(3,4,5-trifluorophenyl)benzamide (D83)

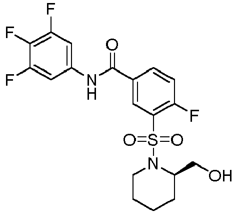

Similarly prepared according to the procedure described for the preparation of compound **D79**, using (R)-Piperidin-2-ylmethanol instead of piperidin-2-ylmethanol, and diisopropylethylamine instead of triethylamine. The title compound **D83** was obtained by purification by flash chromatography on silica (petroleum ether/EtOAc). Method 1; Rt=2.11min, m/z=447.16 (M+H)⁺. ¹H NMR (300 MHz, DMSO-*d*₆) δ ppm 1.18 - 1.38 (m, 2 H) 1.41 - 1.64 (m, 3 H) 1.71 - 1.85 (m, 1 H) 2.98 - 3.20 (m, 1 H) 3.37 - 3.49 (m, 1 H) 3.51 - 3.65 (m, 1 H) 3.66 - 3.82 (m, 1 H) 3.84 - 3.96 (m, 1 H) 4.76 (br s, 1 H) 7.57 - 7.82 (m, 3 H) 8.19 - 8.35 (m, 1 H) 8.42 (dd, *J*=6.88, 2.20 Hz, 1 H) 10.78 (br s, 1 H).

### Description 84: 4-fluoro-3-[(2S)-2-(hydroxymethyl)piperidine-1-sulfonyl]-N-(3,4,5-trifluorophenyl)benzamide (D84)

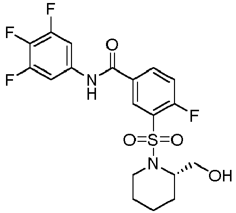

Similarly prepared according to the procedure described for the preparation of compound **D79**, using (S)-Piperidin-2-ylmethanol instead of piperidin-2-ylmethanol, and diisopropylethylamine instead of triethylamine. The title compound **D84** was obtained by purification by flash chromatography on silica (petroleum ether/EtOAc). Method 1; Rt=2.11min, m/z=447.22 (M+H)⁺. ¹HNMR (300 MHz, DMSO-*d*₆) δ ppm 1.01 - 1.18 (m, 1 H) 1.24 - 1.38 (m, 1 H) 1.40 - 1.63 (m, 3 H) 1.72 - 1.88 (m, 1 H) 3.01 - 3.20 (m, 1 H) 3.36 - 3.50 (m, 1 H) 3.51 - 3.64 (m, 1 H) 3.65 - 3.82 (m, 1 H) 3.83 - 3.96 (m, 1 H) 4.61 - 4.91 (m, 1 H) 7.58 - 7.83 (m, 3 H) 8.19 - 8.33 (m, 1 H) 8.41 (dd, *J*=6.69, 2.29 Hz, 1 H) 10.78 (br s, 1 H).

### Description 85: tert-butyl 4-((2-fluoro-5-((3,4,5-trifluorophenyl)carbamoyl)phenyl)sulfonyl)-3-(hydroxymethyl)piperazine-1-carboxylate (D85)

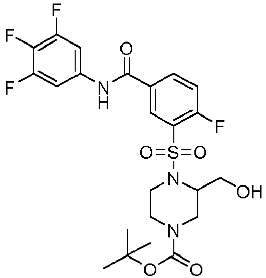

To a solution of **D7** (150mg, 0.41mmol) in MeCN (2.72mL) at 0°C was added DIPEA (0.21mL, 1.22mmol) and 2-methyl-2-propanyl 3-(hydroxymethyl)-1-piperazinecarboxylate (88.23mg, 0.41mmol). The reaction was stirred at room temperature for 10min. The reaction was quenched by addition of water (10mL) and was diluted with DCM (50mL). The organic phase was washed with 5% citric acid acqueous solution and brine, dried over Na₂SO₄, was filtered and concentrated under reduced pressure. The residue was purified by flash cromatography on direct phase (DCM/MeOH), affording the title compound **D85** (190mg).

### Description 86: 4-fluoro-3-[(2-hydroxyethyl)sulfamoyl]-N-(3,4,5-trifluorophenyl)benzamide (D86)

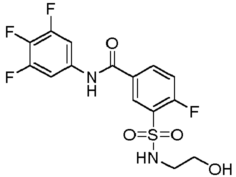

**D7** (200mg, 0.54mmol) was dissolved in MeCN (2mL) and cooled to 0°C. Ethanolamine (0.07mL, 1.09mmol) was added and the reaction mixture was stirred at RT for 1.5h. The reaction was diluted with EtOAc and washed twice with 5% citric acid solution. Organic layer was dried over Na₂SO₄, filtered and concentrated under vacuo. The resulting crude product was purified by flash chromatography on silica (DCM/MeOH) to give the title compound **D86** (165mg) as white solid. Method 3: Rt=3.08min, m/z=393.32 (M+H)⁺. ¹H NMR (300 MHz, DMSO-*d*₆) δ ppm 2.97 (t, *J*=6.30 Hz, 2 H) 3.35 - 3.46 (m, 2 H) 4.66 (br s, 1 H) 7.43 - 7.84 (m, 3 H) 8.14 - 8.32 (m, 1 H) 8.38 (dd, *J*=6.74, 1.79 Hz, 1 H).

### Description 87: 4-fluoro-3-[(3-hydroxypropyl)sulfamoyl]-N-(3,4,5-trifluorophenyl)benzamide (D87)

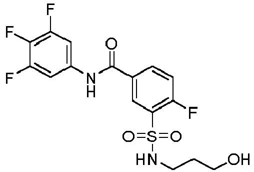

Similarly prepared according to the procedure described for the preparation of compound **D86**, using propanolamine instead of ethanolamine. Method 3: Rt=3.14min, m/z=407.24 (M+H)⁺. ¹H NMR (300 MHz, DMSO-*d*₆) δ ppm 1.44 - 1.62 (m, 2 H) 2.95 (t, *J*=7.24 Hz, 2 H) 3.37 (t, *J*=6.14 Hz, 2 H) 4.48 (br s, 1 H) 7.59 - 7.80 (m, 3 H) 8.02 (br s, 1 H) 8.19 - 8.33 (m, 1 H) 8.38 (dd, *J*=6.88, 2.29 Hz, 1 H) 10.72 (br s, 1 H).

### Description 88: 3-(N-ethyl-N-(4-hydroxybutyl)sulfamoyl)-4-fluoro-N-(3,4,5-trifluorophenyl)benzamide (D88)

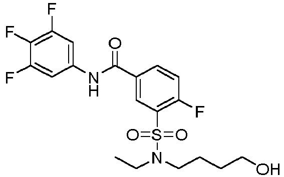

Similarly prepared according to the procedure described for the preparation of compound **D86**, using 4-(ethylamino)butan-1-ol instead of ethanolamine. Method 3: Rt=3.59min, m/z=449.25 (M+H)⁺. ¹H NMR (300 MHz, DMSO-*d*₆) δ ppm 1.05 (t, *J*=7.11 Hz, 3 H) 1.29 - 1.45 (m, 2 H) 1.45 - 1.64 (m, 2 H) 3.19 - 3.31 (m, 4 H) 3.34 - 3.43 (m, 2 H) 4.41 (br s, 1 H) 7.58 - 7.81 (m, 3 H) 8.22 - 8.35 (m, 1 H) 8.41 (dd, *J*=6.74, 2.25 Hz, 1 H) 10.78 (br s, 1 H).

### Description 89: (R)-4-fluoro-3-(N-(1-hydroxy-4-methylpentan-2-yl)sulfamoyl)-N-(3,4,5-trifluorophenyl)benzamide (D89)

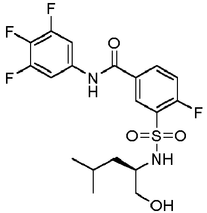

Similarly prepared according to the procedure described for the preparation of compound **D86**, using (R)-2-amino-4-methylpentan-1-ol instead of ethanolamine. Method 3: Rt=3.69min, m/z=449.12 (M+H)⁺. ¹H NMR (300 MHz, DMSO-*d*₆) δ ppm 0.61 (d, *J*=6.42 Hz, 3 H) 0.79 (d, *J*=6.60 Hz, 3 H) 1.10 - 1.40 (m, 2 H) 1.43 - 1.65 (m, 1 H) 3.11 - 3.23 (m, 2 H) 3.23 - 3.30 (m, 1 H) 4.60 (br s, 1 H) 7.55 - 7.80 (m, 3 H) 7.99 (br s, 1 H) 8.18 - 8.32 (m, 1 H) 8.41 (dd, *J*=6.88, 2.29 Hz, 1 H) 10.78 (br s, 1 H).

### Description 90: 4-fluoro-3-[(2-hydroxyethyl)(methyl)sulfamoyl]-N-(3,4,5-trifluorophenyl)benzamide (D90)

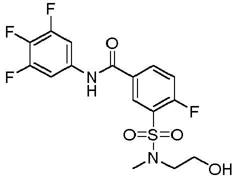

**D7** (80mg, 0.22mmol) was dissolved in MeCN (1mL) and cooled to 0°C. 2-(methylamino)ethanol hydrochloride (48.55mg, 0.440mmol) and triethylamine (0.12mL, 0.870mmol) were added and the reaction mixture was stirred at RT for 1h. The reaction was diluted with EtOAc and washed twice with 5% citric acid solution two times. Organic layer was dried over Na₂SO₄, filtered and concentrated under vacuo. The resulting crude product was purified by flash chromatography on silica (DCM/MeOH), then lyophilized, to give the title compound **D90** (68mg) as white solid. Method 3: Rt=3.30min, m/z=407.17 (M+H)⁺. ¹H NMR (300 MHz, DMSO-*d*₆) δ ppm 2.80 - 2.93 (m, 3 H) 3.13 - 3.25 (m, 2 H) 3.44 - 3.62 (m, 2 H) 4.80 (t, *J*=5.30 Hz, 1 H) 7.53 - 7.81 (m, 3 H) 8.21 - 8.34 (m, 1 H) 8.39 (dd, *J*=6.69, 2.29 Hz, 1 H) 10.79 (br s, 1 H).

### Description 91: 4-amino-3-(N-(2-hydroxyethyl)sulfamoyl)-N-(3,4,5-trifluorophenyl)benzamide (D91)

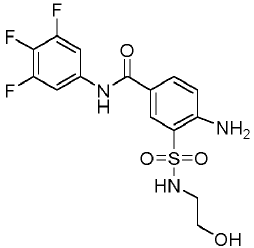

In a vial (5 mL) **D86** (52.55 mg, 0.134 mmol) was weighed and dissolved in 1,4-dioxane (0.4 mL), the vial was sealed. Under stirring ammonia solution 30% (0.3 mL) were added and the mixture was heated at 90 °C. The reaction was carried out for 24h. The solvent was removed under vacuum giving the title compound **D91** (48 mg) as white solid. Method 9: Rt=1.80 min, m/z=390.21 (M+H)⁺.

### Description 92: 4-amino-3-(N-(3-((tert-butyldimethylsilyl)oxy)cyclobutyl)sulfamoyl)-N-(3,4,5-trifluorophenyl)benzamide (D92)

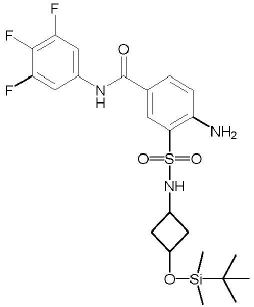

To a stirred solution of **D129** (237mg, 0.57mmol) and DIPEA (198.8uL, 1.14mmol) in 9/1 DCM/DMF (10mL) at 0°C was added portionwise tert-butyldimethylsilyl trifluoromethanesulfonate (144.1uL, 0.63mmol) over 2min. The resulting solution was stirred at 0°C for 5min. The reaction was diluted with DCM and water, poured into a separating funnel, washed with 5% citric acid aq. solution, dried over Na₂SO₄ filtered and finally evaporated yielding the title compound **D92** (248mg, 0.468mmol) as mixture of two diastereoisomers. (1/1 cis/trans). Isomer1: Method 11; Rt: 2.23min. m/z: 530.3(M+H)⁺. Isomer2: Method 11; Rt: 2.27min. m/z: 530.4(M+H)⁺.

### Description 93: 2-(3-((tert-butyldimethylsilyl)oxy)cyclobutyl)-3-oxo-N-(3,4,5-trifluorophenyl)-3,4-dihydro-2H-benzo[e][1,2,4]thiadiazine-7-carboxamide 1,1-dioxide (D93)

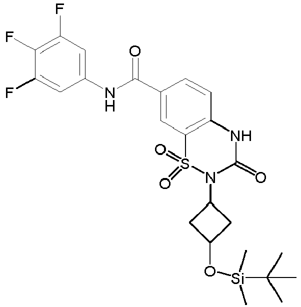

A solution of **D92** (70mg, 0.13mmol) and di(1H-imidazol-1-yl)methanone (171.44mg, 1.06mmol) in DMF (1mL) was treated with a single portion of triethylamine (84.91uL, 0,53mmol) and heated by microwave irradiation at 150°C for 15min. The reaction mixture was diluted with water until precipitation; the slurry was acidified with 5% citric acid aq. solution and extracted with diethylether. The organic layer was dried over Na₂SO₄, filtered and finally evaporated giving the title compound **D93** (55mg, 0.099mmol) as 1/1 mixture of two diastereoisomer (cis/trans). Isomer1: Method 11; Rt: 2.50min. m/z: 556.31(M+H)⁺. Isomer2: Method 11; Rt: 2.58min. m/z: 556.24(M+H)⁺.

### Description 94: cis-2-(4-((tert-butyldimethylsilyl)oxy)cyclohexyl)-3-oxo-N-(3,4,5-trifluorophenyl)-3,4-dihydro-2H-benzo[e][1,2,4]thiadiazine-7-carboxamide 1,1-dioxide (D94)

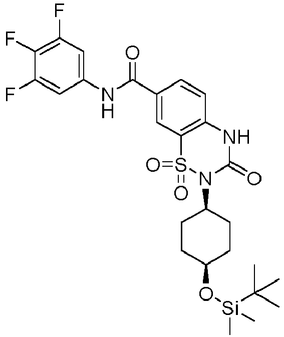

A mixture of **D49** (92mg, 0.16mmol) and di(1H-imidazol-1-yl)methanone (160,5mg, 0.99mmol) in DMF (1.4mL) was treated with triethylamine (0.69mL, 0.49mmol) and heated by microwave irradiation at 150°C for 1h10min. The reaction mixture was cooled to room temperature and EtOAc and water were added. Organic layer was washed with 5% citric acid solution and water, then was dried over Na₂SO₄, filtered and concentrated under vacuo, to obtain crude the title compound **D94** (94mg) that was used in the next step without further purification. Method 11; Rt: 2.74min. m/z: 584.27 (M+H)⁺.

### Description 95: N-(6-chloropyridin-3-yl)-2H-benzo[e][1,2,4]thiadiazine-7-carboxamide 1,1-dioxide (D95)

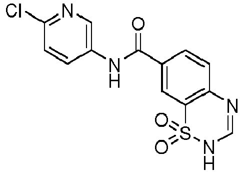

**D60** (22.14 mg, 0.09 mmol) and 6-chloropyridin-3-amine (14.1 mg, 0.11 mmol) were dissolved in dry THF (867 uL) under nitrogen flow and lithium bis(trimethylsilyl)amide (503 uL, 0,51 mmol) was added. The reaction mixture was stirred at room temperature for 1h. Lithium bis(trimethylsilyl)amide (503 uL, 0,51 mmol) was added and the stirring continued for further 3 h. A sat. aq. NH₄Cl solution was added to the reaction mixture and extracted with EtOAc (three times with 25 ml). The combined organic layers were dried on Na₂SO₄, filtered and evaporated under reduced pressure. The crude compound was purified by preparative HPLC (H₂O/CH₃CN+1%TFA) to afford, after lyophilization, the title compound **D95** (3 mg). Method 3; Rt=2.41min, m/z=337.11 (M+H)⁺.

### Description 96: 5-fluoro-N-(3,4,5-trifluorophenyl)-2H-benzo[e][1,2,4]thiadiazine-7-carboxamide 1,1-dioxide (D96)

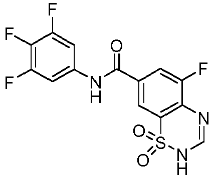

**D27** (207mg, 0,530mmol) was dissolved in DMSO (5mL), cesium carbonate (686mg, 2.11mmol) was added and the reaction mixture was heated at 100°C for 30min under MW irradiation. Mixture was diluted with water and filtered to recover the title compound **D96** (130mg) as white solid. Method 1; Rt=1.87min, m/z=374.07 (M+H)⁺. ¹H NMR (300 MHz, DMSO-*d*₆) δ ppm 7.46 (s, 1 H) 7.67 - 7.84 (m, 3 H) 8.13 - 8.25 (m, 1 H) 10,57 (br s, 1 H).

### Description 97: trans-2-(4-((tert-butyldimethylsilyl)oxy)cyclohexyl)-6-methyl-3-oxo-N-(3,4,5-trifluorophenyl)-3,4-dihydro-2H-benzo[e][1,2,4]thiadiazine-7-carboxamide 1,1-dioxide (D97)

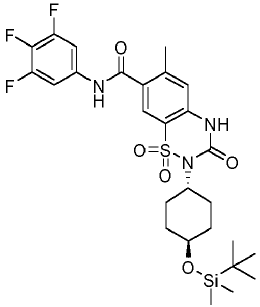

Similary prepared according to the procedure described for the preparation of compound **D94** using compound **D120** as starting material; m/z=374.07 (M+H)⁺.

### Description 98: 4-(methylamino)-3-sulfamoyl-N-(3,4,5-trifluorophenyl)benzamide (D98)

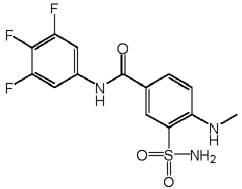

**D22** (58.mg, 0.170mmol) was dissolved in DMSO (1mL, 0.014mol) and treated with methanamine (77.59mg, 2.5mmol) and Triethylamine (346.27uL, 2.5mmol). The reaction solution was stirred overnight at room temperature. A second portion of methanamine (77.59mg, 2.5mmol) was added followed by triethylamine (346.27uL, 2.5mmol). After 5hrs at room temperature MeCN (200uL) were added and the reaction was stirred overnight at room temperature. The reaction was diluted with water and DCM, poured into a separating funnel and the organic layer washed twice with water and brine, dried over MgSO₄ (dry), filtered and finally evaporated yielding a yellowish solid (72mg). DCM (1mL) was added and the resulting white suspension was filtered yielding the title compound **D98** as white solid (33 mg). Method 3; Rt: 3.27min. MS(ES+) m/z: 360.15 (M+H)⁺. ¹H NMR (300 MHz, DMSO-*d*₆) δ ppm 2.93 (d, J=4.86 Hz, 3 H) 6.30 - 6.47 (m, 1 H) 6.86 (d, J=8.89 Hz, 1 H) 7.43 (br s, 2 H) 7.64 - 7.86 (m, 2 H) 8.06(dd, J=8.76, 2.06 Hz, 1 H) 8.33 (d, J=2.20 Hz, 1 H) 10.41 (br s, 1 H)

### Description 99: 4-amino-3-(N-methylsulfamoyl)-N-(3,4,5-trifluorophenyl)benzamide (D99)

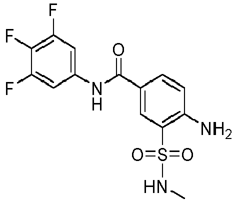

To a solution of crude **D26** (151 mg) in 1,4-dioxane (1.04 mL), Aqueous ammonia (517 uL, 13.27 mmol) was added and the reaction mixture was stirred in a sealed vial at 90°C for 2h, and overnight at room temperature. The solvent was evaporated under reduced pressure. The crude product (40.06 mg) was purified by preparative HPLC (H₂O/CH₃CN+1%TFA) to afford, after lyophilization, the title compound **D99** (17.25 mg) as white powder. The remaining crude title compound was used without purification. Method 3; Rt: 3.23 min. m/z: 360.15 (M+H)⁺. ¹H NMR (300 MHz, DMSO-*d*₆) δ ppm 2.44 (d, *J*=5.10 Hz, 3 H) 6.57 (br s, 2 H) 6.95 (d, *J*=8.70 Hz, 1 H) 7.50 - 7.59 (m, 1 H) 7.68 - 7.81 (m, 2 H) 0.00 (dd, *J*=9.80, 2.10 Hz, 1 H) 0.00 (d, *J*=2.10 Hz, 1 H) 10.36 (br s, 1 H)

### Description 100: 4-amino-3-sulfamoyl-N-(3,4,5-trifluorophenyl)benzamide (D100)

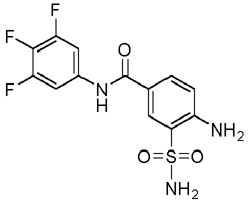

A pressure vessel was charged with **D7** (1.2g, 3.26mmol), 1,4-dioxane (6mL) and 33% aqueous ammonia (4mL, 34mmol). The pressure vessel was sealed and the reaction mixture was stirred 5min at RT, then heated at 95°C for 8h. The reaction was diluted with EtOAc and water, organic layer was dried over Na₂SO₄, filtered and concentrated under vacuo. The resulting crude product was purified by preparative HPLC (H₂O/CH₃CN+1%TFA) to obtain, after lyophilization, the title compound **D100** (840mg) as white solid. Method 3; Rt=3.01min, m/z=346.10 (M+H)⁺. ¹H NMR (300 MHz, DMSO-*d*₆) δ ppm 6.49 (br s, 2 H) 6.88 (d, *J*=8.71 Hz, 1 H) 7.37 (s, 2 H) 7.62 - 7.81 (m, 2 H) 7.88 (dd, *J*=8.80, 2.20 Hz, 1 H) 8.26 (d, *J*=2.11 Hz, 1 H) 10.33 (s, 1 H).

### Description 101: 4-amino-N-(3,4-difluorophenyl)-3-sulfamoylbenzamide (D101)

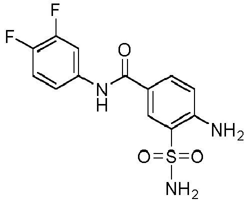

Similarly prepared according to the procedure described for the preparation of **D100**, starting from **D8.** Method 3; Rt=2.79min, m/z=328.20 (M+H)⁺. ¹H NMR (300 MHz, DMSO-*d*₆) δ ppm 6.49 (br s, 2 H) 6.88 (d, *J*=8.71 Hz, 1 H) 7.37 (s, 2 H) 7.62 - 7.81 (m, 2 H) 7.88 (dd, *J*=8.80, 2.20 Hz, 1 H) 8.26 (d, *J*=2.11 Hz, 1 H) 10.33 (s, 1 H).

### Description 102: 4-amino-2-chloro-5-sulfamoyl-N-(3,4,5-trifluorophenyl)benzamide (D102)

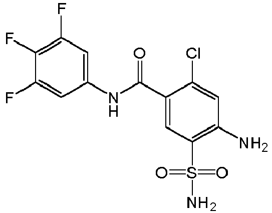

**D23** (250mg, 0.65mmol) was dissolved in 1,4-Dioxane (0,5mL), treated with ammonia (2.mL, 117.44mmol) and heated at 90°C in a closed vial for 12hrs. The yellowish reaction solution was poured, diluted with EtOAc (aprox. 20mL) and water. The organic layer was washed with brine and 0.2N HCl (2mL) then evaporated, giving a residue (220 mg). One amount (20mg) was purified by preparative HPLC (H₂O/CH₃CN+1%TFA) yielding, the title compound **D102** (11mg) as white powder. Method 3; Rt: 3.21min. m/z: 380.15 (M+H)⁺. ¹H NMR (300 MHz, DMSO-*d*₆) δ ppm 6.46 (br s, 2 H) 6.96 (s, 1 H) 7.46 (br s, 2 H) 7.53 - 7.70 (m, 2 H) 7.76 (br s, 1 H) 10.69 (br s, 1 H).

### Description 103: 4-amino-2-bromo-5-sulfamoyl-N-(3,4,5-trifluorophenyl)benzamide (D103)

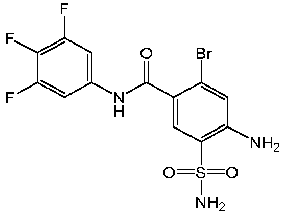

Similary prepared according to procedure described for **D102**, starting from **D24** 2-bromo-4-fluoro-5-sulfamoyl-N-(3,4,5-trifluorophenyl)benzamide and purified by preparative HPLC (H₂O,CH₃CN, 0.1% HCOOH). Method 3; Rt: 3.24min. m/z: 424.12 (M+H)⁺. ¹H NMR (300 MHz, DMSO-*d*₆) δ ppm 6.42 (s, 2 H) 7.14 (s, 1 H) 7.47 (br s, 2 H) 7.60 (dd, *J*=10.22, 6.56 Hz, 2 H) 7.71 (s, 1 H) 10.73 (br s, 1 H).

### Description 104: 4-amino-N-(4-fluoro-3-(trifluoromethyl)phenyl)-3-sulfamoylbenzamide (D104)

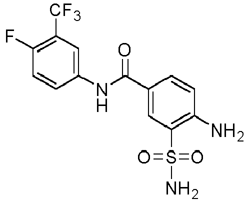

Similarly prepared according to the procedure described for the preparation of compound **D100**, starting from **D12**. Method 3; Rt=3.16min, m/z=378.12 (M+H)⁺. ¹H NMR (300 MHz, DMSO-d6) δ ppm 6.47 (s, 2 H) 6.88 (d, J=8.62 Hz, 1 H) 7.36 (s, 2 H) 7.50 (t, J=9.90 Hz, 1 H) 7.91 (dd, J=8.67, 2.15 Hz, 1 H) 8.05 - 8.13 (m, 1 H) 8.22 (dd, J=6.69, 2.48 Hz, 1 H) 8.29 (d, J=2.11 Hz, 1 H) 10.34 (s, 1 H).

### Description 105: 4-amino-N-(3-cyano-4-fluorophenyl)-3-sulfamoylbenzamide (D105)

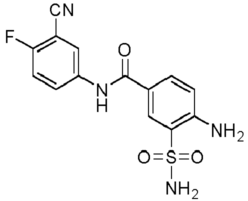

Similarly prepared according to procedure described for the preparation of **D100**, starting from **D13**. Method 3; Rt=2.65min, m/z=334.95, (M+H)⁺. 1H NMR (300 MHz, DMSO-*d*₆) δ ppm 6.44 (br s, 2 H) 6.88 (d, *J*=8.71 Hz, 1 H) 7.37 (s, 2 H) 7.53 (t, *J*=9.17 Hz, 1 H) 7.90 (dd, *J*=8.62, 2.11 Hz, 1 H) 7.99 - 8.13 (m, 1 H) 8.25 (dd, *J*=5.78, 2.66 Hz, 1 H) 8.28 (d, *J*=2.11 Hz, 1 H) 10.36 (s, 1 H).

### Description 106: 4-amino-N-(3-(difluoromethyl)-4-fluorophenyl)-3-sulfamoylbenzamide (D106)

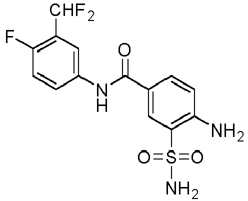

Similarly prepared according to procedure described for the preparation of **D100**, starting from **D14**. Method 3; Rt=2.85, m/z=360.08 (M+H)⁺. 1H NMR (300 MHz, DMSO-d6) δ ppm 6.45 (br s, 2 H) 6.87 (d, J=8.62 Hz, 1 H) 7.01 - 7.45 (m, 4 H) 7.86 - 7.98 (m, 2 H) 8.03 - 8.12 (m, 1 H) 8.28 (d, J=2.11 Hz, 1 H) 10.25 (s, 1 H).

### Description 107: 4-amino-N-(3-chloro-4-fluorophenyl)-3-sulfamoylbenzamide (D107)

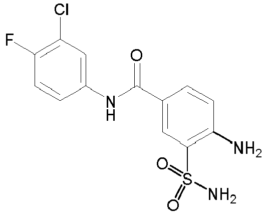

A mixture of **D25** (200.mg, 0,580mmol) 1,4-dioxane (2mL) and aqueous ammonia (2.3mL, 10.38mmol) were heated in a closed vial at 100°C for 10hrs. Solvent was removed, the residue suspended in DCM and filtered giving a off-white solid (110mg). One half of this crude product was purified by preparative HPLC (H₂O/CH₃CN+1%TFA) giving the title compound **D107** (8.4mg)as off-white solid. Method 3; Rt: 2.98min. m/z: 344.07 (M+H)⁺. ¹H NMR (300 MHz, DMSO-*d*₆) δ ppm 6.45 (br s, 2 H) 6.87 (d, *J*=8.71 Hz, 1 H) 7.31 - 7.43 (m, 3 H) 7.64 - 7.77 (m, 1 H) 7.89 (dd, *J*=8.67, 2.16 Hz, 1 H) 8.05 (dd, *J*=6.92, 2.52 Hz, 1 H) 8.26 (d, *J*=2.11 Hz, 1 H) 10.20 (s, 1 H).

### Description 108: 4-amino-N-(6-chloropyridin-3-yl)-3-sulfamoylbenzamide (D108)

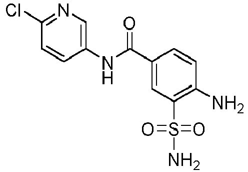

Similarly prepared according to procedure described for the preparation of **D100** starting from **D15**. Method 3; Rt=2.33min, m/z=327.05 (M+H)⁺. 1H NMR (300 MHz, DMSO-d6) δ ppm 6.49 (br s, 2 H) 6.88 (d, J=8.71 Hz, 1 H) 7.37 (s, 2 H) 7.50 (d, J=8.71 Hz, 1 H) 7.91 (dd, J=8.62, 2.02 Hz, 1 H) 8.23 (dd, J=8.71, 2.75 Hz, 1 H) 8.29 (d, J=2.02 Hz, 1 H) 8.77 (d, J=2.57 Hz, 1 H) 10.35 (s, 1 H).

### Description 109: 4-amino-N-(4-fluoro-3-methylphenyl)-3-sulfamoylbenzamide (D109)

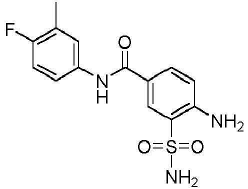

Similarly prepared according to procedure described for the preparation of **D100**, starting from **D17**. Method 3; Rt=2.83, m/z=324.14 (M+H)⁺.

### Description 110: 4-amino-N-(3,5-difluoro-4-methylphenyl)-3-sulfamoylbenzamide (D110)

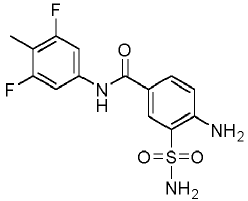

Similarly prepared according to procedure described for the preparation of **D100**, starting from **D18**. Method 3; Rt=3.17, m/z=342.25 (M+H)⁺.

### Description 111: 4-amino-3-sulfamoyl-N-(2,3,4-trifluorophenyl)benzamide (D111)

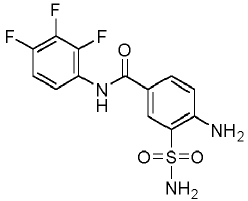

Similarly prepared according to procedure described for the preparation of **D100**, starting from **D19**. Method 3; Rt=2.65, m/z=346.17 (M+H)⁺.

### Description 112: 4-amino-3-sulfamoyl-N-(2,4,5-trifluorophenyl)benzamide (D112)

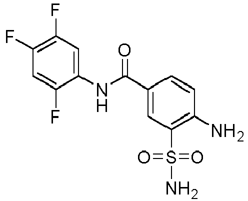

Similarly prepared according to procedure described for the preparation of **D100**, starting from **D20**. Method 3; Rt=2.68, m/z=346.17 (M+H)⁺.

### Description 113: 4-amino-N-(2-chloro-4-fluorophenyl)-3-sulfamoylbenzamide (D113)

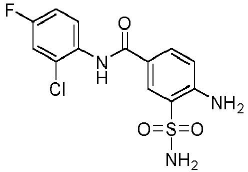

Similarly prepared according to procedure described for the preparation of **D100**, starting from **D21**.

### Description 114: 4-amino-2-methyl-5-sulfamoyl-N-(3,4,5-trifluorophenyl)benzamide (D114)

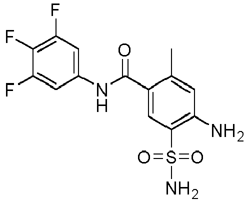

**D16** (150mg, 0.390mmol) was suspended in 1,4-Dioxane (1mL) Aqueous ammonia (0.96mL, 24.6mmol) was added and the reaction mixture was stirred at 100°C for 8h and overnight at RT. More Aqueous ammonia (0.3mL, 7.69mmol) was added and the reaction mixture was stirred at 100°C for another 8h. The reaction was diluted with EtOAc and water, organic layer was dried over Na₂SO₄, filtered and concentrated under vacuo. The resulting crude were purified by preparative HPLC (H₂O/CH₃CN+1% TFA) to obtain, after lyophilization, the title compound **D114** (110mg) as white solid. Method 3; Rt=3.16min, m/z=360.22 (M+H)⁺. ¹H NMR (300 MHz, DMSO-*d*₆) δ ppm 2.32 (s, 3 H) 6.22 (br s, 2 H) 6.68 (s, 1 H) 7.26 (s, 2 H) 7.56 - 7.70 (m, 2 H) 7.74 (s, 1 H) 10.49 (s, 1 H).

### Description 115: (R)-4-amino-N-(3,4,5-trifluorophenyl)-3-(N-(1,1,1-trifluoropropan-2-yl)sulfamoyl)benzamide (D115)

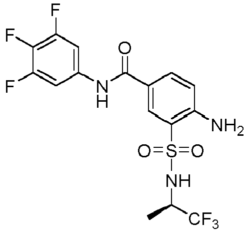

Similarly prepared according to procedure described for the preparation of **D100**, starting from **D31**. Method 3; Rt: 3.75min, m/z: 442.16 (M+H)⁺. ¹H NMR (300 MHz, DMSO-*d*₆) δ ppm 1.06 (d, *J*=6.88 Hz, 3 H) 3.95 - 4.03 (m, 1 H) 6.56 (br s, 2 H) 6.92 (d, *J*=8.71 Hz, 1 H) 7.58 - 7.80 (m, 2 H) 7.91 (dd, *J*=8.71, 2.20 Hz, 1 H) 8.25 (d, *J*=2.11 Hz, 1 H) 8.55 (d, *J*=9.17 Hz, 1 H) 10.35 (s, 1 H).

### Description 116: (S)-4-amino-N-(3,4,5-trifluorophenyl)-3-(N-(1,1,1-trifluoropropan-2-yl)sulfamoyl)benzamide (D116)

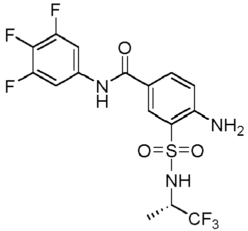

Similarly prepared according to procedure described for the preparation of **D100**, starting from **D32**. Method 3; Rt: 3.75min, m/z: 442.16 (M+H)⁺. ¹H NMR (300 MHz, DMSO-*d*₆) δ ppm 1.06 (d, *J*=6.88 Hz, 3 H) 3.95 - 4.00 (m, 1 H) 6.57 (br s, 2 H) 6.92 (d, *J*=8.80 Hz, 1 H) 7.63 - 7.78 (m, 2 H) 7.91 (dd, *J*=8.80, 2.20 Hz, 1 H) 8.25 (d, *J*=2.20 Hz, 1 H) 8.55 (d, *J*=9.17 Hz, 1 H) 10.35 (s, 1 H).

### Description 117: 4-amino-3-(N-cyclopropylsulfamoyl)-N-(3,4,5-trifluorophenyl)benzamide (D117)

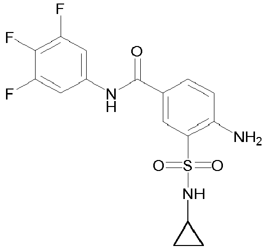

A solution of **D33** (125mg, 0.32mmol) in aqueous ammonia (0.38mL, 3.22mmol)and 1,4-dioxane (1.1mL, 0.013mol) was heated at 100°C for 8hrs in a closed vial. Solvent was removed *in vacuo,* the residue was treated with toluene and further evaporated by high vacuum pump, giving a residue (107mg, off-white solid). A sample of this crude material (24.5mg) was purified by preparative HPLC (H₂O/CH₃CN+1%TFA) to afford, after lyophilization the title compound **D117** (13.2mg) as white solid. Method 3; Rt: 3.55min. m/z: 386.23 (M+H)⁺. ¹H NMR (300 MHz, DMSO-*d*₆) δ ppm 0.35 - 0,50 (m, 4 H) 2.11 (td, *J*=6.51, 3.12 Hz, 1 H) 6.54 (br s, 2 H) 6.91 (d, *J*=8.71 Hz, 1 H) 7.64 - 7.80 (m, 2 H) 7.88 (dd, *J*=8.70, 2.00 Hz, 1 H) 7.95 (dd, *J*=2.50 Hz, 1 H) 8.25 (d, *J*=2.11 Hz, 1 H) 10.35 (br s, 1 H).

### Description 118: trans-4-(1,1-dioxido-3-oxo-7-((3,4,5-trifluorophenyl)carbamoyl)-3,4-dihydro-2H-benzo[e][1,2,4]thiadiazin-2-yl)cyclohexyl 1H-imidazole-1-carboxylate (D118)

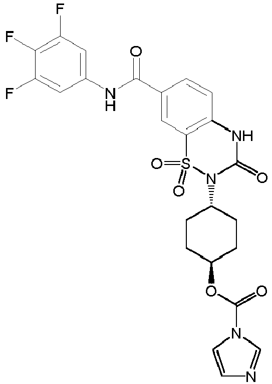

A mixture of **D61** (40mg, 0.09mmol) and di(1H-imidazol-1-yl)methanone (58.51mg, 0.36mmol) in DMF (540uL) was heated by microwave irradiation at 150°C for 11min. The reaction mixture (a solution) was diluted with water, extracted with EtOAc, dried over Na₂SO₄, filtered and finally evaporated giving the title compound **D118** (50mg), as orange gum. Method 1; Rt: 1.87min. m/z: 564.08(M+H)⁺.

### Description 119: cis-4-amino-3-(N-(4-((tert-butyldimethylsilyl)oxy)cyclohexyl)sulfamoyl)-N-(3,4,5-trifluorophenyl)benzamide (D119)

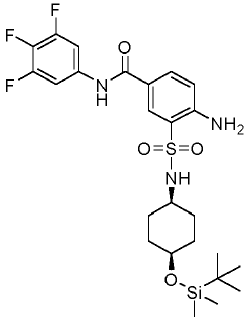

**D49** (138mg, 0.31mmol) was reacted in dry DCM (5mL) under a nitrogen atmosphere. Dry DMF (0.3mL) and DIPEA (0.11mL, 0.62mmol were added and the mixture was cooled in an ice bath. Tert-butyldimethylsilyl trifluoromethanesulfonate (0.11mL, 0.47mmol) was added dropwise. The reaction was stirred at RT for 1.5h, then was diluted with DCM and washed with 5% citric acid solution and brine. The organic layer was dried over Na₂SO₄, filtered and evaporated. The resulting crude product was purified by flash chromatography on silica (PE/EtOAc) to obtain the title compound **D119** (132 mg) as white solid. Method 11; Rt=2.51min, m/z=558.27 (M+H)⁺.

### Description 120: trans-4-amino-5-(N-(4-((tert-butyldimethylsilyl)oxy)cyclohexyl)sulfamoyl)-2-methyl-N-(3,4,5-trifluorophenyl)benzamide (D120)

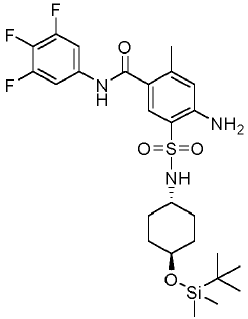

**D50** (103mg, 0.230mmol) was dissolved in DMF (1.2mL)1H-imidazole (46mg, 0.68mmol) and tert-butylchlorodimethylsilane (50.9mg, 0.34mmol)were added, and the reaction mixture was stirred at RT for 1.5h. The reaction was diluted with EtOAc and washed with water, 5% citric acid solution and brine. Organic layer was dried over Na₂SO₄, filtered and concentrated under vacuo. The resulting crude was purified by flash chromatography on silica (PE/EtOAc) to obtain **D120** (105mg) as white solid. Method 9; Rt: 3.07min. m/z: 572.32 (M+H)⁺.

### Description 121: cis-4-amino-3-(N-3-hydroxycyclobutyl)sulfamoyl)-N-(3,4,5-trifluorophenyl)benzamide (D121)

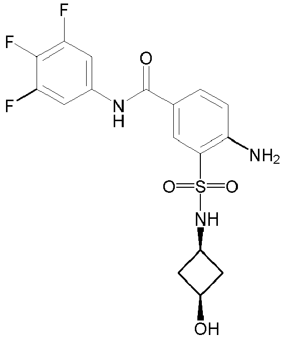

*Cis*-4-fluoro-3-(N-(3-hydroxycyclobutyl)sulfamoyl)-N-(3,4,5-trifluorophenyl)benzamide was prepared from **D7** according to the procedure described for the synthesis of **D45**, using *cis*-3-aminocyclobutanol instead of 3-aminocyclobutanol. The intermediate compound was further reacted with aqueous ammonia in 1,4-dioxane according to the procedure described for the preparation of **D107**. Method 3; Rt: 3.12. m/z: 416.29 (M+H)⁺.

### Description 122: trans-4-amino-3-(N-3-hydroxycyclobutyl)sulfamoyl)-N-(3,4,5-trifluorophenyl)benzamide (D122)

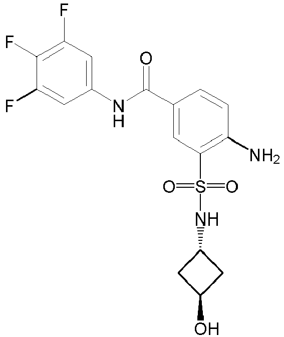

*Trans*-4-fluoro-3-(N-3-yroxycycobutyl)sulfamoyl)-N-(3,4,5-trifluorophenyl)benzamide was prepared from **D7** according to the procedure described for the synthesis of **D45**, using *trans* 3-aminocyclobutanol instead of 3-aminocyclobutanol. The intermediate compound was further reacted with aqueous ammonia in 1,4-dioxane according to the procedure described for the preparation of **D107**. Method 3; Rt: 3.04. m/z: 416.35 (M+H)⁺.

### Description 123: 4-amino-3-(N-((1S,3S)-3-hydroxycyclopentyl)sulfamoyl)-N-(3,4,5-trifluorophenyl)benzamide (D123)

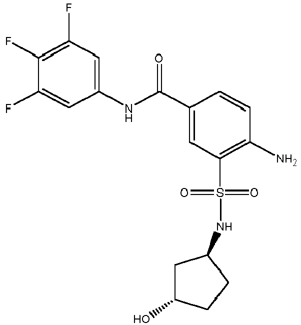

A solution of **D46** (105mg, 0.24mmol) in aqueous ammonia (0.5mL,4.6mmol) and 1,4-Dioxane (0.5mL) was heated for 8hrs at 100°C in a closed vial. The reaction solution was diluted with DCM/EtOAc (about 7/3) and water, then the organic layer was evaporated giving a residue (80mg) that was purified by preparative HPLC (H₂O/CH₃CN+0.1%TFA).Method 3; Rt: 3.11. m/z: 430.27 (M+H)⁺.

### Description 124: tert-butyl 4-((2-amino-5-((3,4,5-trifluorophenyl)carbamoyl)phenyl)sulfonamido)piperidine-1-carboxylate (D124)

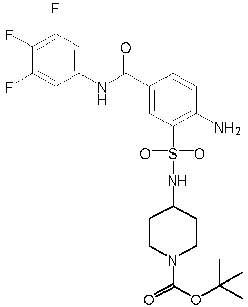

A solution of **D38** (147mg, 0.28mmol) in aqueous ammonia (0.99mL, 2.77mmol) and 1,4-dioxane (1.1mL) was heated at 100°C for 8hrs in a closed vial. Solvent was removed *in vacuo,* the residue was treated with toluene and further evaporated. Traces of solvent were removed by high vacuum pump, giving a residue as off-white solid (107mg). A sample of this crude material (20mg) was purified by preparative HPLC (H₂O/CH₃CN+1%TFA) affording the title compound **D124** (19.86mg) as white solid. Method 3; Rt: 3.93min. m/z: 529.09 (M+H)⁺. ¹H NMR (300 MHz, DMSO-*d*₆) δ ppm 1.13 - 1.32 (m, 2 H) 1.36 (s, 9 H) 1.47 - 1.65 (m, 2 H) 2.75 - 2.95 (m, 2 H) 3.05 - 3.23 (m, 1 H) 3.67 (br d, *J*=13.57 Hz, 2 H) 6.53 (br s, 2 H) 6.89 (d, *J*=8.71 Hz, 1 H) 7.64 - 7.78 (m, 2 H) 7.82 (d, *J*=7.89 Hz, 1 H) 7.90 (dd, *J*=8.67, 2.15 Hz, 1 H) 8.24 (d, *J*=2.11 Hz, 1 H) 10.34 (br s, 1 H).

### Description 125: 4-amino-3-(N-(piperidin-4-yl)sulfamoyl)-N-(3,4,5-trifluorophenyl)benzamide (D125)

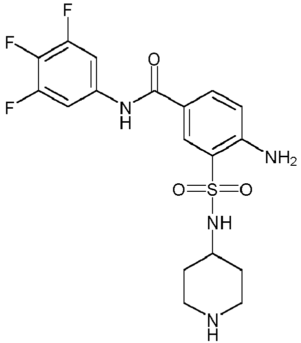

A solution of **D124** in DCM (1mL) was treated at room temperature with TFA (1mL). The yellow reaction solution was magnetically stirred at room temperature for 1h. Solvent was removed *in vacuo* and the residue purified by preparative HPLC (H₂O/CH₃CN+1‰TFA) affording the title compound **D125** (8.6mg) as TFA salt. ¹H NMR (300 MHz, DMSO-*d*₆) δ ppm 1.40 - 1.64 (m, 2 H) 1.64 - 1.87 (m, 2 H) 2.78 - 3.03 (m, 2 H) 3.08 - 3.28 (m, 3 H) 6.54 (br s, 2 H) 6.91 (d, *J*=8.71 Hz, 1 H) 7.72 (dd, *J*=10,59, 6.56 Hz, 2 H) 7.91 (dd, *J*=8.71, 2.11 Hz, 1 H) 8.03 (d, *J*=7.61 Hz, 1 H) 8.17 (br s, 1 H) 8.25 (d, *J*=2.11 Hz, 1 H) 8.41 (br s, 1 H) 10.36 (br s, 1 H). ¹H NMR (300 MHz, DMSO-d*₆*+TFA) δ ppm 1.43 - 1.64 (m, 2 H) 1.75 (br dd, *J*=13.75, 3.30 Hz, 2 H) 2.79 - 3.00 (m, 2 H) 3.08 - 3.22 (m, 2 H) 3.22 - 3.38 (m, 1 H) 6.91 (d, *J*=8.71 Hz, 1 H) 7.61 - 7.81 (m, 2 H) 7.91 (dd, *J*=8.71, 2.20 Hz, 1 H) 8.03 (d, *J*=7.52 Hz, 1 H) 8.13 - 8.34 (m, 2 H) 8.35 - 8.60 (m, 1 H) 10.35 (s, 1 H). Method 3; Rt: 2.48min. m/z: 429.26 (M+H)⁺.

### Description 126: 4-amino-3-((4-hydroxypiperidin-1-yl)sulfonyl)-N-(3,4,5-trifluorophenyl)benzamide (D126)

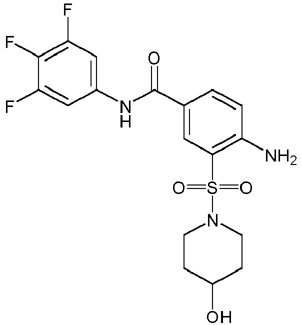

A mixture of 4-fluoranyl-3-(4-oxidanylpiperidin-1-yl)sulfonyl-N-[3,4,5-tris(fluoranyl)phenyl]benzamide (prepared according to WO2013/096744) (50mg, 0.11mmol), 1,4-dioxane (150uL) and aqueous ammonia (0.3mL, 2.29mmol) were heated at 100°C for 8hrs in a sealed tube. The reaction was diluted with EtOAc and water, organic layer was dried over Na₂SO₄, filtered and finally evaporated. The residue was suspended in DCM and filtered affording the title compound **D126** (15.5mg) as white solid. Method 3; Rt: 3.26min. m/z: 430.2 (M+H)⁺. ¹H NMR (300 MHz, DMSO-*d*₆) δ ppm 1.30 - 1.52 (m, 2 H) 1.60 - 1.83 (m, 2 H) 2.77 - 2.92 (m, 2 H) 3.25 (br s, 2 H) 3.45 - 3.65 (m, 1 H) 4.67 (d, *J*=3.76 Hz, 1 H) 6.53 - 6.75 (m, 2 H) 6.93 (d, *J*=8.71 Hz, 1 H) 7.69 (dd, *J*=10,50, 6.46 Hz, 2 H) 7.81 - 7.98 (m, 1 H) 8.08 (d, *J*=2.02 Hz, 1 H) 10.31 (s, 1 H)

### Description 127: 3-((4-hydroxypiperidin-1-yl)sulfonyl)-4-(methylamino)-N-(3,4,5-trifluorophenyl)benzamide (D127)

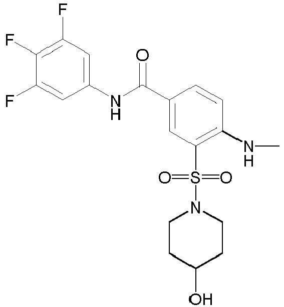

A mixture of 4-fluoranyl-3-(4-oxidanylpiperidin-1-yl)sulfonyl-N-[3,4,5-tris(fluoranyl)phenyl]benzamide (prepared according to WO2013/096744) (50mg, 0.12mmol), methanamine (1.04mL, 2.08mmol) in a DMSO (700uL,0.010mol) and MeCN (140uL,0.003mol) mixture was treated with triethylamine (480.87uL, 3.47mmol) and stirred at room temperature overnight. The reaction solution was purified by preparative HPLC (H₂O/CH₃CN+1%TFA) affording the title compound **D127** (23mg). ¹H NMR (300 MHz, DMSO-*d*₆) δ ppm 1.28 - 1.51 (m, 2 H) 1.58 - 1.81 (m, 2 H) 2.75 - 2.97 (m, 5 H) 3.19 - 3.28 (m, 2 H) 3.46 - 3.65 (m, 1 H) 4.66 (d, *J*=3.90 Hz, 1 H) 6.72 - 6.81 (m, 1 H) 6.89 (d, *J*=8.90 Hz, 1 H) 7.63 - 7.77 (m, 2 H) 8.03 - 8.11 (m, 1 H) 8.15 (d, *J*=2.11 Hz, 1 H) 10.35 (s, 1 H).

### Description 128: 4-amino-3-(N-(pyridin-4-yl)sulfamoyl)-N-(3,4,5-trifluorophenyl)benzamide (D128)

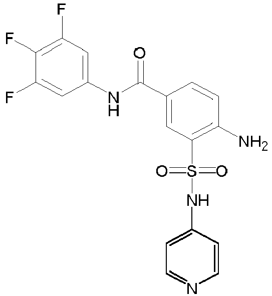

A 5mL vial was charged with **D40** (100mg, 0.24mmol) dioxane (1mL) and aqueous ammonia (2mL). The vial was sealed and heated for 8hrs at 100°C. Solvents were removed by evaporation and the residue partitioned between water and EtOAc. The organic extract were combined and evaporated, the residue was dissolved in 1/2 dioxane/aqueous ammonia (2mL) and heated in a closed vial for 8hrs at 100°C. Solvent was removed *in vacuo* and the residue partitioned betweeen water and EtOAc. The organic extract was evaporated and the residue was purified by flash chromatography on direct phase (EtOAc/MeOH). The fractions containing the product were combined and evaporated affording a residue (20mg) that was purified by preparative HPLC (H₂O/CH₃CN+1‰TFA) affording the title compound **D128** as TFA salt (1.24mg). Method 3; Rt: 2.63min. m/z: 423.1 (M+H)⁺. ¹H NMR (300 MHz, DMSO-*d*₆) δ ppm 6.52 (br s, 1 H) 6.82 (d, *J*=8.62 Hz, 1 H) 7.03 (br d, *J*=6.88 Hz, 2 H) 7.66 - 7.79 (m, 2 H) 7.83 (dd, *J*=8.67, 2.16 Hz, 1 H) 8.03 (d, *J*=6.00 Hz, 2 H) 8.35 (d, *J*=2.11 Hz, 1 H) 10.35 (s, 1 H)

### Description 129: 4-amino-3-(N-(3-hydroxycyclobutyl)sulfamoyl)-N-(3,4,5-trifluorophenyl)benzamide (D129)

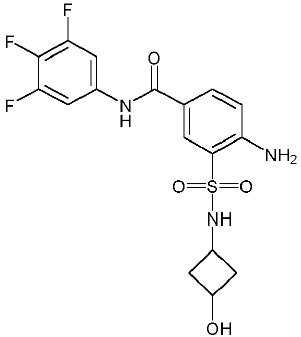

Similarly prepared according to procedure described for the preparation of **D107**, starting from **D45**. Method 1; Rt: 1.81min. m/z: 416.40(M+H)⁺.

### Description 130: 4-amino-3-(N-(oxetan-3-yl)sulfamoyl)-N-(3,4,5-trifluorophenyl)benzamide (D130)

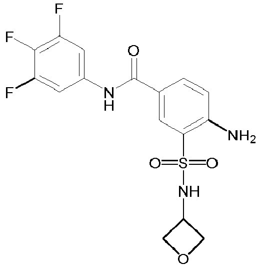

Similarly prepared according to procedure described for the preparation of **D107**, starting from **D43**. Method 3; Rt: 3.20. m/z: 402.30(M+H)⁺.

### Example 1: N-(3,4,5-trifluorophenyl)-3,4-dihydro-2H-benzo[e][1,2,4]thiadiazine-7-carboxamide 1,1-dioxide (E1)

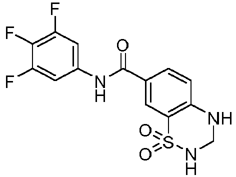

**D100** (30mg, 0.09mmol) was dissolved in 2-propanol (0.9mL), 37% aqueous formaldehyde (0.03mL, 0.350mmol) and one drop of HCl 4N in dioxane were added, and the resulting solution was stirred at 65°C for 1h. Water was added and the mixture was extracted twice with EtOAc. Combined organic layer was dried over Na₂SO₄, filtered and concentrated under vacuo. The resulting crude product was purified by preparative HPLC (H₂O/CH₃CN+1% TFA) to obtain, after lyophilization the title compound **E1** (15mg) as white solid. Method 3; Rt=3.19min, m/z=358.12 (M+H)⁺. 1H NMR (300 MHz, DMSO-*d*₆) δ ppm 4.63 - 4.78 (m, 2 H) 6.88 (d, *J*=8.89 Hz, 1 H) 7.67 - 7.79 (m, 2 H) 7.82 (br s, 1 H) 7.84 - 7.88 (m, 1 H) 7.90 (dd, *J*=8.80, 2.11 Hz, 1 H) 8.26 (br d, *J*=2.10 Hz, 1 H) 10.39 (s, 1 H).

### Example 2: N-(3,4,5-trifluorophenyl)-3,4-dihydro-2H-benzo[e][1,2,4]thiadiazine-3,3-d₂-7-carboxamide 1,1-dioxide (E2)

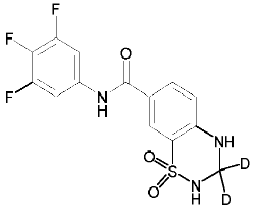

**D100** (55mg, 0.16mmol) was suspended in CD₃OD (600uL), heated to 100°C and treated with formaldehyde-d₂ solution (20% wt% in D₂O, 100uL) in a closed vial. The suspension became a yellowish solution after 10min; the reaction mixture was additionally heated at 100°C for 5hrs. Solvent was removed and the residue was purified by flash chromatography on direct phase (EtOAc/Cyclohexane) giving the title compound (25mg). This batch was further purified by preparative HPLC to give the title compound **E2**. Method 1; Rt: 1.93min. m/z: 360.22(M+H)⁺. ¹H NMR (300 MHz, DMSO-d6) δ ppm 6.88 (d, J=8.80 Hz, 1 H) 7.55 - 8.03 (m, 5 H) 8.26 (d, J=1.93 Hz, 1 H) 10.42 (br s, 1 H).

### Example 3: 4-methyl-N-(3,4,5-trifluorophenyl)-3,4-dihydro-2H-benzo[e][1,2,4]thiadiazine-7-carboxamide 1,1-dioxide (E3)

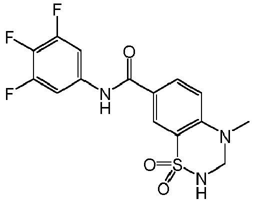

A 5mL vial was charged with **D98** (22 mg, 0.060mmol), IPA (600uL) and formaldehyde (18.75uL, 0.240mmol). The vial was sealed and 4N HCl in dioxane (50uL) was added in a single portion. The resulting white suspension was stirred at 70°C for 1.5hrs. Solvent was removed and the residue (18mg) purified by preparative HPLC (H₂O/CH₃CN 0.1% TFA) affording the title compound **E3** (5mg), as white solid. Method 3; Rt: 3.37min. m/z: 372.24 (M+H)⁺. ¹H NMR (300 MHz, DMSO-*d*₆) δ ppm 3.05 (s, 3 H) 4.79 (d, J =8.07 Hz, 2 H) 6.99 (d, J=9.08 Hz, 1 H) 7.68 - 7.81 (m, 2 H) 8.04 (dd, J=9.03, 2.25 Hz, 1 H) 8.22 (t, J=8.12 Hz, 1 H) 8.30 (d, J=2.20 Hz, 1 H) 10.44 (br s, 1 H).

### Example 4: 4-hydroxy-N-(3,4,5-trifluorophenyl)-3,4-dihydro-2H-benzo[e][1,2,4]thiadiazine-7-carboxamide 1,1-dioxide (E4)

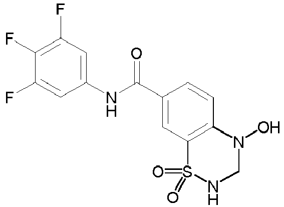

**D63** (48mg, 0.13mmol) was dissolved in EtOH (600uL), treated with formaldehyde (29.1uL, 0.19mmol) and aqueous 10% HCl (147uL, 0.04mmol). The vial was sealed and heated at 90°C for 15min. The reaction solution was purified by preparative HPLC, yielding the title compound **E4** (4.5mg). Method 3; Rt: 3.36min. m/z: 374.13 (M+H)⁺. ¹H NMR (300 MHz, DMSO-*d*₆) δ ppm 4.87 (d, *J*=8.07 Hz, 2 H) 7.40 (d, *J*=8.89 Hz, 1 H) 7.75 - 7.95 (m, 2 H) 8.20 (dd, *J*=8.89, 2.11 Hz, 1 H) 8.36 (d, J=2.11Hz, 1 H) 8.96 (t, *J*=8.02 Hz, 1 H) 10.39 (s, 1 H) 10.64 (s, 1 H) 8.22 (t, *J*=8.12 Hz, 1 H) 8.30 (d, *J*=2.20 Hz, 1 H) 10.44 (br s, 1 H).

### Example 5: 2-hydroxy-N-(3,4,5-trifluorophenyl)-3,4-dihydro-2H-benzo[e][1,2,4]thiadiazine-7-carboxamide 1,1-dioxide (E5)

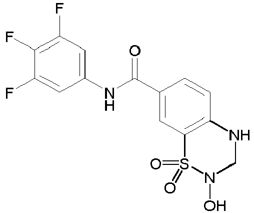

**D52** (49.mg, 0.11mmol) was dissolved in IPA (2mL), charged in a 5mL vial, and treated with formaline (82.uL, 0.1 1mmol) and (4N) HCl (30uL, 0.11mmol). The vial was sealed and heated by conventional heating at 65°C for 1h and additionally for 1h30min at 85-90°C and stored at room temperature overnight. The clear yellow reaction solution was treated with (4N) HCl in dioxane (60uL, 0.22mmol) and water (80uL) and heated at 90°C for 1h. The reaction solution was purified by preparative HPLC (H₂O,CH₃CN 0.1% TFA) to give the title compound **E5**. Method 3; Rt: 3.35min. m/z:374.13 (M+H)⁺. Isomer A: ¹H NMR (300 MHz, DMSO-*d*₆) δ ppm 4.97 (br s, 2 H) 6.90 (d, *J*=8.90 Hz, 1 H) 7.63 - 8.04 (m,4 H) 8.29 (d, *J*=2.02 Hz, 1 H) 10.42 (br s, 1 H) 10,51 (s, 1 H). Isomer B: ¹H NMR (300 MHz, DMSO-*d*₆) δ ppm 4.70 (dd, *J*=7.93, 2.43 Hz, 2 H) 6.87 (d, *J*=8.80 Hz, 1 H) 7.63 - 8.04 (m, 4 H) 8.25 (d, *J*=2.00 Hz, 1 H) 10.38 (br s, 1 H) 10.42 (br s, 1 H).

### Example 6: 2-methyl-N-(3,4,5-trifluorophenyl)-3,4-dihydro-2H-benzo[e][1,2,4]thiadiazine-7-carboxamide 1,1-dioxide (E6)

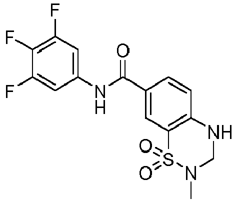

To a solution of **D99**, (52.55 mg, 0.15 mmol) in IPA (1.44 mL), formaldehyde (16.12 uL, 0,59 mmol) and 4N HCl in dioxane (1 drop) were added and the reaction mixture was stirred in a sealed vial at 65°C for 2h. Water (15 mL) was added and the reaction mixture was extracted with EtOAc. The combined organic layers were dried on Na₂SO₄, filtered and evaporated under reduced pressure. The crude product was purified by preparative HPLC (H₂O/CH₃CN+1%TFA) to afford, after lyophilization, the title compound **E6** (12.37 mg) as white powder. Method 3; Rt: 3.49 min. m/z: 372.18 (M+H)⁺. ¹H NMR (300 MHz, DMSO-d6) δ ppm 2.69 (s, 3 H) 4.91 (d, J=2.90 Hz, 2 H) 6.99 (d, J=8.80 Hz, 1 H) 7.68 - 7.85 (m, 2 H) 7.88 - 8.03 (m, 2 H) 8.30 (d, J=2.00 Hz, 1 H) 10.45 (br s, 1 H).

### Example 7: 6-chloro-N-(3,4,5-trifluorophenyl)-3,4-dihydro-2H-benzo[e][1,2,4]thiadiazine-7-carboxamide 1,1-dioxide (E7)

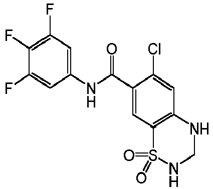

**D102** (40.mg, 0.110mmol), IPA (2mL, 0.026mol), Formaldehyde (81.79uL, 0.42mmol) and 4N HCl in dioxane (30.uL, 0.120mmol) were heated at 65°C for 15min. Solvent was removed by evaporation and the residue (50mg), purified by preparative HPLC (H₂O,CH₃CN 0.1% TFA) After liophyilization (17mg, white solid), the compound was suspended in DCM, sonicated and evap. for 3 times, giving pure the title compound **E7** (11mg) as white solid. Method 3; Rt: 3.33min. m/z: 392.17 (M+H)⁺. ¹H NMR (300 MHz, DMSO-*d*₆) δ ppm 4.71 (d, J=2.02 Hz, 2 H) 6.94 (s, 1 H) 7.54 - 7.67 (m, 2 H) 7.74 (s, 1 H) 7.77 - 7.83 (m, 1 H) 7.84 - 7.96 (m, 1 H) 10.71 (br s, 1 H).

### Example 8: 6-bromo-N-(3,4,5-trifluorophenyl)-3,4-dihydro-2H-benzo[e][1,2,4]thiadiazine-7-carboxamide 1,1-dioxide (E8)

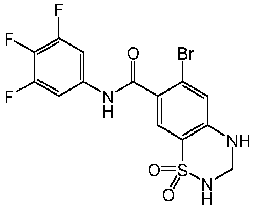

Similary prepared according to the procedure described for the preparation of **E7**, starting from **D103**. Purified by flash chromatography (direct phase, eluent DCM/MeOH) to give the title compound **E8** (25mg) as white solid. Method 3; Rt: 3.37min. m/z: 437.97 (M+H)⁺. ¹H NMR (300 MHz, DMSO-*d*₆) δ ppm 4.70 (br s, 2 H) 7.13 (s, 1 H) 7.54 - 7.66 (m, 2 H) 7.69 (s, 1 H) 7.75 (br s, 1 H) 7.88 (br s, 1 H) 10.72 (s, 1 H).

### Example 9: 6-methyl-N-(3,4,5-trifluorophenyl)-3,4-dihydro-2H-benzo[e][1,2,4]thiadiazine-7-carboxamide 1,1-dioxide (E9)

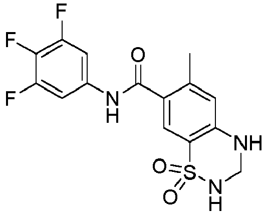

Similary prepared according to the procedure described for the preparation of **E1**, starting from **D114**. Method 3: Rt=3.32min, m/z=372.18 (M+H)⁺. ¹H NMR (300 MHz, DMSO-*d*₆) δ ppm 2.35 (s, 3 H) 4.66 (dd, *J*=7.98, 2.48 Hz, 2 H) 6.66 (s, 1 H) 7.49 - 7.58 (m, 1 H) 7.59 - 7.78 (m, 4 H) 10.52 (s, 1 H).

### Example 10: 6-chloro-3-methyl-N-(3,4,5-trifluorophenyl)-3,4-dihydro-2H-benzo[e][1,2,4]thiadiazine-7-carboxamide 1,1-dioxide (E10)

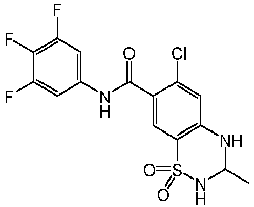

Similary prepared according to the procedure described for the preparation of **E7**, using acetaldehyde instead of formaldehyde and purified by preparative HPLC (H₂O, CH₃CN 0.1% TFA) to give the title compound **E10** (21mg) as white solid. Method 3; Rt: 3.46min. m/z: 406.02 (M+H)⁺. ¹H NMR (300 MHz, DMSO-*d₆*) δ ppm 1.46 (d, J=6.14 Hz, 3 H) 4.84-4.98 (m, 1 H) 6.92 (s, 1 H) 7.54-7.67 (m, 2 H) 7.71-7.85 (m, 3 H) 10.71 (s, 1 H).

### Example 11: N-(3,4-difluorophenyl)-2H,4H-spiro[benzo[e][1,2,4]thiadiazine-3,3'-oxetane]-7-carboxamide 1,1-dioxide (E11)

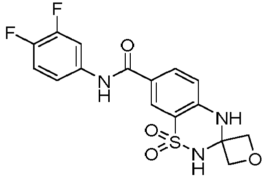

**D101** (53mg, 0.160mmol) was suspended in 2-propanol (1.5mL), 3-oxetanone (0.32mL, 4.86mmol) and two drops of HCl 4N in dioxane were added, and the reaction mixture was stirred at 65°C for 2.5h. EtOAc was added and the resulting solution was washed with 5% citric acid solution and sat. NaHCO₃ solution. Organic layer was dried over Na₂SO₄, filtered and concentrated under vacuo. The resulting crude product was purified by preparative HPLC (H₂O/CH₃CN) to obtain, after lyophilization, the title compound **E11** (15mg) as white solid. Method 3; Rt=2.99min, m/z=382.17 (M+H)⁺. ¹H NMR (300 MHz, DMSO-*d₆*) δ ppm 4.65 (d, *J*=6.97 Hz, 2 H) 4.86 (d, *J*=6.97 Hz, 2 H) 6.92 (d, *J*=8.80 Hz, 1 H) 7.34 - 7.49 (m, 1 H) 7.49 - 7.59 (m, 1 H) 7.86 - 8.01 (m, 2 H) 8.24 (d, *J*=2.02 Hz, 1 H) 8.47 (br s, 1 H) 8.75 (s, 1 H) 10.32 (s, 1 H).

### Example 12: N-(3,4-difluorophenyl)-2H,4H-spiro[benzo[e] [1,2,4]thiadiazine-3,1'-cyclobutane]-7-carboxamide 1,1-dioxide (E12)

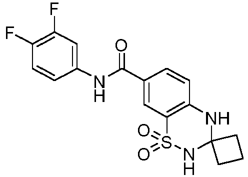

**D101** (40mg, 0.120mmol) was dissolved in cyclobutanone (0.9mL, 12.04mmol) and two drops of HCl 4N in dioxane were added. The resulting solution was stirred at 100°C for 30min. The reaction was concentrated under vacuo, and the residue was purified by preparative HPLC (H₂O/CH₃CN) to obtain, after lyophilisation the title compound **E12** (10mg) as white solid. Method 3; Rt=3.34min, m/z=380.28 (M+H)⁺ . ¹H NMR (300 MHz, DMSO-*d₆*) δ ppm 1.68 - 1.95 (m, 2 H) 2.16 - 2.31 (m, 2 H) 2.56 - 2.68 (m, 2 H) 6.83 (d, *J*=8.80 Hz, 1 H) 7.34 - 7.48 (m, 1 H) 7.49 - 7.59 (m, 1 H) 7.87 - 7.98 (m, 2 H) 8.02 (br s, 1 H) 8.20 (s, 1 H) 8.23 (d, *J*=2.02 Hz, 1 H) 10.28 (s, 1 H).

### Example 13: N-(3,4,5-trifluorophenyl)-2',3',5',6'-tetrahydro-2H,4H-spiro[benzo[e] [1,2,4]thiadiazine-3,4'-pyran]-7-carboxamide1,1-dioxide (E13)

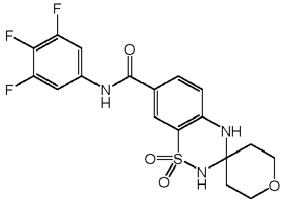

A mixture of **D100** (30mg, 0.09mmol), tetrahydro-4H-pyran-4-one (32.1uL, 0.35mmol) in 1,4-Dioxane (1.2mL) was treated with magnesium sulfate (104.58mg, 0.87mmol) and 4-methylbenzenesulfonic acid hydrate (9.92mg, 0.05mmol). The suspension was heated at 100°C for 30min by microwave irradiation. Solvent was removed *in vacuo* and the residue purified by preparative HPLC (H₂O, CH₃CN) to give the title compound **E13.** Method 3; Rt: 3.30min. m/z: 428.24 (M+H)⁺. ¹H NMR (300 MHz, DMSO-*d₆*) δ ppm 1.64 - 1.85 (m, 2 H) 2.16 - 2.34 (m, 2 H) 3.53 - 3.88 (m, 4 H) 6.89 (d, *J*=8.89 Hz, 1 H) 7.66 - 7.82 (m, 2 H) 7.82 - 7.99 (m, 3 H) 8.26 (d, *J*=2.02 Hz, 1 H) 10.39 (br s, 1 H).

### Example 14: N-(3,4,5-trifluorophenyl)-2H,4H-spiro[benzo[e][1,2,4]thiadiazine-3,4'-piperidine]-7-carboxamide 1,1-dioxide (E14)

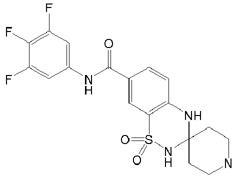

**D100** (30.mg, 0.09mmol), magnesium sulfate (104.58mg, 0.87mmol), 4-methylbenzenesulfonic acid hydrate (9.92mg, 0.05mmol) and piperidine-4,4-diol-hydrogen chloride (1/1) (40.18mg,0.26mmol) were charged in a 5 mL vial. The vial was sealed, evacuated, charged with 1,4-dioxane and heated at 100°C for 30min giving a white suspension. The reaction was cooled to room temperature and filtered and the solid filtrate washed with 1,4-dioxane. The precipitate was suspended in 1,4-dioxane and heated to 115°C until partial dissolution, then filtered. The solid obtained was washed with MeOH and the liquid part was concentrated and the residue (80mg) purified by preparative HPLC (H₂O,CH₃CN 0.1% TFA), yielding the title compound E14 as trifluoroacetate salt. Method 3; Rt: 2.99min m/z: 427.23 (M+H)⁺. ¹H NMR (300 MHz, DMSO-d6) δ = 14.85 - 13.64 (m, 1H), 10.49 (s, 1H), 8.97 (br s, 1H), 8.48 (br d, *J=* 10.3 Hz, 1H), 8.42 - 8.27 (m, 1H), 8.09 - 7.95 (m, 1H), 7.82 - 7.69 (m, 4H), 7.07 (dd, *J*= 1.5, 8.9 Hz, 1H), 6.16 (dd, *J* = 0.7, 10.4 Hz, 1H).

### Example 15: 3-(trifluoromethyl)-N-(3,4,5-trifluorophenyl)-3,4-dihydro-2H-benzo[e][1,2,4]thiadiazine-7-carboxamide1,1-dioxide (E15)

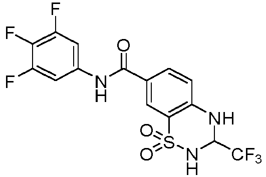

4-amino-3-sulfamoyl-N-(3,4,5-trifluorophenyl)benzamide **D100** (36.mg, 0.100mmol) was dissolved in 2-propanol (1.1mL), Trifluoroacetaldehyde monohydrate 72% aqueous solution (0.06mL,0.630mmol) and two drops of HCl 4N in dioxane were added and the reaction mixture was stirred at 100°C for 3h. The reaction mixture was directly purified by preparative HPLC (H₂O/CH₃CN+0.1%TFA) to obtain, after lyophilization, the title compound E15 (15mg) as light-yellow solid. Method 3; Rt=3.54min, m/z=426.22 (M+H)⁺. ¹H NMR (300 MHz, DMSO-*d₆*) δ ppm 5.52 - 5.82 (m, 1 H) 7.16 (d, *J*=8.89 Hz, 1 H) 7.65 - 7.83 (m, 2 H) 8.00 (dd, J=8.80, 2.11 Hz, 1 H) 8.31 (d, *J*=2.02 Hz, 1 H) 8.42 (s, 1 H) 8.75 (d, *J*=11.65 Hz, 1 H) 10.48 (s, 1 H).

### Example 16: 3-(2-isopropoxyethyl)-N-(3,4,5-trifluorophenyl)-3,4-dihydro-2H-benzo[e][1,2,4]thiadiazine-7-carboxamide 1,1-dioxide (E16)

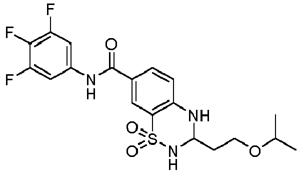

Similary prepared according to the procedure described for the preparation of E15 using 3,3-diethoxypropan-1-ol instead of trifluoroacetaldehyde monohydrate 72% aqueous solution. Method 3; Rt: 3.72 min. m/z: 444.25 (M+H)⁺. ¹H NMR (300 MHz, DMSO-*d₆*) δ ppm 1.12 (s, 3 H) 1.16 (s, 3 H) 1.91 - 2.11 (m, 2 H) 3.47 - 3.71 (m, 3 H) 4.84 - 5.00 (m, 1 H) 6.95 (d, *J*=8.80 Hz, 1 H) 7.62 - 7.86 (m, 4 H) 7.95 (dd, J=8.90, 2.20 Hz, 1 H) 8.30 (d, *J*=2.20 Hz, 1 H) 10.43 (s, 1 H).

### Example 17: 3-(difluoromethyl)-N-(3,4,5-trifluorophenyl)-3,4-dihydro-2H-benzo[e][1,2,4]thiadiazine-7-carboxamide1,1-dioxide (E17)

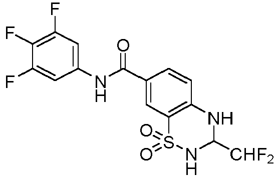

**D100** (30mg, 0.090mmol) was dissolved in 1,4-dioxane (0.9mL), 1-Ethoxy-2,2-difluoroethanol (43.82mg, 0.350mmol) and two drops of HCl 4N in dioxane were added, and the reaction mixture was stirred at 100°C for 1h. The reaction mixture was directly purified by preparative HPLC (H₂O/CH₃CN+0.1%TFA) to obtain, after lyophilization, the title compound E17 (18mg) as white solid. Method 3; Rt=3.45min, m/z=408.18 (M+H)⁺. ¹H NMR (300 MHz, DMSO-*d₆*) δ ppm 5.05 - 5.24 (m, 1 H) 6.28 (td, *J*=54.47, 4.31 Hz, 1 H) 7.09 (d, J=8.90 Hz, 1 H) 7.65 - 7.82 (m, 2 H) 7.97 (dd, J=8.80, 2.11 Hz, 1 H) 8.20 (s, 1 H) 8.29 (d, *J*=2.02 Hz, 1 H) 8.37 (d, J=10.64 Hz, 1 H) 10.45 (s, 1 H).

### Example 18: 2-methyl-3-(trifluoromethyl)-N-(3,4,5-trifluorophenyl)-3,4-dihydro-2H-benzo[e][1,2,4]thiadiazine-7-carboxamide1,1-dioxide (E18)

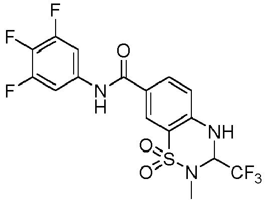

**D99** (41mg, 0.11mmol) was dissolved in 2-propanol (1mL), Trifluoroacetaldehyde monohydrate 72% aqueous solution (0.13mL, 1.37mmol) and two drops of HCl 4N in dioxane were added, and the reaction mixture was heated under microwave irradiation at 150°C for 2.5h. The reaction mixture was directly purified by preparative HPLC (H₂O/CH₃CN+0.1%TFA) to obtain, after lyophilization, the title compound **E18** (10mg) as white solid. Method 3; Rt=3.92min, m/z=440.13 (M+H)⁺.¹H NMR (300 MHz, DMSO-*d₆*) δ ppm 2.77 (s, 3 H) 5.94 - 6.13 (m, 1 H) 7.18 (d, *J*=8.80 Hz, 1 H) 7.61 - 7.83 (m, 2 H) 8.04 (dd, J=8.90, 2.10 Hz, 1 H) 8.33 (d, *J*=2.02 Hz, 1 H) 8.60 - 8.80 (m, 1 H) 10,51 (s, 1 H).

Examples **E19** to **E26** were prepared according to the procedure described for the preparation of example **E1**

### Example 19: N-(3,4-difluorophenyl)-3,4-dihydro-2H-benzo[e][1,2,4]thiadiazine-7-carboxamide 1,1-dioxide (E19)

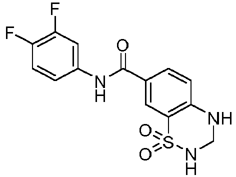

Compound **D101** was used as starting material. Method 3; Rt=2.94min, m/z=340.09 (M+H)⁺. ¹H NMR (300 MHz, DMSO-*d₆*) δ ppm 4.63 - 4.77 (m, 2 H) 6.87 (d, *J*=8.90 Hz, 1 H) 7.35 - 7.48 (m, 1 H) 7.49 - 7.61 (m, 1 H) 7.74 - 7.86 (m, 2 H) 7.87 - 8.01 (m, 2 H) 8.26 (d, *J*=2.11 Hz, 1 H) 10.29 (s, 1 H).

### Example 20: N-(3,4-difluorophenyl)-3-methyl-3,4-dihydro-2H-benzo[e][1,2,4]thiadiazine-7-carboxamide 1,1-dioxide (E20)

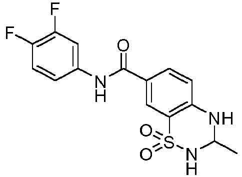

Acetaldehyde was used instead of formaldehyde, and compound **D101** was used as starting material. Method 3; Rt=3.08min, m/z=354.14 (M+H)⁺. ¹H NMR (300 MHz, DMSO-*d₆*) δ ppm 1.47 (d, *J*=6.14 Hz, 3 H) 4.84 - 4.99 (m, 1 H) 6.88 (d, J=8.80 Hz, 1 H) 7.34 - 7.48 (m, 1 H) 7.49 - 7.60 (m, 1 H) 7.69 (d, *J*=11.65 Hz, 1 H) 7.81 (s, 1 H) 7.86 - 8.02 (m, 2 H) 8.20 - 8.31 (m, 1 H) 10.31 (s, 1 H).

### Example 21: 3-methyl-N-(3,4,5-trifluorophenyl)-3,4-dihydro-2H-benzo[e][1,2,4]thiadiazine-7-carboxamide 1,1-dioxide (E21)

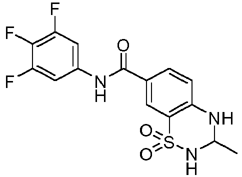

Compound **D100** was used as starting material. Acetaldehyde was used instead of formaldehyde, and the crude title compound was purified by preparative HPLC (H₂O/CH₃CN+0.1%HCOOH). Method 3; Rt=3.32min, m/z=372.11 (M+H)⁺. ¹H NMR (300 MHz, DMSO-*d₆*) δ ppm 1.47 (d, *J*=6.14 Hz, 3 H) 4.82 - 5.03 (m, 1 H) 6.88 (d, *J*=8.80 Hz, 1 H) 7.62 - 7.81 (m, 3 H) 7.86 (s, 1 H) 7.91 (dd, J=8.80, 2.02 Hz, 1 H) 8.26 (d, *J*=1.93 Hz, 1 H) 10.41 (s, 1 H).

### Example 22: 3-(2-hydroxyethyl)-N-(3,4,5-trifluorophenyl)-3,4-dihydro-2H-benzo[e][1,2,4]thiadiazine-7-carboxamide 1,1-dioxide (E22)

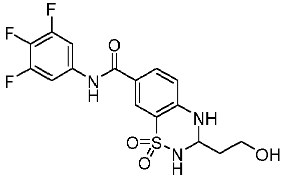

Compound **D100** was used as starting material. 3,3-diethoxypropan-1-ol was used instead of formaldehyde and 1,4-dioxane was used instead of 2-propanol. Method 3; Rt: 3.02 min. m/z: 402.10 (M+H)⁺. ¹H NMR (300 MHz, DMSO-*d₆*) δ ppm 1.92 - 2.06 (m, 2 H) 3.59 - 3.72 (m, 2 H) 4.72 - 4.82 (m, 1 H) 4.89 - 5.02 (m, 1 H) 6.96 (d, J=8.70 Hz, 1 H) 7.68 (d, *J*=11.60 Hz, 1 H) 7.72 - 7.82 (m, 3 H) 7.94 (dd, J=8.90, 2.30 Hz, 1 H) 8.29 (br d, *J*=2.10 Hz, 1 H) 10.42 (s, 1 H).

### Example 23: 3-((S)-1-hydroxyethyl)-N-(3,4,5-trifluorophenyl)-3,4-dihydro-2H-benzo[e][1,2,4]thiadiazine-7-carboxamide 1,1-dioxide (E23)

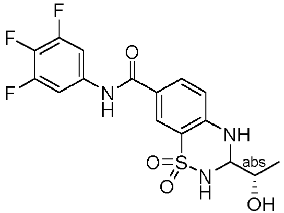

Compound **D100** was used as starting material. D-lactaldehyde solution was used instead of formaldehyde and 1,4-dioxane was used instead of 2-propanol. Method 3; Rt: 3.30 min. m/z: 402.17 (M+H)⁺. ¹H NMR (300 MHz, DMSO-*d*₆; mixture of diastereoisomers) δ ppm 1.23 - 1.31 (m, 6 H) 3.75 - 3.93 (m, 1 H, a) 3.93 - 4.10 (m, 1 H, b) 4.50 (dd, *J*=11.92, 7.70 Hz, 1 H, a) 4.77 (dd, *J*=12.20, 2.84 Hz, 1 H, b) 5.37 (br s, 1 H) 7.10 (d, *J*=9.00 Hz, 1 H, a or b) 7.19 (d, *J*=8.89 Hz, 1 H, a or b) 7.29 (d, *J*=12.38 Hz, 1 H, b) 7.63 - 7.84 (m, 7 H) 7.91 (t, *J*=2.20 Hz, 1 H, a or b) 7.94 (t, *J*=2.30 Hz, 1 H, a or b) 8.26 - 8.30 (m, 2 H) 10.41 (br s, 1 H, a or b) 10.43 (br s, 1 H, a or b)

### Example 24: 3-((R)-1-hydroxyethyl)-N-(3,4,5-trifluorophenyl)-3,4-dihydro-2H-benzo[e][1,2,4]thiadiazine-7-carboxamide 1,1-dioxide (E24)

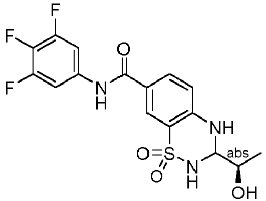

Compound **D100** was used as starting material. L-Lactaldehyde solution was used instead of formaldehyde and 1,4-dioxane was used instead of 2-propanol. Method 3; Rt: 3.56 min. m/z: 402.24 (M+H)⁺. ¹H NMR (mixture of diastereoisomers, 300 MHz, DMSO-*d₆*) δ ppm 1.26 (d, *J*=5.41 Hz, 3 H, a or b) 1.28 (d, *J*=5.60 Hz, 3 H, a or b) 3.78 - 3.89 (m, 1 H, a) 3.96 - 4.07 (m, 1 H, b) 4.50 (dd, *J*=11. 90, 7.70 Hz, 1 H, a) 4.77 (dd, *J*=12.30, 3.20 Hz, 1 H, b) 5.05 - 5.23 (m, 1 H, a or b) 5.28 - 5.45 (m, 1 H, a or b) 7.10 (d, *J*=8.90 Hz, 1 H, a or b) 7.20 (d, *J*=8.90 Hz, 1 H, a or b) 7.29 (d, *J*=12.20 Hz, 1 H, b) 7.67 (d, *J*=11.80 Hz, 1 H, a) 7.70 - 7.83 (m, 6 H) 7.91 (t, *J*=2.30 Hz, 1 H, a or b) 7.94 (t, *J*=2.40 Hz, 1 H, a or b) 8.28 (d, *J*=2.00 Hz, 1 H, a or b) 8.28 (d, *J*=2.10 Hz, 1 H, a or b) 10.41 (s, 1 H, a or b) 10.43 (s, 1 H, a or b).

### Example 25: (S)-3-(1-hydroxyethyl)-N-(3,4,5-trifluorophenyl)-4H-benzo[e][1,2,4]thiadiazine-7-carboxamide1,1-dioxide (E25)

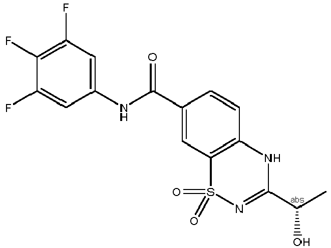

Compound **D100** was used as starting material. D-(+)-Glyceraldehyde was used instead of formaldehyde and 1,4-dioxane was used instead of 2-propanol. Method 3; Rt: 3.26 min. m/z: 400.14 (M+H)⁺. ¹H NMR (300 MHz, DMSO-*d₆*) δ ppm 1.46 (d, *J*=6.70 Hz, 3 H) 4.46 - 4.62 (m, 1 H) 6.32 (d, *J*=4.80 Hz, 1 H) 7.71 - 7.84 (m, 2 H) 7.88 (d, *J*=8.80 Hz, 1 H) 8.27 (dd, *J*=8.80, 2.00 Hz, 1 H) 8.54 (d, *J*=2.00 Hz, 1 H) 10.78 (s, 1 H) 12.11 (s, 1 H).

### Example 26: 3-((R)-1,2-dihydroxyethyl)-N-(3,4,5-trifluorophenyl)-3,4-dihydro-2H-benzo[e][1,2,4]thiadiazine-7-carboxamide 1,1-dioxide (E26)

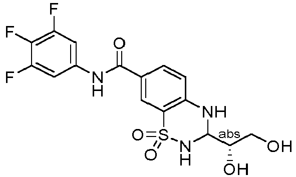

Similary prepared according to the procedure described for the preparation of **E25** but at room temperature instead of 65°C. Method 3; Rt: 3.28 min. m/z: 418.18 (M+H)⁺. 417.06. ¹H NMR (300 MHz, DMSO-*d*₆; mixture of diastereoisomers) δ ppm 3.51 - 3.61 (m,1 H, a) 3.65 - 3.80 (m, 2 H, b) 3.80 - 3.91 (m, 1 H, a) 4.72 - 4.88 (m, 3 H, a and b) 4.90 - 4.99 (m, 1 H, a) 5.26 (d, *J*=5.10 Hz, 1 H, a) 5.42 (d, *J*=6.00 Hz, 1 H, b) 7.03 (d, *J*=8.80 Hz, 1 H, b) 7.16 (d, *J*=12.00 Hz, 1 H, a) 7.20 (d, *J*=9.30 Hz, 1 H, a) 7.59 (br s, 1 H, a or b) 7.65 (d, *J*=11.90 Hz, 1 H, b) 7.69 - 7.83 (m, 5 H, a and b) 7.88 - 7.98 (m, 2 H, a and b) 8.28 (br s, 2 H, a and b) 10.42 (br s, 2 H, a and b).

### Example 27: 3,3-dimethyl-N-(3,4,5-trifluorophenyl)-3,4-dihydro-2H-benzo[e][1,2,4]thiadiazine-7-carboxamide 1,1-dioxide (E27)

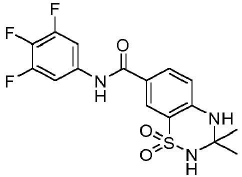

4-amino-3-sulfamoyl-N-(3,4,5-trifluorophenyl)benzamide **D100** (31.75 mg, 0.09 mmol) was dissolved in acetone (1.6 mL) and HCl 4N in dioxane (2 drops) was added. The solution was stirred at 65°C for 30 min. The solvent was evaporated under reduced pressure. The crude product was purified by flash chromatography (DCM:MeOH) to afford the title compound E27 (13.1 mg) as white powder. Method 3; Rt: 3.43 min m/z: 386.23 (M+H)⁺. ¹H NMR (300 MHz, DMSO-*d₆*) δ ppm 1.58 (s, 6 H) 6.86 (d, *J*=8.90 Hz, 1 H) 7.71 - 7.83 (m, 2 H) 7.87 (br s, 2 H) 7.92 (dd, *J*=8.80, 2.10 Hz, 1 H) 8.30 (s, 1 H) 10.41 (s, 1 H).

### Example 28: 3-(hydroxymethyl)-N-(3,4,5-trifluorophenyl)-3,4-dihydro-2H-benzo[e][1,2,4]thiadiazine-7-carboxamide 1,1-dioxide (E28)

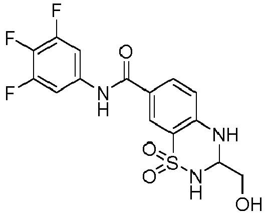

4-amino-3-sulfamoyl-N-(3,4,5-trifluorophenyl)benzamide **D100** (31.75 mg, 0.09 mmol) was dissolved in 1,4-dioxane (950 uL), 2-((tert-butyldimethylsilyl)oxy)acetaldehyde-1-ol (70 uL, 0.37 mmol) and HCl 4N in dioxane (2 drops) were added. The reaction mixture was stirred in a sealed vial at 65°C for 30 min. 6M HCl solution (153 uL) was added and the stirring was continued at room temperature for 30 min. The reaction was directly purified by preparative HPLC (H₂O/CH₃CN+1%TFA) to afford, after lyophilization, 5.4 mg of the title compound E28 as white powder. Method 3; Rt: 3.03 min. m/z: 388.19 (M+H)⁺: 387.05. ¹H NMR (300 MHz, DMSO-*d₆*) δ ppm 3.62 - 3.83 (m, 2 H) 4.76 - 4.89 (m, 1 H) 5.28 - 5.38 (m, 1 H) 7.06 (d, *J*=8.70 Hz, 1 H) 7.69 (d, *J*= 11.65 Hz, 1 H) 7.71 - 7.87 (m, 3 H) 7.93 (dd, *J*=8.90, 2.30 Hz, 1 H) 8.28 (d, *J*=2.10 Hz, 1 H) 10.44 (s, 1 H).

### Example 29: 3-(aminomethyl)-N-(3,4,5-trifluorophenyl)-3,4-dihydro-2H-benzo[e][1,2,4]thiadiazine-7-carboxamide 1,1-dioxide (E29)

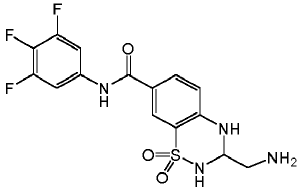

To a solution of **D100** (30 mg, 0.090 mmol) in IPA (1 mL) was added N-Boc-2 aminoacetaldehyde (55 mg, 0.350 mmol) and 4N HCl in dioxane (30 µL, 0.120 mmol). The reaction was stirred at 65°C for 2h. 4N HCl in dioxane (287 µL, 1.148 mmol) was added and stirring was continued at room temperature over night. The reaction mixture was concentrated under reduced pressure and the residue purified by preparative HPLC (H₂O, CH₃CN 0.1% HCOOH). The pure fractions were combined and lyophilized to afford the title comppund E29 (16 mg) as white solid. Method 3; Rt: 2.43 min. m/z: 387.10 (M+H)⁺. ¹H NMR (300 MHz, DMSO-*d₆*) δ ppm 2.83 - 3.02 (m, 2 H) 4.73 (t, *J*=5.69 Hz, 1 H) 6.96 (d, *J*=8.80 Hz, 1 H) 7.68 - 7.83 (m, 3 H) 7.92 (dd, *J*=8.80, 2.11 Hz, 1H) 8.20 - 8.28 (m, 2 H) 10.41 (s, 1 H).

### Example 30: 3-(acetamidomethyl)-N-(3,4,5-trifluorophenyl)-3,4-dihydro-2H-benzo[e][1,2,4]thiadiazine-7-carboxamide 1,1-dioxide (E30)

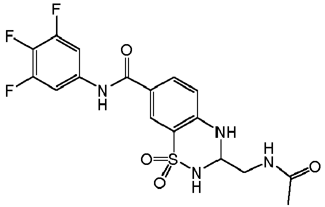

To a solution of **E29** (12.6 mg, 0.030 mmol) in DCM (3 mL) was added acetic anhydride (0.004 mL, 0.040 mmol) and triethylamine (0.009 mL, 0.070 mmol). After 30 min. volatiles were evaporated under reduced pressure and the residue purified by preparative HPLC (H₂O/CH₃CN). The pure fractions were combined and lyophilized to afford the title compound **E30** (4.0 mg) as a white solid. Method 3; Rt: 3.00 min. m/z: 429.19 (M+H)⁺. ¹H NMR (300 MHz, DMSO-*d₆*) δ ppm 1.89 (s, 3 H) 3.23 - 3.31 (m, 1 H) 3.63 (dt, *J*=13.66, 5.55 Hz, 1 H) 3.72 (br d, *J*=8.25 Hz, 1 H) 4.73 - 4.85 (m, 1 H) 6.93 (d, *J*=8.80 Hz, 1 H) 7.68 - 7.95 (m, 5 H) 8.12 - 8.29 (m, 2 H) 10.41 (s, 1 H).

### Example 31: tert-butyl 4-(1,1-dioxido-7-((3,4,5-trifluorophenyl)carbamoyl)-3,4-dihydro-2H-benzo[e] [1,2,4]thiadiazin-3-yl)piperidine-1-carboxylate (E31)

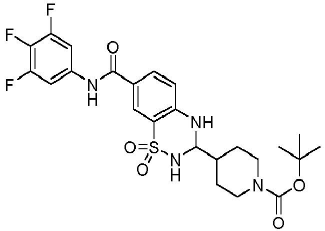

Similary prepared according to the procedure described for the preparation of **E1.** Compound **D100** was used as starting material and tert-butyl 4-formylpiperidine-1-carboxylate was used instead of formaldehyde, and purified by preparative HPLC (H₂O/CH₃CN+0.1%HCOOH). Method 3; Rt = 3.89 min, m/z = 541.11, 11 (M+H)⁺. ¹H NMR (300 MHz, DMSO-*d₆*) δ ppm 1.20 - 1.34 (m, 2 H) 1.42 (s, 9 H) 1.79 - 1.92 (m, 3 H) 2.62 - 2.82 (m, 2 H) 3.92 - 4.11 (m, 2 H) 4.58 - 4.68 (m, 1 H) 7.01 (d, *J*=8.80 Hz, 1 H) 7.54 (br s, 1 H) 7.64 - 7.79 (m, 3 H) 7.91 (dd, *J*=8.89, 2.11 Hz, 1 H) 8.27 (d, *J*=2.11 Hz, 1 H) 10.40 (s, 1 H)

### Example 32: 3-(piperidin-4-yl)-N-(3,4,5-trifluorophenyl)-3,4-dihydro-2H-benzo[e][1,2,4]thiadiazine-7-carboxamide 1,1-dioxide (E32)

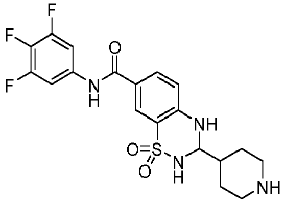

**D100** (30 mg, 0.087 mmol) and tert-butyl 4-formylpiperidine-1-carboxylate (74 mg, 0.348 mmol) were dissolved in i-PrOH (0.9 mL); 3 drops of 4N HCl in dioxane were added and the solution was heated to 65°C in a closed vial for 30 min; more 4N HCl in dioxane (0.2 mL) was added and heating was continued for 1h. The crude product was purified by preparative HPLC (H₂O/CH₃CN+0.1%HCOOH) to obtain, after lyophilisation the title compound E32 (19.9mg) as white solid. Method 3; Rt = 2.56 min, m/z = 441.21, 11 (M+H)⁺. ¹H NMR(300 MHz, DMSO-*d₆*) δ ppm 1.34 - 1.55 (m, 2 H) 1.75 - 2.04 (m, 3 H) 2.54 - 2.79 (m, 2 H) 3.10 - 3.26 (m, 2 H) 4.54 - 4.65 (m, 1 H) 7.02 (d, *J*=8.90 Hz, 1 H) 7.63 - 7.81 (m, 3 H) 7.92 (dd, *J*=8.85, 2.15 Hz, 1 H) 8.27 (d, *J*=2.11 Hz, 1 H) 8.34 (s, 1 H) 10.42 (s, 1 H).

### Example 33: 2-methyl-3-(piperidin-4-yl)-N-(3,4,5-trifluorophenyl)-3,4-dihydro-2H-benzo[e][1,2,4]thiadiazine-7-carboxamide 1,1-dioxide (E33)

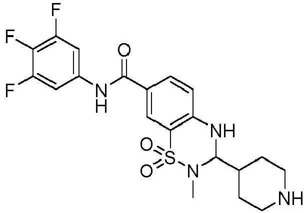

Similarly prepared according to procedure described for the preparation of **E32**, starting from **D99.** The title compound **E33** was obtained as HCOOH salt. Method 3; Rt= 2.74min, m/z=455.33 (M+H)⁺. ¹H NMR (300 MHz, DMSO-*d₆*) δ ppm 1.15 - 1.43 (m, 2 H) 1.76 - 1.87 (m, 1 H) 1.89 - 2.06 (m, 1 H) 2.09 - 2.23 (m, 1 H) 2.47 (s, 3 H) 2.59 - 2.78 (m, 2 H) 3.11 - 3.24 (m, 2 H) 4.80 (dd, *J*=9.95, 2.15 Hz, 1 H) 7.09 (d, *J*=8.90 Hz, 1 H) 7.64 - 7.81 (m, 3 H) 7.97 (dd, *J*=8.90, 2.10 Hz, 1 H) 8.28 (d, *J*=2.02 Hz, 1 H) 8.36 (s, 1 H) 10.47 (s, 1 H)

### Example 34: 6-chloro-N-(3,4,5-trifluorophenyl)-2H,4H-spiro[benzo[e] [1,2,4]thiadiazine-3,1'-cyclobutane]-7-carboxamide 1,1-dioxide (E34)

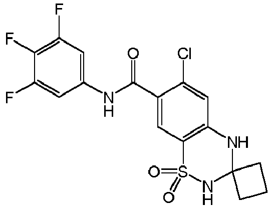

A mixture of **D102** (40mg, 0.11mmol), cyclobutanone (0.01mL, 0.11mmol) in 1,4-Dioxane (1.5mL) was treated with 4-methylbenzenesulfonic acid hydrate (5.01mg, 0.03mmol) and heated at 100°C for 30min by microwave irradiation. Solvent was removed in vacuo. The residue was purified by preparative LC-MS (H₂O,CH₃CN 0.1% HCOOH) giving the title compound **E34** (5.29mg). Method 3; Rt: 3.70min. m/z: 432.09 (M+H)⁺. ¹H NMR (300 MHz, DMSO-*d₆*) δ ppm 1.69 - 1.95 (m, 2 H) 2.11 - 2.32 (m, 2 H) 2.55 - 2.68 (m, 2 H) 6.88 (s, 1 H) 7.46 - 7.66 (m, 2 H) 7.66 - 7.78 (m, 1 H) 8.13 (br s, 1 H) 8.23 (s, 1 H) 10,51 - 10.85 (m, 1 H).

### Example 35: 3-(2,6-difluorophenyl)-N-(3,4,5-trifluorophenyl)-3,4-dihydro-2H-benzo[e][1,2,4]thiadiazine-7-carboxamide 1,1-dioxide (E35)

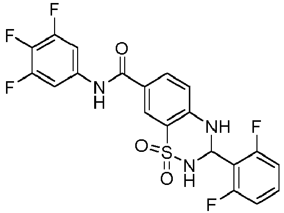

In a vial (5 mL) **D100** (22.0 mg, 0.064 mmol), was dissolved in 0,5 mL of IPA and 2,6-Difluorobenzaldehyde (0.027 mL, 0.255 mmol), 1 drop of HCl 4N in 1,4-dioxane were added. The vial was sealed and the reaction mixture was stirred and heated at 65°C for 1 h. The mixture was purified by HPLC/MS prep to yield the title compound **E35** (17.26 mg) as white powder. Method 3: Rt=3.81min, m/z= 470.06, (M+H)⁺. ¹H NMR (DMSO-d₆, 300 MHz): δ = 10.45 (s, 1H), 8.33 (d, *J* = 1.8 Hz, 2H), 8.18 (br d, *J* = 2.8 Hz, 1H), 7.95 (dd, *J* = 8.8, 2.0 Hz, 1H), 7.84-7.84 (m, 1H), 7.75 (dd, *J* = 10,5, 6.6 Hz, 2H), 7.54-7.67 (m, 1H), 7.25 (t, *J* = 8.8 Hz, 2H), 6.94 (d, *J* = 8.8 Hz, 1H), 6.28 ppm (br s, 1H)

### Example 36: 6-chloro-3-(thiazol-2-yl)-N-(3,4,5-trifluorophenyl)-3,4-dihydro-2H-benzo[e][1,2,4]thiadiazine-7-carboxamide 1,1-dioxide (E36)

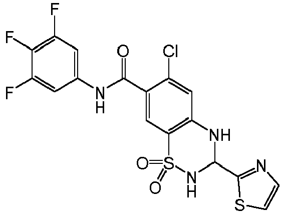

Similary prepared according to the procedure described for the preparation of **E7**, using thiazole-2-carbaldehyde instead of formaldehyde. Title compound **E36** (19mg) was purified from the reaction mixture by filtration and obtained as white solid. Method 3; Rt: 3.59min. m/z: 474.99 (M+H)⁺. ¹H NMR (300 MHz, DMSO-*d₆*) δ ppm 6.26 (dd, *J*=11.32, 0.78 Hz, 1 H) 7.30 (s, 1 H) 7.54 - 7.68 (m, 2 H) 7.82 (s, 1 H) 7.95 (q, *J*=3.21 Hz, 1 H) 8.45 (s, 1 H) 8.74 (d, *J*=11.37 Hz, 1 H) 10.78 (s, 1 H).

### Example 37: 3-(1-methyl-1H-imidazol-5-yl)-N-(3,4,5-trifluorophenyl)-3,4-dihydro-2H-benzo[e][1,2,4]thiadiazine-7-carboxamide 1,1-dioxide (E37)

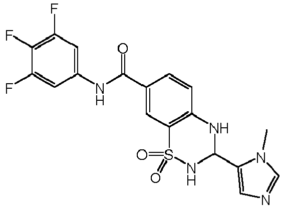

A mixture of **D100** (30.mg, 0.09mmol) and 1-methyl-1H-imidazole-5-carbaldehyde (52mg, 0.47mmol) in IPA (2mL) was treated with 4N HCl in dioxane (22.5uL, 0.61mmol) and heated at 70°C for 5min. Solvent was removed in vacuo and the residue purified by preparative HPLC (H₂O,CH₃CN 0.1% HCOOH, giving the title compound **E37** (17mg) as white solid. Method 3; Rt: 2.54min. m/z: 438.11 (M+H)⁺. ¹H NMR (300 MHz, DMSO-*d₆*) δ ppm 3.70 (s, 3 H) 6.08 (br d, J=7.79 Hz, 1 H) 7.08 (d, *J*=8.89 Hz, 1 H) 7.18 (s, 1 H) 7.64 - 7.84 (m, 3 H) 7.96 (dd, *J*=8.80, 2.02 Hz, 1 H) 8.18 (br s, 2 H) 8.32 (d, *J*=1.83 Hz, 1 H) 10.44 (s, 1 H)

### Example 38: N-(3,4-difluorophenyl)-3-(2,4-dimethyloxazol-5-yl)-3,4-dihydro-2H-benzo[e][1,2,4]thiadiazine-7-carboxamide 1,1-dioxide (E38)

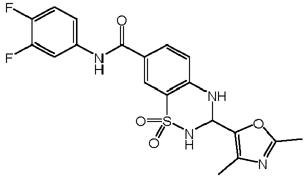

A mixture of **D101** (30mg, 0.09mmol) and 2,4-dimethyloxazole-5-carbaldehyde (43.49mg,0.35mmol) in IPA (2mL) was treated with 4N HCl in dioxane (4.82uL,0.13mmol). The reaction mixture was heated at 70°C for 10min. Solvent was removed in vacuo and the residue purified by preparative LC_MS (H₂O,CH₃CN) to give the title compound **E38.** Method 3; Rt: 3.12min. m/z: 435.20 (M+H)⁺. ¹H NMR (300 MHz, DMSO-*d₆*) δ ppm 2.15 (s, 3 H) 2.41 (s, 3 H) 6.04 (s, 1 H) 6.96 (d, *J*=8.80 Hz, 1 H) 7.36 - 7.49 (m, 1 H) 7.50 - 7.60 (m, 1 H) 7.88 - 8.01 (m, 2 H) 8.13 (br s, 1 H) 8.27 - 8.32 (m, 1 H) 8.33 - 8.44 (m, 1 H) 10.35 (s, 1 H).

### Example 39: 3-methyl-3-(pyridin-4-yl)-N-(3,4,5-trifluorophenyl)-3,4-dihydro-2H-benzo[e][1,2,4]thiadiazine-7-carboxamide 1,1-dioxide (E39)

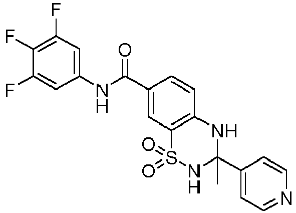

**D100** (20mg, 0.06mmol) was dissolved in 2-propanol (0.9mL), 4-acetylpyridine (0.03mL,0.23mmol) and two drops of HCl 4N in dioxane were added, and the reaction mixture was stirred at 100°C for 10h. The reaction mixture was directly purified by preparative HPLC (H₂O/CH₃CN+0.1%HCOOH) to obtain, after lyophilisation the title compound **E39** (9mg) as white solid. Method 3; Rt=2.51min, m/z=449.12 (M+H)⁺. ¹H NMR (300 MHz, DMSO-*d₆*) δ ppm 1.74 (s, 3 H) 7.11 (d, *J*=8.80 Hz, 1 H) 7.37 - 7.49 (m, 2 H) 7.64 - 7.79 (m, 2 H) 7.98 (dd, *J*=8.62, 2.20 Hz, 1 H) 8.18 (d, *J*=2.02 Hz, 1 H) 8.38 - 8.62 (m, 4 H) 10.38 (s, 1 H).

Examples **E40** to **E51** were prepared according to the procedure described for the preparation of **E1**

### Example 40: 3-(thiazol-5-yl)-N-(3,4,5-trifluorophenyl)-3,4-dihydro-2H-benzo[e][1,2,4]thiadiazine-7-carboxamide 1,1-dioxide (E40)

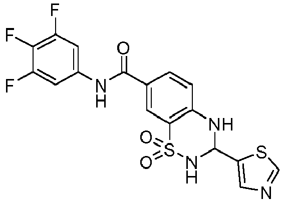

Compound **D100** was used as starting material. Thiazole-5-carbaldehyde was used instead of formaldehyde. Purified by preparative HPLC (H₂O/CH₃CN+0.1%HCOOH). Method 3; Rt=3.30min, m/z=441.08 (M+H)⁺. ¹H NMR (300 MHz, DMSO-*d₆*) δ ppm 6.37 (s, 1 H) 7.02 (d, *J*=8.80 Hz, 1 H) 7.67 - 7.82 (m, 2 H) 7.96 (dd, *J*=8.99, 2.20 Hz, 1 H) 8.22 (s, 1 H) 8.27 - 8.38 (m, 2 H) 8.57 (br s, 1 H) 9.20 (s, 1 H) 10.46 (s, 1 H).

### Example 41: 3-(thiazol-4-yl)-N-(3,4,5-trifluorophenyl)-3,4-dihydro-2H-benzo[e][1,2,4]thiadiazine-7-carboxamide 1,1-dioxide (E41)

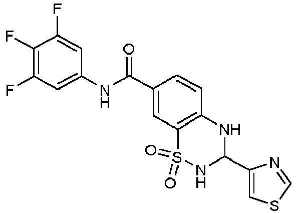

Compound **D100** was used as starting material. Thiazole-4-carbaldehyde was used instead of formaldehyde. Purified by preparative HPLC (H₂O/CH₃CN+0.1%HCOOH). Method 3; Rt=3.39 min, m/z=441.08 (M+H)⁺. ¹H NMR (300 MHz, DMSO-*d₆*) δ ppm 6.11 (s, 1 H) 7.09 (d, *J*=8.89 Hz, 1 H) 7.69 - 7.80 (m, 2 H) 7.94 (dd, *J*=8.89, 2.11 Hz, 1 H) 7.99 (d, *J*=1.56 Hz, 1 H) 8.19 (s, 1 H) 8.32 (d, *J*=2.02 Hz, 2 H) 9.23 (d, *J*=1.93 Hz, 1 H) 10.45 (s, 1 H).

### Example 42: 3-(6-(trifluoromethyl)pyridin-3-yl)-N-(3,4,5-trifluorophenyl)-3,4-dihydro-2H-benzo[e][1,2,4]thiadiazine-7-carboxamide 1,1-dioxide (E42)

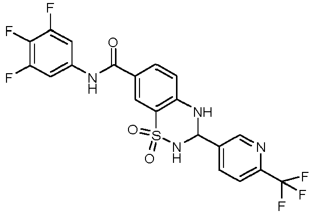

Compound **D100** was used as starting material. 6-(trifluoromethyl)pyridine-3-carboxaldehyde was used instead of formaldehyde. Purified by preparative HPLC (H₂O/CH₃CN+0.1%HCOOH). Method 3; Rt=3.73 min, m/z=503.09 (M+H)⁺. ¹H NMR (300 MHz, DMSO-*d₆*) δ ppm 6.19 (s, 1 H) 7.01 (d, *J*=8.80 Hz, 1 H) 7.73 (br d, *J*=6.60 Hz, 1 H) 7.77 (br d, *J*=6.60 Hz, 1 H) 7.98 (dd, J=8.89, 2.02 Hz, 1 H) 8.09 (d, *J*=8.07 Hz, 1 H) 8.25 (s, 1 H) 8.34 - 8.49 (m, 3 H) 9.07 (s, 1 H) 10.47 (s, 1 H).

### Example 43: 3-(thiophen-2-yl)-N-(3,4,5-trifluorophenyl)-3,4-dihydro-2H-benzo[e][1,2,4]thiadiazine-7-carboxamide 1,1-dioxide (E43)

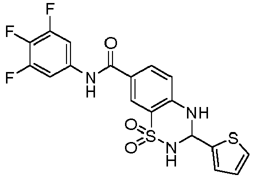

Compound **D100** was used as starting material. Thiophene-2-carbaldehyde was used instead of formaldehyde. Title compound **E43** was recovered by filtration from the reaction mixture. Method 3; Rt: 3.72 min. m/z: 440.06 (M+H)⁺. ¹H NMR (300 MHz, DMSO-*d₆*) δ ppm 0.00 (d, *J*=*11.60* Hz, 1 H) 7.09 (d, *J*=8.89 Hz, 1 H) 7.16 (dd, *J*=*5.00,* 3.60 Hz, 1 H) 7.50 (d, *J*=3.20 Hz, 1 H) 7.70 (dd, *J*=5.00, 1.00 Hz, 1 H) 7.73 - 7.84 (m, 2 H) 0.00 (dd, *J*=8.90, 2.10 Hz, 1 H) 8.29 (br s, 1 H) 8.32 - 8.40 (m, 2 H) 10.49 (br s, 1 H).

### Example 44: 3-(pyridin-2-yl)-N-(3,4,5-trifluorophenyl)-3,4-dihydro-2H-benzo[e][1,2,4]thiadiazine-7-carboxamide 1,1-dioxide (E44)

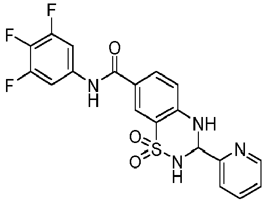

Compound **D100** was used as starting material. Picolinaldehyde was used instead of formaldehyde. Title compound **E44** was recovered by filtration from the reaction mixture. Method 3; Rt: 3.30 min. m/z: 435.27 (M+H)⁺. ¹H NMR (300 MHz, DMSO-*d₆*) δ ppm 5.93 - 6.06 (m, 1 H) 7.21 (d, *J*=8.90 Hz, 1 H) 7.49 - 7.63 (m, 1 H) 7.70 - 7.87 (m, 3 H) 7.93 - 8.09 (m, 2 H) 8.20 - 8.32 (m, 2 H) 8.38 (br s, 1 H) 8.72 (br d, *J*=3.70 Hz, 1 H) 10.48 (br s, 1 H).

### Example 45: 3-(1,2,3-thiadiazol-5-yl)-N-(3,4,5-trifluorophenyl)-3,4-dihydro-2H-benzo[e][1,2,4]thiadiazine-7-carboxamide 1,1-dioxide (E45)

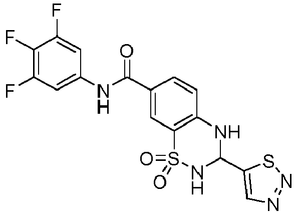

Compound **D100** was used as starting material. 1,2,3-thiadiazole-5-carbaldehyde was used instead of formaldehyde. Title compound **E45** was recovered by filtration from the reaction mixture. Method 3; Rt: 3.40 min. m/z: 442.09 (M+H)⁺. ¹H NMR (300 MHz, DMSO-*d₆*) δ ppm 6.58 (d, J=11.74 Hz, 1 H) 7.07 (d, *J*=8.90 Hz, 1 H) 7.75 (dd, J=10,55, 6.60 Hz, 2 H) 7.98 (dd, J=8.85, 2.16 Hz, 1 H) 8.36 (d, *J*=2.02 Hz, 1 H) 8.45 (s, 1 H) 8.61 (d, *J*=11.74 Hz, 1 H) 9.42 (s, 1 H) 10.48 (s, 1 H).

### Example 46: 3-(1H-pyrazol-5-yl)-N-(3,4,5-trifluorophenyl)-3,4-dihydro-2H-benzo[e][1,2,4]thiadiazine-7-carboxamide 1,1-dioxide (E46)

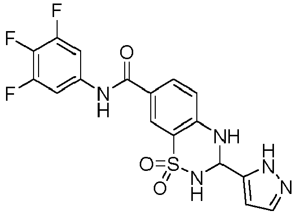

Compound **D100** was used as starting material. 1H-pyrazole-5-carbaldehyde was used instead of formaldehyde. Purified by preparative HPLC (H₂O/CH₃CN+0.1%HCOOH) to give the title compound **E46.** Method 3; Rt=3.19min. m/z= 424.12 (M+H)⁺. ¹H NMR (300 MHz, DMSO-*d₆*) δ ppm 5.93 (br s, 1 H) 6.57 (d, *J*=2.20 Hz, 1 H) 7.05 (d, *J*=8.80 Hz, 1 H) 7.68 - 7.85 (m, 3 H) 7.93 (dd, *J*=8.89, 2.02 Hz, 1 H) 8.02 - 8.25 (m, 2 H) 8.28 - 8.35 (m, 1 H) 10.45 (s, 1 H) 13.06 (br s, 1 H)

### Example 47: N-(3,4-difluorophenyl)-3-(pyridin-3-yl)-3,4-dihydro-2H-benzo[e][1,2,4]thiadiazine-7-carboxamide 1,1-dioxide (E47)

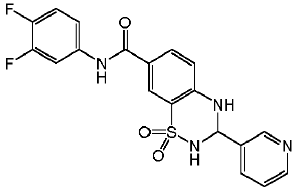

Compound **D101** was used as starting material and nicotinaldehyde was used instead of folrmaldehyde. Method 3; Rt: 2.46 min. m/z: 417.17 (M+H)⁺. ¹H NMR (300 MHz, DMSO-*d₆*) δ 6.05 (d, *J*=11.74 Hz, 1H), 7.01 (d, *J*=8.89 Hz, 1H), 7.36-7.49 (m, 1H), 7.52-7.67 (m, 2H), 7.86-8.05 (m, 2H), 8.15-8.24 (m, 2H), 8.31 (d, *J*=11.83 Hz, 1H), 8.36 (d, *J*=1.93 Hz, 1H), 8.72 (d, *J*=4.13 Hz, 1H), 8.92 (s, 1H), 10.37 (s, 1H).

### Example 48: 3-(pyridin-3-yl)-N-(3,4,5-trifluorophenyl)-3,4-dihydro-2H-benzo[e][1,2,4]thiadiazine-7-carboxamide 1,1-dioxide (E48)

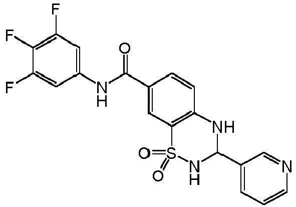

Compound **D100** was used as starting material. Nicotinaldehyde was used instead of formaline. Purified by preparative HPLC (H₂O/MeCN). Method 3; Rt: min.2.68 m/z: 435.00 (M+H)⁺. ¹H NMR (300 MHz, DMSO-*d₆*) δ 6.02 (br s, 1H), 7.01 (d,*J*=8.80 Hz, 1H), 7.54 (dd, *J*=7.93, 4.81 Hz, 1H), 7.75 (dd, J=10,50, 6.56 Hz, 2H), 7.97 (dd, *J*=8.71, 1.93 Hz, 1H), 8.11 (br d, *J*=7.89 Hz, 1H), 8.20 (s, 3H), 8.67 (dd, *J*=4.72, 1.24 Hz, 1H), 8.88 (d, *J*=1.74 Hz, 1H), 10.47 (s, 1H).

### Example 49: 3-(thiazol-2-yl)-N-(3,4,5-trifluorophenyl)-3,4-dihydro-2H-benzo[e][1,2,4]thiadiazine-7-carboxamide 1,1-dioxide (E49)

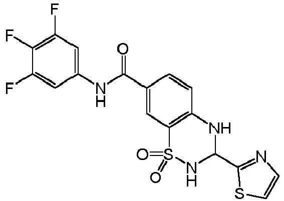

Compound **D100** was used as starting material; thiazole-2-carbaldehyde was used instead of formaline. Product was recovered by filtration of the reaction mixture. Method 3; Rt: min.3.46 m/z: 441.08 (M+H)⁺. ¹H NMR (300 MHz, DMSO-*d₆*) δ 6.25 (dd, *J*=1 1.42, 0.78 Hz, 1H), 7.20 (d, *J*=8.90 Hz, 1H), 7.75 (dd, J=10,59, 6.65 Hz, 2H), 7.89-8.00 (m, 3H), 8.32 (d, *J*=2.02 Hz, 1H), 8.47 (s, 1H), 8.65 (d, *J*=11.46 Hz, 1H), 10.46 (s, 1H).

### Example 50: N-(3,4-difluorophenyl)-3-(thiazol-2-yl)-3,4-dihydro-2H-benzo[e][1,2,4]thiadiazine-7-carboxamide 1,1-dioxide (E50)

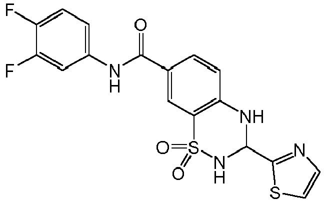

Compound **D101** was used as starting material and thiazole-2-carbaldehyde was used instead of formaline. Purified by preparative HPLC (H₂O, CH₃CN 0.1% TFA) to give the title compound **E50** (5.01mg). Method 3; Rt: 3.22min. m/z: 423.11 (M+H)⁺. ¹H NMR (300 MHz, DMSO-*d₆*) δ 6.25 (d, *J*=11.37 Hz, 1H), 7.19 (d, *J*=8.80 Hz, 1H), 7.37-7.48 (m, 1H), 7.51-7.65 (m, 1H), 7.84-8.06 (m, 4H), 8.32 (d, *J*=1.74 Hz, 1H), 8.43 (s, 1H), 8.64 (d, *J*=11.46 Hz, 1H), 10.37 (s, 1H).

### Example 51: 3-(1H-imidazol-5-yl)-N-(3,4,5-trifluorophenyl)-3,4-dihydro-2H-benzo[e][1,2,4]thiadiazine-7-carboxamide 1,1-dioxide (E51)

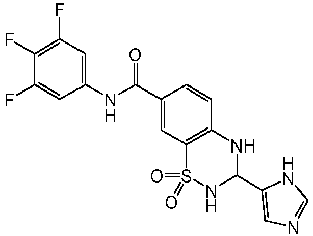

Compound **D100** was used as starting material. 1H-imidazole-5-carbaldehyde was used instead of formaline. The title compound **E51** was recovered by filtration of the reaction mixture. Method 3; Rt: 2.49min. m/z: 424.19 (M+H)⁺. ¹H NMR (300 MHz, DMSO-*d₆*) δ 6.16 (dd, *J*=10.36, 0.73 Hz, 1H), 7.07 (dd, *J*=8.94, 1.51 Hz, 1H), 7.69-7.82 (m, 4H), 7.95-8.09 (m, 1H), 8.27-8.42 (m, 1H), 8.48 (br d, *J*=10.27 Hz, 1H), 8.97 (br s, 1H), 10.49 (s, 1H), 13.64-14.85 (m, 1H).

### Example 52: 3-(6-chloropyridin-3-yl)-N-(3,4,5-trifluorophenyl)-3,4-dihydro-2H-benzo[e][1,2,4]thiadiazine-7-carboxamide 1,1-dioxide (E52)

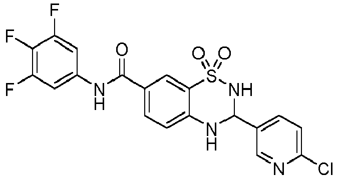

In a vial (5 mL) **D100** (28.2 mg, 0.082 mmol) was dissolved in 0,5 mL of IPA and 6-chloronicotinaldehyde (0.037 mL, 0.327 mmol) and 1 drop of HCl 4N in 1,4-dioxane were added. The vial was sealed and the reaction mixture was stirred and heated at 65°C for 1 h. The reaction mixture was directly purified by HPLC/MS preparative giving the title compound **E52** as white powder (19.91 mg). Method 3, RT=3.61min, m/z= 469,04, (M+H)⁺.¹H NMR (DMSO-d₆, 300 MHz): δ = 10.46 (s, 1H), 8.72 (d, *J* = 2.3 Hz, 1H), 8.34 (d, *J* = 1.9 Hz, 1H), 8.12-8.20 (m, 2H), 7.96 (dd, *J* = 8.8, 2.0 Hz, 1H), 7.75 (dd, *J* = 10,5, 6.6 Hz, 2H), 7.69 (d, *J* = 8.3 Hz, 1H), 6.99 (d, *J* = 8.8 Hz, 1H), 6.06 ppm (s, 1H).

### Example 53: 3-(5-chloropyridin-2-yl)-3-methyl-N-(3,4,5-trifluorophenyl)-3,4-dihydro-2H-benzo[e][1,2,4]thiadiazine-7-carboxamide 1,1-dioxide (E53)

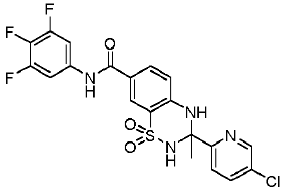

**D100** (32mg, 0.09 mmol) was dissolved in 2-propanol (900 uL), 1-(5-chloropyridin-2-yl) ethan-1-one (57.67mg, 0.37 mmol) and HCl 4N in dioxane (100 uL, 0.40 mmol) were added, and the solution was stirred at 95°C for 4h and overnight at room temperature. The reaction mixture was directly purified by preparative HPLC (H₂O/CH₃CN+0.1%HCOOH) to afford the title compound **E53** (15.82 mg) as white powder. Method 3; Rt: 3.67 min. m/z: 483.02 (M+H)⁺. ¹H NMR (300 MHz, DMSO-*d₆*) δ ppm 1.81 (s, 3 H) 7.13 (d, *J*=8.70 Hz, 1 H) 7.63 (d, *J*=8.60 Hz, 1 H) 7.68 - 7.83 (m, 2 H) 7.89 - 8.03 (m, 2 H) 8.21 (d, *J*=2.00 Hz, 1 H) 8.38 - 8.55 (m, 2 H) 8.59 (d, *J*=2.20 Hz, 1 H) 10.40 (br s, 1 H).

### Example 54: 3-(pyrimidin-2-yl)-N-(3,4,5-trifluorophenyl)-3,4-dihydro-2H-benzo[e][1,2,4]thiadiazine-7-carboxamide 1,1-dioxide (E54)

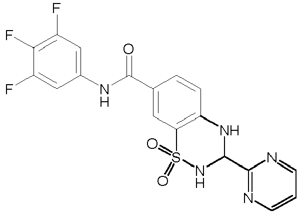

A mixture of **D100** (30mg, 0.09mmol) and pyrimidine-2-carbaldehyde (28.18mg, 0.26mmol) in IPA (600uL) was heated in a closed vial for 1h at 65°C. Purified by preparative HPLC (H₂O/CH₃CN+1%TFA), yielding the title compound **E54** (14mg). Method 3; Rt: 3.28min. m/z: 436.15 (M+H)⁺. ¹H NMR (300 MHz, DMSO-*d₆*) δ ppm 5.90 - 6.17 (m, 1 H) 7.07 - 7.31 (m, 1 H) 7.65 (t, *J*=4.91 Hz, 1 H) 7.75 (dd, *J*=10,55, 6.60 Hz, 2 H) 7.90 - 8.03 (m, 1 H) 8.21 - 8.32 (m, 3 H) 8.99 (d, *J*=4.95 Hz, 2 H) 10.33 - 10,54 (m, 1 H).

### Example 55: 3-methyl-3-(pyrazin-2-yl)-N-(3,4,5-trifluorophenyl)-3,4-dihydro-2H-benzo[e][1,2,4]thiadiazine-7-carboxamide 1,1-dioxide (E55)

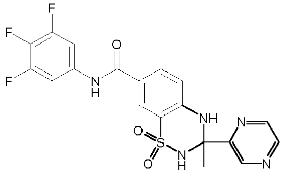

A mixture of **D100** (31mg, 0.09mmol) and 2-acetylpyrazine (51.53mg, 0.42mmol) in IPA (1.5mL) was treated with 4N HCl (314.22uL,1.26mmol), heated to 80°C in a closed vial, treated with 4N HCl (100uL) and heated at 140°C for 15min. The reaction was further treated with 4N HCl (200uL) and stirred overnight at room temperature. Solvent was removed *in vacuo* and the residue was purified by flash chromatography on direct phase (DCM/MeOH), the title compound **E55** (1.47mg). Method 3; Rt: 3.15min. m/z: 450.1 (M+H)⁺. ¹H NMR (300 MHz, DMSO-*d₆*) δ ppm 1.84 (s, 3 H) 7.11 (d, *J*=8.89 Hz, 1 H) 7.62 - 7.81 (m, 2 H) 7.96 (dd, *J*=8.85, 2.15 Hz, 1 H) 8.17 (d, *J*=2.02 Hz, 1 H) 8.51 - 8.60 (m, 4 H) 8.85 (d, *J*=1.28 Hz, 1 H) 10.37 (s, 1 H).

### Example 56: 2'-oxo-N-(3,4,5-trifluorophenyl)-2H,4H-spiro[benzo[e][1,2,4]thiadiazine-3,3'-indoline]-7-carboxamide 1,1-dioxide (E56)

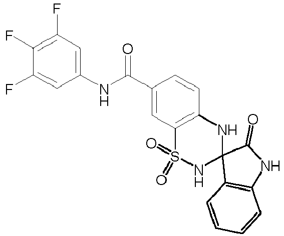

A mixture of **D100** (30mg, 0.09mmol), indoline-2,3-dione (51.13mg, 0.35mmol) in IPA (1.5mL) was treated with 4N HCl (20uL, 1eq) and heated at 80°C in a closed vial for 2-2.5hrs. More 4N HCl was added (100uL) in a single portion and the vial was heated at 100°C for 1h. The reaction solution was filtered giving the title compound **E56** (11.84mg). Method 3; Rt: 3.47min. m/z: 475.12 (M+H)⁺. ¹H NMR (300 MHz, DMSO-*d₆*) δ ppm 6.87 - 6.97 (m, 1 H) 6.87 - 6.97 (m, 1 H) 7.04 (td, *J*=7.61, 0.73 Hz, 1 H) 7.34 (td, *J*=7.70, 1.19 Hz, 1 H) 7.56 - 7.68 (m, 1 H) 7.75 (dd, J=10,55, 6.60 Hz, 2 H) 7.97 (dd, *J*=8.76, 2.06 Hz, 1 H) 8.29 - 8.36 (m, 2 H) 8.61 (s, 1 H) 10.43 - 10,54 (m, 1 H) 10.67 - 10.78 (m, 1 H).

The racemic mixture was dissolved in 200 µL of methanol in order to separate the two enantiomers (herein reported as **E129** and **E130**) by chiral phase HPLC. Chiral Separation Method: isocratic analysis with Chiral HPLC Analytical column , Chiralpack IB-N5 (Daicel corporation), 0.46 cm I.D.x 25 cm L at 20% B (Phase A: n-Heptane and Phase B: 2-Propanol). Semiprep-scale-up: isocratic analysis with Chiral HPLC Semiprep column , Chiralpack IB-N5 (Daicel corporation), 1cm I.D.x 25 cm L at 20% B (Phase A: n-Heptane and Phase B: 2-Propanol).

### Example 57: 2'-oxo-N-(3,4,5-trifluorophenyl)-1',2'-dihydro-2H,4H-spiro[benzo[e][1,2,4]thiadiazine-3,3'-pyrrolo[2,3-b]pyridine]-7-carboxamide 1,1-dioxide (E57)

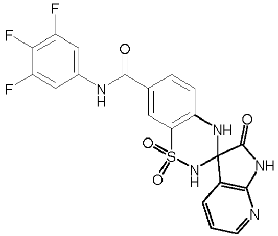

Similary prepared according to the procedure described for the preparation of **E56,** but using at room temperature 1H-Pyrrolo[2,3-b]pyridine-2,3-dione instead of 2,3-Indolinedione. Purification was performed by preparative HPLC (H₂O/CH₃CN+ 0.1%HCOOH). Method 3; Rt: 3.27. m/z: 476.27y (M+H)⁺. ¹H NMR (300 MHz, DMSO-d6) δ ppm 6.87 - 6.97 (m, 1 H) 6.87 - 6.97 (m, 1 H) 7.04 (td, J=7.61, 0.73 Hz, 1 H) 7.34 (td, J=7.70, 1.19 Hz, 1 H) 7.56 - 7.68 (m,1 H) 7.75 (dd, J=10.55, 6.60 Hz, 2 H) 7.97 (dd, J=8.76, 2.06 Hz, 1 H) 8.29 - 8.36 (m, 2 H) 8.61 (s, 1 H) 10.43 - 10.54 (m, 1 H) 10.67 - 10.78 (m, 1 H).

### Example 58: trans-2-(4-hydroxycyclohexyl)-N-(3,4,5-trifluorophenyl)-3,4-dihydro-2H-benzo[e][1,2,4]thiadiazine-7-carboxamide 1,1-dioxide (E58)

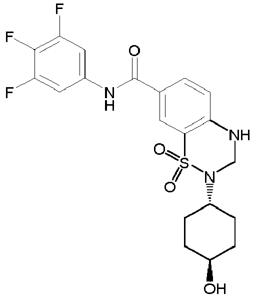

A mixture of **D61** (35mg, 0.08mmol) and formaldehyde (22.68uL, 0.32mmol) in IPA (2mL) was treated with 4N HCl in dioxane (21.71uL, 0.09mmol) and heated in a closed vial at 65°C for 30min. The reaction solution was purified by preparative HPLC (H₂O/CH₃CN+1%TFA) affording the title compound **E58** (14mg). Method 3; Rt: 3.32min. m/z: 456.14 (M+H)⁺. ¹H NMR (300 MHz, DMSO-*d₆*) δ ppm 0.98 - 1.33 (m, 2 H) 1.50 - 1.72 (m, 4 H) 1.72 - 1.91 (m, 2 H) 3.44 - 3.62 (m, 3H) 4.96 (d, *J*=2.84 Hz, 2 H) 6.88 (d, *J*=8.80 Hz, 1 H) 7.67 - 7.76 (m, 2 H) 7.77 - 7.83 (m, 1 H) 7.90 (dd, *J*=8.85, 2.06 Hz, 1 H) 7.99 - 8.05 (m, 1 H) 8.24 (d, *J*=2.02 Hz, 1 H) 10.39 (s, 1 H). ¹H NMR (300 MHz, DMSO-*d*₆+TFA) δ ppm 0.98 - 1.31 (m, 3 H) 1.52 - 1.72 (m, 4 H) 1.72 - 1.92 (m, 2 H) 2.06 (s, 1 H) 3.21 - 3.42 (m, 1 H) 3.51 (br t, *J*=7.02 Hz, 1 H) 4.96 (s, 2 H) 6.87 (d, *J*=8.80 Hz, 1 H) 7.59 - 7.84 (m, 3 H) 7.89 (dd, *J*=8.89, 2.11 Hz, 1 H) 8.24 (d, *J*=2.11 Hz, 1 H) 10.38 (s, 1 H).

### Example 59: cis-2-(4-hydroxycyclohexyl)-N-(3,4,5-trifluorophenyl)-3,4-dihydro-2H-benzo[e][1,2,4]thiadiazine-7-carboxamide 1,1-dioxide (E59)

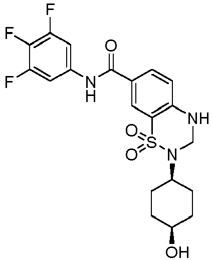

Similarly prepared according to the procedure described for the preparation of compound **E1**, starting from **D49**. Method 3: Rt=3.47min, m/z=456.21 (M+H)⁺. ¹H NMR (300 MHz, DMSO-*d*₆+TFA) δ ppm 1.27 - 1.49 (m, 4 H) 1.55 - 1.71 (m, 2 H) 1.85 - 2.04 (m, 2 H) 3.47 - 3.64 (m, 1 H) 3.66 - 3.81 (m, 1 H) 4.96 (s, 2 H) 6.87 (d, *J*=8.80 Hz, 1 H) 7.63 - 7.78 (m, 2 H) 7.78 - 8.04 (m, 2 H) 8.24 (d, *J*=2.02 Hz, 1 H) 10.38 (s, 1 H).

### Example 60: 2-cyclopropyl-N-(3,4,5-trifluorophenyl)-3,4-dihydro-2H-benzo[e][1,2,4]thiadiazine-7-carboxamide 1,1-dioxide (E60)

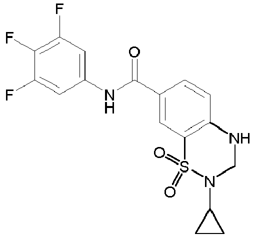

**D117** (30mg, 0.08mmol) and formaldehyde (22.37uL, 0.31mmol) in IPA (2mL) was treated with 4N HCl (21uL, 0.086mmol, 1.1eq) and heated at 65°C for 1h. The reaction solution was purified by preparative HPLC (H₂O/CH₃CN+1%TFA) affording, the title compound **E60** (12mg). Method 3; Rt: 3.71min. m/z: 398.18 (M+H)⁺. ¹H NMR (300 MHz, DMSO-*d₆*) δ ppm 0.66 - 0.84 (m, 4 H) 2.15 (tt, *J*=6.63, 3.37 Hz, 1 H) 3.43 (br s, 5 H) 4.86 (d, *J*=2.75 Hz, 2 H) 6.96 (d, *J*=8.80 Hz, 1 H) 7.67 - 7.78 (m, 2 H) 7.95 (dd, *J*=8.80, 2.30 Hz, 1 H) 7.99 - 8.06 (m, 1 H) 8.30 (d, *J*=2.11 Hz, 1 H) 10.43 (br s, 1 H).

### Example 61: 2-(piperidin-4-yl)-N-(3,4,5-trifluorophenyl)-3,4-dihydro-2H-benzo[e][1,2,4]thiadiazine-7-carboxamide 1,1-dioxide (E61)

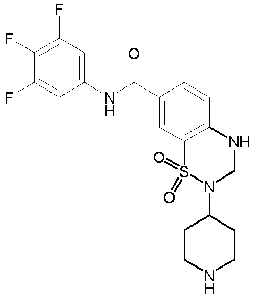

In a 5mL vial, solution of **D124** (30mg, 0.06mmol) and formaldehyde (44.07uL, 0.23mmol) in IPA (2mL) was treated with 4N HCl in dioxane (15.61uL, 0.06mmol). The reaction was heated at 65°C for 1h, cooled to room temperature, treated with 37% HCl (500uL) and heated at 100°C for 30min. The reaction mixture was cooled to room temperature and purified by preparative HPLC (H₂O/CH₃CN+1%TFA) affording the title compound **E61** (4.41mg) as TFA salt. Method 3; Rt: 2.63min. m/z: 441.21 (M+H)⁺. ¹H NMR (300 MHz, DMSO-*d₆*) δ ppm 1.75 - 2.07 (m, 4 H) 2.80 - 3.08 (m, 2 H) 3.21 - 3.28 (m, 1 H) 3.71 - 3.96 (m, 1 H) 4.97 (br d, *J*=1.83 Hz, 2 H) 6.92 (d, *J*=8.89 Hz, 1 H) 7.72 (dd, *J*=10,55, 6.60 Hz, 2 H) 7.89 - 7.97 (m, 1 H) 8.00 (br s, 1 H) 8.11 - 8.25 (m, 1 H) 8.29 (d, *J*=1.93 Hz, 1 H) 8.37 - 8.67 (m, 1 H) 10.41 (s, 1 H).

### Example 62: 2-(2-hydroxyethyl)-N-(3,4,5-trifluorophenyl)-3,4-dihydro-2H-benzo[e][1,2,4]thiadiazine-7-carboxamide 1,1-dioxide (E62)

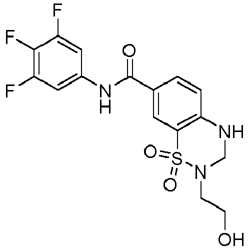

In a vial (5 mL) **D91** (24 mg, 0.062 mmol) was dissolved in IPA (0,5 mL) then formaldehyde, 37% aq., (0.037 mL) and 1 drop of HCl 4N in 1,4-dioxane were added. The vial was sealed and the reaction mixture was stirred and heated at 65°C for 1.5 h. The crude product was purified preparative HPLC (H₂O/CH₃CN+0.1% TFA) giving the title compound E62 as white powder (11.46 mg). Method 3: Rt= 3.13min, m/z=402.10 (M+H)⁺.
¹H NMR (DMSO-d₆, 300 MHz): δ = 10.41 (s, 1H), 8.26 (d, *J=* 2.1 Hz, 1H), 7.89-7.98 (m, 2H), 7.72 (dd, *J=* 10,5, 6.6 Hz, 2H), 6.95 (d, *J=* 8.9 Hz, 1H), 4.96 (d, *J=* 2.8 Hz, 2H), 3.63 (br t, *J=* 5.9 Hz, 3H), 2.97 ppm (t, *J* = 5.8 Hz, 2H).

### Example 63: 2-(2-hydroxyethyl)-3-methyl-N-(3,4,5-trifluorophenyl)-3,4-dihydro-2H-benzo[e][1,2,4]thiadiazine-7-carboxamide 1,1-dioxide (E63)

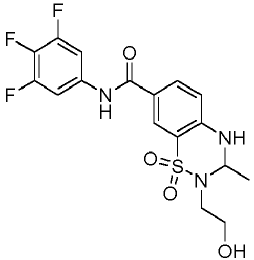

In a vial (5 mL) **D91** (24 mg, 0.062 mmol) was dissolved in IPA (0,5 mL) then of acetaldehyde (0.017 mL, 0.308 mmol) and 1 drop of HCl 4N in 1,4-dioxane were added. The vial was sealed and the reaction mixture was stirred and heated at 65°C for 1.5 h. The crude product was purified by preparative HPLC (H₂O/CH₃CN) giving the title compound **E63** (8.86 mg) Method 3: Rt= 3.21min, m/z=416.15 (M+H)⁺. ¹H NMR (DMSO-d₆, 300 MHz): δ = 10.42 (s, 1H), 8.28 (d,*J* = 1.9 Hz, 1H), 7.88-8.01 (m, 2H), 7.71 (dd, *J*= 10.6, 6.6 Hz, 2H), 6.93 (d, *J* = 8.8 Hz, 1H), 5.35 (q, *J* = 6.0 Hz, 1H), 4.79 (br s, 1H), 3.43-3.63 (m, 2H), 2.97 (dt, *J=* 14.5, 7.3 Hz, 1H), 2.72-2.87 (m, 1H), 1.50 ppm (d, *J* = 6.4 Hz, 3H).

### Example 64: N-(3,4,5-trifluorophenyl)-2,3,10,10a-tetrahydro-1H-benzo[e]pyrrolo[1,2-b][1,2,4]thiadiazine-7-carboxamide 5,5-dioxide (E64)

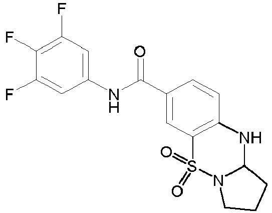

**D51** (60mg, 0.12mmol) was dissolved in dimethoxyethane (1mL) and treated at room temperature with a single portion of 37% HCl (200uL). The reaction was stirred at room temperature for 15min. The reaction was partitioned between water and DCM. The organic layer was washed with brine, dried over Na₂SO₄, filtered and finally evaporated giving a residue (50mg, white solid). This residue was suspended in DCM and filtered giving the title compound **E64** (14mg)Method 3; Rt: 3.69min. m/z: 398.25 (M+H)⁺. ¹H NMR (300 MHz, DMSO-*d₆*) δ ppm 1.88 - 2.35 (m, 4 H) 2.84 - 2.98 (m, 1 H) 3.35 - 3.48 (m, 1 H) 5.34 (d, *J*=4.31 Hz, 1 H) 6.87 (d, *J*=8.80 Hz, 1 H) 7.65 - 7.80 (m, 3 H) 7.92 (dd, *J*=8.85, 2.15 Hz, 1 H) 8.25 (d, *J*=2.02 Hz, 1 H) 10.40 (s, 1 H).

### Example 65: N-(3-chloro-4-fluorophenyl)-3,4-dihydro-2H-benzo[e][1,2,4]thiadiazine-7-carboxamide 1,1-dioxide (E65)

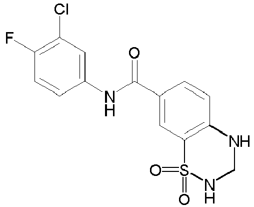

A mixture of **D107** (50mg, 0.15mmol) in IPA (2mL) was treated with formaldehyde (112.94uL, 0,58mmol) and 4N HCl (4N in dioxane, 40uL) the mixture was heated at 70°C in a closed vial for 4hrs. Solvent was removed *in vacuo* and the residue purified by preparative HPLC (H₂O/CH₃CN+1% TFA) to give the title compound **E65.** Method 3; Rt: 3.17min. m/z: 355.77 (M+H)⁺. ¹H NMR (300 MHz, DMSO-*d₆*) δ ppm 4.70 (br s, 2 H) 6.87 (d, *J*=8.80 Hz, 1 H) 7.40 (t, *J*=9.12 Hz, 1 H) 7.70 - 7.85 (m, 3 H) 7.91 (dd, *J*=8.80, 2.11 Hz, 1 H) 8.06 (dd, *J*=6.97, 2.57 Hz, 1 H) 8.27 (d, *J*=2.02 Hz, 1 H) 10.28 (s, 1 H).

### Example 66: N-(3,4-difluorophenyl)-2H-benzo[e][1,2,4]thiadiazine-7-carboxamide 1,1-dioxide (E66)

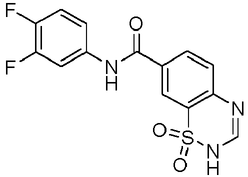

In a sealed microwave vial **D101** (43 mg, 0.131 mmol) was dissolved in formic acid (2 mL) and the solution heated at 110°C for 2.5h. The crude product was purified by preparative HPLC (H₂O/CH₃CN+0.1% TFA) to give the title compound **E66** as white powder (24.18 mg). Method 3; Rt=2.80 min, m/z=337.99 (M+H)⁺. ¹H NMR (300 MHz, DMSO-*d₆*) δ ppm 7.41 - 7.53 (m, 2 H) 7.53 - 7.60 (m, 1 H) 7.94 (ddd, *J*=13.30, 7.52, 2.48 Hz, 1 H) 8.08 (s, 1 H) 8.25 (dd, *J*=8.71, 2.02 Hz, 1 H) 8.54 (d, *J*=1.93 Hz, 1 H) 10.68 (s, 1 H) 12.57 (br s, 1 H).

### Example 67: N-(3,4-difluorophenyl)-3-methyl-2H-benzo[e][1,2,4]thiadiazine-7-carboxamide 1,1-dioxide (E67)

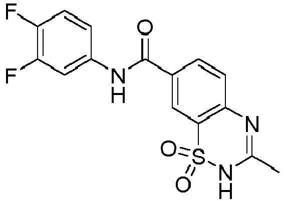

In a round bottom flask **D101** (40 mg, 0.122mmol) was treated at 80 °C overnight in CH₃COOH but the reaction did not occur. The reaction was performed in a sealed vial by means of a microwave reactor and was heated for 10 hours at 150°C after addition of 2 mL of acetic anhydride. The crude product was purified by preparative HPLC (H₂O/CH₃CN+0.1% TFA) to give the title compound **E67** (1.1 mg) as white powder. Method 3: Rt=2.85 min, m/z= 352.18 (M+H)⁺. ¹H NMR (DMSO-d₆, 300 MHz): δ ppm 12.06-12.63 (m, 1H), 10.67 (br s, 1H), 8.50 (br s, 1H), 8.23 (br d, J = 8.3 Hz, 1H), 7.93 (br dd, J = 11.3, 7.5 Hz, 1H), 7.49-7.61 (m, 1H), 7.44 (br d, J = 8.3 Hz, 2H), 2.35 ppm (s, 3H).

### Example 68: 6-fluoro-N-(3,4,5-trifluorophenyl)-2H-benzo[e] [1,2,4]thiadiazine-7-carboxamide 1,1-dioxide (E68)

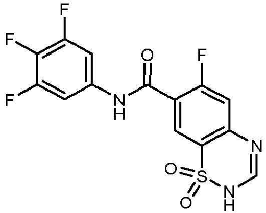

In a microwave vial **D28** (90 mg, 0.229mmol) was dissolved in DMSO (4.5 mL), cesium carbonate (300 mg, 0.921mmol) was added, the vial sealed and heated at 100°C for 30 min under MW. Mixture was diluted with AcOEt and washed with brine two times. Organic layer was dried over Na₂SO₄, filtered and solvent removed under reduced pressure. The crude title compound was purified by preparative HPLC (H₂O/CH₃CN+0.1% TFA) to give the title compound **E68** (5.12 mg) as white powder. Method 3; Rt: 3.11 min. m/z: 374.07 (M+H)⁺. ¹H NMR (300 MHz, DMSO-*d*₆) δ ppm 7.26 (d, *J*=10.82 Hz, 1 H) 7.53 - 7.75 (m, 2 H) 8.10 (s, 1 H) 8.20 (d, *J*=7.06 Hz, 1 H) 10.91 (s, 1 H) 12.58 (br s, 1 H).

### Example 69: 3-amino-N-(3,4,5-trifluorophenyl)-2H-benzo[e] [1,2,4]thiadiazine-7-carboxamide 1,1-dioxide (E69)

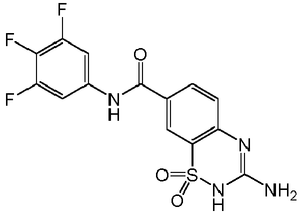

**D29** (212.33mg, 0,54mmol) was dissolved in DMSO (2mL), treated with cesium carbonate (600mg, 1.83mmol) and heated to 90°C for 3hrs. The reaction was diluted with water and extracted with DCM. The acqueous layer was acidified to pH= 7 with 6N HCl and further extracted with DCM. The combined organic extracts were dried over MgSO₄ (dry), filtered and finally evaporated. The residue was purified by preparative HPLC (H₂O, CH₃CN 0.1% TFA) yielding the title compound **E69** (31mg) as white solid. Method 3; Rt: 2.82min. m/z: 371.09 (M+H)⁺. ¹H NMR (300 MHz, DMSO-*d₆*) δ ppm 7.11 - 7.27 (m, 1 H) 7.32 (d, *J*=8.71 Hz, 1 H) 7.67 - 7.83 (m, 2 H) 8.13 (dd, *J*=8.62, 2.02 Hz, 1 H) 8.41 (d, *J*=1.93 Hz, 1 H) 10.61 - 10.75 (m, 1 H) 11.11 (s, 1 H).

### Example 70: 3-acetamido-N-(3,4,5-trifluorophenyl)-2H-benzo[e] [1,2,4]thiadiazine-7-carboxamide 1,1-dioxide (E70)

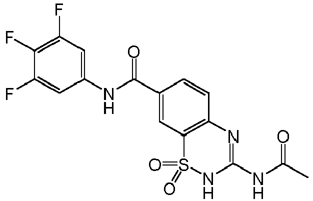

Similary prepared according to the procedure described for the preparation of compound **E69,** using as starting material **D30.** Method 3; Rt: 3.18min. m/z: 413.11 (M+H)⁺. ¹H NMR (300 MHz, DMSO-*d₆*) δ ppm 2.20 (s, 3 H) 7.68 - 7.83 (m, 3 H) 8.22 (dd, *J*=8.71, 2.02 Hz, 1 H) 8.48 (d, *J*=1.93 Hz, 1 H) 10.76 (s, 1 H) 11.65 (br s, 1 H) 12.16 (br s, 1 H).

### Example 71: 6-fluoro-N-(3,4,5-trifluorophenyl)-3,4-dihydro-2H-benzo[e][1,2,4]thiadiazine-7-carboxamide 1,1-dioxide (E71)

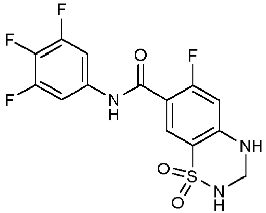

To a stirred solution of **E68** (28 mg, 0.075 mmol) in dry THF (1 mL), sodium borohydride (28.4 mg, 0.751 mmol) and methanol (0.050 mL) were added. To have complete conversion two additions of sodium borohydride (15 mg, 0.396 mmol) were made during 7.5h. Mixture was diluted with slow addition of water and TFA in order to purify crude with preparative HPLC (H₂O/CH₃CN+0.1% TFA) to give the title compound **E71** as white powder (8.95 mg). Method 3; Rt: 3.28 min. m/z: 376.09 (M+H)⁺. ¹H NMR (300 MHz, DMSO-*d*₆) δ ppm 4.72 (dd, *J*=7.98, 2.38 Hz, 2 H) 6.65 (d, *J*=13.11 Hz, 1 H) 7.58 - 7.71 (m, 2 H) 7.84 - 8.01 (m, 3 H) 10.43 (s, 1 H).

### Example 72: 5-fluoro-N-(3,4,5-trifluorophenyl)-3,4-dihydro-2H-benzo[e][1,2,4]thiadiazine-7-carboxamide 1,1-dioxide (E72)

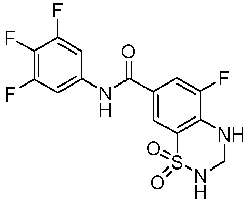

**D96** (49mg, 0.130mmol) was dissolved in water (1.5mL) and THF (1mL), sodium tetrahydroborate (59.59mg, 1.58mmol) was added, and the reaction mixture was stirred at room temperature for 3.5h. More sodium tetrahydroborate (59.59mg, 1.58mmol) was added, and the reaction mixture was stirred at room temperature overnight. The reaction mixture was directly purified by preparative HPLC (H₂O/CH₃CN+1%TFA) to obtain, after lyophilisation the title compound **E72** (18mg) as white solid. Method 3; Rt=3.45min, m/z=376.16 (M+H)⁺. ¹H NMR (300 MHz, DMSO-*d*₆) δ ppm 4.62 - 4.86 (m, 2 H) 7.63 - 7.80 (m, 2 H) 7.80 - 7.95 (m, 2 H) 8.04 (br s, 1 H) 8.12 - 8.23 (m, 1 H) 10.45 (s, 1 H).

### Example 73: 6-fluoro-4-methyl-N-(3,4,5-trifluorophenyl)-4H-benzo[e][1,2,4]thiadiazine-7-carboxamide 1,1-dioxide (E73)

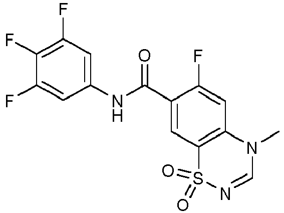

To a stirred solution of sodium **E68** (31.7 mg, 0.080 mmol) in dry DMF (1 mL), iodomethane (5 uL, 0.088 mmol) was added. To have complete conversion more iodomethane (15 uL, 0.264 mmol) was added during 3h. Crude was diluted with water and acetonitrile for purification with preparative HPLC (H₂O/CH₃CN+0.1% TFA) to give the title compound **E73** as white powder (8.62 mg). Method 3; Rt: 3.21 min. m/z: 388.05 (M+H)⁺. ¹H NMR (300 MHz, DMSO-*d*₆) δ ppm 3.65 (s, 3 H) 7.56 - 7.72 (m, 3 H) 8.16 (s, 1 H) 8.25 (d, *J*=7.24 Hz, 1 H) 10.94 (s, 1 H).

### Example 74: N-(3,4-difluorophenyl)-3-oxo-3,4-dihydro-2H-benzo[e][1,2,4]thiadiazine-7-carboxamide 1,1-dioxide (E74)

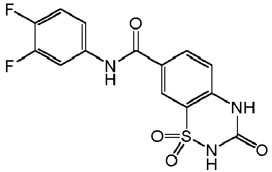

**D101** (40mg, 0.12mmol) was suspended in DCM (0.8mL, 0.012mol) and N-ethyl-N-isopropylpropan-2-amine (72.37uL, 0.42mmol) was added. The suspension was cooled to 0°C. Triphosgene (15.23mg, 0.05mmol) was added and the reaction was stirred at 0°C for 1h. The resulting yellow solution become a white suspension; water (100uL) was added in a single portion at 0°C, the mixture was stirred 2min then filtered. The precipitate was purified by preparative HPLC (H₂O/CH₃CN 0.1% TFA) giving the title compound **E74** (6.7mg) as white solid. Method 3; Rt: 2.57min. m/z: 354.00 (M+H)⁺. ¹H NMR (300 MHz, DMSO-*d*₆) δ ppm 7.33 (d, *J*=8.80 Hz, 1 H) 7.39 - 7.51 (m, 1 H) 7.52 - 7.60 (m, 1 H) 7.93 (ddd, *J*=13.27, 7.54, 2.48 Hz, 1 H) 8.19 (dd, *J*=8.50, 1.90 Hz, 1 H) 8.44 (d, *J*=1.74 Hz, 1 H) 10,59 (s, 1 H) 11.36 (br s, 1 H).

### Example 75: 3-oxo-N-(3,4,5-trifluorophenyl)-3,4-dihydro-2H-benzo[e][1,2,4]thiadiazine-7-carboxamide 1,1-dioxide (E75)

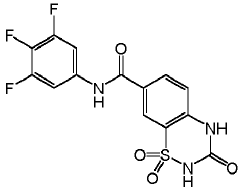

Prepared similary according to the procedure described for the preparation of **E74** using as starting material **D100** and purified by preparative HPLC (H₂O,CH₃CN 0.1% TFA). Method 3; Rt: 2.85min. m/z: 369.92 (M-H)⁻. ¹H NMR (300 MHz, DMSO-d6) δ ppm 6.01 - 6.05 (m, 1 H) 7.23 (d, J=8.62 Hz, 1 H) 7.75 (dd, J=10.45, 6.60 Hz, 2 H) 8.08 (dd, J=8.62, 1.74 Hz, 1 H) 8.35 - 8.39 (m, 1 H) 10.62 (s, 1 H) 10.69 - 10.95 (m, 1 H).

### Example 76: 2-methyl-3-oxo-N-(3,4,5-trifluorophenyl)-3,4-dihydro-2H-benzo[e][1,2,4]thiadiazine-7-carboxamide 1,1-dioxide (E76)

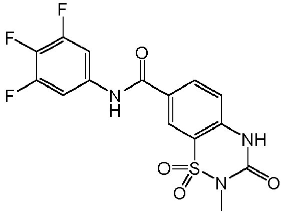

**D99** (60.mg,0.170mmol) was dissolved in DCM (0.8mL), treated with DIPEA (233uL, 8eq), cooled to 0°C and treated with triphosgene (148.66mg, 0,5mmol) added in 3 equal portion over 1.5hrs. The reaction was poured in water and extracted with DCM. The combned organic extracts were dried over MgSO₄ (dry) filtered and evaporated, giving a residue, that was purified by prepartative HPLC (H₂O, CH₃CN 0.1% TFA). Obtained the title compound **E76** (7.9mg). Method 3; Rt: 3.50min. m/z: 383.97 (M-H)⁻. ¹H NMR (300 MHz, DMSO-*d*₆) δ ppm 3.22 - 3.28 (m, 3 H) 7.42 (d, *J*=8.71 Hz, 1 H) 7.63 - 7.82 (m, 2 H) 8.25 - 8.31 (m, 1 H) 8.53 (d, *J*=1.93 Hz, 1 H) 10.73 (br s, 1 H) 11.81 (br s, 1 H).

### Example 77: (R)-3-oxo-N-(3,4,5-trifluorophenyl)-2-(1,1,1-trifluoropropan-2-yl)-3,4-dihydro-2H-benzo[e] [1,2,4]thiadiazine-7-carboxamide 1,1-dioxide (E77)

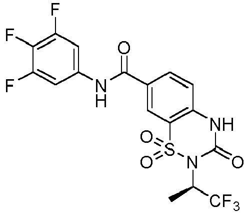

A mixture of **D115** (25.5mg, 0.06mmol) and 1,1'-carbonyldiimidazole (37.47mg, 0.23mmol) in DMF (0,5mL) was treated with triethylamine (0.16mL, 0.12mmol) and heated by microwave irradiation at 150°C for 45min. The reaction mixture was cooled to room temperature and purified by preparative HPLC (H₂O/CH₃CN+0.1%TFA) to obtain, after lyophilization, the title compound **E77** (7mg) as white solid. Method 3; Rt: 3.97min, m/z: 468.16 (M+H)⁺. ¹H NMR (300 MHz, DMSO-*d*₆) δ ppm 1.71 (d, *J*=6.80 Hz, 3 H) 5.29 (br s, 1 H) 7.47 (d, *J*=8.62 Hz, 1 H) 7.62 - 7.79 (m, 2 H) 8.30 (dd, *J*=8.53, 1.93 Hz, 1 H) 8.51 (d, *J*=1.74 Hz, 1 H) 10.75 (s, 1 H) 12.01 (br s, 1 H).

### Example 78: (S)-3-oxo-N-(3,4,5-trifluorophenyl)-2-(1,1,1-trifluoropropan-2-yl)-3,4-dihydro-2H-benzo[e][1,2,4]thiadiazine-7-carboxamide 1,1-dioxide (E78)

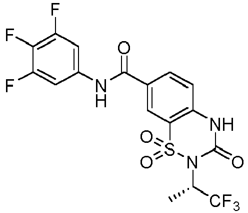

Similarly prepared according to the procedure described for the preparation of compound **E77**, starting from **D116.** Method 3; Rt: 3.97min. m/z: 468.16 (M+H)⁺. ¹H NMR (300 MHz, DMSO-*d*₆) δ ppm 1.71 (d, *J*=6.90 Hz, 3 H) 5.34 (br s, 1 H) 7.46 (d, *J*=8.62 Hz, 1 H) 7.60 - 7.83 (m, 2 H) 8.30 (dd, *J*=8.53, 2.02 Hz, 1 H) 8.51 (d, *J*=1.83 Hz, 1 H) 10.75 (s, 1 H) 12.00 (br s, 1 H).

### Example 79: 2-cyclopropyl-3-oxo-N-(3,4,5-trifluorophenyl)-3,4-dihydro-2H-benzo[e][1,2,4]thiadiazine-7-carboxamide 1,1-dioxide (E79)

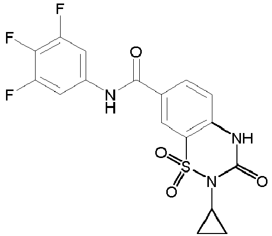

A mixture of **D117** (40mg, 0.1mmol) and di(1H-imidazol-1-yl)methanone (43.2mg, 0.27mmol) in DMF (0.62mL) was treated with triethylamine (18.47uL, 0.13mmol) and heated by microwave irradiation at 150°C for 11min. The reaction mixture was cooled to room temperature and the reaction solution purified by preparative HPLC (H₂O/CH₃CN+1 TFA) to afford, after lyophilization the title compound **E79** (12.2mg). ¹H NMR (300 MHz, DMSO-*d*₆) δ ppm 0.75 - 1.13 (m, 4 H) 2.08 (s, 1 H) 2.75 - 2.85 (m, 1 H) 7.37 (d, *J*=8.62 Hz, 1 H) 7.62 - 7.82 (m, 2 H) 8.25 (dd, *J*=8.62, 2.02 Hz, 1 H) 8.49 (d, *J*=1.93 Hz, 1 H) 10.71 (br s, 1 H) 11.72 (br s, 1 H). Method 3; Rt: 3.68min. m/z: 412.17 (M+H)⁺.

### Example 80: trans-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide (E80)

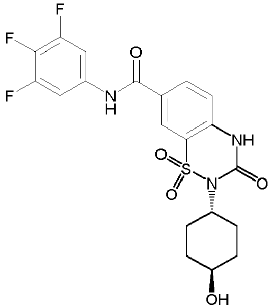

A solution of **D118** (50mg, 0.09mmol) in DMSO (1.5mL) was treated with 37% HCl (3 drops) and heated at 50°C for 1h. The reaction was diluted with water and extracted with DCM 3 times. The combined organic extracts were washed with NaHCO₃ (sat. solution), dried over Na₂SO₄, filtered and finally evaporated under reduced pressure, giving a residue which was purified by preparative HPLC (H₂O/CH₃CN+1%TFA) affording the title compound **E80** (3.59mg). ¹H NMR (300 MHz, DMSO-*d*₆) δ ppm 1.17 - 1.47 (m, 2 H) 1.56 - 2.10 (m, 4 H) 2.16 - 2.42 (m, 2 H) 3.39 - 3.53 (m, 1 H) 4.25 - 4.42 (m, 1 H) 4.43 - 4.97 (m, 1 H) 7.39 (d, *J*=8.62 Hz, 1 H) 7.57 - 7.84 (m, 2 H) 8.25 (dd, *J*=8.62, 1.93 Hz, 1 H) 8.46 (d, *J*=1.83 Hz, 1 H) 10.72 (s, 1 H) 11.59 - 11.80 (m, 1 H). ¹H NMR (300 MHz, DMSO-*d5*+*TFA)p*pm 2.22 - 2.32 (m, 2 H) 2.49 (dd, *J*=18.87, 4.38 Hz, 4 H) 2.63 - 2.72 (m, 2 H) 3.07 - 3.17 (m, 1 H) 3.44 - 3.54 (m, 1 H) 4.71 (d, *J*=3.45 Hz, 1 H) 4.79 - 4.88 (m, 2 H) 5.05 (dd, *J*=3.45, 0.77 Hz, 1 H) 5.13 (d, *J*=0.73 Hz, 1 H) 6.04 (s, 1 H) 6.43 (s, 1 H). Method 3; Rt: 3.42min. m/z: 468.32 (M-H)-.

### Example 81: 3-oxo-2-(piperidin-4-yl)-N-(3,4,5-trifluorophenyl)-3,4-dihydro-2H-benzo[e][1,2,4]thiadiazine-7-carboxamide 1,1-dioxide (E81)

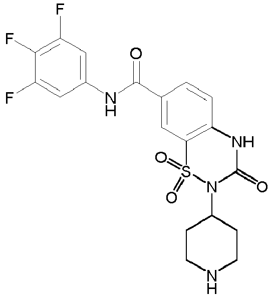

A solution of **D62** in DCM (1mL) was treated at room temperature with TFA (1mL). The yellow reaction solution was magnetically stirred at room temperature for 1h. The solvent was removed *in vacuo* and the residue purified by preparative HPLC (H₂O/CH₃CN+1%TFA) to give the title compound **E81** (25.9mg) as TFA salt. ¹H NMR (300 MHz, DMSO-*d*₆) δ ppm 1.98 (br d, *J*=11.92 Hz, 2 H) 2.67 (br dd, *J*=12.88, 3.26 Hz, 2 H) 3.12 (m, *J*=10.00 Hz, 2 H) 3.41 (br s, 2 H) 4.59 - 4.86 (m, 1 H) 7.42 (d, *J*=8.62 Hz, 1 H) 7.72 (dd, *J*=10.32, 6.56 Hz, 2 H) 8.22 - 8.43 (m, 1 H) 8.49 (d, *J*=1.83 Hz, 1 H) 8.73 (br s, 1 H) 10,59 - 10.86 (m, 1 H) 11.85 (s, 1 H). ¹H NMR (300 MHz, DMSO-*d*₆+TFA) δ ppm 1.90 - 2.04 (m, 2 H) 2.57 - 2.84 (m, 2 H) 3.01 - 3.24 (m, 2 H) 3.38 (br d, *J*=11.92 Hz, 2 H) 4.52 - 4.93 (m, 1 H) 7.41 (d, *J*=8.62 Hz, 1 H) 7.58 - 7.85 (m, 2 H) 8.28 (dd, *J*=8.67, 1.97 Hz, 1 H) 8.31 - 8.44 (m, 1 H) 8.49 (d, *J*=1.83 Hz, 1 H) 8.72 (br d, *J*=9.90 Hz, 1 H) 10.73 (br s, 1 H) 11.83 (br s, 1 H). Method 3; Rt: 2.64min. m/z: 455.13 (M+H)⁺.

### Example 82: 2-(oxetan-3-yl)-3-oxo-N-(3,4,5-trifluorophenyl)-3,4-dihydro-2H-benzo[e][1,2,4]thiadiazine-7-carboxamide 1,1-dioxide (E82)

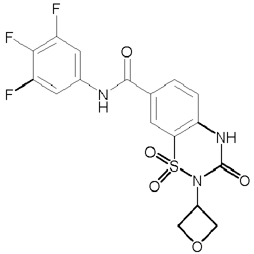

A solution of **D130** (40mg, 0.1mmol) in DMF (0.750mL) was treated with di(1H-imidazol-1-yl)methanone (64.64mg, 0.4mmol) and triethylamine (27.63uL, 0.2mmol). The resulting bright yellow solution was heated by microwave irradiation at 150°C for 10min (2run). The reaction solution was diluted with water, acidified with 5% citric acid and extracted with diethylether. Solvent was removed giving a residue (100mg) which was purified by preparative HPLC affording the title compound **E82**. Method 1; Rt: 2.08min. m/z: 428.24(M+H)⁺. ¹H NMR (300 MHz, DMSO-d6) δ ppm 4.66 - 4.79 (m, 2 H) 4.86 (t, J=7.20 Hz, 2 H) 5.26 (t, J=7.47 Hz, 1 H) 7.40 (d, J=8.62 Hz, 1 H) 7.73 (dd, J=10.36, 6.60Hz, 2 H) 8.27 (dd, J=8.62, 1.56 Hz, 1 H) 8.48 (d, J=1.83 Hz, 1 H) 10.72 (s, 1 H) 11.97 (br s, 1 H).

### Example 83: tert-butyl (S)-3-(1,1-dioxido-3-oxo-7-((3,4,5-trifluorophenyl)carbamoyl)-3,4-dihydro-2H-benzo[e][1,2,4]thiadiazin-2-yl)pyrrolidine-1-carboxylate (E83)

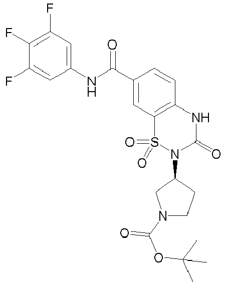

tert-butyl (S)-3-((2-amino-5-((3,4,5-trifluorophenyl)carbamoyl)phenyl)sulfonamido)pyrrolidine-1-carboxylate was prepared similary as described for the preparation of **D107** using as starting material **D44** and purified by preparative HPLC (H₂O/CH₃CN+ 0.1%HCOOH),. Method 3; Rt: 3.81min. m/z: 515.24(M+H)⁺. ¹H NMR (300 MHz, DMSO-*d*₆) δ ppm 1.35 (br d, *J*=5.69 Hz, 9 H) 1.55 - 1.77 (m, 1 H) 1.78 - 2.00 (m, 1 H) 2.88 - 3.05 (m, 1 H) 3.07 - 3.31 (m, 4 H) 3.64 (br s, 1 H) 6.56 (br s, 2 H) 6.91 (d, *J*=8.71 Hz, 1 H) 7.64 - 7.79 (m, 2 H) 7.92 (dd, *J*=8.71, 2.11 Hz, 1 H) 8.07 (br s, 1 H) 8.24 (d, *J*=2.02 Hz, 1 H) 10.37 (s, 1 H). A solution of compound from previous step (200mg, 0.39mmol) in DMF (3mL, 0.039mol) was treated with di(1H-imidazol-1-yl)methanone (252.1mg, 1.55mmol) and triethylamine (107.76uL, 0.78mmol). The resulting bright yellow solution was heated by microwave irradiation at 150°C for 21min (2runs). The reaction was diluted with water, acidified with 5% citric acid and extracted with diethylether. Solvent was removed by evaporation giving a residue (200mg). One amount (40mg) was purified by preparative HPLC (H₂O/CH₃CN+ 0.1% HCOOH) affording the title compound **E83** (15mg) as white solid. Method 3; Rt: 4.07. m/z: 485.05 (M+H)⁺. ¹H NMR (300 MHz, DMSO-*d*₆+TFA) δ ppm 1.40 (s, 9 H) 2.10 - 2.26 (m, 1 H) 3.19 - 3.43 (m, 1 H) 3.50 - 3.65 (m, 2 H) 5.10 (t, *J*=8.21 Hz, 1 H) 7.42 (d, *J*=8.62 Hz, 1 H) 7.63 - 7.81 (m, 2 H) 8.27 (dd, *J*=8.62, 2.02 Hz, 1 H) 8.51 (d, *J*=1.83 Hz, 1 H) 10.72 (s, 1 H) 11.82 (s, 1 H).

### Example 84: (S)-3-oxo-2-(pyrrolidin-3-yl)-N-(3,4,5-trifluorophenyl)-3,4-dihydro-2H-benzo[e][1,2,4]thiadiazine-7-carboxamide 1,1-dioxide (E84)

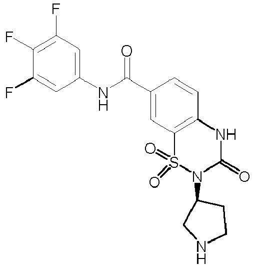

A solution of **E83** (160mg, 0.3mmol) in DCM (1mL) was treated at room temperature with a single portion of trifluoroacetic acid (2.27mL, 29.6mmol). The reaction was stirred at room temperature for 1h. Solvent was removed *in vacuo* giving the residue (217mg) which was partitioned between EtOAc/(1M)NaOH. The organic layer was dried over Na₂SO₄, filtered and evaporated giving a residue (95mg) of which one amount (31mg) was purified by preparative HPLC (H₂O/CH₃CN 0.1%TFA) affording the title compound **E84** as TFA salt. Method 3; Rt: 2.64min. m/z: 441.21 (M+H)⁺. ¹H NMR (300 MHz, DMSO-*d*₆+TFA) δ ppm 1.40 (s, 9 H) 2.10 - 2.26 (m, 1 H) 3.19 - 3.43 (m, 1 H) 3.50 - 3.65 (m, 2 H) 5.10 (t, *J*=8.21 Hz, 1 H) 7.42 (d, *J*=8.62 Hz, 1 H) 7.63 - 7.81 (m, 2 H) 8.27 (dd, *J*=8.62, 2.02 Hz, 1 H) 8.51 (d, *J*=1.83 Hz, 1 H) 10.72 (s, 1 H) 11.82 (s, 1 H).

### Example 85: (S)-2-(1-methylpyrrolidin-3-yl)-3-oxo-N-(3,4,5-trifluorophenyl)-3,4-dihydro-2H-benzo[e][1,2,4]thiadiazine-7-carboxamide 1,1-dioxide (E85)

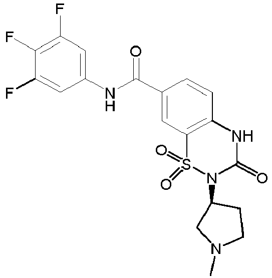

To a stirred mixture of **E84** (31mg, 0.07mmol) and formaldehyde (37% in water, 57.13uL, 0.7mmol) in methanol (1mL)was added at room temperature sodium cyanoborohydride (26.54mg, 0.42mmol) in a single portion. The yellowish solution was stirred at room temperature for 10min. The reaction solution was purified by preparative HPLC (H₂O/CH₃CN 0.1%TFA) giving the title compound **E85** as TFA salt Method 3; Rt: 2.66min. m/z: 455.26(M+H)⁺. ¹H NMR (300 MHz, DMSO-*d*₆) δ ppm 2.51 (m, 2 H) 2.87 (s, 3 H) 2.95-3.93 (m, 4 H) 5.32 (m, 1 H) 7.47 (d, J=8.62 Hz, 1 H) 7.73 (dd, J=10.32, 6.56 Hz, 2 H) 8.32 (dd, J=8.67, 1.97 Hz, 1 H) 8.56 (d, J=1.83 Hz, 1 H) 9.10 (s, 1 H) 10.75 (s, 1 H) 11.97 (br s, 1 H).

### Example 86: 2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide (E86)

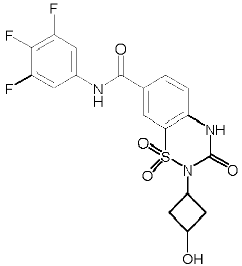

A solution of **D93** in THF (1mL) was treated with a single portion of tetrabutylammonium fluoride (1M in THF, 395.9uL, 0.4mmol). The solution was stirred at room temperature overnight. The reaction was diluted with water and 5% citric acid aq. solution, extracted with DCM and washed with NaHCO₃ (sat. solution). The organic layer was evaporated giving a residue that was purified by preparative HPLC. Method 1; Rt: 1.97min. m/z: 442.23 (M+H)⁺. ¹H NMR (300 MHz, DMSO-d6 + TFA) δ ppm 2.15 - 2.33 (m, 1.5 H) 2.54 - 2.77 (m, 2.5 H) 2.87 - 3.07 (m, 1 H) 3.81 - 3.98 (m, 0.5 H) 4.12 - 4.30 (m, 0.5 H) 4.31 - 4.44 (m, 0.5 H) 5.08 - 5.29 (m, 0.5 H) 7.31 - 7.47 (m, 1 H) 7.61 - 7.81 (m, 2 H) 8.25 (dd, J=8.57, 1.97 Hz, 1 H) 8.41 - 8.54 (m, 1 H) 10.71 (s, 1 H) 11.70 (s, 1 H).

### Example 87. cis-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide (E87)

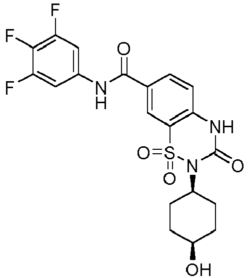

**D94** (94mg,0.16mmol) was dissolved in THF (0,5mL) 1M tetrabutylammonium fluoride in THF (0.64mL, 0.64mmol) was added, and the solution was stirred at room temperature for 2h. 4N HCl in dioxane (1.53mL, 6.12mmol) was added and the reaction was stirred at room temperature for another 1.5h. EtOAc was added and the mixture was washed with water, sat. NaHCO₃ solution and brine. Organic layer was dried over Na₂SO₄, filtered and concentrated under vacuo. The resulting crude product was purified by flash chromatography on silica (cyclohexane/EtOAc), then lyophilized, to obtain the title compound **E87** (41mg) as white solid. Method 3: Rt=3.49min, m/z=470.33 (M+H)⁺. ¹H NMR (300 MHz, DMSO-*d*₆) δ ppm 1.40 - 1.62 (m, 4 H) 1.68 - 1.87 (m, 2 H) 2.60 - 2.79 (m, 2 H) 3.83 (br s, 1 H) 4.28 - 4.47 (m, 2 H) 7.38 (d, *J*=8.62 Hz, 1 H) 7.62 - 7.86 (m, 2 H) 8.24 (dd, *J*=8.53, 1.93 Hz, 1 H) 8.45 (d, *J*=1.93 Hz, 1 H) 10.65 (s, 1 H) 11.67 (br s, 1 H).

### Example 88: 5-methyl-N-(3,4,5-trifluorophenyl)-3,4-dihydro-2H-benzo[e][1,2,4]thiadiazine-7-carboxamide 1,1-dioxide (E88)

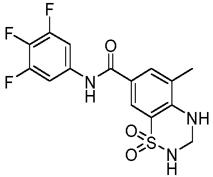

A pressure vessel was charged with **D54** (217mg, 0.600mmol), 1,4-dioxane (1.5mL) and 33% aqueous ammonia (0.75mL,6.36mmol). The pressure vessel was sealed and the reaction mixture was heated at 95°C for 7.5h, then stirred at RT overnight. More 33% aqueous ammonia (0,5mL, 4.24mmol) was added, and the reaction mixture was stirred at 100°C for another 8.5h. The reaction was diluted with EtOAc and water, organic layer was dried over Na₂SO₄, filtered and concentrated under vacuo. The residue was triturated with DCM, then was purified by preparative HPLC (H₂O/CH₃CN+1%TFA) to obtain, after lyophilisation compound 4-amino-3-methyl-5-sulfamoyl-N-(3,4,5-trifluorophenyl)benzamide (86mg) as off-white solid. Method 3; Rt=3.19min, m/z=360.15 (M+H)⁺. ¹H NMR (300 MHz, DMSO-*d*₆) δ ppm 2.22 (s, 3 H) 6.21 (s, 2 H) 7.41 (s, 2 H) 7.65 - 7.79 (m, 2 H) 7.80 - 7.89 (m, 1 H) 8.19 (d, *J*=2.02 Hz, 1 H) 10.33 (s, 1 H).

The purified compounds was reacted according to the procedure described for the preparation of **E1**. The title compound **E88** was recovered by filtration from the reaction mixture. Method 3; Rt=3.37min, m/z=372.04 (M+H)⁺. ¹H-NMR (300 MHz, DMSO-*d*₆) δ ppm 2.19 (s, 3 H) 4.73 (d, *J*=2.66 Hz, 2 H) 6.98 - 7.32 (m, 1 H) 7.66 - 7.79 (m, 2 H) 7.79 - 7.97 (m, 2 H) 8.17 (d, *J*=1.83 Hz, 1 H) 10.37 (brs, 1 H).

### Example 89: 5-methyl-3-(thiazol-2-yl)-N-(3,4,5-trifluorophenyl)-3,4-dihydro-2H-benzo[e][1,2,4]thiadiazine-7-carboxamide 1,1-dioxide (E89)

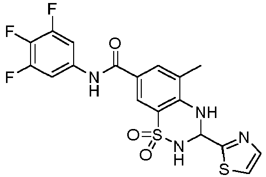

Similary prepared according to the procedure described for the preparation of example **E1**, using as starting material compound 4-amino-3-methyl-5-sulfamoyl-N-(3,4,5-trifluorophenyl)benzamide prepared as in **E88** and treating with thiazole-2-carbaldehyde instead of formaldehyde. The title compound was recovered by filtration from the reaction mixture. Method 3; Rt=3.67, m/z=455.13 (M+H)⁺. ¹H NMR (300 MHz, DMSO-*d*₆) δ ppm 2.31 (s, 3 H) 6.13 - 6.36 (m, 1 H) 7.27 (br s, 1 H) 7.64 - 7.84 (m, 2 H) 7.85 - 8.01 (m, 3 H) 8.14 - 8.30 (m, 1 H) 8.54 - 8.79 (m, 1 H) 10.47 (s, 1 H).

### Example 90: N-(3-cyano-4-fluorophenyl)-2H-benzo[e][1,2,4]thiadiazine-7-carboxamide 1,1-dioxide (E90)

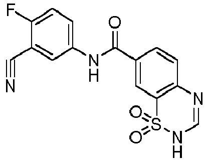

To a solution of **D58** (40mg, 0.18 mmol) in DCM (2 mL), were added oxalyl dichloride (22.93 uL, 0.26 mmol) followed by DMF (a few drops). The reaction mixture was stirred at room temperature for 50 min, then was cooled to 0°C and DIPEA (152.64 uL, 0.88 mmol) and 5-Amino-2-fluorobenzonitrile (71.58 mg, 0,53 mmol) were added. The reaction mixture was warmed to room temperature and stirred for the weekend. The mixture was diluted with DCM (5 mL) and washed with 5% citric acid solution (5mL). The product was present in both phases. All the solvents were evaporated under reduced pressure. The residue was dissolved in water and eluted on PoraPak reversed phase cartridge (5 g). The crude product was purified by preparative HPLC (H₂O/CH₃CN+1%TFA) to afford, after lyophilization, the title compound **E90** (2.89 mg) as white powder. Method 3; Rt: 2.68 min. m/z: 344.82 (M+H)⁺. ¹H NMR (300 MHz, DMSO-*d*₆) δ ppm 7.51 (d, *J*=8.71 Hz, 1 H) 7.61 (t, *J*=9.20 Hz, 1 H) 8.10 (s, 2 H) 8.25 - 8.36 (m, 2 H) 8.59 (d, *J*=1.70 Hz, 1 H) 10.83 (s, 1 H) 12.61 (br s, 1 H)

### Example 91: N-(3-cyano-4-fluorophenyl)-3,4-dihydro-2H-benzo[e] [1,2,4]thiadiazine-7-carboxamide 1,1-dioxide (E91)

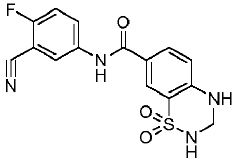

To a mixture of **D58** (40 mg, 0.18 mmol) and 2-(2,3-dihydro-1H-benzo[d][1,2,3]triazol-1-yl)-1,1,3,3-tetramethylisouronium hexafluorophosphate(V) (66.64 mg, 0.18 mmol) in dry DMF (353 uL) under nitrogen, 5-Amino-2-fluorobenzonitrile (23.86 mg, 0.18 mmol) and N-ethyl-N-isopropylpropan-2-amine (91.58 uL, 0,53 mmol) were added. The reaction mixture was stirred at room temperature for the weekend. Water and EtOAc were added and the two phases separated.

The organic layer was washed with sat. aq. NaHCO₃ solution (15 mL), 2N HCl solution (15 mL) and Brine (15 mL), then dried on Na₂SO₄, filtered and evaporated under reduced pressure. The crude title compound was purified by preparative HPLC (H₂O/CH₃CN+1%TFA) to afford, after lyophilization, 23.2 mg of the title compound **E91.** Method 3; Rt:2.80 min. m/z: 347.04 (M+H)⁺. ¹H NMR (300 MHz, DMSO-*d*₆) δ ppm 4.74 (dd, *J*=8.20, 2.70 Hz, 2 H) 6.91 (d, *J*=9.40 Hz, 1 H) 7.57 (t, *J*=9.30 Hz, 1 H) 7.79 - 7.91 (m, 2 H) 7.95 (dd, *J*=8.70, 2.00 Hz, 1 H) 8.07 - 8.16 (m, 1 H) 8.26 - 8.36 (m, 2 H) 10.42 - 10,51 (m, 1 H).

### Example 92: N-(6-chloropyridin-3-yl)-3,4-dihydro-2H-benzo[e][1,2,4]thiadiazine-7-carboxamide 1,1-dioxide (E92)

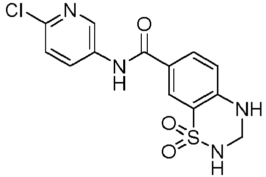

**D95** (2 mg, 0.006 mmol, 1 eq) was dissolved in water (200 uL) and THF (300 uL), NaBH₄ (10 mg, 0.264 mmol, 44.5 eq) was added, and the reaction mixture was stirred at room temperature overnight. NaBH₄ (10 mg, 0.264 mmol) was added and after total 44 h the reaction was almost complete. Water (1 mL) and MeOH (1 mL) were added and the solvents were evaporated under reduced pressure. The crude compound was purified by preparative HPLC (H₂O/CH₃CN+1%TFA) to afford the title compound **E92** (1.91 mg) as white powder. Method 3; Rt: 2.53. m/z: 339.14 (M+H)⁺. ¹H NMR (300 MHz, DMSO-*d*₆) δ ppm 4.67 - 4.81 (m, 2 H) 6.91 (d, *J*=8.90 Hz, 1 H) 7.54 (d, *J*=8.70 Hz, 1 H) 7.80 - 7.92 (m, 2 H) 7.96 (dd, *J*=8.80, 2.10 Hz, 1 H) 8.28 (dd, *J*=8.80, 2.80 Hz, 1 H) 8.32 (d, *J*=2.00 Hz, 1 H) 8.82 (d, *J*=2.60 Hz, 1 H) 10.47 (s, 1 H).

### Example 93: N-(3,4,5-trifluorophenyl)-3,4-dihydro-2H-pyrido[2,3-e][1,2,4]thiadiazine-7-carboxamide 1,1-dioxide (E93)

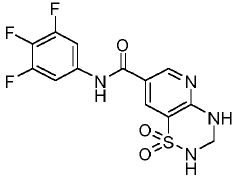

6-Amino-5-sulfamoyl-N-(3,4,5-trifluorophenyl)nicotinamide was prepared according to the procedure described for the preparation of compound **D100**, starting from **D56**. Method 3: Rt=2.82min, m/z=347.04 (M+H)⁺. ¹H NMR (300 MHz, DMSO-*d*₆) δ ppm 7.07 (br s, 2 H) 7.60 (s, 2 H) 7.65 - 7.81 (m, 2 H) 8.46 (d, *J*=2.20 Hz, 1 H) 8.80 (d, *J*=2.20 Hz, 1 H) 10.48 (s, 1 H). The isolated compound (50mg, 0.140mmol) was suspended in 2-propanol (1.5mL), 37% aqueous formaldehyde (0.04mL, 0,580mmol) and two drops of HCl 4N in dioxane were added, and the reaction mixture was stirred 30min at 65°C, then 1h at 100°C. The reaction was filtered and the solid was washed with iPrOH, to yield the title compound **E93** (15mg) as off-white solid. Method 3: Rt=3.07min, m/z=359.20 (M+H)⁺. ¹H NMR (300 MHz, DMSO-*d*₆) δ ppm 4.64 - 4.79 (m, 2 H) 7.62 - 7.80 (m, 2 H) 8.05 (br s, 1 H) 8.52 (d, *J*=2.20 Hz, 1 H) 8.66 (br s, 1 H) 8.82 (d, *J*=2.38 Hz, 1 H) 10.46 (brs, 1 H).

### Example 94: N-(3,4,5-trifluorophenyl)-2,3,4,5-tetrahydrobenzo[f][1,2,5]thiadiazepine-8-carboxamide 1,1-dioxide (E94)

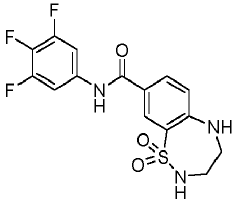

**D48** (HCl salt, 68mg, 0.160mmol) was dissolved in DMSO (2mL) and cesium carbonate (207.2mg, 0.636mmol) was added. The reaction mixture was heated under microwave irradiation at 130°C for 1.5h. The reaction mixture was diluted with water and purified by preparative HPLC (H₂O/CH₃CN+1%TFA) to obtain, after lyophilization, the title compound **E94** (28mg) as white solid. Method 3; Rt=3.17min, m/z=372.24 (M+H)⁺. ¹H NMR (300 MHz, DMSO-*d*₆) δ ppm 3.16 - 3.28 (m, 2 H) 3.41 - 3.57 (m, 2 H) 7.00 (d, *J*=8.71 Hz, 1 H) 7.13 - 7.25 (m, 1 H) 7.63 - 7.80 (m, 2 H) 7.81 - 7.97 (m, 2 H) 8.27 (d, *J*=2.11 Hz, 1 H) 10.43 (s, 1 H).

### Example 95: (3aS,10aS)-N-(3,4,5-trinuorophenyl)-2,3,3a,4,10,10a-hexahydro-1H-benzo[f]cyclopenta[c][1,2,5]thiadiazepine-7-carboxamide 5,5-dioxide (E95)

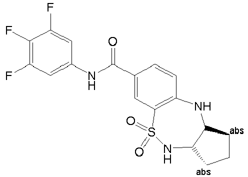

A solution of **D42** (50.mg, 0.120mmol) in DMSO (2mL) was treated with cesium carbonate (226.58mg, 0.700mmol) and heated for 10hrs at 130°C. The reaction solution was purified by preparative HPLC affording the title compound **E95** (0.500mg) as white solid. Method 3; Rt: 3.52. m/z: 412.23 (M+H)⁺. ¹H NMR (300 MHz, DMSO-*d*₆+TFA) δ ppm 1.41 - 1.86 (m, 4 H) 1.92 - 2.24 (m, 2 H) 3.06 - 3.30 (m, 1 H) 3.39 (br d, *J*=8.53 Hz, 1 H) 6.52 - 7.04 (m, 1 H) 7.17 (d, *J*=8.53 Hz, 1 H) 7.72 (dd, *J*=10.59, 6.56 Hz, 3 H) 7.92 (dd, *J*=8.67, 2.06 Hz, 1 H) 8.37 (d, *J*=2.02 Hz, 1 H) 10.46 (s, 1 H).

### Example 96: N-(3,4,5-trifluorophenyl)-3,4-dihydro-2H-benzo[b][1,4,5]oxathiazepine-8-carboxamide 1,1-dioxide (E96)

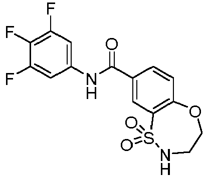

**D86** (50mg, 0.130mmol) was dissolved in dry DMF (1mL) and NaH 60% dispersion on mineral oil (20.4mg, 0,510mmol) was added. The reaction mixture was stirred under N₂ flow at RT for 10min, then heated under microwave irradiation at 130°C for 1h. The reaction mixture was diluted with H₂O and extracted twice with EtOAc. The combined organic layer was washed with H₂O and brine, then dried over Na₂SO₄, filtered and concentrated under vacuo. The resulting crude product was purified by flash chromatography on silica (DCM/MeOH) to obtain the title compound **E96** (27mg) as white solid. Method 3; Rt=3.33, m/z=373.05 (M+H)⁺. ¹H NMR (300 MHz, DMSO-*d*₆) δ ppm 3.42 - 3.56 (m, 2 H) 4.12 - 4.28 (m, 2 H) 7.40 (d, *J*=8.44 Hz, 1 H) 7.65 - 7.81 (m, 2 H) 8.14 (dd, *J*=8.44, 2.29 Hz, 1 H) 8.35 (d, *J*=2.29 Hz, 1 H).

### Example 97: N-(3,4,5-trifluorophenyl)-2,3,4,5-tetrahydrobenzo[b] [1,4,5]oxathiazocine-9-carboxamide 1,1-dioxide (E97)

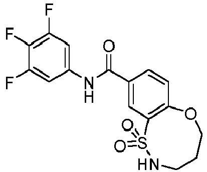

Similarly prepared according to the procedure described for the preparation of compound **E96** starting from **D87,** and purified by preparative HPLC (H₂O/CH₃CN+1%TFA). Method 3; Rt=3.29min, m/z=387.17 (M+H)⁺. ¹H NMR (300 MHz, DMSO-*d*₆) δ ppm 1.60 - 1.83 (m, 2 H) 3.37 - 3.50 (m, 2 H) 4.34 (t, *J*=6.10 Hz, 2 H) 7.34 - 7.66 (m, 2 H) 7.66 - 7.82 (m, 2 H) 8.20 (dd, *J*=8.44, 2.29 Hz, 1 H) 8.38 (d, *J*=2.20 Hz, 1 H) 10.46 - 10.97 (m, 1 H).

### Example 98: 2-ethyl-N-(3,4,5-trifluorophenyl)-3,4,5,6-tetrahydro-2H-benzo[b][1,4,5]oxathiazonine-10-carboxamide 1,1-dioxide (E98)

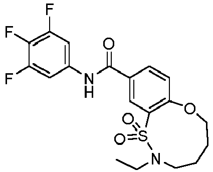

Similarly prepared according to the procedure described for the preparation of compound **E96** starting from **D88**, and purified by preparative HPLC (H₂O/CH₃CN+1%TFA). Method 3; Rt=3.92min, m/z=429.32 (M+H)⁺. ¹H NMR (300 MHz, DMSO-*d*₆) δ ppm 1.09 (t, *J*=6.97 Hz, 3 H) 1.30 - 1.51 (m, 2 H) 1.65 - 1.86 (m, 2 H) 2.90 - 3.14 (m, 2 H) 3.62 - 3.96 (m, 2 H) 4.42 - 4.59 (m, 2 H) 7.61 (d, *J*=8.71 Hz, 1 H) 7.65 - 7.80 (m, 2 H) 8.13 - 8.24 (m, 1 H) 8.34 (d, *J*=2.29 Hz, 1 H) 10.67 (s, 1 H).

### Example 99: (R)-3-isobutyl-N-(3,4,5-trifluorophenyl)-3,4-dihydro-2H-benzo[b][1,4,5]oxathiazepine-8-carboxamide 1,1-dioxide (E99)

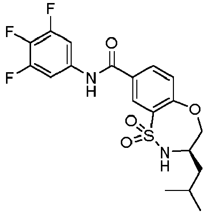

Similarly prepared according to the procedure described for the preparation of compound **E96**, starting from **D89**, and purified by preparative HPLC (H₂O/CH₃CN+1% TFA). Method 3; Rt=4.00min, m/z=429.12 (M+H)⁺. ¹H NMR (300 MHz, DMSO-*d*₆) δ ppm 0.93 (dd, *J*=6.56, 0.78 Hz, 6 H) 1.16 - 1.31 (m, 1 H) 1.32 - 1.53 (m, 1 H) 1.69 - 1.97 (m, 1 H) 3.63 - 3.88 (m, 2 H) 4.39 - 4.61 (m, 1 H) 7.39 (d, *J*=8.44 Hz, 1 H) 7.62 - 7.94 (m, 3 H) 8.14 (dd, *J*=8.48, 2.34 Hz, 1 H) 8.36 (d, *J*=2.29 Hz, 1 H) 10.73 (s, 1 H).

### Example 100: 2-methyl-N-(3,4,5-trifluorophenyl)-3,4-dihydro-2H-benzo[b][1,4,5]oxathiazepine-8-carboxamide 1,1-dioxide (E100)

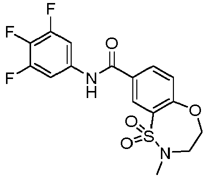

Similarly prepared according to the procedure described for the preparation of compound **E96** starting from **D90**, and purified by preparative HPLC (H₂O/CH₃CN+1‰TFA). Method 3; Rt=3.60min, m/z=387.24 (M+H)⁺. ¹H NMR (300 MHz, DMSO-*d*₆) δ ppm 2.75 (s, 3 H) 3.65 - 3.82 (m, 2 H) 4.18 - 4.38 (m, 2 H) 7.45 (d, *J*=8.44 Hz, 1 H) 7.62 - 7.83 (m, 2 H) 8.20 (dd, *J*=8.44, 2.38 Hz, 1 H) 8.32 (d, *J*=2.29 Hz, 1 H) 10.75 (s, 1 H).

### Example 101: N-(3,4,5-trifluorophenyl)-1,3,4,11,12,12a-hexahydro-2H-benzo[f]pyrido[1,2-b][1,2,5]thiadiazepine-8-carboxamide 6,6-dioxide (E101)

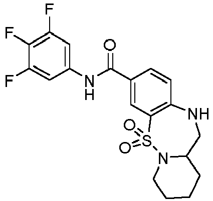

Similarly prepared according to the procedure described for the preparation of compound **E94** starting from **D65.** Method 3; Rt=3.85min, m/z=426.20 (M+H)⁺. ¹H NMR (300 MHz, DMSO-*d*₆+TFA) δ ppm 1.39 - 1.65 (m, 4 H) 1.66 - 1.83 (m, 2 H) 2.95 - 3.14 (m, 1 H) 3.28 - 3.41 (m, 1 H) 3.42 - 3.53 (m, 1 H) 3.53 - 3.73 (m, 2 H) 7.01 (d, *J*=8.62 Hz, 1 H) 7.32 (br s, 1 H) 7.62 - 7.81 (m, 2 H) 7.89 (dd, *J*=8.71, 2.20 Hz, 1 H) 8.25 (d, *J*=2.20 Hz, 1 H) 10.43 (s, 1 H).

### Example 102: 11-methyl-N-(3,4,5-trifluorophenyl)-1,3,4,11,12,12a-hexahydro-2H-benzo[f]pyrido[1,2-b][1,2,5]thiadiazepine-8-carboxamide 6,6-dioxide (E102)

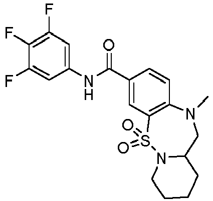

**E101** (10mg, 0.020mmol) was suspended in MeOH (0.6mL) and THF (0.15mL), 1 drop of AcOH and 37% aqueous formaldehyde (0.02mL, 0.280mmol) were added. The reaction mixture was stirred at RT for 1h, then sodium cyanoborohydride (29.54mg, 0.470mmol) was added, and the reaction was stirred at RT for 3h. More 37% aqueous formaldehyde (0.03mL, 0.403mmol) was added, and after 30min more sodium cyanoborohydride (42mg, 0.668mmol) was added, and the reaction mixture was stirred for another 16h, then quenched with 2 drops of 1N HCl, and purified by preparative HPLC (H₂O/CH₃CN+1%TFA) to obtain, after lyophilization, the title compound **E102** (4.7mg) as white solid. Method 3; Rt=4.57min, m/z=440.27 (M+H)⁺. ¹H NMR (300 MHz, DMSO-*d*₆+TFA) δ ppm 1.40 - 1.67 (m, 4 H) 1.70 - 1.86 (m, 2 H) 2.99 - 3.14 (m, 4 H) 3.14 - 3.27 (m, 1 H) 3.41 - 3.57 (m, 1 H) 3.57 - 3.79 (m, 2 H) 7.18 (d, *J*=8.80 Hz, 1 H) 7.65 - 7.80 (m, 2 H) 8.07 (dd, *J*=8.80, 2.29 Hz, 1 H) 8.35 (d, *J*=2.20 Hz, 1 H) 10,52 (s, 1 H).

### Example 103: (S)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide (E103)

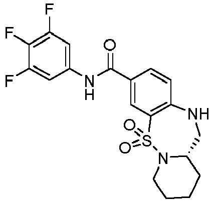

Similarly prepared according to the procedure described for the preparation of compound **E94,** starting from **D67.** Method 3; Rt=3.84min, m/z=426.28 (M+H)⁺. ¹H NMR (300 MHz, DMSO-*d*₆+TFA) δ ppm 1.37 - 1.66 (m, 4 H) 1.66 - 1.83 (m, 2 H) 2.96 - 3.17 (m, 1 H) 3.28 - 3.53 (m, 2 H) 3.53 - 3.76 (m, 2 H) 7.01 (d, *J*=8.71 Hz, 1 H) 7.30 (br s, 1 H) 7.62 - 7.80 (m, 2 H) 7.89 (dd, *J*=8.62, 2.20 Hz, 1 H) 8.25 (d, *J*=2.11 Hz, 1 H) 10.43 (s, 1 H).

### Example 104: (R)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide (E104)

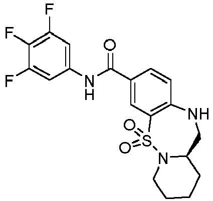

**D69** (54mg, 0.110mmol) was dissolved in DMSO (1.8mL) and diisopropylethylamine (0.08mL, 0.450mmol) was added. The reaction mixture was heated at 65°C for 1h, then was purified by preparative HPLC (H₂O/CH₃CN+1%TFA) to obtain, after lyophilization, the title compound **E104** (38mg) as white solid. Method 3; Rt=3.84min, m/z=426.28 (M+H)⁺. ¹H NMR (300 MHz, DMSO-*d*₆+TFA) δ ppm 1.42 - 1.66 (m, 4 H) 1.66 - 1.83 (m, 2 H) 2.96 - 3.18 (m, 1 H) 3.26 - 3.53 (m, 2 H) 3.53 - 3.75 (m, 2 H) 7.01 (d, *J*=8.62 Hz, 1 H) 7.31 (br s, 1 H) 7.60 - 7.79 (m, 2 H) 7.89 (dd, *J*=8.53, 2.02 Hz, 1 H) 8.25 (d, *J*=2.11 Hz, 1 H) 10.43 (s, 1 H).

### Example 105: (5S)-N-(3,4,5-trifluorophenyl)-3,4,5,6-tetrahydro-2,5-methanobenzo[g][1,2,6]thiadiazocine-9-carboxamide 1,1-dioxide (E105)

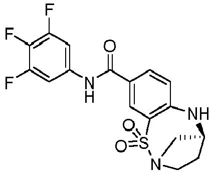

Similarly prepared according to the procedure described for the preparation of compound **E94,** starting from **D71.** Method 3; Rt=3.91min, m/z=398.32 (M+H)⁺. ¹H NMR (300 MHz, DMSO-*d*₆+TFA) δ ppm 1.52 - 1.73 (m, 1 H) 2.01 - 2.21 (m, 1 H) 3.06 - 3.35 (m, 2 H) 3.37 - 3.57 (m, 1 H) 3.70 (br d, *J*=13.48 Hz, 1 H) 4.02 - 4.20 (m, 1 H) 7.00 (d, *J*=8.71 Hz, 1 H) 7.54 (br s, 1 H) 7.66 - 7.77 (m, 2 H) 7.90 (dd, *J*=8.71, 2.20 Hz, 1 H) 8.33 (d, *J*=2.11 Hz, 1 H) 10.48 (s, 1 H).

### Example 106: N-(3,4,5-trifluorophenyl)-1,2,3,10,11,11a-hexahydrobenzo[f]pyrrolo[1,2-b][1,2,5]thiadiazepine-7-carboxamide 5,5-dioxide (E106)

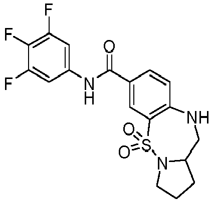

Similarly prepared according to the procedure described for the preparation of compound **E94,** starting from **D73.** Method 3; Rt=3.70min, m/z=412.17 (M+H)⁺. ¹H NMR (300 MHz, DMSO-*d*₆+TFA) δ ppm 1.74 - 2.19 (m, 4 H) 2.77 - 2.92 (m, 1 H) 3.02 - 3.22 (m, 1 H) 3.26 - 3.40 (m, 1 H) 3.78 - 3.93 (m, 1 H) 4.28 - 4.42 (m, 1 H) 6.90 (d, *J*=8.71 Hz, 1 H) 7.42 (br s, 1 H) 7.63 - 7.78 (m, 2 H) 7.83 (dd, *J*=8.71, 2.20 Hz, 1 H) 8.22 (d, *J*=2.11 Hz, 1 H) 10.40 (s, 1 H).

### Example 107: N-(3,4,5-trifluorophenyl)-1,9,10,10a-tetrahydro-2H-azeto[1,2-b]benzo[f][1,2,5]thiadiazepine-6-carboxamide 4,4-dioxide (E107)

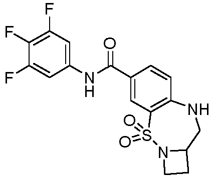

Similarly prepared according to the procedure described for the preparation of compound **E94,** starting from **D75**. Method 3; Rt=3.50min, m/z=398.12 (M+H)⁺. ¹H NMR (300 MHz, DMSO-*d*₆) δ ppm 2.22 - 2.37 (m, 1 H) 3.19 - 3.31 (m, 1 H) 3.61 - 3.74 (m, 1 H) 3.75 - 3.88 (m, 1 H) 4.24 - 4.39 (m, 1 H) 4.40 - 4.53 (m, 1 H) 6.98 (d, *J*=8.80 Hz, 1 H) 7.50 - 7.62 (m, 1 H) 7.64 - 7.78 (m, 2 H) 7.87 (dd, *J*=8.70, 2.20 Hz, 1 H) 8.20 (d, *J*=2.11 Hz, 1 H) 10.42 (s, 1 H).

### Example 108: N-(3,4,5-trifluorophenyl)-1,3,4,11,12,12a-hexahydrobenzo[f][1,4]oxazino[4,3-b][1,2,5]thiadiazepine-8-carboxamide 6,6-dioxide (E108)

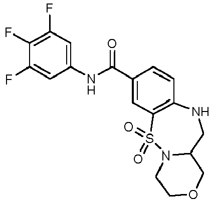

Similarly prepared according to the procedure described for the preparation of compound **E94,** starting from **D77** and purified by preparative HPLC (H₂O/CH₃CN+0.1%HCOOH). Method 3; Rt=3.44 min, m/z=428.24 (M+H)⁺. ¹H NMR (300 MHz, DMSO-*d*₆) δ ppm 3.13 - 3.23 (m, 2 H) 3.53 (br d, *J*=5.96 Hz, 1 H) 3.43 - 3.52 (m, 1 H) 3.59 - 3.71 (m, 2 H) 3.71 - 3.92 (m, 3 H) 6.95 - 7.07 (m, 1 H) 7.12 (d, *J*=8.62 Hz, 1 H) 7.64 - 7.81 (m, 2 H) 7.96 (dd, *J*=8.62, 2.20 Hz, 1 H) 8.28 (d, *J*=2.11 Hz, 1 H) 10.49 (s, 1 H).

### Example 109: N-(3,4,5-trifluorophenyl)-10,11-dihydrobenzo[f]imidazo[1,2-b][1,2,5]thiadiazepine-7-carboxamide 5,5-dioxide (E109)

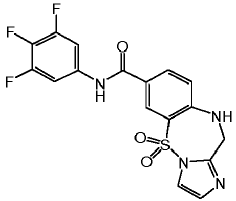

A 5mL vial was charged with **D78** (90mg, 0.18mmol), toluene (600uL) and phosphoric acid (360.78uL, 4.78mmol). The resulting milky suspension was stirred at room temperature for 30min then MeCN (4drops) was added, followed by NaHCO₃ (sat. solution). The solution was poured into a separating funnel and extracted with DCM. The organic layer was washed with brine, dried over MgSO₄ (dry), filtered and evaporated. The residue was dissolved in DMF, treated with triethylamine (300uL, 2.16mmol) and stirred at 50°C for 30min. Solvents were removed *in vacuo* and the residue was partitioned between water and diethyl ether /EtOAc. The organic layer was dried over MgSO₄ (dry), filtered and finally evaporated. The residue was purified by flash chromatography over silica gel (EtOAc/DCM), giving the title compound **E109** (1.94mg) as white solid. Method 3; Rt: 3.64min. m/z: 409.19 (M+H)⁺. ¹H NMR (300 MHz, DMSO-*d*₆) δ ppm 4.76 - 4.93 (m, 2 H) 6.99 (d, *J*=8.99 Hz, 1 H) 7.09 - 7.20 (m, 1 H) 7.62 - 7.76 (m, 3 H) 7.91 - 8.02 (m, 1 H) 8.38 (d, *J*=2.11 Hz, 1 H) 8.42 - 8.55 (m, 1 H) 10,50 (br s, 1 H).

### Example 110: N-(3,4-difluorophenyl)-3,4-dihydro-2,5-ethanobenzo[f][1,2,5]thiadiazepine-8-carboxamide 1,1-dioxide (E110)

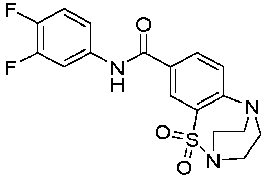

In a vial (3 mL) **D82** (4.4 mg, 0.011 mmol) was weighed together with triphenylphosphane (8.7 mg, 0.033mmol) and DIAD (5,5 mg, 0.028 mmol) then THF dry (0.3 mL) was added. The vial was sealed and the supsension was stirred at room temperature for 1 h. Then the solvent was removed under vacuum and the crude product purified by preparative HPLC twice (H₂O/CH₃CN+0.1% TFA) to give the title compound **E110** (0.67 mg) as white powder. Method 3; Rt=3.32 min, m/z=380.35 (M+H)⁺. ¹H NMR (acetonitrile-d₃, 300 MHz): δ = 9.05 (br s, 1H), 8.36 (d, *J*= 2.1 Hz, 1H), 8.09 (dd, *J*= 8.3, 2.2 Hz, 1H), 7.83 (ddd, *J*= 13.0, 7.5, 2.6 Hz, 1H), 7.37-7.57 (m, 2H), 7.15-7.37 (m, 1H), 3.60-3.76 (m, 2H), 3.28-3.45 (m, 4H), 3.14-3.28 ppm (m, 2H).

### Example 111: N-(3,4,5-trifluorophenyl)-1,2,3,4,12,12a-hexahydrobenzo[b]pyrido[1,2-e][1,4,5]oxathiazepine-8-carboxamide 6,6-dioxide (E111)

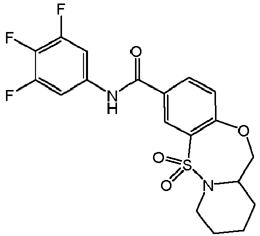

A microwave vial was charged with sodium hydride (40mg, 1mmol); the vial was sealed and evacuated. A solution of **D79** (40.mg,0.09mmol) in DMF (3mL, dry) was added in a single portion and the resulting suspension stirred at room temperature for 1h30min then heated by microwave irradiation for 30min at 130°C. Sovent was removed *in vacuo.* The residue was partitioned between water and DCM. The organic layer was evaporated giving the crude product (60mg, white solid) which was purified by preparative HPLC (H₂O/CH₃CN 0.1% TFA) to obtain the title compound **E111** (16mg) as white solid. Method 3; Rt: 3.96min. m/z: 427.30 (M+H)⁺. ¹H NMR (300 MHz, DMSO-*d*₆) δ ppm 1.11 - 1.36 (m, 1 H) 1.41 - 1.62 (m, 2 H) 1.62 - 1.91 (m, 3 H) 2.60 - 2.72 (m, 1 H) 3.33 - 3.44 (m, 1 H) 4.29 - 4.43 (m, 3 H) 7.44 (d, *J*=8.44 Hz, 1 H) 7.72 (dd, *J*=10.36, 6.60 Hz, 2 H) 8.21 (br dd, *J*=8.44, 2.38 Hz, 1 H) 8.30 (d, *J*=2.20 Hz, 1 H) 10.75 (br s, 1 H).

### Example 112: (R)-N-(3,4,5-trifluorophenyl)-1,2,3,4,12,12a-hexahydrobenzo[b]pyrido[1,2-e][1,4,5]oxathiazepine-8-carboxamide 6,6-dioxide (E112)

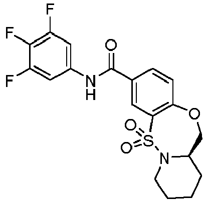

**D83** (49 mg, 0.110mmol) was dissolved in dry DMF (1.5mL) and NaH 60% dispersion on mineral oil (17.6mg, 0.439mmol) was added. The reaction mixture was stirred at RT for 2h, then diluted with H₂O and extracted with EtOAc. Organic layer was washed with H₂O, dried over Na₂SO₄, filtered and concentrated under vacuo. The resulting crude product was purified by preparative HPLC (H₂O/CH₃CN+1%TFA) to obtain, after lyophilization, the title compound **E112** (39mg) as white solid. Method 3; Rt=3.96min, m/z= 427.16 (M+H)⁺. ¹H NMR (300 MHz, DMSO-*d*₆) δ ppm 1.13 - 1.37 (m, 1 H) 1.42 - 1.63 (m, 2 H) 1.63 - 1.90 (m, 3 H) 2.59 - 2.72 (m, 1 H) 3.33 - 3.43 (m, 1 H) 4.26 - 4.49 (m, 3 H) 7.45 (d, *J*=8.44 Hz, 1 H) 7.65 - 7.80 (m, 2 H) 8.21 (dd, *J*=8.44, 2.29 Hz, 1 H) 8.30 (d, *J*=2.20 Hz, 1 H) 10.74 (s, 1 H).

### Example 113: (S)-N-(3,4,5-trifluorophenyl)-1,2,3,4,12,12a-hexahydrobenzo[b]pyrido[1,2-e][1,4,5]oxathiazepine-8-carboxamide 6,6-dioxide (E113)

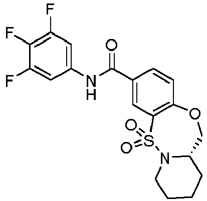

Similarly prepared according to the procedure described for the preparation of compound **E112** starting from **D84**. Method 3; Rt=3.96min, m/z=427.37 (M+H)⁺. ¹H NMR (300 MHz, DMSO-*d*₆) δ ppm 1.11 - 1.36 (m, 1 H) 1.42 - 1.62 (m, 2 H) 1.63 - 1.91 (m, 3 H) 2.60 - 2.72 (m, 1 H) 3.31 - 3.44 (m, 1 H) 4.30 - 4.45 (m, 3 H) 7.45 (d, *J*=8.44 Hz, 1 H) 7.75 (m, *J*=6.50 Hz, 2 H) 8.21 (dd, *J*=8.44, 2.29 Hz, 1 H) 8.30 (d, *J*=2.20 Hz, 1 H) 10.75 (s, 1 H).

### Example 114: tert-butyl 8-((3,4,5-trifluorophenyl)carbamoyl)-3,4,12,12a-tetrahydrobenzo[b]pyrazino[1,2-e][1,4,5]oxathiazepine-2(1H)-carboxylate 6,6-dioxide (E114)

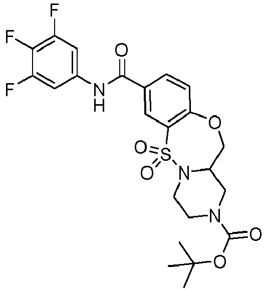

To a solution of **D85** (83mg, 0.15mmol) in DMF (1.5mL) was added sodium hydride (60% in mineral oil, 18.19mg, 0.45mmol) at room temperature and the mixture was stirred for 1h. The reaction was poured on ice and was extracted with EtOAc (3x40mL), washed with brine, dried over Na₂SO₄, filtered and concentrated under reduced pressure to yield the crude product (80mg) of which one amount (20mg) was purified by HPLC (H₂O/CH₃CN), affording the title compound **E114** (0.6mg) as white solid. Method 3: Rt= 4.11, m/z-56= 472.08; m/z-100= 428.11. ¹H NMR (300 MHz, DMSO-*d*₆+ TFA 20uL) δ 1.37 (s, 9H), 2.61-2.78 (m, 1H), 2.86-3.08 (m, 1H), 3.10-3.26 (m, 1H), 3.28-3.43 (m, 1H), 3.85-3.99 (m, 1H), 4.01-4.21 (m, 2H), 4.28-4.37 (m, 1H), 4.39-4.53 (m, 1H), 7.44 (d, *J*=8.44 Hz, 1H), 7.72 (dd, *J*=10.41, 6.56 Hz, 2H), 8.22 (dd, *J*=8.44, 2.20 Hz, 1H), 8.30 (d, *J*=2.11 Hz, 1H), 10.75 (s, 1H).

### Example 115: N-(3,4,5-trifluorophenyl)-1,2,3,4,12,12a-hexahydrobenzo[b]pyrazino[1,2-e][1,4,5]oxathiazepine-8-carboxamide 6,6-dioxide (E115)

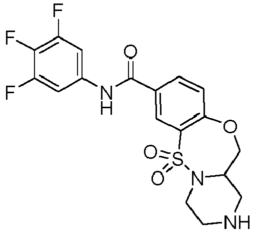

To a solution of **E114** (70mg, 0.13mmol) in 1,4-dioxane (0,5mL) was added 4M HCl in dioxane (0.33mL,1.33mmol). The reaction was stirred at room temperature overnight. The reaction mixture was diluted with water (15mL) and DCM (15mL). The acqueous phase was basified with 5% NaOH until pH=10 (by paper) and was extracted with DCM (3x20mL), washed with brine, dried over Na₂SO₄, filtered and concentrated under reduced pressure to give the title compound **E115** (37mg) as white powder. Method 3; Rt: 2.59. m/z: 428.11 (M+H)⁺. ¹H NMR (300 MHz, DMSO-*d*₆) δ = 2.55 (m, 3H), 2.97 - 2.81 (m, 2H), 3.10 - 3.01 (m, 1H), 3.31 - 3.21 (m, 1H), 4.26 - 4.03 (m, 1H), 4.46 - 4.37 (m, 2H), 7.47 (d, *J* = 8.3 Hz, 1H), 7.79 - 7.66 (m, 2H), 8.22 (dd, *J* = 2.3, 8.4 Hz, 1H), 8.30 (d, *J* = 2.2 Hz, 1H), 10.75 (s, 1H).

### Example 116: 2-acetyl-N-(3,4,5-trifluorophenyl)-1,2,3,4,12,12a-hexahydrobenzo[b]pyrazino[1,2-e][1,4,5]oxathiazepine-8-carboxamide 6,6-dioxide (E116)

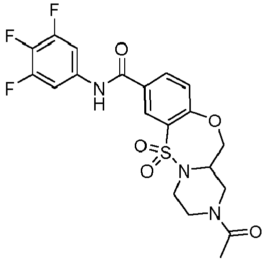

To a solution of **E115** (15mg, 0.03mmol) in DCM (0,5mL) and triethylamine (0.01mL, 0.05mmol) at 0°C was added a solution of acetic anhydride (0.003mL, 0.030mmol) in DCM (0.1mL). The reaction was stirred at room temperature for 1h. The reaction was quenched by addition of water and was extracted with EtOAc. The organic phase was washed with 5% citric acid aq. solution, NaHCO₃ sat. solution, brine, dried over Na₂SO₄, filtered and concentrated under reduced pressure to yield the title compound **E116** (4.8mg). Method 3; Rt: 3.33min. m/z: 470.19 (M+H)⁺.

### Example 117: N-(3,4,5-trifluorophenyl)-3,4-dihydro-2,4-methanobenzo[b] [1,4,5]oxathiazepine-8-carboxamide 1,1-dioxide (E117)

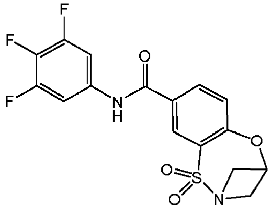

To a solution of 4-fluoro-3-((3-hydroxyazetidin-1-yl)sulfonyl)-N-(3,4,5-trifluorophenyl)benzamide (prepared as described in WO2013/096744) (45.mg, 0.110mmol) in DMF (10mL) was added sodium hydride, 60% mineral oil (35.61mg, 0.890mmol). The reaction was stirred at 80°C for 2hrs. The reaction was quenched with water and was concentrated under reduced pressure. The crude product was purifed by HPLC (H₂O/CH₃CN+1%TFA) to afford the title compound E117 (2.65mg). Method 3; Rt=3.54min, m/z= 385.15 (M+H)⁺. ¹H NMR (300 MHz, DMSO-*d₆*) δ = 10.72 (s, 1H), 8.46 (d, *J* = 2.2 Hz, 1H), 8.19 (dd, *J* = 2.3, 8.9 Hz, 1H), 7.81 - 7.75 (m, 1H), 7.75 - 7.70 (m, 1H), 7.34 (d, *J* = 8.9 Hz, 1H), 5.28 (t, *J* = 2731.7 Hz, 1H), 4.80 - 4.74 (m, 1H), 4.74 - 4.70 (m, 1H), 4.12 - 4.06 (m, 1H), 4.06 - 4.01 (m, 1H).

### Example 118: N-(3,4,5-trifluorophenyl)-4,5-dihydro-3H-2,5-methanobenzo[b][1,4,5]oxathiazocine-9-carboxamide 1,1-dioxide (E118)

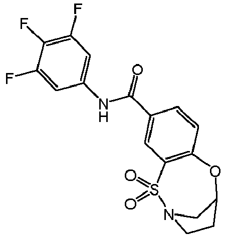

Similarly prepared according to the procedure described for the preparation of compound **E117** starting from (S)-4-fluoro-3-((3-hydroxypyrrolidin-1-yl)sulfonyl)-N-(3,4,5-trifluorophenyl)benzamide (prepared as described in WO2013/096744). Method 3; Rt=3.63min, m/z=399.3 (M+H)⁺. ¹H NMR (300 MHz, DMSO-d₆+TFA) δ = 10.87 - 10.65 (m, 1H), 8.41 (d, *J* = 2.2 Hz, 1H), 8.16 (dd, *J* = 2.3, 8.5 Hz, 1H), 7.72 (dd, *J* = 6.6, 10.4 Hz, 2H), 7.33 (d, *J* = 8.5 Hz, 1H), 5.36 - 5.07 (m, 1H), 4.16 - 4.01 (m, 1H), 3.49 (br s, 4H), 2.25 - 2.03 (m, 1H), 1.88 - 1.70 (m, 1H).

The examples shown in Table 1 were prepared according to the synthetic methods described above. The general synthetic strategies, the appropriate intermediate materials and the relevant reaction steps (were appropriate), are indicated in Table 1 by referring to the appropriate Scheme.

**Table 1:**

| **Example** | **Compound Name** | **Structure** | **(ES+) m/z** | **Scheme, Intermediate, Reaction Steps** |
|---|---|---|---|---|
| **E119** | N-(3,4-difluorophenyl)-3-(hydroxymethyl)-3,4-dihydro-2H-benzo[e][1,2,4]thiadiazine-7-carboxamide 1,1-dioxide | 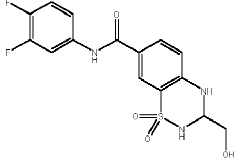 | 370 | Scheme 1: D8 → (3) → (6) |
| **E120** | N-(3,4-difluorophenyl)-3,3-dimethyl-3,4-dihydro-2H-benzo[e][1,2,4]thiadiazine-7-carboxamide 1,1-dioxide | 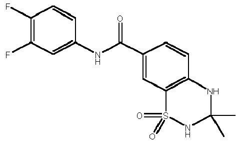 | 368 | Scheme 1: D8 → (3) → (6) |
| **E121** | trans-2-(4-hydroxycyclohexyl)- 3-oxo-N-(3,4,5-trifluorophenyl)-3,4-dihydro-2H-pyrido[2,3-e][1,2,4]thiadiazine-7-carboxamide 1,1-dioxide | 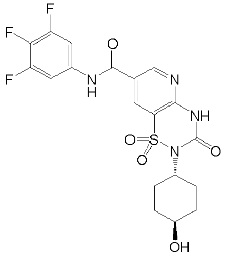 | 469 (ES -) | Scheme 1 |
| **E122** | trans-2-(4-hydroxycyclohexyl)-6-methyl-3-oxo-N-(3,4,5-trifluorophenyl)-3,4-dihydro-2H-benzo[e][1,2,4]thiadiazine-7-carboxamide 1,1-dioxide | 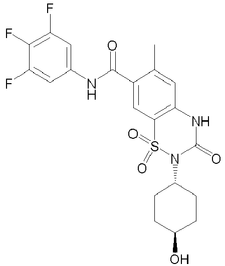 | 484 | Scheme 1: D16 → (3) → (4) |
| **E123** | N-(3-chloro-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide | 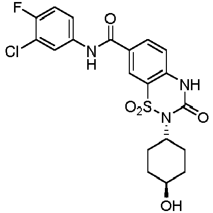 | 468 | Scheme 1: D11 → (3) → (4) |
| **E124** | Cis-2-(3-hydroxycyclobutyl)-3- oxo-N-(3,4,5-trifluorophenyl)-3,4-dihydro-2H-benzo[e][1,2,4]thiadiazine-7-carboxamide 1,1-dioxide | 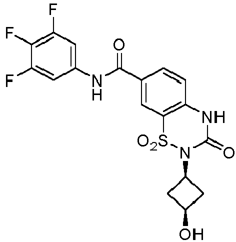 | 442 | Scheme 1: D7 → (3) → (4) |
| **E125** | Trans-2(3 -hydroxycyclobutyl)-3 - oxo-N-(3,4,5-trifluorophenyl)-3,4-dihydro-2H-benzo[e][1,2,4]thiadiazine-7-carboxamide 1,1-dioxide | 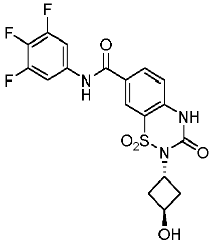 | 442 | Scheme 1: D7 → (3) → (4) |
| **E126** | Cis-2-(3-hydroxycyclobutyl)-N-(3,4,5-trifluorophenyl)-3,4-dihydro-2H-benzo[e][1,2,4]thiadiazine-7-carboxamide 1,1-dioxide | 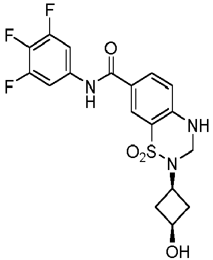 | 428 | Scheme 1: D7 → (3) → (6) |
| **E127** | Trans- 2-(3 - hydroxycyclobutyl)- N-(3,4,5-trifluorophenyl)-3,4-dihydro-2H-benzo[e][1,2,4]thiadiazine-7-carboxamide 1,1-dioxide | 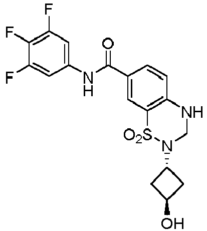 | 428 | Scheme 1: D7 → (3) → (6) |
| **E128** | 3-oxo-2-(pyridin-3-yl)-N-(3,4,5-trifluorophenyl)-3,4-dihydro-2H-benzo[e][1,2,4]thiadiazine-7-carboxamide 1,1-dioxide | 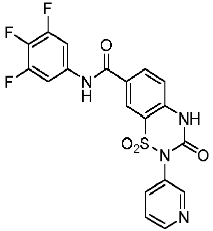 | 449 | Scheme 1: D7 → (3) → (4) |
| **E129** | 2'-oxo-N-(3,4,5-trifluorophenyl)-2H,4H-spiro[benzo[e][1,2,4]thiadiazine-3,3'-indoline]-7-carboxamide 1,1-dioxide (single enantiomer) | 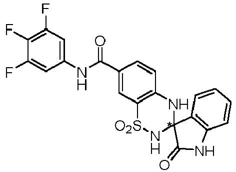 | 475 | E57 (chiral separation)^{a} |
| **E130** | (R)-2'-oxo-N-(3,4,5-trifluorophenyl)-2H,4H-spiro[benzo[e][1,2,4]thiadiazine-3,3'-indoline]-7-carboxamide 1,1-dioxide (single enantiomer) | 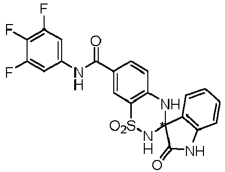 | 475 | E57 (chiral separation)^{a} |
| **E131** | N-(3-(difluoromethyl)-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide | 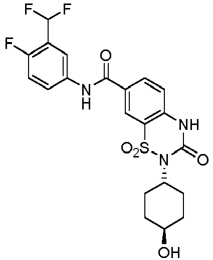 | 484 | Scheme 1: D14→(3) → (4) |
| **E132** | N-(4-fluoro-3-methylphenyl)-2-((1r,4r)-4-hydroxycyclohexyl)-3-oxo-3,4-dihydro-2H-benzo[e][1,2,4]thiadiazine-7-carboxamide 1,1-dioxide | 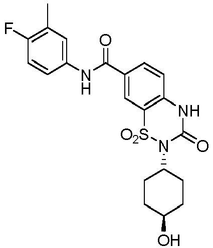 | 448 | Scheme 1: D17→(3) → (4) |
| **E133** | 2-(1-methylpiperidin-4-yl)-3-oxoN-(3,4,5-trifluorophenyl)-3,4-dihydro-2H-benzo[e][1,2,4]thiadiazine-7-carboxamide 1,1-dioxide | 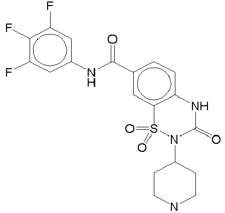 | 469 | Scheme 1: D7→(3) → (4) |
| **E134** | 2-(4-(hydroxymethyl)cyclohex-3-en-1-yl)-3-oxo-N-(3,4,5-trifluorophenyl)-3,4-dihydro-2H-benzo[e][1,2,4]thiadiazine-7-carboxamide 1,1-dioxide | 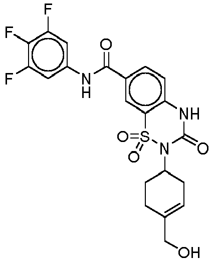 | 482 | Scheme 1: D7→(3) → (4) |
| **E135** | 3-(hydroxymethyl)-N-(3,4,5-trifluorophenyl)-3,4-dihydro-2H-benzo[b][1,4,5]oxathiazepine-8-carboxamide 1,1-dioxide | 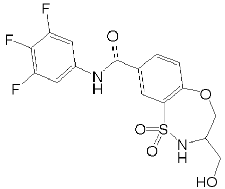 | 403 | Scheme 6: D7 → a) |
| **E136** | (3aR,10aS)-N-(3,4,5-trifluorophenyl)-2,3,3a,4,10,10a-hexahydro-1H-benzo[f]cyclopenta[c][1,2,5]thiadia zepine-7-carboxamide 5,5-dioxide | 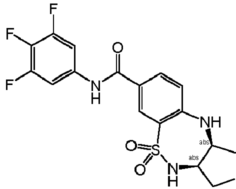 | 412 | Scheme 6: D7 → b) |
| **E137** | (3aS,10aR)-N-(3,4,5-trifluorophenyl)-2,3,3a,4,10,10a-hexahydro-1H-benzo[f]cyclopenta[c][1,2,5]thiadia zepine-7-carboxamide 5,5-dioxide | 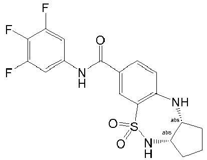 | 412 | Scheme 6: D7 → b) |
| **E138** | N-(3,4,5-trifluorophenyl)-4H-benzo[e][1,2,4]triazolo[5,1-c][1,2,4]thiadiazine-7-carboxamide 5,5-dioxide | 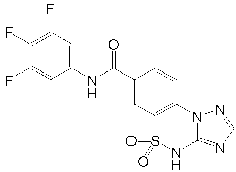 | 396 | Scheme 8 |
| **E139** | 4-(hydroxymethyl)-N-(3,4,5-trifluorophenyl)-3,4-dihydro-2H-benzo[b][1,4,5]oxathiazepine-8-carboxamide 1,1-dioxide | 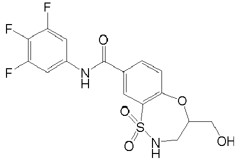 | 403 | Scheme 6: D7 → a) |
| **E140** | N-(3,4,5-trifluorophenyl)-2H,4H-spiro[benzo[b][1,4,5]oxathiazepine-3,3'-oxetane]-8-carboxamide 1,1-dioxide | 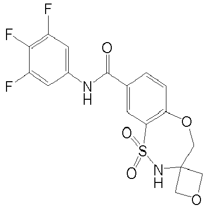 | 415 | Scheme 6: D7 → a) |
| **E141** | 3-(4-methylthiazol-5-yl)-N-(3,4,5-trifluorophenyl)-3,4-dihydro-2H-benzo[e][1,2,4]thiadiazine-7-carboxamide 1,1-dioxide | 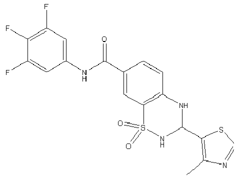 | 455 | Scheme 1: D7 → (3) → (6) |
| **E142** | 9-methyl-N-(3,4,5-trifluorophenyl)-1,3,4,11,12,12a-hexahydro-2H-benzo[f]pyrido[1,2-b][1,2,5]thiadiazepine-8-carboxamide 6,6-dioxide | 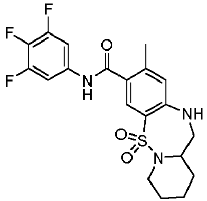 | 440 | Scheme 6: D16 → c) |
| **E143** | 2-(4-hydroxy-4-(hydroxymethyl)cyclohexyl)-3-oxo-N-(3,4,5-trifluorophenyl)-3,4-dihydro-2H-benzo[e][1,2,4]thiadiazine-7-carboxamide 1,1-dioxide | 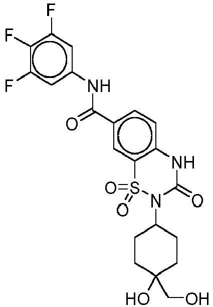 | 500 | Scheme 1: D7→(3) → (4) |

| | | | | |
|---|---|---|---|---|
| ^{a} **Chiral separation conditions:** Chiralpack IB-N5 (Daicel corporation), 1cm I.D.x 25 cm L at 20% B (Phase A: n-Heptane and Phase B: 2-Propanol). | | | | |

### Biology

### Assay

### Cells and culture conditions

HepAD38 cell line (Ladner et al., Antimicrob Agents Chemother, 1997, 41, 1715-20) was used for HBV inhibition assays. HepAD38 is a subclone, derived from Hepatoblastoma cell line HepG2 (ATCC® Number: HB-8065™), that expresses HBV genome under the control of a tetracycline-responsive promoter in a TET-OFF system: addition of tetracycline suppresses HBV replication, while its removal switches on the process allowing HBV viral particles release in the cell supernatant. HepAD38 cell line is maintained in DMEM/F12, supplemented with 10% of fetal bovine serum, 1% of glutamine, 1% of penicillin/streptomycin, 0.4 mg/ml G418 and 0,3 ug/ml tetracycline. For the HBV inhibition assay, doxycycline-free medium is used in order to allow virion production.

### Anti-HBV activity in vitro

HBV inhibition activity in vitro was performed in 96 multiwell plates. During the initial (primary) screening compounds were first tested in triplicates at concentrations of 0.1µM, 0.5µM amd 1µM. For selected compounds, an 8-point dose-response curve can be obtained using 1:2 serial dilutions (starting from 2.5 µM, 1.25µM or 0.4 µM, depending on the degree of inhibition observed during the primary screening). From the dose-response curves, half maximal effective concentration (EC₅₀) can be calculated (see also below).

In more detail, compounds - typically dissolved in DMSO stock solutions - are diluted to 2x the final desired concentration in 100 µl of the above medium (without doxycycline) and plated in three replicates in the 96-well plates.

Simultaneously, HepAD38 cells - extensively pre-washed in tetracycline-free medium in order to induce HBV production - are suspended at 2*10⁴ cells in 100 µl of tetracycline-free medium and added to each well of the plate, to yield a final assay volume of 200 µl DMSO - used for stock solutions and compounds dilutions - which is always present in the assays at a final concentration of 0.5%.

Plates are then incubated 96 hours at 37°C and then subjected to cell viability assay in order to define compounds cytotoxicity and to extracellular HBV quantification in order to evaluate antiviral activity of compounds. Cytotoxicity is assessed by a commercial fluorescence assay that measures the metabolic activity of cells, directly related to cell viability (Cell Titer Blue, Promega). Anti-HBV activity was evaluated by quantification of extracellular HBV DNA with direct qPCR. In particular, supernatant was collected and centrifuged for cell debris clarification, viral DNA was extracted from virions by addition of lysis buffer (1 mM 1,4-dithiothreitol, 0.2% sodium dodecyl sulphate) and incubated at 95°C for 10 min. Samples were then diluted 1:40 and real time PCR amplification was performed with SYBR green assay (Power SYBR™ Green PCR Master Mix-Thermo Fisher Scientific) and specific HBV primer (HBV-DF:5'-ATTTGTTCAGTGGTTCGTAGGG-3' (SEQ ID No. 1), HBV-DR:5'-CGGTAAAAAGGGACTCAAGATG-3' (SEQ ID No. 2)).

All HBV inhibition or antiviral activity data are typically reported in percent (%) relative to a non-treated reference sample. Excel and Graphpad Prism programs are typically used for data elaboration and EC₅₀ calculation.

### RESULTS

The exemplified compounds described herein were tested in the assays described above. All the compounds displayed no measurable cytotoxicity at the tested compound concentration.

Results for HBV inhibition are reported in the following Table 2.

Legend: A indicates HBV inhibition greater than 50% at the concentration indicated in the table or EC₅₀ less than 1µM; B indicates HBV inhibition less than 50% at the concentration indicated in the table or EC₅₀ greater than 1µM.

**Table 2**

| **Example** | **Compound Name** | **Anti HBV Activity (conc µM)** | **HBV inh EC₅₀ (µM)** |
|---|---|---|---|
| **E1** | N-(3,4,5-trifluorophenyl)-3,4-dihydro-2H-benzo[e][1,2,4]thiadiazine-7-carboxamide 1,1-dioxide | A(1) | - |
| **E2** | N-(3,4,5-trifluorophenyl)-3,4-dihydro-2H-benzo[e][1,2,4]thiadiazine-3,3-d₂-7-carboxamide 1,1-dioxide | A (0.5) | - |
| **E3** | 4-methyl-N-(3,4,5-trifluorophenyl)-3,4-dihydro-2H-benzo[e][1,2,4]thiadiazine-7-carboxamide 1,1-dioxide | B (1) | - |
| **E4** | 4-hydroxy-N-(3,4,5-trifluorophenyl)-3,4-dihydro-2H-benzo[e][1,2,4]thiadiazine-7-carboxamide 1,1-dioxide | B (0.5) | - |
| **E5** | 2-hydroxy-N-(3,4,5-trifluorophenyl)-3,4-dihydro-2H-benzo[e][1,2,4]thiadiazine-7-carboxamide 1,1-dioxide | B (0.5) | - |
| **E6** | 2-methyl-N-(3,4,5-trifluorophenyl)-3,4-dihydro-2H-benzo[e][1,2,4]thiadiazine-7-carboxamide 1,1-dioxide | B (1) | - |
| **E7** | 6-chloro-N-(3,4,5-trifluorophenyl)-3,4-dihydro-2H-benzo[e][1,2,4]thiadiazine-7-carboxamide 1,1-dioxide | - | A |
| **E8** | 6-bromo-N-(3,4,5-trifluorophenyl)-3,4-dihydro-2H-benzo[e][1,2,4]thiadiazine-7-carboxamide 1,1-dioxide | A (1) | - |
| **E9** | 6-methyl-N-(3,4,5-trifluorophenyl)-3,4-dihydro-2H-benzo[e][1,2,4]thiadiazine-7-carboxamide 1,1-dioxide | A (0.5) | - |
| **E10** | 6-chloro-3-methyl-N-(3,4,5-trifluorophenyl)-3,4-dihydro-2H-benzo[e][1,2,4]thiadiazine-7 -carboxamide 1,1-dioxide | - | A |
| **E11** | N-(3,4-difluorophenyl)-2H,4H-spiro[benzo[e][1,2,4]thiadiazine-3,3'-oxetane]-7-carboxamide 1,1-dioxide | B (1) | - |
| **E12** | N-(3,4-difluorophenyl)-2H,4H-spiro[benzo[e][1,2,4]thiadiazine-3,1'-cyclobutane]-7-carboxamide 1,1-dioxide | A (1) | - |
| **E13** | N-(3,4,5-trifluorophenyl)-2',3',5',6'-tetrahydro-2H,4H-spiro[benzo[e][1,2,4]thiadiazine-3,4'-pyran]-7-carboxamide 1,1-dioxide | A (1) | - |
| **E14** | N-(3,4,5-trifluorophenyl)-2H,4H-spiro[benzo[e][1,2,4]thiadiazine-3,4'-piperidine]-7-carboxamide 1,1-dioxide | A (0.5) | - |
| **E15** | 3-(trifluoromethyl)-N-(3,4,5-trifluorophenyl)-3,4-dihydro-2H-benzo[e][1,2,4]thiadiazine-7-carboxamide 1,1-dioxide | B (1) | - |
| **E16** | 3-(2-isopropoxyethyl)-N-(3,4,5-trifluorophenyl)-3,4-dihydro-2H-benzo[e][1,2,4]thiadiazine-7-carboxamide 1,1-dioxide | A (1) | - |
| **E17** | 3-(difluoromethyl)-N-(3,4,5-trifluorophenyl)-3,4-dihydro-2H-benzo[e][1,2,4]thiadiazine-7-carboxamide 1,1-dioxide | A (1) | - |
| **E18** | 2-methyl-3-(trifluoromethyl)-N-(3,4,5-trifluorophenyl)-3,4-dihydro-2H-benzo[e][1,2,4]thiadiazine-7-carboxamide 1,1-dioxide | B (0.5) | - |
| **E19** | N-(3,4-difluorophenyl)-3,4-dihydro-2H-benzo[e][1,2,4]thiadiazine-7-carboxamide 1,1-dioxide | A (1) | - |
| **E20** | N-(3,4-difluorophenyl)-3-methyl-3,4-dihydro-2H-benzo[e][1,2,4]thiadiazine-7-carboxamide 1,1-dioxide | A (1) | - |
| **E21** | 3-methyl-N-(3,4,5-trifluorophenyl)-3,4-dihydro-2H-benzo[e][1,2,4]thiadiazine-7-carboxamide 1,1-dioxide | - | A |
| **E22** | 3-(2-hydroxyethyl)-N-(3,4,5-trifluorophenyl)-3,4-dihydro-2H-benzo[e][1,2,4]thiadiazine-7-carboxamide 1,1-dioxide | A (0.5) | - |
| **E23** | 3-((S)-1-hydroxyethyl)-N-(3,4,5-trifluorophenyl)-3,4-dihydro-2H-benzo[e][1,2,4]thiadiazine-7-carboxamide 1,1-dioxide | A (0.5) | - |
| **E24** | 3-((R)-1,2-dihydroxyethyl)-N-(3,4,5-trifluorophenyl)-3,4-dihydro-2H-benzo[e][1,2,4]thiadiazine-7-carboxamide 1,1-dioxide | A (0.5) | - |
| **E25** | (S)-3-(1-hydroxyethyl)-N-(3,4,5-trifluorophenyl)-4H-benzo[e][1,2,4]thiadiazine-7-carboxamide 1,1-dioxide | B (0.5) | - |
| **E26** | 3-((R)-1-hydroxyethyl)-N-(3,4,5-trifluorophenyl)-3,4-dihydro-2H-benzo[e][1,2,4]thiadiazine-7-carboxamide 1,1-dioxide | A (0.5) | - |
| **E27** | 3,3-dimethyl-N-(3,4,5-trifluorophenyl)-3,4-dihydro-2H-benzo[e][1,2,4]thiadiazine-7-carboxamide 1,1-dioxide | A (0.5) | - |
| **E28** | 3-(hydroxymethyl)-N-(3,4,5-trifluorophenyl)-3,4-dihydro-2H-benzo[e][1,2,4]thiadiazine-7-carboxamide 1,1-dioxide | A (0.5) | - |
| **E29** | 3-(aminomethyl)-N-(3,4,5-trifluorophenyl)-3,4-dihydro-2H-benzo[e][1,2,4]thiadiazine-7-carboxamide 1,1-dioxide | A (1) | - |
| **E30** | 3-(acetamidomethyl)-N-(3,4,5-trifluorophenyl)-3,4-dihydro-2H-benzo[e][1,2,4]thiadiazine-7-carboxamide 1,1-dioxide | A (1) | - |
| **E31** | *tert*-butyl 4-(1,1-dioxido-7-((3,4,5-trifluorophenyl)carbamoyl)-3,4-dihydro-2H-benzo[e][1,2,4]thiadiazin-3-yl)piperidine-1-carboxylate | A (1) | - |
| **E32** | 3-(piperidin-4-yl)-N-(3,4,5-trifluorophenyl)-3,4-dihydro-2H-benzo[e][1,2,4]thiadiazine-7-carboxamide 1,1-dioxide | - | A |
| **E33** | 2-methyl-3-(piperidin-4-yl)-N-(3,4,5-trifluorophenyl)-3,4-dihydro-2H-benzo[e][1,2,4]thiadiazine-7-carboxamide 1,1-dioxide | B (0.5) | - |
| **E34** | 6-chloro-N-(3,4,5-trifluorophenyl)-2H,4H-spiro[benzo[e][1,2,4]thiadiazine-3,1'-cyclobutane]-7-carboxamide 1,1-dioxide | B (1) | - |
| **E35** | 3-(2,6-difluorophenyl)-N-(3,4,5-trifluorophenyl)-3,4-dihydro-2H-benzo[e][1,2,4]thiadiazine-7-carboxamide 1,1-dioxide | - | A |
| **E36** | 6-chloro-3-(thiazol-2-yl)-N-(3,4,5-trifluorophenyl)-3,4-dihydro-2H-benzo[e][1,2,4]thiadiazine-7-carboxamide 1,1-dioxide | A (1) | - |
| **E37** | 3-(1-methyl-1H-imidazol-5-yl)-N-(3,4,5-trifluorophenyl)-3,4-dihydro-2H-benzo[e][1,2,4]thiadiazine-7-carboxamide 1,1-dioxide | - | A |
| **E38** | N-(3,4-difluorophenyl)-3-(2,4-dimethyloxazol-5-yl)-3,4-dihydro-2H-benzo[e][1,2,4]thiadiazine-7-carboxamide 1,1-dioxide | A (1) | - |
| **E39** | 3-methyl-3-(pyridin-4-yl)-N-(3,4,5-trifluorophenyl)-3,4-dihydro-2H-benzo[e][1,2,4]thiadiazine-7-carboxamide 1,1-dioxide | A (1) | - |
| **E40** | 3-(thiazol-5-yl)-N-(3,4,5-trifluorophenyl)-3,4-dihydro-2H-benzo[e][1,2,4]thiadiazine-7-carboxamide 1,1-dioxide | A (1) | - |
| **E41** | 3-(thiazol-4-yl)-N-(3,4,5-trifluorophenyl)-3,4-dihydro-2H-benzo[e][1,2,4]thiadiazine-7-carboxamide 1,1-dioxide | A (1) | - |
| **E42** | 3-(6-(trifluoromethyl)pyridin-3-yl)-N-(3,4,5-trifluorophenyl)-3,4-dihydro-2H-benzo[e][1,2,4]thiadiazine-7-carboxamide 1,1-dioxide | A (1) | - |
| **E43** | 3-(thiophen-2-yl)-N-(3,4,5-trifluorophenyl)-3,4-dihydro-2H-benzo[e][1,2,4]thiadiazine-7-carboxamide 1,1-dioxide | A (1) | - |
| **E44** | 3-(pyridin-2-yl)-N-(3,4,5-trifluorophenyl)-3,4-dihydro-2H-benzo[e][1,2,4]thiadiazine-7-carboxamide 1,1-dioxide | A (1) | - |
| **E45** | 3-(1,2,3-thiadiazol-5-yl)-N-(3,4,5-trifluorophenyl)-3,4-dihydro-2H-benzo[e][1,2,4]thiadiazine-7-carboxamide 1,1-dioxide | A (1) | - |
| **E46** | 3-(1H-pyrazol-5-yl)-N-(3,4,5-trifluorophenyl)-3,4-dihydro-2H-benzo[e][1,2,4]thiadiazine-7-carboxamide 1,1-dioxide | A (1) | - |
| **E47** | N-(3,4-difluorophenyl)-3-(pyridin-3-yl)-3,4-dihydro-2H-benzo[*e*][1,2,4]thiadiazine-7-carboxamide 1,1-dioxide | A (1) | - |
| **E48** | 3-(pyridin-3-yl)-N-(3,4,5-trifluorophenyl)-3,4-dihydro-2H-benzo[*e*][1,2,4]thiadiazine-7-carboxamide 1,1-dioxide | A (1) | - |
| **E49** | 3-(thiazol-2-yl)-N-(3,4,5-trifluorophenyl)-3,4-dihydro-2H-benzo[*e*][1,2,4]thiadiazine-7-carboxamide 1,1-dioxide | - | A |
| **E50** | N-(3,4-difluorophenyl)-3-(thiazol-2-yl)-3,4-dihydro-2H-benzo[*e*][1,2,4]thiadiazine-7-carboxamide 1,1-dioxide | A (1) | - |
| **E51** | 3-(1H-imidazol-5-yl)-N-(3,4,5-trifluorophenyl)-3,4-dihydro-2H-benzo[e][1,2,4]thiadiazine-7-carboxamide 1,1-dioxide | B (1) | - |
| **E52** | 3-(6-chloropyridin-3-yl)-N-(3,4,5-trifluorophenyl)-3,4-dihydro-2H-benzo[e][1,2,4]thiadiazine-7-carboxamide 1,1-dioxide | A (1) | - |
| **E53** | 3-(5-chloropyridin-2-yl)-3-methyl-N-(3,4,5-trifluorophenyl)-3,4-dihydro-2H-benzo[e][1,2,4]thiadiazine-7-carboxamide 1,1-dioxide | A (1) | - |
| **E54** | 3-(pyrimidin-2-yl)-N-(3,4,5-trifluorophenyl)-3,4-dihydro-2H-benzo[e][1,2,4]thiadiazine-7-carboxamide 1,1-dioxide | A (0.5) | - |
| **E55** | 3-methyl-3-(pyrazin-2-yl)-N-(3,4,5-trifluorophenyl)-3,4-dihydro-2H-benzo[e][1,2,4]thiadiazine-7-carboxamide 1,1-dioxide | A (0.5) | - |
| **E56** | 2'-oxo-N-(3,4,5-trifluorophenyl)-2H,4H-spiro[benzo[e][1,2,4]thiadiazine-3,3'-indoline]-7-carboxamide 1,1-dioxide | A (0.5) | - |
| **E57** | 2'-oxo-N-(3,4,5-trifluorophenyl)-1',2'-dihydro-2H,4H-spiro[benzo[e][1,2,4]thiadiazine-3,3'-pyrrolo[2,3-b]pyridine]-7-carboxamide 1, 1-dioxide | A (0.5) | - |
| **E58** | *trans*-2-(4-hydroxycyclohexyl)-N-(3,4,5-trifluorophenyl)-3,4-dihydro-2H-benzo[e] [1,2,4]thiadiazine-7-carboxamide 1,1-dioxide | B (0.5) | - |
| **E59** | *cis*-2-(4-hydroxycyclohexyl)-N-(3,4,5-trifluorophenyl)-3,4-dihydro-2H-benzo[e][1,2,4]thiadiazine-7-carboxamide 1,1-dioxide | B (0.5) | - |
| **E60** | 2-cyclopropyl-N-(3,4,5-trifluorophenyl)-3,4-dihydro-2H-benzo[e][1,2,4]thiadiazine-7-carboxamide 1,1-dioxide | B (0.5) | - |
| **E61** | 2-(piperidin-4-yl)-N-(3,4,5-trifluorophenyl)-3,4-dihydro-2H-benzo[e][1,2,4]thiadiazine-7-carboxamide 1,1-dioxide | B (0.5) | - |
| **E62** | 2-(2-hydroxyethyl)-N-(3,4,5-trifluorophenyl)-3,4-dihydro-2H-benzo[e][1,2,4]thiadiazine-7-carboxamide 1,1-dioxide | B (1) | - |
| **E63** | 2-(2-hydroxyethyl)-3 -methyl-N-(3,4,5-trifluorophenyl)-3,4-dihydro-2H-benzo[e][1,2,4]thiadiazine-7-carboxamide 1,1-dioxide | B (1) | - |
| **E64** | N-(3,4,5-trifluorophenyl)-2,3,10,10a-tetrahydro-1H-benzo[e]pyrrolo[1,2-b][1,2,4]thiadiazine-7-carboxamide 5,5-dioxide | B (0.5) | - |
| **E65** | N-(3-chloro-4-fluorophenyl)-3,4-dihydro-2H-benzo[e][1,2,4]thiadiazine-7-carboxamide 1,1-dioxide | A (0.5) | - |
| **E66** | N-(3,4-difluorophenyl)-2H-benzo[e] [1,2,4]thiadiazine-7-carboxamide 1,1-dioxide | B (1) | - |
| **E67** | N-(3,4-difluorophenyl)-3-methyl-2H-benzo[e][1,2,4]thiadiazine-7-carboxamide 1,1-dioxide | B (1) | - |
| **E68** | 6-fluoro-N-(3,4,5-trifluorophenyl)-2H-benzo[e][1,2,4]thiadiazine-7-carboxamide 1,1-dioxide | B (1) | - |
| **E69** | 3-amino-N-(3,4,5-trifluorophenyl)-2H-benzo[e][1,2,4]thiadiazine-7-carboxamide 1,1-dioxide | B (1) | - |
| **E70** | 3-acetamido-N-(3,4,5-trifluorophenyl)-2H-benzo[e][1,2,4]thiadiazine-7-carboxamide 1,1-dioxide | B (1) | - |
| **E71** | 6-fluoro-N-(3,4,5-trifluorophenyl)-3,4-dihydro-2H-benzo[e][1,2,4]thiadiazine-7-carboxamide 1,1-dioxide | - | A |
| **E72** | 5-fluoro-N-(3,4,5-trifluorophenyl)-3,4-dihydro-2H-benzo[e][1,2,4]thiadiazine-7-carboxamide 1,1-dioxide | - | B |
| **E73** | 6-fluoro-4-methyl-N-(3,4,5-trifluorophenyl)-4H-benzo[e][1,2,4]thiadiazine-7-carboxamide 1,1-dioxide | B (1) | - |
| **E74** | N-(3,4-difluorophenyl)-3-oxo-3,4-dihydro-2H-benzo[e][1,2,4]thiadiazine-7-carboxamide 1,1-dioxide | B (1) | - |
| **E75** | 3-oxo-N-(3,4,5-trifluorophenyl)-3,4-dihydro-2H-benzo[e][1,2,4]thiadiazine-7-carboxamide 1,1-dioxide | B (1) | - |
| **E76** | 2-methyl-3-oxo-N-(3,4,5-trifluorophenyl)-3,4-dihydro-2H-benzo[e][1,2,4]thiadiazine-7-carboxamide 1,1-dioxide | A (1) | - |
| **E77** | (R)-3-oxo-N-(3,4,5-trifluorophenyl)-2-(1,1,1-trifluoropropan-2-yl)-3,4-dihydro-2H-benzo[e][1,2,4]thiadiazine-7-carboxamide 1,1-dioxide | B (0.5) | - |
| **E78** | (S)-3-oxo-N-(3,4,5-trifluorophenyl)-2-(1,1,1-trifluoropropan-2-yl)-3,4-dihydro-2H-benzo[e][1,2,4]thiadiazine-7-carboxamide 1,1-dioxide | A (0.5) | - |
| **E79** | 2-cyclopropyl-3-oxo-N-(3,4,5-trifluorophenyl)-3,4-dihydro-2H-benzo[e][1,2,4]thiadiazine-7-carboxamide 1,1-dioxide | B (1) | - |
| **E80** | *trans*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide | A (1) | - |
| **E81** | 3-oxo-2-(piperidin-4-yl)-N-(3,4,5-trifluorophenyl)-3,4-dihydro-2H-benzo[e][1,2,4]thiadiazine-7-carboxamide 1,1-dioxide | A (1) | - |
| **E82** | 2-(oxetan-3-yl)-3-oxo-N-(3,4,5-trifluorophenyl)-3,4-dihydro-2H-benzo[e][1,2,4]thiadiazine-7-carboxamide 1,1-dioxide | A (0.5) | - |
| **E83** | tert-butyl (S)-3-(1,1-dioxido-3-oxo-7-((3,4,5-trifluorophenyl)carbamoyl)-3,4-dihydro-2H-benzo[e][1,2,4]thiadiazin-2-yl)pyrrolidine-1-carboxylate | B (0.5) | - |
| **E84** | (S)-3-oxo-2-(pyrrolidin-3-yl)-N-(3,4,5-trifluorophenyl)-3,4-dihydro-2H-benzo[e][1,2,4]thiadiazine-7-carboxamide 1,1-dioxide | B (0.1) | - |
| **E85** | (S)-2-(1-methylpyrrolidin-3-yl)-3-oxo-N-(3,4,5-trifluorophenyl)-3,4-dihydro-2H-benzo[e][1,2,4]thiadiazine-7-carboxamide 1,1-dioxide | B (0.1) | - |
| **E86** | 2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide | A (0.5) | - |
| **E87** | *cis*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide | A (0.5) | - |
| **E88** | 5-methyl-N-(3,4,5-trifluorophenyl)-3,4-dihydro-2H-benzo[e][1,2,4]thiadiazine-7-carboxamide 1,1-dioxide | - | B |
| **E89** | 5-methyl-3-(thiazol-2-yl)-N-(3,4,5-trifluorophenyl)-3,4-dihydro-2H-benzo[e][1,2,4]thiadiazine-7-carboxamide 1,1-dioxide | A (1) | - |
| **E90** | N-(3-cyano-4-fluorophenyl)-2H-benzo[e][1,2,4]thiadiazine-7-carboxamide 1,1-dioxide | B (1) | - |
| **E91** | N-(3-cyano-4-fluorophenyl)-3,4-dihydro-2H-benzo[e][1,2,4]thiadiazine-7-carboxamide 1,1-dioxide | B (1) | - |
| **E92** | N-(6-chloropyridin-3-yl)-3,4-dihydro-2H-benzo[e][1,2,4]thiadiazine-7-carboxamide 1,1-dioxide | B (0.5) | - |
| **E93** | N-(3,4,5-trifluorophenyl)-3,4-dihydro-2H-pyrido[2,3-e][1,2,4]thiadiazine-7-carboxamide 1,1-dioxide | B (0.5) | - |
| **E94** | N-(3,4,5-trifluorophenyl)-2,3,4,5-tetrahydrobenzo[*f*][1,2,5]thiadiazepine-8-carboxamide 1,1-dioxide | - | B |
| **E95** | (3aS, 10aS)-N-(3,4,5-trifluorophenyl)-2,3,3a,4,10,10a-hexahydro-1H-benzo[f]cyclopenta[c][1,2,5]thiadiazepine-7-carboxamide 5,5-dioxide | B (0.5) | - |
| **E96** | N-(3,4,5-trifluorophenyl)-3,4-dihydro-2H-benzo[b][1,4,5]oxathiazepine-8-carboxamide 1,1-dioxide | B | - |
| **E101** | N-(3,4,5-trifluorophenyl)-1,3,4,11,12,12a-hexahydro-2H-benzo[f]pyrido[1,2-b][1,2,5]thiadiazepine-8-carboxamide 6,6-dioxide | A (1) | - |
| **E102** | 11-methyl-N-(3,4,5-trifluorophenyl)-1,3,4,11,12,12a-hexahydro-2H-benzo[f]pyrido[1,2-b][1,2,5]thiadiazepine-8-carboxamide 6,6-dioxide | B (1) | - |
| **E103** | (S)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide | B (1) | - |
| **E104** | (R)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide | A (1) | - |
| **E105** | (5S)-N-(3,4,5-trifluorophenyl)-3,4,5,6-tetrahydro-2,5-methanobenzo[g][1,2,6]thiadiazocine-9-carboxamide 1,1-dioxide | B (1) | - |
| **E106** | N-(3,4,5-trifluorophenyl)-1,2,3,10,11,11a-hexahydrobenzo[f]pyrrolo[1,2-b][1,2,5]thiadiazepine-7-carboxamide 5,5-dioxide | B (1) | - |
| **E107** | N-(3,4,5-trifluorophenyl)-1,9,10,10a-tetrahydro-2H-azeto[1,2-b]benzo[f][1,2,5]thiadiazepine-6-carboxamide 4,4-dioxide | B (1) | - |
| **E108** | N-(3,4,5-trifluorophenyl)-1,3,4,11,12,12a-hexahydrobenzo[f][1,4]oxazino[4,3-b][1,2,5]thiadiazepine-8-carboxamide 6,6-dioxide | B (1) | - |
| **E109** | N-(3,4,5-trifluorophenyl)-10,11-dihydrobenzo[f]imidazo[1,2-b][1,2,5]thiadiazepine-7-carboxamide 5,5-dioxide | B (1) | - |
| **E110** | N-(3,4-difluorophenyl)-3,4-dihydro-2,5-ethanobenzo[f][1,2,5]thiadiazepine-8-carboxamide 1,1-dioxide | B (1) | - |
| **E111** | N-(3,4,5-trifluorophenyl)-1,2,3,4,12,12a-hexahydrobenzo[b]pyrido[1,2-e][1,4,5]oxathiazepine-8-carboxamide 6,6-dioxide | B (1) | - |
| **E112** | (R)-N-(3,4,5-trifluorophenyl)-1,2,3,4,12,12a-hexahydrobenzo[b]pyrido[1,2-e][1,4,5]oxathiazepine-8-carboxamide 6,6-dioxide | B (1) | - |
| **E113** | (S)-N-(3,4,5-trifluorophenyl)-1,2,3,4,12,12a-hexahydrobenzo[b]pyrido[1,2-e][1,4,5]oxathiazepine-8-carboxamide 6,6-dioxide | B (1) | - |
| **E114** | *tert*-butyl 8-((3,4,5-trifluorophenyl)carbamoyl)-3,4,12,12a-tetrahydrobenzo[b]pyrazino[1,2-e][1,4,5]oxathiazepine-2(1H)-carboxylate 6,6-dioxide | B (1) | - |
| **E115** | N-(3,4,5-trifluorophenyl)-1,2,3,4,12,12a-hexahydrobenzo[b]pyrazino[1,2-e][1,4,5]oxathiazepine-8-carboxamide 6,6-dioxide | B (1) | - |
| **E116** | 2-acetyl-N-(3,4,5-trifluorophenyl)-1,2,3,4,12,12a-hexahydrobenzo[b]pyrazino[1,2-e][1,4,5]oxathiazepine-8-carboxamide 6,6-dioxide | B (1) | - |
| **E117** | N-(3,4,5-trifluorophenyl)-3,4-dihydro-2,4-methanobenzo[b][1,4,5]oxathiazepine-8-carboxamide 1,1-dioxide | B (1) | - |
| **E118** | N-(3,4,5-trifluorophenyl)-4,5-dihydro-3H-2,5-methanobenzo[b][1,4,5]oxathiazocine-9-carboxamide 1,1-dioxide | B (1) | - |
| **E119** | N-(3,4-difluorophenyl)-3-(hydroxymethyl)-3,4-dihydro-2H-benzo[e][1,2,4]thiadiazine-7-carboxamide 1,1-dioxide | A (1) | - |
| **E120** | N-(3,4-difluorophenyl)-3,3-dimethyl-3,4-dihydro-2H-benzo[e][1,2,4]thiadiazine-7-carboxamide 1,1-dioxide | A (1) | - |
| **E121** | trans-2-(4-hydroxycyclohexyl)-3-oxo-N-(3,4,5-trifluorophenyl)-3,4-dihydro-2H-pyrido[2,3-e][1,2,4]thiadiazine-7-carboxamide 1,1-dioxide | B (0.5) | - |
| **E122** | trans-2-(4-hydroxycyclohexyl)-6-methyl-3-oxo-N-(3,4,5-trifluorophenyl)-3,4-dihydro-2H-benzo[e][1,2,4]thiadiazine-7-carboxamide 1,1-dioxide | A (0.1) | - |
| **E123** | N-(3-chloro-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide | A (0.1) | - |
| **E124** | Cis-2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide | A (0.5) | - |
| **E125** | Trans-2(3-hydroxycyclobutyl)-3-oxo-N-(3,4,5-trifluorophenyl)-3,4-dihydro-2H-benzo[e][1,2,4]thiadiazine-7-carboxamide 1,1-dioxide | A (0.5) | - |
| **E126** | Cis-2-(3-hydroxycyclobutyl)-N-(3,4,5-trifluorophenyl)-3,4-dihydro-2H-benzo[e][1,2,4]thiadiazine-7-carboxamide 1,1-dioxide | B (0.5) | - |
| **E127** | Trans-2-(3-hydroxycyclobutyl)-N-(3,4,5-trifluorophenyl)-3,4-dihydro-2H-benzo[e][1,2,4]thiadiazine-7-carboxamide 1,1-dioxide | B (0.5) | - |
| **E128** | 3-oxo-2-(pyridin-3-yl)-N-(3,4,5-trifluorophenyl)-3,4-dihydro-2H-benzo[e][1,2,4]thiadiazine-7-carboxamide 1,1-dioxide | B (0.5) | - |
| **E129** | (S)-2'-oxo-N-(3,4,5-trifluorophenyl)-2H,4H-spiro[benzo[e][1,2,4]thiadiazine-3,3'-indoline]-7-carboxamide 1,1-dioxide (single enantiomer; first eluted) | - | B |
| **E130** | (R)-2'-oxo-N-(3,4,5-trifluorophenyl)-2H,4H-spiro[benzo[e][1,2,4]thiadiazine-3,3'-indoline]-7-carboxamide 1,1-dioxide (single enantiomer; second eluted) | - | A |
| **E131** | N-(3-(difluoromethyl)-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide | A (0.5) | - |
| **E132** | N-(4-fluoro-3-methylphenyl)-2-((1r,4r)-4-hydroxycyclohexyl)-3-oxo-3,4-dihydro-2H-benzo[e][1,2,4]thiadiazine-7-carboxamide 1,1-dioxide | A (0.5) | - |
| **E133** | 2-(1-methylpiperidin-4-yl)-3-oxo-N-(3,4,5-trifluorophenyl)-3,4-dihydro-2H-benzo[e][1,2,4]thiadiazine-7-carboxamide 1,1-dioxide | A (1) | - |
| **E134** | 2-(4-(hydroxymethyl)cyclohex-3-en-1-yl)-3-oxo-N-(3,4,5-trifluorophenyl)-3,4-dihydro-2H-benzo[e][1,2,4]thiadiazine-7-carboxamide 1,1-dioxide | A (0.5) | - |
| **E135** | 3-(hydroxymethyl)-N-(3,4,5-trifluorophenyl)-3,4-dihydro-2H-benzo[b][1,4,5]oxathiazepine-8-carboxamide 1,1-dioxide | B (1) | - |
| **E136** | (3aR,10aS)-N-(3,4,5-trifluorophenyl)-2,3,3a,4,10,10a-hexahydro-1H-benzo[f]cyclopenta[c][1,2,5]thiadiazepine-7-carboxamide 5,5-dioxide | B (1) | - |
| **E137** | (3aS,10aR)-N-(3,4,5-trifluorophenyl)-2,3,3a,4,10,10a-hexahydro-1H-benzo[f]cyclopenta[c][1,2,5]thiadiazepine-7-carboxamide 5,5-dioxide | B (1) | - |
| **E138** | N-(3,4,5-trifluorophenyl)-4H-benzo[e][1,2,4]triazolo[5,1-c][1,2,4]thiadiazine-7-carboxamide 5,5-dioxide | B (1) | - |
| **E139** | 4-(hydroxymethyl)-N-(3,4,5-trifluorophenyl)-3,4-dihydro-2H-benzo[b][1,4,5]oxathiazepine-8-carboxamide 1,1-dioxide | B (1) | - |
| **E140** | N-(3,4,5-trifluorophenyl)-2H,4H-spiro[benzo[b][1,4,5]oxathiazepine-3,3'-oxetane]-8-carboxamide 1,1-dioxide | B (1) | - |

## Claims

1. A compound of general formula (I): 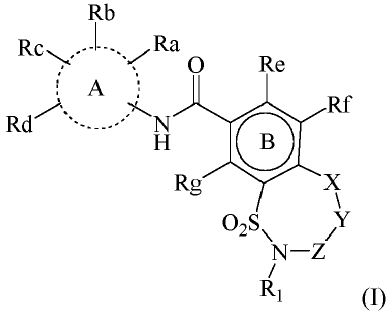 wherein:
A is aryl or heteroaryl;
B is a 6-membered aryl optionally containing one or more N atoms;
X is N optionally substituted with R₄; or is O;
Y is a single bond; a double bond; a C₁₋₄alkanediyl or a C₂₋₄alkenediyl each of said C₁₋₄alkanediyl or C₂₋₄alkenediyl being independently optionally substituted with R₅;
Z is CR₂R₃ or C(O);
R₁ is selected from:
- hydrogen;
- OH;
- C₁₋₆alkyl optionally substituted with one or more substituents each independently selected from the group consisting of OH, halogen, CN, NH₂, NH(R₆), N(R₆)₂, aryl, heteroaryl, 3-7 membered saturated ring and 5-7 membered unsaturated ring, each of said saturated or unsaturated ring optionally containing one or more heteroatoms selected from the group consisting of O, N, S and each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
- aryl or heteroaryl ring, each of said aryl or heteroaryl ring being optionally substituted with one or more substituents selected from OH, halogen, haloC₁₋₆alkyl, CN, haloC₁₋₆alkoxy and C₁₋₆alkoxy; and
- a 3-8 membered saturated or partially unsaturated ring optionally containing one or more heteroatoms each independently selected from the group consisting of O, S and N, the 3-8 membered saturated or partially unsaturated ring being optionally substituted with one, two or more substituents each independently selected from the group consisting of OH, halogen, CN, C₁-₄alkyl, hydroxyC₁-₄alkyl, C(O)OR₆, C(O)R₆, haloC₁₋₆alkyl, haloC₁₋₆alkoxy and C₁₋₆alkoxy;
R₂ is selected from:
- hydrogen;
- deuterium;
- C₁₋₆alkyl; C₁₋₆alkoxy; C₂₋₆alkenyl; each of said C₁₋₆alkyl, C₁₋₆alkoxy or C₂₋₆alkenyl being optionally substituted with one or more substituents independently selected from OH, halogen, NH₂, NH(R₆), N(R₆)₂, NHC(O)R₆, C₁₋₆alkoxy, haloC₁₋₆alkoxy, CN, aryl, heteroaryl, 3-7 membered saturated ring and 5-7 membered unsaturated ring, each of said 3-7 membered saturated or 5-7 membered unsaturated ring optionally containing one or more heteroatoms selected from the group consisting of O, N, S, each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
- NH₂;
- NHC(O)R₆;
- a 3-8 membered saturated ring optionally containing one or more heteroatoms each independently selected from the group consisting of O, S and N, the 3-8 membered saturated ring being optionally substituted with one or more substituents each independently selected from the group consisting of OH, halogen, C(O)OR₆, C(O)R₆, C₁₋₆alkyl and CN; and
- an aryl or heteroaryl ring optionally substituted with one, two, three or more substituents each independently selected from the group consisting of C₁₋₆alkyl, haloC₁₋₆alkyl, halogen, C₁₋₆alkoxy and haloC₁₋₆alkoxy;
R₃ is absent, hydrogen, deuterium or C₁-₆alkyl;
R₄ is hydrogen, C₁₋₄alkyl, C₃₋₆cycloalkyl or OH;
R₅ is C₁₋₃alkyl optionally substituted with OH;
R₆ is C₁-₄alkyl;
or R₁ and R₂ taken together form with the atoms to which they are respectively attached a heterocyclic ring optionally substituted with one or more substituents each independently selected from the group consisting of C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl ring optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or any two residues of R₁, R₂, R₃, R₄, R₅ which are not geminal or vicinal group, taken together represent a single bond, or represent a C₁₋₄alkanediyl or a C₂₋₄alkenediyl, each of said C₁₋₄alkanediyl or a C₂₋₄alkenediyl being independently optionally substituted with one or more substituents each independently selected from C₁₋₄ alkyl and halogen; said single bond or said optionally substituted C₁₋₄alkanediyl or C₂₋₄alkenediyl forming together with the bridging atoms to which they are respectively linked a 4-10 membered saturated or partially unsaturated ring;
or R₂ and R₃ taken together form with the carbon atom to which they are attached a 3-7 membered saturated ring optionally containing one or more heteroatoms each independently selected from the group consisting of O, S and N, the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)R₆;
or R₂ and R₃ taken together form with the carbon atom to which they are attached an indoline-2-one or an azaindoline-2-one, each being optionally substituted with one or more substituents selected from halogen, hydroxy, C₁₋₆alkyl, haloC₁₋₆alkyl, C₁₋₆alkoxy and haloC₁₋₆alkoxy;
or R₂ and R₅ taken together form with the carbon atoms to which they are respectively attached a 3-8 membered saturated ring or a 5-10 membered partially saturated ring, each ring optionally containing one or more heteroatoms each independently selected from the group consisting of O, S and N, each ring optionally being substituted with one, two or more substituents each independently selected from the group consisting of halogen, OH, CN, C(O)OR₆, C(O)R₆, C₁₋₆alkyl, C₁₋₆alkoxy, haloC₁₋₆alkyl and haloC₁₋₆alkoxy;
or, when Y is a bond, R₂ and R₄ taken together form with the atoms to which they are attached a 5-6 membered heteroaryl ring optionally substituted with C₁₋₃alkyl;
Ra is selected from the group consisting of hydrogen, halogen, CN, C₁₋₃alkyl, C₁₋₃alkoxy, haloC₁₋₃alkyl and haloC₁₋₃alkoxy; or is absent;
Rb is selected from the group consisting of hydrogen, halogen, CN, C₁₋₃alkyl, C₁₋₃alkoxy, haloC₁₋₃alkyl and haloC₁₋₃alkoxy; or is absent;
Rc is selected from the group consisting of hydrogen, halogen, CN, C₁₋₃alkyl, C₁₋₃alkoxy, haloC₁₋₆alkyl and haloC₁₋₃alkoxy; or is absent
Rd is selected from the group consisting of hydrogen, halogen, CN, C₁₋₃alkyl, C₁₋₃alkoxy, haloC₁₋₃alkyl and haloC₁₋₃alkoxy; or is absent;
Re is selected from the group consisting of hydrogen, halogen, C₁₋₃alkyl, NH₂, NH(CH₃), N(CH₃)₂, NHC(O)CH₃, OH, and CN; or is absent;
Rf is selected from the group consisting of hydrogen, halogen, C₁₋₃alkyl, haloC₁₋₃alkyl, C₁₋₃alkoxy and haloC₁₋₃alkoxy; or is absent;
Rg is selected from the group consisting of hydrogen, halogen, C₁₋₃alkyl, haloC₁₋₃alkyl, C₁₋₃alkoxy and haloC₁₋₃alkoxy; or is absent;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate or 4-cyclopropyl-N-phenyl-3,4-dihydro-2H-benzo[e][1,2,4]thiadiazine-7-carboxamide 1,1-dioxide
and pharmaceutically acceptable salts, tautomers, isomers, stereoisomers thereof.

2. The compound of general formula (I) according to claim 1 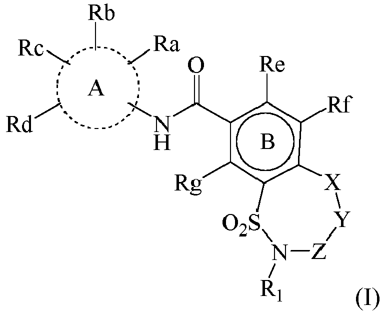 wherein:
A is aryl or heteroaryl;
B is phenyl or pyridyl;
X is N optionally substituted with R₄; or is O;
Y is a single bond; a double bond; or a C₁₋₄ alkanediyl optionally substituted with R₅;
Z is CR₂R₃ or C(O);
R₁ is selected from:
- hydrogen;
- OH;
- C₁₋₆alkyl; hydroxyC₁₋₆alkyl, haloC₁₋₆alkyl; and
- a 3-8 membered saturated or partially unsaturated ring optionally containing one or more heteroatoms each independently selected from the group consisting of O, S and N, the 3-8 membered saturated or partially unsaturated ring being optionally substituted with one, two, three or more substituents each independently selected from the group consisting of OH, halogen, CN, C₁-₆alkyl, hydroxyC₁₋₄alkyl, C(O)OR₆, C(O)CH₃, haloC₁₋₆alkyl, haloC₁₋₆alkoxy and C₁₋₆alkoxy;
R₂ is selected from:
- hydrogen;
- C₁₋₆alkyl optionally substituted with one or more substituents selected from OH, halogen, NH₂, NHC(O)CH₃ and C₁₋₆alkoxy;
- NH₂;
- NHC(O)CH₃;
- a 3-8 membered saturated ring optionally containing one or more heteroatoms each independently selected from the group consisting of O, S and N, the 3-8 membered saturated ring optionally substituted with one or more substituents each independently selected from the group consisting of OH, halogen and C₁₋₆alkyl; and
- an aryl or heteroaryl ring, each of said aryl or heteroaryl ring optionally substituted with one, two, three or more substituents each independently selected from C₁₋₆alkyl, haloC₁₋₆alkyl and halogen;
R₃ is absent, hydrogen or C₁-₆alkyl;
R₄ is hydrogen, C₁₋₄alkyl, C₃₋₆cycloalkyl or OH;
R₅ is C₁₋₃alkyl;
R₆ is C₁-₄alkyl;
or R₁ and R₂ taken together form with the atoms to which they are respectively attached a heterocyclic ring selected from the group consisting of aziridinyl, azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy; or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₁ and R₄, or R₁ and R₅ taken together represent a C₁₋₄ alkanediyl optionally substituted with one or more substituents each independently selected from C₁₋₄ alkyl and halogen;
said C₁₋₄ alkanediyl forming together with the bridging atoms to which it is linked a 4-10 heterocyclic ring;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
or, when Y is a bond, R₂ and R₄ taken together form with the atoms to which they are attached a 5-6 membered heteroaryl ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl; or is absent when B is pyridyl;
Rf is selected from the group consisting of hydrogen, halogen and methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide; or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate; or 4-cyclopropyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

3. The compound of general formula (I) according to claim 1 or 2 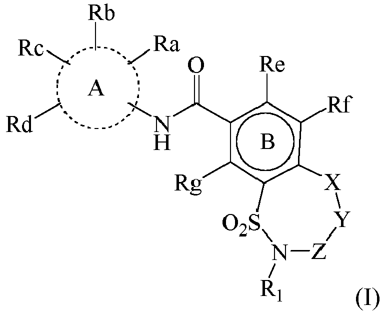 wherein:
A is phenyl or pyridyl;
B is phenyl or pyridyl;
X is N optionally substituted with R₄; or is O;
Y is a single bond, C₁₋₂alkanediyl optionally substituted with R₅;
Z is CR₂R₃ or C(O);
R₁ is selected from:
- hydrogen;
- OH;
- C₁₋₆alkyl, hydroxyC₁₋₆alkyl, haloC₁₋₆alkyl; and
- a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one, two, three or more substituents each independently selected from halogen, C₁-₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
- hydrogen;
- C₁₋₆alkyl optionally substituted with one or more substituents selected from OH, halogen, NH₂, NHC(O)CH₃ and C₁₋₆alkoxy;
- NH₂;
- NHC(O)CH₃;
- a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one or more substituents each independently selected from OH, halogen, C(O)OR₆, C(O)CH₃, C₁₋₆alkyl and CN; and
- an aryl or heteroaryl ring selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl, each of said aryl or heteroaryl ring being optionally substituted with one, two, three or more substituents each independently selected from C₁₋₆alkyl, haloC₁₋₆alkyl and halogen;
R₃ is absent, hydrogen or methyl;
R₄ is hydrogen, C₁₋₄alkyl or OH;
R₅ is C₁₋₃alkyl;
R₆ is C₁-₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄, or R₁ and R₅ taken together represent a C₁₋₄ alkanediyl optionally substituted with one or more substituents each independently selected from C₁₋₄ alkyl and halogen; said C₁₋₄ alkanediyl forming together with the bridging atoms to which it is linked a 4-10 heterocyclic ring;
or R₂ and R₅ taken together form with the carbon atoms to which they are respectively attached a 3-8 membered saturated ring selected from the group consisting of cyclobutyl, cyclopentyl and cyclohexyl, said 3-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
Ra is selected from the group consisting of hydrogen, halogen and CN;
Rb is selected from the group consisting of hydrogen, halogen and CN;
Rc is selected from the group consisting of hydrogen, halogen and CN;
Rd is selected from the group consisting of hydrogen, halogen and CN;
Re is selected from the group consisting of hydrogen, halogen, C₁₋₃alkyl, NH₂, NH(CH₃), N(CH₃)₂, NHC(O)CH₃, OH and CN; or is absent when B is pyridyl;
Rf is selected from the group consisting of hydrogen, halogen and methyl; or is absent when B is pyridyl;
Rg is hydrogen; or is absent when B is pyridyl;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

4. The compound according to any one of claims 1-3 having formula (Ia): 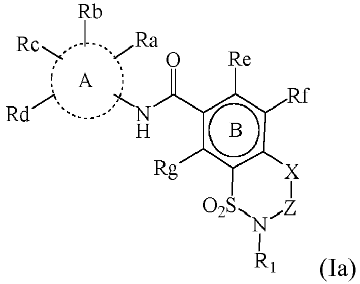 wherein:
A is phenyl or pyridyl;
B is phenyl or pyridyl;
Z is CR₂R₃ or C(O);
X is N optionally substituted with R₄; or is O;
R₁ is selected from:
- hydrogen;
- OH;
- C₁₋₆alkyl, hydroxyC₁₋₆alkyl or haloC₁₋₆alkyl; and
- a 3-8 membered saturated ring selected from the group consisting of cyclopropyl, oxetanyl, piperidinyl, cyclobutanolyl, cyclohexanolyl, pyrrolidinyl and 1-methylpyrrolidinyl, said 3-8 membered saturated ring being optionally substituted with one, two, three or more substituents each independently selected from halogen, C₁-₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
- hydrogen;
- C₁₋₆alkyl optionally substituted with one or more substituents selected from halogen, OH, NH₂, NHC(O)CH₃, C₁₋₆alkoxy;
- NH₂;
- NHC(O)CH₃;
- piperidinyl optionally substituted with C(O)OR₆; and
- an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from C₁₋₆alkyl, haloC₁₋₆alkyl and halogen;
R₃ is absent, hydrogen, or methyl;
R₄ is hydrogen or methyl;
R₆ is C₁-₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a 5-membered heteroaryl optionally substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclobutyl, oxetanyl, piperidinyl and tetrahydro-2H-pyranyl; said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄ taken together represent a C₁₋₄ alkanediyl forming together with the bridging atoms to which it is linked a 4-10 heterocyclic ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide; or tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

5. The compound according to any one of claims 1-3 having formula (Ib): 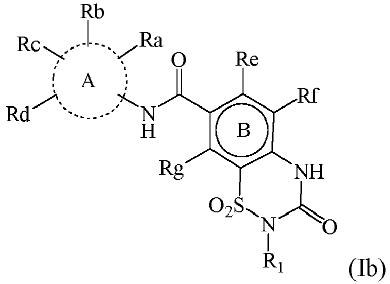 wherein:
A is phenyl;
B is phenyl;
R₁ is selected from:
- hydrogen;
- methyl;
- CH(CH₃)CF₃; and
- a 3-8 membered saturated ring selected from cyclopropyl, oxetanyl, piperidinyl, pyrrolidinyl, 1-methylpyrrolidinyl, cyclobutanolyl and cyclohexanolyl, said 3-8 membered saturated ring being substituted with one, two or more substituents each independently selected from the group consisting of C₁-₄alkyl, OH, C(O)OR₆ and C(O)CH₃;
R₆ is C₁-₄ alkyl;
Ra is selected from the group consisting of hydrogen, methyl and halogen;
Rb is selected from the group consisting of hydrogen, methyl and halogen;
Rc is selected from the group consisting of hydrogen, methyl and halogen;
Rd is hydrogen;
Re, Rf and Rg are each hydrogen;
provided that the compound is not: tert-butyl 4-((4-methyl-1,1-dioxido-3-oxo-7-(phenylcarbamoyl)-3,4-dihydro-2H-benzo[e][1,2,4]thiadiazin-2-yl)methyl)benzoate;
and pharmaceutically acceptable salts, tautomers, isomers, stereoisomers thereof.

6. The compound according to any one of claims 1-3 having formula (Ic): 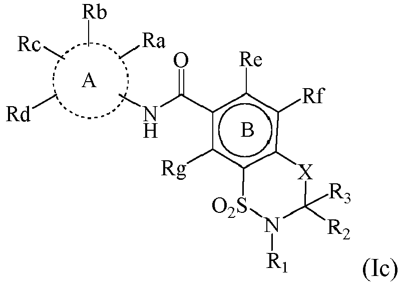 wherein:
A is phenyl or pyridyl;
B is phenyl or pyridyl;
X is N optionally substituted with R₄;
R₁ is selected from hydrogen, methyl, OH, CH₂CH₂OH, cyclopropyl, cyclohexanolyl and piperidinyl;
R₂ is selected from:
- hydrogen;
- methyl, CF₃, CHF₂, CH₂OH, CH₂CH₂OH, CHOHCH₂OH, CH(CH)₃OH, CH₂NH₂, CH₂NHC(O)CH₃, CH₂CH₂OCH(CH₃)₂;
- piperidinyl optionally substituted with C(O)OR₆; and
- an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from methyl, CF₃, Cl, F and Br;
R₃ is hydrogen or methyl;
R₄ is hydrogen, methyl or OH;
R₆ is C₁-₄ alkyl;
or R₁ and R₂ taken together form with the atom to which they are respectively attached a pyrrolidinyl;
or R₂ and R₃ taken together with the atom to which they are respectively attached form a ring selected from the group consisting of cyclobutyl, oxetanyl, piperidinyl and tetrahydro-2H-pyranyl;
or R₂ and R₃ taken together form with the carbon atom to which they are attached an indoline-2-one or an azaindoline-2-one;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
provided that the compound is not: 4-ethyl-N-phenyl-3,4-dihydro-2H-benzo[e][1,2,4]thiadiazine-7-carboxamide 1,1-dioxide, or 4-methyl-N-phenyl-3,4-dihydro-2H-benzo[e][1,2,4]thiadiazine-7-carboxamide 1,1-dioxide;
and pharmaceutically acceptable salts, tautomers, isomers, stereoisomers thereof.

7. The compound according to claim 1 being selected from the following list:
- N-(3,4,5-trifluorophenyl)-3,4-dihydro-2H-benzo[e][1,2,4]thiadiazine-7-carboxamide 1,1-dioxide;
- N-(3,4,5-trifluorophenyl)-3,4-dihydro-2H-benzo[e][1,2,4]thiadiazine-3,3-d₂-7-carboxamide 1,1-dioxide;
- 6-chloro-N-(3,4,5-trifluorophenyl)-3,4-dihydro-2H-benzo[e][1,2,4]thiadiazine-7-carboxamide 1,1-dioxide;
- 6-bromo-N-(3,4,5-trifluorophenyl)-3,4-dihydro-2H-benzo[e][1,2,4]thiadiazine-7-carboxamide 1,1-dioxide;
- 6-methyl-N-(3,4,5-trifluorophenyl)-3,4-dihydro-2H-benzo[e][1,2,4]thiadiazine-7-carboxamide 1,1-dioxide;
- 6-chloro-3-methyl-N-(3,4,5-trifluorophenyl)-3,4-dihydro-2H-benzo[e][1,2,4]thiadiazine-7-carboxamide 1,1-dioxide;
- N-(3,4-difluorophenyl)-2H,4H-spiro[benzo[e][1,2,4]thiadiazine-3,1'-cyclobutane]-7-carboxamide 1,1-dioxide;
- N-(3,4,5-trifluorophenyl)-2',3',5',6'-tetrahydro-2H,4H-spiro[benzo[e][1,2,4]thiadiazine-3,4'-pyran]-7-carboxamide 1,1-dioxide;
- N-(3,4,5-trifluorophenyl)-2H,4H-spiro[benzo[e][1,2,4]thiadiazine-3,4'-piperidine]-7-carboxamide 1,1-dioxide;
- 3-(2-isopropoxyethyl)-N-(3,4,5-trifluorophenyl)-3,4-dihydro-2H-benzo[e][1,2,4]thiadiazine-7-carboxamide 1,1-dioxide;
- 3-(difluoromethyl)-N-(3,4,5-trifluorophenyl)-3,4-dihydro-2H-benzo[e][1,2,4]thiadiazine-7-carboxamide 1,1-dioxide;
- N-(3,4-difluorophenyl)-3,4-dihydro-2H-benzo[e][1,2,4]thiadiazine-7-carboxamide 1,1-dioxide;
- N-(3,4-difluorophenyl)-3-methyl-3,4-dihydro-2H-benzo[e][1,2,4]thiadiazine-7-carboxamide 1,1-dioxide;
- 3-methyl-N-(3,4,5-trifluorophenyl)-3,4-dihydro-2H-benzo[e][1,2,4]thiadiazine-7-carboxamide 1, 1-dioxide;
- 3-(2-hydroxyethyl)-N-(3,4,5-trifluorophenyl)-3,4-dihydro-2H-benzo[e][1,2,4]thiadiazine-7-carboxamide 1,1-dioxide;
- 3-((S)-1-hydroxyethyl)-N-(3,4,5-trifluorophenyl)-3,4-dihydro-2H-benzo[e][1,2,4]thiadiazine-7-carboxamide 1,1-dioxide;
- 3-((R)-1,2-dihydroxyethyl)-N-(3,4,5-trifluorophenyl)-3,4-dihydro-2H-benzo[e][1,2,4]thiadiazine-7-carboxamide 1,1-dioxide;
- 3,3-dimethyl-N-(3,4,5-trifluorophenyl)-3,4-dihydro-2H-benzo[e][1,2,4]thiadiazine-7-carboxamide 1,1-dioxide;
- 3-((R)-1-hydroxyethyl)-N-(3,4,5-trifluorophenyl)-3,4-dihydro-2H-benzo[e][1,2,4]thiadiazine-7-carboxamide 1,1-dioxide;
- 3-(hydroxymethyl)-N-(3,4,5-trifluorophenyl)-3,4-dihydro-2H-benzo[e][1,2,4]thiadiazine-7-carboxamide 1,1-dioxide;
- 3-(aminomethyl)-N-(3,4,5-trifluorophenyl)-3,4-dihydro-2H-benzo[e][1,2,4]thiadiazine-7-carboxamide 1,1-dioxide;
- 3-(acetamidomethyl)-N-(3,4,5-trifluorophenyl)-3,4-dihydro-2H-benzo[e][1,2,4]thiadiazine-7-carboxamide 1,1-dioxide;
- *tert*-butyl 4-(1,1-dioxido-7-((3,4,5-trifluorophenyl)carbamoyl)-3,4-dihydro-2H-benzo[e][1,2,4]thiadiazin-3-yl)piperidine-1-carboxylate;
- 3-(piperidin-4-yl)-N-(3,4,5-trifluorophenyl)-3,4-dihydro-2H-benzo[e][1,2,4]thiadiazine-7-carboxamide 1,1-dioxide;
- 3-(2,6-difluorophenyl)-N-(3,4,5-trifluorophenyl)-3,4-dihydro-2H-benzo[e][1,2,4]thiadiazine-7-carboxamide 1,1-dioxide;
- 6-chloro-3-(thiazol-2-yl)-N-(3,4,5-trifluorophenyl)-3,4-dihydro-2H-benzo[e][1,2,4]thiadiazine-7-carboxamide 1,1-dioxide;
- 3-(1-methyl-1H-imidazol-5-yl)-N-(3,4,5-trifluorophenyl)-3,4-dihydro-2H-benzo[e][1,2,4]thiadiazine-7-carboxamide 1,1-dioxide;
- N-(3,4-difluorophenyl)-3-(2,4-dimethyloxazol-5-yl)-3,4-dihydro-2H-benzo[e][1,2,4]thiadiazine-7-carboxamide 1,1-dioxide;
- 3-methyl-3-(pyridin-4-yl)-N-(3,4,5-trifluorophenyl)-3,4-dihydro-2H-benzo[e][1,2,4]thiadiazine-7-carboxamide 1,1-dioxide;
- 3-(thiazol-5-yl)-N-(3,4,5-trifluorophenyl)-3,4-dihydro-2H-benzo[e][1,2,4]thiadiazine-7-carboxamide 1,1-dioxide;
- 3-(thiazol-4-yl)-N-(3,4,5-trifluorophenyl)-3,4-dihydro-2H-benzo[e][1,2,4]thiadiazine-7-carboxamide 1,1-dioxide;
- 3-(6-(trifluoromethyl)pyridin-3-yl)-N-(3,4,5-trifluorophenyl)-3,4-dihydro-2H-benzo[e][1,2,4]thiadiazine-7-carboxamide 1,1-dioxide;
- 3-(thiophen-2-yl)-N-(3,4,5-trifluorophenyl)-3,4-dihydro-2H-benzo[e][1,2,4]thiadiazine-7-carboxamide 1,1-dioxide;
- 3-(pyridin-2-yl)-N-(3,4,5-trifluorophenyl)-3,4-dihydro-2H-benzo[e][1,2,4]thiadiazine-7-carboxamide 1,1-dioxide;
- 3-(1,2,3-thiadiazol-5-yl)-N-(3,4,5-trifluorophenyl)-3,4-dihydro-2H-benzo[e][1,2,4]thiadiazine-7-carboxamide 1,1-dioxide;
- 3-(1H-pyrazol-5-yl)-N-(3,4,5-trifluorophenyl)-3,4-dihydro-2H-benzo[e][1,2,4]thiadiazine-7-carboxamide 1,1-dioxide;
- N-(3,4-difluorophenyl)-3-(pyridin-3-yl)-3,4-dihydro-2H-benzo[e][1,2,4]thiadiazine-7-carboxamide 1,1-dioxide;
- 3-(pyridin-3-yl)-N-(3,4,5-trifluorophenyl)-3,4-dihydro-2H-benzo[e][1,2,4]thiadiazine-7-carboxamide 1,1-dioxide;
- 3-(thiazol-2-yl)-N-(3,4,5-trifluorophenyl)-3,4-dihydro-2H-benzo[e][1,2,4]thiadiazine-7-carboxamide 1,1-dioxide;
- N-(3,4-difluorophenyl)-3-(thiazol-2-yl)-3,4-dihydro-2H-benzo[e][1,2,4]thiadiazine-7-carboxamide 1,1-dioxide;
- 3-(6-chloropyridin-3-yl)-N-(3,4,5-trifluorophenyl)-3,4-dihydro-2H-benzo[e][1,2,4]thiadiazine-7-carboxamide 1,1-dioxide;
- 3-(5-chloropyridin-2-yl)-3-methyl-N-(3,4,5-trifluorophenyl)-3,4-dihydro-2H-benzo[e][1,2,4]thiadiazine-7-carboxamide 1,1-dioxide;
- 3-(pyrimidin-2-yl)-N-(3,4,5-trifluorophenyl)-3,4-dihydro-2H-benzo[e][1,2,4]thiadiazine-7-carboxamide 1,1-dioxide;
- 3-methyl-3-(pyrazin-2-yl)-N-(3,4,5-trifluorophenyl)-3,4-dihydro-2H-benzo[e][1,2,4]thiadiazine-7-carboxamide 1,1-dioxide;
- 2'-oxo-N-(3,4,5-trifluorophenyl)-2H,4H-spiro[benzo[e][1,2,4]thiadiazine-3,3'-indoline]-7-carboxamide 1,1-dioxide;
- 2'-oxo-N-(3,4,5-trifluorophenyl)-1',2'-dihydro-2H,4H-spiro[benzo[e][1,2,4]thiadiazine-3,3'-pyrrolo[2,3-b]pyridine]-7-carboxamide 1,1-dioxide;
- N-(3-chloro-4-fluorophenyl)-3,4-dihydro-2H-benzo[e][1,2,4]thiadiazine-7-carboxamide 1,1-dioxide;
- 6-fluoro-N-(3,4,5-trifluorophenyl)-3,4-dihydro-2H-benzo[e][1,2,4]thiadiazine-7-carboxamide 1,1-dioxide;
- 2-methyl-3-oxo-N-(3,4,5-trifluorophenyl)-3,4-dihydro-2H-benzo[e][1,2,4]thiadiazine-7-carboxamide 1,1-dioxide;
- (S)-3-oxo-N-(3,4,5-trifluorophenyl)-2-(1,1,1-trifluoropropan-2-yl)-3,4-dihydro-2H-benzo[e][1,2,4]thiadiazine-7-carboxamide 1,1-dioxide;
- *trans*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide;
- 3-oxo-2-(piperidin-4-yl)-N-(3,4,5-trifluorophenyl)-3,4-dihydro-2H-benzo[e][1,2,4]thiadiazine-7-carboxamide 1,1-dioxide;
- 2-(oxetan-3-yl)-3-oxo-N-(3,4,5-trifluorophenyl)-3,4-dihydro-2H-benzo[e][1,2,4]thiadiazine-7-carboxamide 1,1-dioxide;
- 2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- *cis*-2-(4-hydroxycyclohexyl)-3-oxo-N-(3,4,5-trifluorophenyl)-3,4-dihydro-2H-benzo[e][1,2,4]thiadiazine-7-carboxamide 1,1-dioxide;
- 5-methyl-3-(thiazol-2-yl)-N-(3,4,5-trifluorophenyl)-3,4-dihydro-2H-benzo[e][1,2,4]thiadiazine-7-carboxamide 1,1-dioxide;
- N-(3,4,5-trifluorophenyl)-1,3,4,11,12,12a-hexahydro-2H-benzo[f]pyrido[1,2-b][1,2,5]thiadiazepine-8-carboxamide 6,6-dioxide;
- (R)-N-(3,4,5-trifluorophenyl)-1,3,4,11,12,12a-hexahydro-2H-benzo[f]pyrido[1,2-b][1,2,5]thiadiazepine-8-carboxamide 6,6-dioxide;
- N-(3,4-difluorophenyl)-3-(hydroxymethyl)-3,4-dihydro-2H-benzo[e][1,2,4]thiadiazine-7-carboxamide 1,1-dioxide;
- N-(3,4-difluorophenyl)-3,3-dimethyl-3,4-dihydro-2H-benzo[e][1,2,4]thiadiazine-7-carboxamide 1,1-dioxide;
- trans-2-(4-hydroxycyclohexyl)-6-methyl-3-oxo-N-(3,4,5-trifluorophenyl)-3,4-dihydro-2H-benzo[e][1,2,4]thiadiazine-7-carboxamide 1,1-dioxide;
- N-(3-chloro-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide;
- Cis-2-(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- Trans-2(3-hydroxycyclobutyl)-3-oxo-N-(3,4,5-trifluorophenyl)-3,4-dihydro-2H-benzo[e][1,2,4]thiadiazine-7-carboxamide 1,1-dioxide;
- (R)-2'-oxo-N-(3,4,5-trifluorophenyl)-2H,4H-spiro[benzo[e][1,2,4]thiadiazine-3,3'-indoline]-7-carboxamide 1,1-dioxide (single enantiomer; second eluted);
- N-(3-(difluoromethyl)-4-fluorophenyl)-2-((1r,4r)-4-hydroxycyclohexyl)-3-oxo-3,4-dihydro-2H-benzo[e][1,2,4]thiadiazine-7-carboxamide 1,1-dioxide;
- N-(4-fluoro-3-methylphenyl)-2-((1r,4r)-4-hydroxycyclohexyl)-3-oxo-3,4-dihydro-2H-benzo[e][1,2,4]thiadiazine-7-carboxamide 1,1-dioxide;
- 2-(1-methylpiperidin-4-yl)-3-oxo-N-(3,4,5-trifluorophenyl)-3,4-dihydro-2H-benzo[e][1,2,4]thiadiazine-7-carboxamide 1,1-dioxide;
- 2-(4-(hydroxymethyl)cyclohex-3-en-1-yl)-3-oxo-N-(3,4,5-trifluorophenyl)-3,4-dihydro-2H-benzo[e][1,2,4]thiadiazine-7-carboxamide 1,1-dioxide
and pharmaceutically acceptable salts, tautomers, isomers, stereoisomers thereof.

8. A compound as defined in any one of previous claims for medical use.

9. A compound as defined in claim 8 for use in the treatment and/or prevention of a HBV infection.

10. A compound of general formula (I): 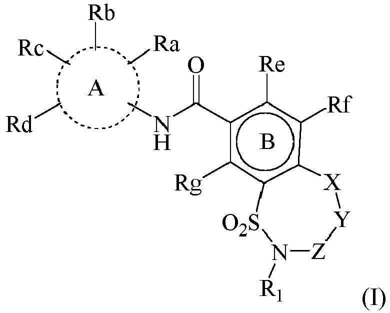 wherein:
A is aryl or heteroaryl;
B is a 6-membered aryl optionally containing one or more N atoms;
X is N optionally substituted with R₄; or is O;
Y is a single bond; a double bond; a C₁₋₄alkanediyl or a C₂₋₄alkenediyl each of said C₁₋₄alkanediyl or C₂₋₄alkenediyl being independently optionally substituted with R₅;
Z is CR₂R₃ or C(O);
R₁ is selected from:
- hydrogen;
- OH;
- C₁₋₆alkyl optionally substituted with one or more substituents each independently selected from the group consisting of OH, halogen, CN, NH₂, NH(R₆), N(R₆)₂, aryl, heteroaryl, 3-7 membered saturated ring and 5-7 membered unsaturated ring, each of said saturated or unsaturated ring optionally containing one or more heteroatoms selected from the group consisting of O, N, S and each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
- aryl or heteroaryl ring, each of said aryl or heteroaryl ring being optionally substituted with one or more substituents selected from OH, halogen, haloC₁₋₆alkyl, CN, haloC₁₋₆alkoxy and C₁₋₆alkoxy; and
- a 3-8 membered saturated or partially unsaturated ring optionally containing one or more heteroatoms each independently selected from the group consisting of O, S and N, the 3-8 membered saturated or partially unsaturated ring being optionally substituted with one, two or more substituents each independently selected from the group consisting of OH, halogen, CN, C₁-₄alkyl, hydroxyC₁-₄alkyl, C(O)OR₆, C(O)R₆, haloC₁₋₆alkyl, haloC₁₋₆alkoxy and C₁₋₆alkoxy;
R₂ is selected from:
- hydrogen;
- deuterium;
- C₁₋₆alkyl; C₁₋₆alkoxy; C₂₋₆alkenyl; each of said C₁₋₆alkyl, C₁₋₆alkoxy or C₂₋₆alkenyl being optionally substituted with one or more substituents independently selected from OH, halogen, NH₂, NH(R₆), N(R₆)₂, NHC(O)R₆, C₁₋₆alkoxy, haloC₁₋₆alkoxy, CN, aryl, heteroaryl, 3-7 membered saturated ring and 5-7 membered unsaturated ring, each of said 3-7 membered saturated or 5-7 membered unsaturated ring optionally containing one or more heteroatoms selected from the group consisting of O, N, S, each of said aryl, heteroaryl, 3-7 membered saturated or 5-7 membered unsaturated ring being optionally substituted with one or more substituents each independently selected from OH, halogen, haloC₁₋₆alkyl, CN, haloC₁₋₆alkoxy and C₁₋₆alkoxy;
- NH₂;
- NHC(O)R₆;
- a 3-8 membered saturated ring optionally containing one or more heteroatoms each independently selected from the group consisting of O, S and N, the 3-8 membered saturated ring being optionally substituted with one or more substituents each independently selected from the group consisting of OH, halogen, C(O)OR₆, C(O)R₆, C₁₋₆alkyl and CN; and
- an aryl or heteroaryl ring optionally substituted with one, two, three or more substituents each independently selected from the group consisting of C₁₋₆alkyl, haloC₁₋₆alkyl, halogen, C₁₋₆alkoxy and haloC₁₋₆alkoxy;
R₃ is absent, hydrogen, deuterium or C₁₋₆alkyl;
R₄ is hydrogen, C₁₋₄alkyl, C₃₋₆cycloalkyl or OH;
R₅ is C₁₋₃alkyl optionally substituted with OH;
R₆ is C₁-₄alkyl;
or R₁ and R₂ taken together form with the atoms to which they are respectively attached a heterocyclic ring optionally substituted with one or more substituents each independently selected from the group consisting of C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl ring optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or any two residues of R₁, R₂, R₃, R₄, R₅ which are not geminal or vicinal group, taken together represent a single bond, or represent a C₁₋₄alkanediyl or a C₂₋₄alkenediyl, each of said C₁₋₄alkanediyl or a C₂₋₄alkenediyl being independently optionally substituted with one or more substituents each independently selected from C₁₋₄ alkyl and halogen; said single bond or said optionally substituted C₁₋₄alkanediyl or C₂₋₄alkenediyl forming together with the bridging atoms to which they are respectively linked a 4-10 membered saturated or partially unsaturated ring;
or R₂ and R₃ taken together form with the carbon atom to which they are attached a 3-7 membered saturated ring optionally containing one or more heteroatoms each independently selected from the group consisting of O, S and N, the 3-7 membered saturated ring optionally being substituted with one or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)R₆;
or R₂ and R₃ taken together form with the carbon atom to which they are attached an indoline-2-one or an azaindoline-2-one, each being optionally substituted with one or more substituents selected from halogen, hydroxy, C₁₋₆alkyl, haloC₁₋₆alkyl, C₁₋₆alkoxy and haloC₁₋₆alkoxy;
or R₂ and R₅ taken together form with the carbon atoms to which they are respectively attached a 3-8 membered saturated ring or a 5-10 membered partially saturated ring, each ring optionally containing one or more heteroatoms each independently selected from the group consisting of O, S and N, each ring optionally being substituted with one, two or more substituents each independently selected from the group consisting of halogen, OH, CN, C(O)OR₆, C(O)R₆, C₁₋₆alkyl, C₁₋₆alkoxy, haloC₁₋₆alkyl and haloC₁₋₆alkoxy;
or, when Y is a bond, R₂ and R₄ taken together form with the atoms to which they are attached a 5-6 membered heteroaryl ring optionally substituted with C₁₋₃alkyl;
Ra is selected from the group consisting of hydrogen, halogen, CN, C₁₋₃alkyl, C₁₋₃alkoxy, haloC₁₋₃alkyl and haloC₁₋₃alkoxy; or is absent;
Rb is selected from the group consisting of hydrogen, halogen, CN, C₁₋₃alkyl, C₁₋₃alkoxy, haloC₁₋₃alkyl and haloC₁₋₃alkoxy; or is absent;
Rc is selected from the group consisting of hydrogen, halogen, CN, C₁₋₃alkyl, C₁₋₃alkoxy, haloC₁₋₆alkyl and haloC₁₋₃alkoxy; or is absent
Rd is selected from the group consisting of hydrogen, halogen, CN, C₁₋₃alkyl, C₁₋₃alkoxy, haloC₁₋₃alkyl and haloC₁₋₃alkoxy; or is absent;
Re is selected from the group consisting of hydrogen, halogen, C₁₋₃alkyl, NH₂, NH(CH₃), N(CH₃)₂, NHC(O)CH₃, OH, and CN; or is absent;
Rf is selected from the group consisting of hydrogen, halogen, C₁₋₃alkyl, haloC₁₋₃alkyl, C₁₋₃alkoxy and haloC₁₋₃alkoxy; or is absent;
Rg is selected from the group consisting of hydrogen, halogen, C₁₋₃alkyl, haloC₁₋₃alkyl, C₁₋₃alkoxy and haloC₁₋₃alkoxy; or is absent;
and pharmaceutically acceptable salts, tautomers, isomers, stereoisomers thereof, for use in the treatment and/or prevention of a HBV infection.

11. The compound for use according to claim 10, wherein:
A is aryl or heteroaryl;
B is phenyl or pyridyl;
X is N optionally substituted with R₄; or is O;
Y is a single bond; a double bond; or a C₁₋₄ alkanediyl optionally substituted with R₅;
Z is CR₂R₃ or C(O);
R₁ is selected from:
- hydrogen;
- OH;
- C₁₋₆alkyl; hydroxyC₁₋₆alkyl, haloC₁₋₆alkyl; and
- a 3-8 membered saturated or partially unsaturated ring optionally containing one or more heteroatoms each independently selected from the group consisting of O, S and N, the 3-8 membered saturated or partially unsaturated ring being optionally substituted with one, two, three or more substituents each independently selected from the group consisting of OH, halogen, CN, C₁₋₆alkyl, hydroxyC₁₋₄alkyl, C(O)OR₆, C(O)CH₃, haloC₁₋₆alkyl, haloC₁₋₆alkoxy and C₁₋₆alkoxy;
R₂ is selected from:
- hydrogen;
- C₁₋₆alkyl optionally substituted with one or more substituents selected from OH, halogen, NH₂, NHC(O)CH₃ and C₁₋₆alkoxy;
- NH₂;
- NHC(O)CH₃;
- a 3-8 membered saturated ring optionally containing one or more heteroatoms each independently selected from the group consisting of O, S and N, the 3-8 membered saturated ring optionally substituted with one or more substituents each independently selected from the group consisting of OH, halogen and C₁₋₆alkyl; and
- an aryl or heteroaryl ring, each of said aryl or heteroaryl ring optionally substituted with one, two, three or more substituents each independently selected from C₁₋₆alkyl, haloC₁₋₆alkyl and halogen;
R₃ is absent, hydrogen or C₁-₆alkyl;
R₄ is hydrogen, C₁₋₄alkyl, C₃₋₆cycloalkyl or OH;
R₅ is C₁₋₃alkyl;
R₆ is C₁-₄alkyl;
or R₁ and R₂ taken together form with the atoms to which they are respectively attached a heterocyclic ring selected from the group consisting of aziridinyl, azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₁ and R₄, or R₁ and R₅ taken together represent a C₁₋₄ alkanediyl optionally substituted with one or more substituents each independently selected from C₁₋₄ alkyl and halogen;
said C₁₋₄ alkanediyl forming together with the bridging atoms to which it is linked a 4-10 heterocyclic ring;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₂ and R₅ taken together form with the carbon atoms to which they are respectively attached a 4-8 membered saturated ring optionally containing one or more heteroatoms each independently selected from the group consisting of O, S and N, the 4-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
or, when Y is a bond, R₂ and R₄ taken together form with the atoms to which they are attached a 5-6 membered heteroaryl ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl; or is absent when B is pyridyl;
Rf is selected from the group consisting of hydrogen, halogen and methyl; or is absent when B is pyridyl;
Rg is hydrogen; or is absent when B is pyridyl;
and pharmaceutically acceptable salts, tautomers, isomers, stereoisomers thereof.

12. The compound for use according to any of claims 10 or 11 wherein:
A is phenyl or pyridyl;
B is phenyl or pyridyl;
X is N optionally substituted with R₄; or is O;
Y is a single bond, C₁₋₂alkanediyl optionally substituted with R₅;
Z is CR₂R₃ or C(O);
R₁ is selected from:
- hydrogen;
- OH;
- C₁₋₆alkyl, hydroxyC₁₋₆alkyl, haloC₁₋₆alkyl; and
- a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one, two, three or more substituents each independently selected from halogen, C₁-₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
- hydrogen;
- C₁₋₆alkyl optionally substituted with one or more substituents selected from OH, halogen, NH₂, NHC(O)CH₃ and C₁₋₆alkoxy;
- NH₂;
- NHC(O)CH₃;
- a 3-8 membered saturated ring selected from the group consisting of cyclopropyl, cyclobutyl, cyclopentyl, cyclohexyl, cycloheptyl, cyclooctyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl and oxazocanyl, said 3-8 membered saturated ring being optionally substituted with one or more substituents each independently selected from OH, halogen, C(O)OR₆, C(O)CH₃, C₁₋₆alkyl and CN; and
- an aryl or heteroaryl ring selected from the group consisting of phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl, each of said aryl or heteroaryl ring being optionally substituted with one, two, three or more substituents each independently selected from C₁₋₆alkyl, haloC₁₋₆alkyl and halogen;
R₃ is absent, hydrogen or methyl;
R₄ is hydrogen, C₁₋₄alkyl or OH;
R₅ is C₁₋₃alkyl;
R₆ is C₁-₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a heteroaryl optionally being substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclopropyl, cyclobutyl, cyclopentyl, cyclohexyl, cycloheptyl, oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide and pyrrolidinyl, said 3-7 membered saturated ring optionally being substituted with one or more substituents independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄, or R₁ and R₅ taken together represent a C₁₋₄ alkanediyl optionally substituted with one or more substituents each independently selected from C₁₋₄ alkyl and halogen; said C₁₋₄ alkanediyl forming together with the bridging atoms to which it is linked a 4-10 heterocyclic ring;
or R₂ and R₅ taken together form with the carbon atoms to which they are respectively attached a 3-8 membered saturated ring selected from the group consisting of cyclobutyl, cyclopentyl and cyclohexyl, said 3-8 membered saturated ring optionally being substituted with one, two or more substituents each independently selected from the group consisting of halogen, OH, haloC₁₋₆alkyl, CN, C₁₋₆alkyl, C₁₋₆alkoxy, haloC₁₋₆alkoxy, C(O)OR₆ and C(O)CH₃;
Ra is selected from the group consisting of hydrogen, halogen and CN;
Rb is selected from the group consisting of hydrogen, halogen and CN;
Rc is selected from the group consisting of hydrogen, halogen and CN;
Rd is selected from the group consisting of hydrogen, halogen and CN;
Re is selected from the group consisting of hydrogen, halogen, C₁₋₃alkyl, NH₂, NH(CH₃), N(CH₃)₂, NHC(O)CH₃, OH and CN; or is absent when B is pyridyl;
Rf is selected from the group consisting of hydrogen, halogen and methyl; or is absent when B is pyridyl;
Rg is hydrogen; or is absent when B is pyridyl;
and pharmaceutically acceptable salts, tautomers, isomers, stereoisomers thereof.

13. The compound for use according to any of claims 10-12 having formula (Ia): 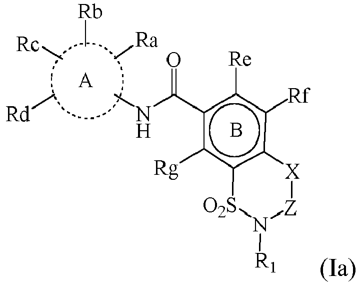 wherein:
A is phenyl or pyridyl;
B is phenyl or pyridyl;
Z is CR₂R₃ or C(O);
X is N optionally substituted with R₄; or is O;
R₁ is selected from:
- hydrogen;
- OH;
- C₁₋₆alkyl, hydroxyC₁₋₆alkyl or haloC₁₋₆alkyl; and
- a 3-8 membered saturated ring selected from the group consisting of cyclopropyl, oxetanyl, piperidinyl, cyclobutanolyl, cyclohexanolyl, pyrrolidinyl and 1-methylpyrrolidinyl, said 3-8 membered saturated ring being optionally substituted with one, two, three or more substituents each independently selected from halogen, C₁₋₄alkyl, OH, C(O)OR₆, C(O)CH₃, C₁₋₆alkyl, haloC₁₋₆alkyl, CN, haloC₁₋₆alkoxy and C₁₋₆alkoxy;
R₂ is selected from:
- hydrogen;
- C₁₋₆alkyl optionally substituted with one or more substituents selected from halogen, OH, NH₂, NHC(O)CH₃, C₁₋₆alkoxy;
- NH₂;
- NHC(O)CH₃;
- piperidinyl optionally substituted with C(O)OR₆; and
- an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from C₁₋₆alkyl, haloC₁₋₆alkyl and halogen;
R₃ is absent, hydrogen, or methyl;
R₄ is hydrogen or methyl;
R₆ is C₁₋₄alkyl;
or R₁ and R₂ taken together form with the atoms to which they are respectively attached a heterocyclic ring selected from the group consisting of azetidinyl, piperazinyl, piperidinyl, morpholinyl, thiomorpholinyl, thiazolidinyl and pyrrolidinyl, the heterocyclic ring optionally being substituted with one or more substituents each independently selected from C(O)OR₆, C(O)CH₃, halogen, OH, haloC₁₋₆alkyl, CN, C₁₋₆alkyl, C₁₋₆alkoxy and haloC₁₋₆alkoxy;
or R₃ is absent and R₁ and R₂ taken together form with the atoms to which they are respectively attached a 5-membered heteroaryl optionally substituted with one, two or more substituents each independently selected from the group consisting of halogen, OH, haloC₁₋₆alkyl, CN, C₁₋₆alkyl, C₁₋₆alkoxy and haloC₁₋₆alkoxy;
or R₂ and R₃ taken together form with the carbon atom to which they are attached a 3-7 membered saturated ring selected from the group consisting of cyclobutyl, oxetanyl, piperidinyl and tetrahydro-2H-pyranyl; said 3-7 membered saturated ring optionally being substituted with one or more substituents each independently selected from halogen, OH, haloC₁₋₆alkyl, CN, C₁₋₆alkyl, haloC₁₋₆alkoxy, C(O)OR₆ and C(O)CH₃;
or R₂ and R₃ taken together form with the carbon atom to which they are attached an indoline-2-one or an azaindoline-2-one;
or R₁ and R₄ taken together represent a C₁₋₄ alkanediyl forming together with the bridging atoms to which it is linked a 4-10 heterocyclic ring;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
and pharmaceutically acceptable salts, tautomers, isomers, stereoisomers thereof.

14. The compound for use according to anyone of claims 10-12 having formula (Ib): 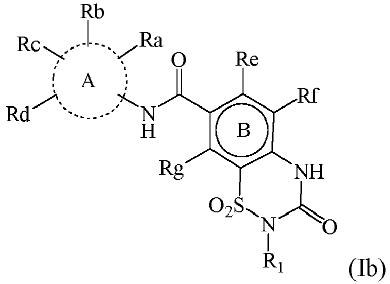 wherein:
A is phenyl;
B is phenyl;
R₁ is selected from:
- hydrogen;
- methyl;
- CH(CH₃)CF₃; and
- a 3-8 membered saturated ring selected from cyclopropyl, oxetanyl, piperidinyl, pyrrolidinyl, 1-methylpyrrolidinyl, cyclobutanolyl and cyclohexanolyl, said 3-8 membered saturated ring being substituted with one, two or more substituents each independently selected from the group consisting of C₁₋₄alkyl, OH, C(O)OR₆ and C(O)CH₃;
R6 is C₁₋₄ alkyl;
Ra is selected from the group consisting of hydrogen, methyl and halogen;
Rb is selected from the group consisting of hydrogen, methyl and halogen;
Rc is selected from the group consisting of hydrogen, methyl and halogen;
Rd is hydrogen;
Re, Rf and Rg are each hydrogen;
and pharmaceutically acceptable salts, tautomers, isomers, stereoisomers thereof.

15. The compound for use according to anyone of claims 10-12 having formula (Ic): 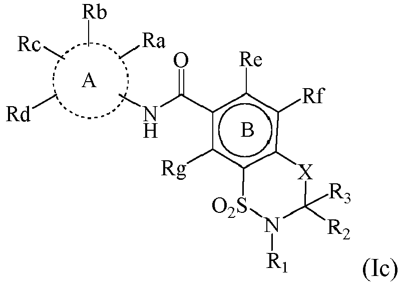 wherein:
A is phenyl or pyridyl;
B is phenyl or pyridyl;
X is N optionally substituted with R₄;
R₁ is selected from hydrogen, methyl, OH, CH₂CH₂OH, cyclopropyl, cyclohexanolyl and piperidinyl;
R₂ is selected from:
- hydrogen;
- methyl, CF₃, CHF₂, CH₂OH, CH₂CH₂OH, CHOHCH₂OH, CH(CH)₃OH, CH₂NH₂, CH₂NHC(O)CH₃, CH₂CH₂OCH(CH₃)₂;
- piperidinyl optionally substituted with C(O)OR₆;
- an aryl ring selected from the group consisting of phenyl, pyridyl, pyrimidinyl, pyrazinyl, thienyl, thiazolyl, thiadiazolyl, pyrazolyl, imidazolyl, isoxazolyl and oxazolyl, said aryl being optionally substituted with one, two, three or more substituents each independently selected from methyl, CF₃, Cl, F and Br;
R₃ is hydrogen or methyl;
R₄ is hydrogen, methyl or OH;
R₆ is C₁₋₄ alkyl;
or R₁ and R₂ taken together form with the atom to which they are respectively attached a pyrrolidinyl;
or R₂ and R₃ taken together with the atom to which they are respectively attached form a ring selected from the group consisting of cyclobutyl, oxetanyl, piperidinyl and tetrahydro-2H-pyranyl;
or R₂ and R₃ taken together form with the carbon atom to which they are attached an indoline-2-one or an azaindoline-2-one;
Ra is selected from the group consisting of hydrogen, methyl, halogen and CN;
Rb is selected from the group consisting of hydrogen, methyl, halogen and CN;
Rc is selected from the group consisting of hydrogen, methyl, halogen and CN;
Rd is selected from the group consisting of hydrogen, methyl, halogen and CN;
Re is selected from the group consisting of hydrogen, halogen and methyl;
Rf is selected from the group consisting of hydrogen, halogen and methyl; or is absent when B is pyridyl;
Rg is hydrogen;
and pharmaceutically acceptable salts, tautomers, isomers, stereoisomers thereof.

16. The compound according to anyone of claims 9-15 for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

17. The compound for use according to any one of claim 9-16, for use in combination with at least one further therapeutic agent, preferably the at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-α), pegylated interferon alpha (PEG-IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

18. A pharmaceutical composition comprising a compound as defined in anyone of claims 1-7 or 10-15, alone or in combination with at least one further therapeutic agent, and at least one pharmaceutically acceptable excipient, wherein the at least one further therapeutic agent is preferably selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX-110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl] amino }methyl)phenyl] acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-α), pegylated interferon alpha (PEG-IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1-chloro-3-oxo-1-phenyl-3-(piperidin-1-yl)prop-1-en-2-yl)benzamide), AT130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

19. The pharmaceutical composition according to claim 18 for use in the treatment and/or prevention of a HBV infection, preferably for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.
